# EUROPEAN PATENT SPECIFICATION

(11) **EP 2 680 883 B1**
(45) Date of publication and mention of the grant of the patent: **05.09.2018**
(21) Application number: 12711453.6
(22) Date of filing: 28.02.2012
(51) Int. Cl.: A61K 39/00, C07K 17/00, C07K 7/04, C07K 14/47, C12N 9/64

(54) **PCSK9 VACCINE**
PCSK9-IMPFSTOFF
VACCIN À BASE DE PCSK9

(30) Priority: 02.03.2011 US 201161448398 P
(43) Date of publication of application: 08.01.2014
(73) Proprietor: Pfizer Inc., New York, NY 10017 (US)
(72) Inventor: CHAMPION, Brian, Robert, La Jolla, CA 92037 (US); FRASER, James, Downey, San Diego, CA 92129 (US); SMITH III, George Joseph, San Diego, CA 92130 (US); LEES, Clare, Solana Beach, CA 92075 (US)
(74) Representative: Pfizer
(86) International application number: PCT/IB2012/050924
(87) International publication number: WO 2012/131504

(56) References cited:
- WO-A1-2011/072263
- WO-A1-2011/117401
- WO-A2-2011/027257
- US-A1- 2007 099 833
- US-A1- 2009 275 504
- NI YAN G ET AL: "A proprotein convertase subtilisin-like/kexin type 9 (PCSK9) C-terminal domain antibody antigen-binding fragment inhibits PCSK9 internalization and restores low density lipoprotein uptake", JOURNAL OF BIOLOGICAL CHEMISTRY, AMERICAN SOCIETY FOR BIOCHEMISTRY AND MOLECULAR BIOLOGY, INC, BETHESDA, MD, USA, vol. 285, no. 17, 23 April 2010 (2010-04-23), pages 12882-12891, XP002619048, ISSN: 1083-351X, DOI: 10.1074/JBC.M110.113035 [retrieved on 2010-02-19]
- JOYCE C Y CHAN ET AL: "A proprotein convertase subtilisin/kexin type 9 neutralizing antibody reduces serum cholesterol in mice and nonhuman primates", PROCEEDINGS OF THE NATIONAL ACADEMY OF SCIENCES, NATIONAL ACADEMY OF SCIENCES, vol. 106, no. 24, 16 June 2009 (2009-06-16), pages 9820-9825, XP002657619, ISSN: 0027-8424, DOI: 10.1073/PNAS.0903849106 [retrieved on 2009-05-14]
- DUFF CHRISTOPHER J ET AL: "Antibody-mediated disruption of the interaction between PCSK9 and the low-density lipoprotein receptor", BIOCHEMICAL JOURNAL, THE BIOCHEMICAL SOCIETY, LONDON, GB, vol. 419, no. 3, 1 May 2009 (2009-05-01), pages 577-584, XP002619050, ISSN: 0264-6021
- THILINA DEWPURA ET AL: "PCSK9 is phosphorylated by a Golgi casein kinase-like kinase ex?vivo and circulates as a phosphoprotein in humans", FEBS JOURNAL, vol. 275, no. 13, 1 July 2008 (2008-07-01) , pages 3480-3493, XP055028224, ISSN: 1742-464X, DOI: 10.1111/j.1742-4658.2008.06495.x

## Description

### FIELD OF THE INVENTION

The present invention relates to the provision of novel immunogens comprising an antigenic PCSK9 peptide linked to an immunogenic carrier for the prevention, treatment or alleviation of PCSK9-related disorders. The disclosure further relates to immunogenic compositions and pharmaceutical compositions thereof and their use in medicine.

### BACKGROUND

Proprotein convertase subtilisin-kexin type 9 (hereinafter called "PCSK9"), also known as neural apoptosis-regulated convertase 1 ("NARC-I"), is a proteinase K-like subtilase identified as the 9th member of the mammalian PCSK family; see Seidah et al, 2003 PNAS 100:928-933. The gene for PCSK9 localizes to human chromosome 1p33-p34.3. PCSK9 is expressed in cells capable of proliferation and differentiation including, for example, hepatocytes, kidney mesenchymal cells, intestinal ileum, and colon epithelia as well as embryonic brain telencephalon neurons.

Original synthesis of PCSK9 is in the form of an inactive enzyme precursor, or zymogen, of ∼72-kDa which undergoes autocatalytic, intramolecular processing in the endoplasmic reticulum ("ER") to activate its functionality. The gene sequence for human PCSK9, which is -22-kb long with 12 exons encoding a 692 amino acid protein, can be found, for example, at Deposit No. NP_777596.2. Human, mouse and rat PCSK9 nucleic acid sequences have been deposited; see, e.g., GenBank Accession Nos.: AX127530 (also AX207686), AX207688, and AX207690, respectively.

Human PCSK9 is a secreted protein expressed primarily in the kidneys, liver and intestines. It has three domains: an inhibitory pro-domain (amino acids 1-152; including a signal sequence at amino acids 1-30), a catalytic domain (amino acids 153-448), and a C-terminal domain 210 residues in length (amino acids 449-692), which is rich in cysteine residues. PCSK9 is synthesized as a zymogen that undergoes autocatalytic cleavage between the pro- domain and catalytic domain in the endoplasmic reticulum. The pro-domain remains bound to the mature protein after cleavage, and the complex is secreted. The cysteine-rich domain may play a role analogous to the P-(processing) domains of other Furin/Kexin/Subtilisin-like serine proteases, which appear to be essential for folding and regulation of the activated protease. Mutations in PCSK9 are associated with abnormal levels of low density lipoprotein cholesterol (LDL-c) in the blood plasma (Horton et al., 2006 Trends. Biochem. Sci. 32(2):71-77).

PCSK9 has been ascribed a role in the differentiation of hepatic and neuronal cells (Seidah et al., supra), is highly expressed in embryonic liver, and has been strongly implicated in cholesterol homeostasis.

The identification of compounds and/or agents effective in the treatment of cardiovascular affliction is highly desirable. Reductions in LDL cholesterol levels have already demonstrated in clinical trials to be directly related to the rate of coronary events; see Law et al., 2003 BMJ 326: 1423-1427. More recently, moderate lifelong reduction in plasma LDL cholesterol levels has been shown to be substantially correlated with a substantial reduction in the incidence of coronary events; see Cohen et al., supra. This was found to be the case even in populations with a high prevalence of non-lipid-related cardiovascular risk factors.

Expression or upregulation of PCSK9 is associated with increased plasma levels of LDL cholesterol, and inhibition or the lack of expression of PCSK9 is associated with low LDL cholesterol plasma levels. Significantly, lower levels of LDL cholesterol associated with sequence variations in PCSK9 have conferred protection against coronary heart disease; see Cohen, 2006 N. Engl. J. Med. 354: 1264-1272.

US2009275504 dislcoses PCSK9 polypeptides, fragments thereof and methods of modulating PCSK9 phosphorylation and low density lipoprotein degradation.

Accordingly, it is of great importance to indentify therapeutic agents permitting the control of LDL cholesterol levels. Further, it is of great importance to produce a medicament that inhibits or antagonizes the activity of PCSK9 and the corresponding role PCSK9 plays in various therapeutic conditions.

### SUMMARY OF THE INVENTION

The present disclosure includes an immunogen comprising at least one antigenic PCSK9 peptide containing a phosphorylation site (with or without phosphorylation of the site), or a functionally active variant thereof, linked to an immunogenic carrier. In a further embodiment, the immunogenic carrier is selected from Diphtheria Toxoid, CRM197 or a VLP selected from HBcAg, HBsAg, Qbeta, PP7, PPV or Norwalk Virus VLP.

In another embodiment, the antigenic PCSK9 peptide containing a phosphorylation site (with or without phosphorylation of the site) is selected from a portion of PCSK9 which participates in the interaction of PCSK9 with the LDL receptor. Ina further embodiment, the antigenic PCSK9 peptide containing a phosphorylation site (with or without phosphorylation of the site) is selected from a portion of PCSK9 which participates in the interaction of PCSK9 with the EGF domain of the LDL receptor.

In still another embodiment, the antigenic PCSK9 peptide containing a phosphorylation site (with or without phosphorylation of the site) as herein described is selected from the prodomain of PCSK9, or the C-terminal domain of PCSK9.

In yet another embodiment, the antigenic PCSK9 peptide containing a phosphorylation site (with or without phosphorylation of the site) comprises from 4 to 20 amino acids. In another embodiment, the antigenic PCSK9 peptide containing a phosphorylation site (with or without phosphorylation of the site) comprises from 4 to 20 amino acids, and one or more of said amino acids are modified by phosphorylation.

In a further embodiment, the immunogen as herein described further comprises at its C-terminus a linker having the formula (G)ₙC, (G)ₙSC or (G)ₙK; and/or said antigenic PCSK9 peptide containing a phosphorylation site (with or without phosphorylation of the site) further comprises at its N-terminus a linker having the formula C(G)ₙ, CS(G)ₙ or K(G)ₙ, wherein n is independently 0, 1, 2, 3, 4, 5 ,6, 7, 8, 9 or 10.

In still another embodiment, the antigenic PCSK9 peptide containing a phosphorylation site (with or without phosphorylation of the site) as herein described further comprises a cysteine at its C-terminus, and/or CG or a cysteine at its N-terminus.

In another embodiment, the immunogen as herein described includes an antigenic PCSK9 peptide containing a phosphorylation site (with or without phosphorylation of the site) that further comprises CGG at its N-terminus, and/or GGC at its C-terminus.

In yet another embodiment, the immunogen as herein described includes an antigenic PCSK9 peptide containing a phosphorylation site (with or without phosphorylation of the site) that is cyclised and further comprises cysteine, or a (G)ₙC or C(G)ₙ fragment, wherein n is independently 0, 1, 2, 3, 4, 5 ,6, 7, 8, 9 or 10. In still another embodiment, the immunogen as herein described includes an antigenic PCSK9 peptide containing a phosphorylation site (with or without phosphorylation of the site) that is cyclised and further comprises a cysteine or a GC or CG fragment.

In a further embodiment, the immunogen as herein described further comprises an antigenic PCSK9 peptide containing a phosphorylation site (with or without phosphorylation of the site) which further comprises KG or KGG at its N-terminus. In still another embodiment, the immunogen further comprises an antigenic PCSK9 peptide containing a phosphorylation site (with or without phosphorylation of the site) that is conformationally constrained.

In still another embodiment, the immunogen as herein described is able, when administered to a subject, to lower the LDL-cholesterol level in blood of a subject by at least 2%, 5%, 10%, 20%, 30% or 50%.

In another embodiment, the disclosure comprises a composition comprising at least two immunogens as herein described.

In a further embodiment, the disclosure includes a pharmaceutical composition comprising the immunogen as herein described, or a composition of immunogens as herein described, and a pharmaceutically acceptable excipient. In still another embodiment, the disclosure includes a pharmaceutical composition as herein described for use as a medicament.

In another embodiment, the disclosure includes an immunogen or a composition as herein described, for preventing, alleviating or treating a PCSK9-related disorder. In still another embodiment, the PCSK9-related disorder is elevated LDL-cholesterol or a condition associated with elevated LDL-cholesterol. In a further embodiment, the PCSK9-related disorder is a lipid disorder selected from hyperlipidemia, type I, type II, type III, type IV, or type V hyperlipidemia, secondary hypertriglyceridemia, hypercholesterolemia, familial hypercholesterolemia, xanthomatosis, and cholesterol acetyltransferase deficiency; an arteriosclerotic conditions (e.g., atherosclerosis), a coronary artery disease, and a cardiovascular disease. In still another embodiment, the PCSK9-related disorder is Alzheimer's disease.

In yet another embodiment, the disclosure includes a method for preventing, alleviating or treating a PCSK9-related disorder in an individual, comprising administering a therapeutically effective amount of the immunogen, or a composition of immunogens, as herein described.

In a further embodiment, the disclosure includes an immunogen comprising at least one antigenic PCSK9 peptide containing a phosphorylation site (with or without phosphorylation of the site), wherein said antigenic PCSK9 peptide containing a phosphorylation site (with or without phosphorylation of the site) is selected from the group consisting of SEQ ID Nos. 149 to 172, and 434 to 468, or a functionally active variant thereof; and said antigenic PCSK9 peptide containing a phosphorylation site (with or without phosphorylation of the site) is linked to an immunogenic carrier.

In a further embodiment, the disclosure includes an immunogen comprising at least one antigenic PCSK9 peptide containing a phosphorylation site (with or without phosphorylation of the site), wherein said antigenic PCSK9 peptide containing a phosphorylation site (with or without phosphorylation of the site) is selected from the group consisting of SEQ ID Nos. 4 to 29, and 287 to 320, or a functionally active variant thereof; and said antigenic PCSK9 peptide containing a phosphorylation site (with or without phosphorylation of the site) is linked to an immunogenic carrier.

In still another embodiment, the disclosure includes an immunogen as herein described wherein said immunogenic carrier is selected from Diphtheria Toxoid, CRM197 or a VLP selected from HBcAg, HBsAg, Qbeta, PP7, PPV or Norwalk Virus VLP. In yet another embodiment, the immunogen as herein described further comprises at least one adjuvant. In yet another embodiment, the adjuvant is selected from alum, CpG ODN, QS21 and Iscomatrix. In a further embodiment, the adjuvant is selected from QS21 in combination with CpG ODN, alum in combination with CpG ODN, or Iscomatrix in combination with CpG ODN, and/or wherein the adjuvant is alum in combination with CpG ODN. In still another embodiment, the immunogen as herein described further comprises CpG ODN selected from 5' TCGTCGTTTTTCGGTGCTTTT 3', 5' TCGTCGTTTTTCGGTCGTTTT 3', and 5' TCGTCGTTTTGTCGTTTTGTCGTT 3'.

In yet another embodiment, the immunogen as herein described comprises at least one antigenic PCSK9 peptide containing a phosphorylation site (with or without phosphorylation of the site), wherein said antigenic PCSK9 peptide containing a phosphorylation site (with or without phosphorylation of the site) is selected from the group consisting of SEQ ID Nos. 173 to 198, and 527 to 547, or a functionally active variant thereof; and said antigenic PCSK9 peptide containing a phosphorylation site (with or without phosphorylation of the site) is linked to an immunogenic carrier.

In yet another embodiment, the immunogen as herein described comprises at least one antigenic PCSK9 peptide containing a phosphorylation site (with or without phosphorylation of the site), wherein said antigenic PCSK9 peptide containing a phosphorylation site (with or without phosphorylation of the site) is selected from the group consisting of SEQ ID Nos. 30 to 54, and 379 to 399, or a functionally active variant thereof; and said antigenic PCSK9 peptide containing a phosphorylation site (with or without phosphorylation of the site) is linked to an immunogenic carrier.

In another embodiment, the immunogen as herein described comprises an immunogenic carrier selected from Diphtheria Toxoid, CRM197 or a VLP selected from HBcAg, HBsAg, Qbeta, PP7, PPV or Norwalk Virus VLP. In yet another embodiment, the immunogen as herein described further comprises at least one adjuvant.

In a further embodiment, the present disclosure includes a PCSK9 peptide containing a phosphorylation site (with or without phosphorylation of the site) selected from the group consisting of SEQ ID Nos. 149 to 286, and 434 to 581.

In a further embodiment, the present disclosure includes a PCSK9 peptide containing a phosphorylation site (with or without phosphorylation of the site) selected from the group consisting of SEQ ID Nos. 4 to 148, and 287 to 433.

In one or more embodiments, the PCSK9 peptide containing a phosphorylation site (with or without phosphorylation of the site) as herein described contains a phosphorylated serine residue. In some embodiments, the PCSK9 peptide containing a phosphorylation site (with or without phosphorylation of the site) is phosphorylated at the residue corresponding to position 47 in human PCSK9. In some embodiments, the PCSK9 peptide containing a phosphorylation site (with or without phosphorylation of the site) contains a phosphorylated serine residue such as in, for example, SEQ ID Nos. 149 to 286, and 434 to 581.

In one or more embodiments, the present disclosure as herein described includes: a nucleic acid encoding the immunogen as herein described; an expression vector as herein described; and/or a host cell comprising the expression vector as herein described.

In an embodiment, the present disclosure further relates to an immunogen comprising an antigenic PCSK9 peptide derived from the prodomain or C-terminal domain of PCSK9 (which may contain potential phosphorylation sites and amino acids that may be modified by phosphorylation) and optionally an immunogenic carrier. In another embodiment, the present disclosure also relates to methods for producing such antigenic PCSK9 peptide optionally linked to an immunogenic carrier.

In still another embodiment, the present disclosure as herein described also relates to immunogenic compositions comprising such antigenic PCSK9 peptide optionally linked to an immunogenic carrier, optionally comprising one or several adjuvants, preferably one or two adjuvants.

In a further embodiment, the disclosure as herein described relates to pharmaceutical compositions comprising an antigenic PCSK9 peptide according to the disclosure, or an immunogenic composition thereof, as well as to medical uses of such compositions.

The antigenic PCSK9 peptides of the disclosure are particularly suitable for treating human patients having, or at risk for, elevated LDL-cholesterol or a condition associated with elevated LDL-cholesterol, e.g., a lipid disorder (e.g., hyperlipidemia, type I, type II, type III, type IV, or type V hyperlipidemia, secondary hypertriglyceridemia, hypercholesterolemia, familial hypercholesterolemia, xanthomatosis, cholesterol acetyltransferase deficiency). Antigenic PCSK9 peptide of the disclosure are also suitable for treating human patients having arteriosclerotic conditions (e.g., atherosclerosis), coronary artery disease, cardiovascular disease, and patients at risk for these disorders, e.g., due to the presence of one or more risk factors (e.g., hypertension, cigarette smoking, diabetes, obesity, or hyperhomocysteinemia).

In yet another embodiment, the present disclosure as herein described provides the use of an antigenic PCSK9 peptide of the disclosure or of an immunogenic composition or a pharmaceutical composition thereof, in the manufacture of a medicament for the treatment of Alzheimer's disease.

In one embodiment, the antigenic PCSK9 peptide or an immunogenic composition or a pharmaceutical composition thereof as herein described is administered together with another agent.

### BRIEF DESCRIPTION OF THE DRAWINGS

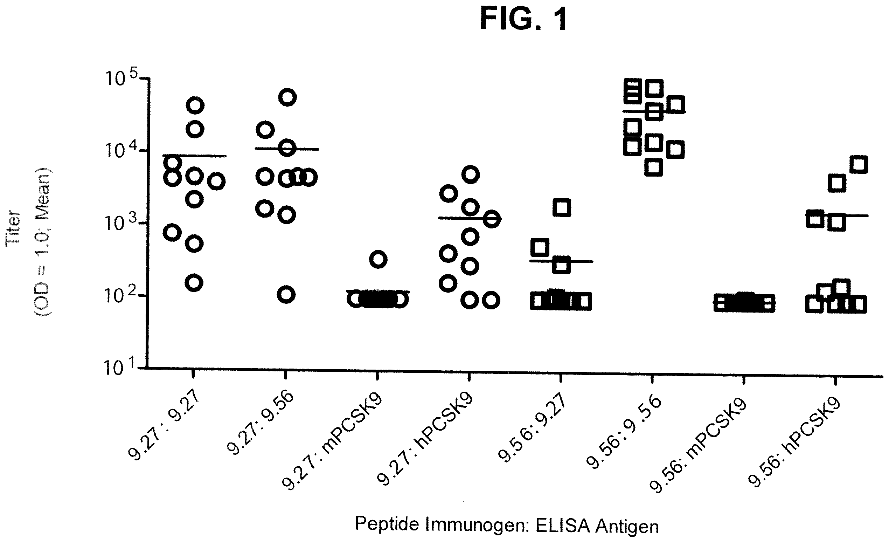

**Figure 1****:** Mice were immunized with peptides 9.27 or 9.56 conjugated to CRM₁₉₇ with Alum plus CpG as adjuvant. Antibody responses to peptides and full-length recombinant mouse and human PCSK9 were measured by titrating sera in an ELISA assay. Results are shown as reciprocal titers for each of 10 mice per group, with the reciprocal titer measured as the dilution of serum giving an optical density reading of 1.

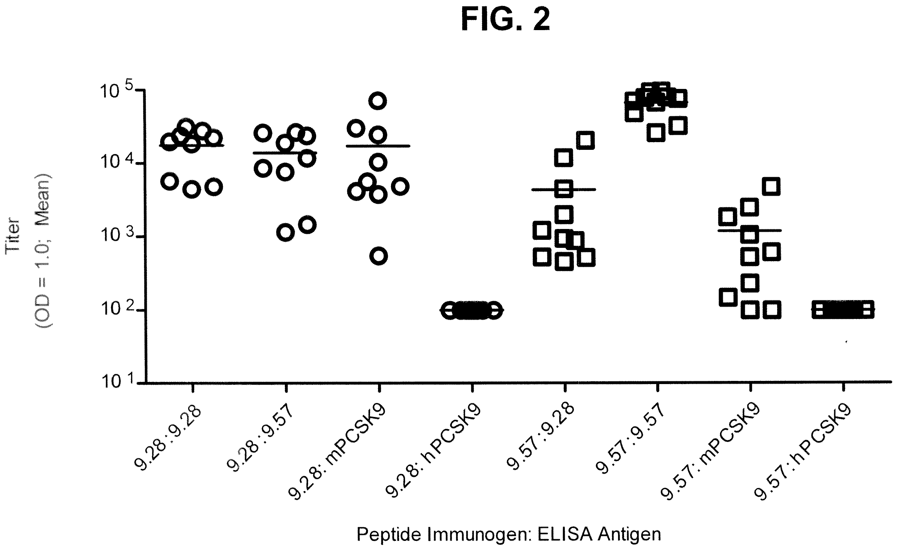

**Figure 2****:** Mice were immunized with peptides 9.28 or 9.57 conjugated to CRM₁₉₇ with Alum plus CpG as adjuvant. Antibody responses to peptide and full-length recombinant mouse and human PCSK9 were measured by titrating sera in an ELISA assay. Results are shown as reciprocal titers for each of 10 mice per group, with the reciprocal titer measured as the dilution of serum giving an optical density reading of 1.

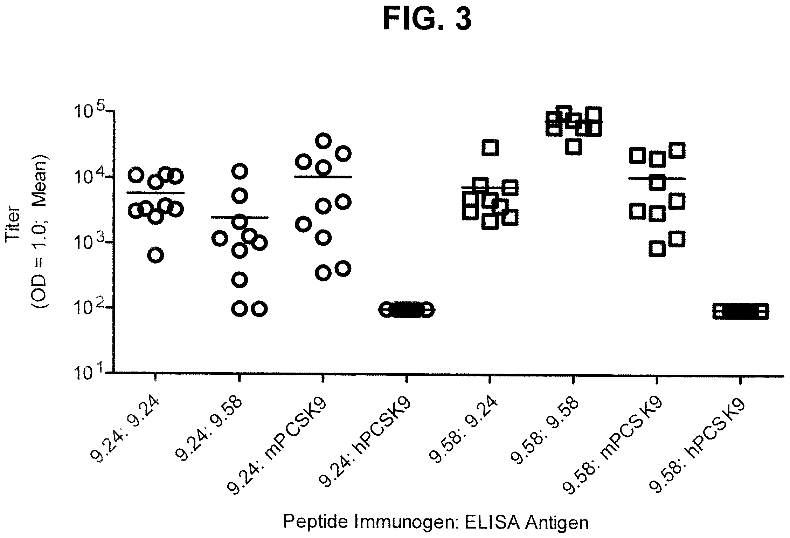

**Figure 3****:** Mice were immunized with peptides 9.24 or 9.58 conjugated to CRM₁₉₇ with Alum plus CpG as adjuvant. Antibody responses to peptide and full-length recombinant mouse and human PCSK9 were measured by titrating sera in an ELISA assay. Results are shown as reciprocal titers for each of 10 mice per group, with the reciprocal titer measured as the dilution of serum giving an optical density reading of 1.

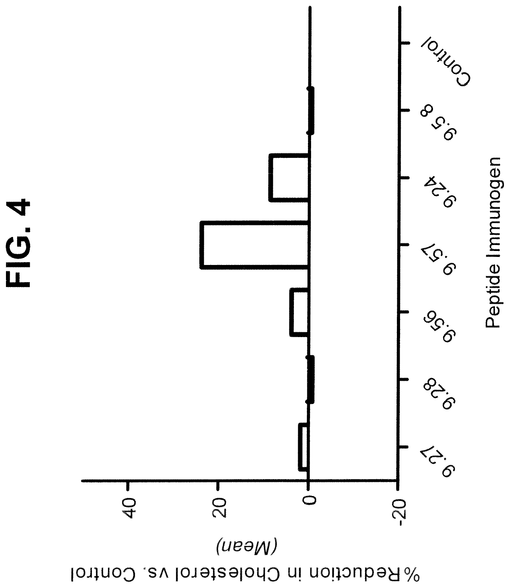

**Figure 4****:** Mice were immunized with peptides 9.24, 9.27, 9.28, 9.56, 9.57 or 9.58 conjugated to CRM₁₉₇, or CRM₁₉₇ alone with Alum plus CpG as adjuvant. Cholesterol levels were measured in the sera of vaccinated mice (same samples as used for antibody assays in Figures 1-3). Percentage reduction of cholesterol compared to control (CRM₁₉₇ only immunisation) is shown.

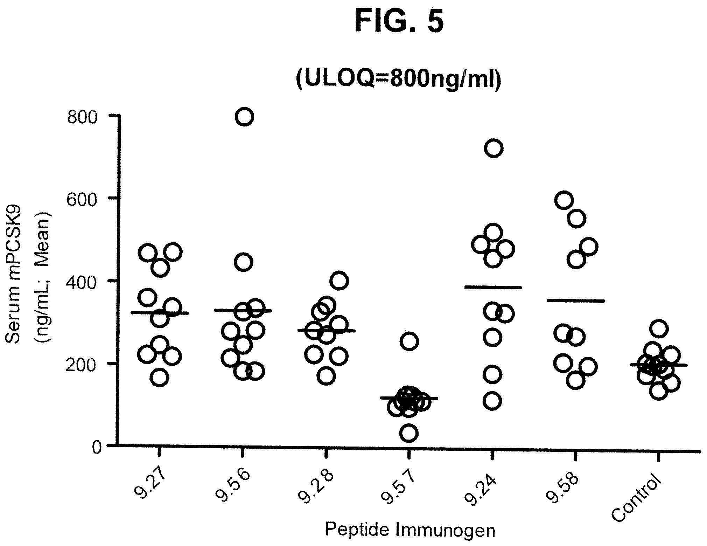

**Figure 5****:** Serum mouse PCSK9 levels measured in the sera of vaccinated mice (same samples as used for assays in Figures 1-4).

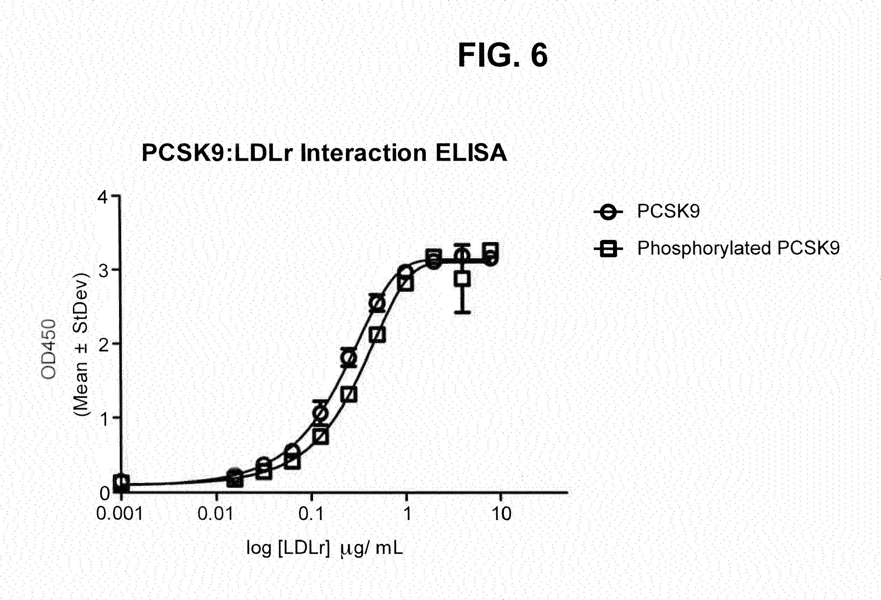

**Figure 6****:** Binding of recombinant and in vitro phosphorylated, recombinant human PCSK9 to extracellular domain of the LDL receptor as determined by ELISA.

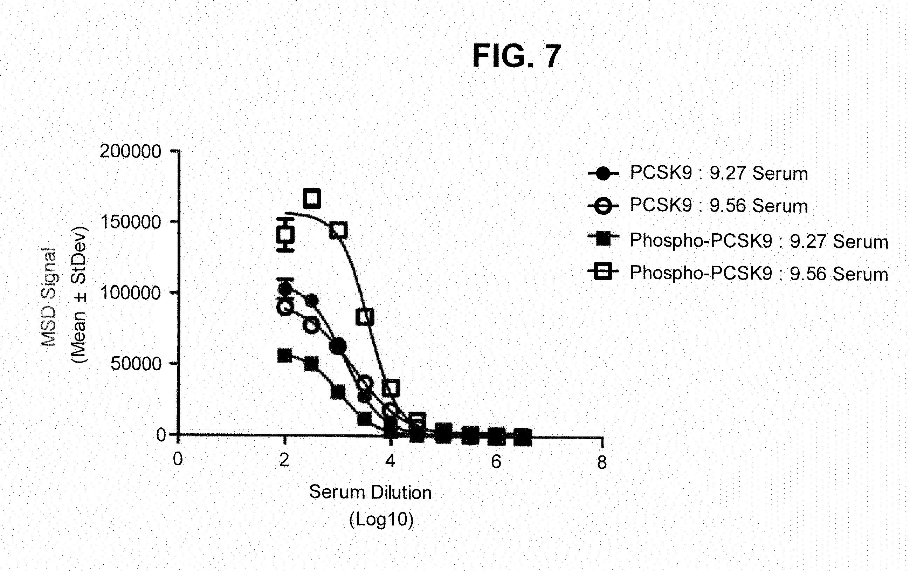

**Figure 7****:** Serum from mice immunized with peptides 9.27 or 9.56 conjugated to CRM₁₉₇ with Alum plus CpG as adjuvant were tested in MSD assay for the presence of antibodies able to bind recombinant human PCSK9 and in vitro phosphorylated recombinant human PCSK9. Results are shown from pooled serum (from 10 mice per sample).

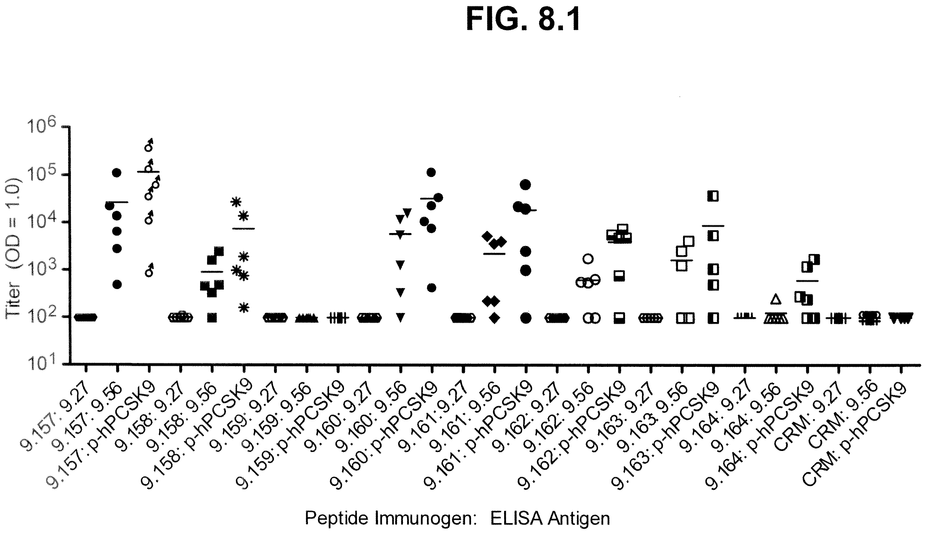

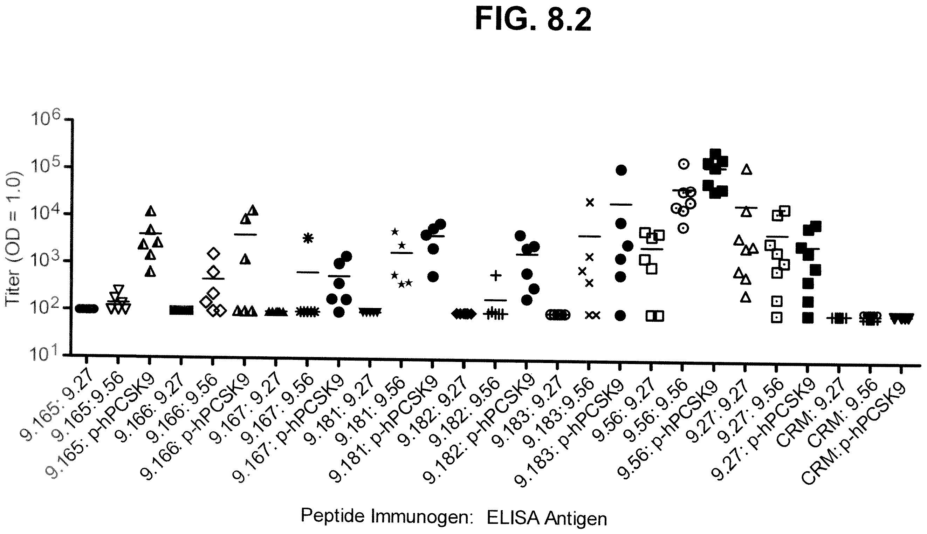

**Figure 8****:** Mice were immunized with peptides conjugated to CRM₁₉₇ with Alum plus CpG as adjuvant. Antibody responses to peptides and in vitro phosphorylated full-length recombinant human PCSK9 were measured by titrating sera in an ELISA assay. Results are shown as reciprocal titers for each of 6 to 8 mice per group, with the reciprocal titer measured as the dilution of serum giving an optical density reading of 1.

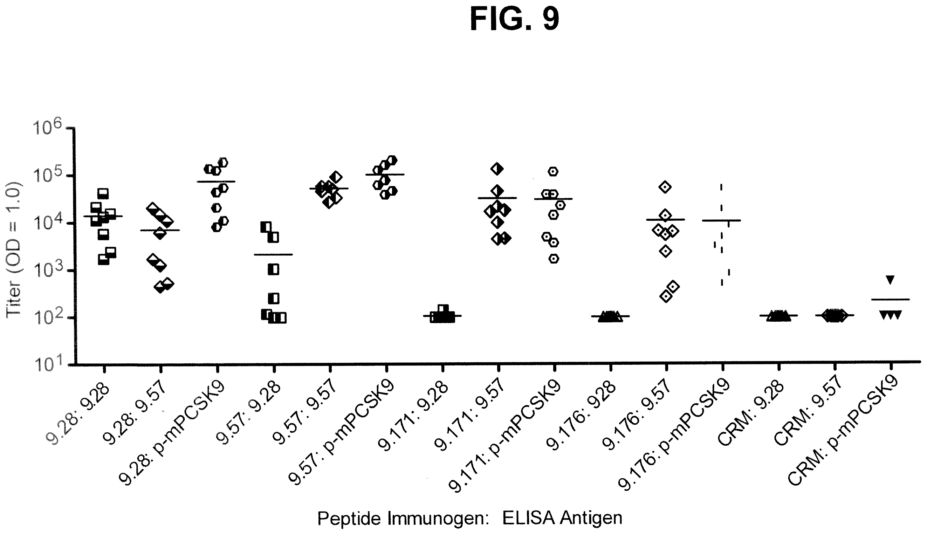

**Figure 9****:** Mice were immunized with peptides conjugated to CRM₁₉₇ with Alum plus CpG as adjuvant. Antibody responses to peptides and in vitro phosphorylated full-length recombinant mouse PCSK9 were measured by titrating sera in an ELISA assay. Results are shown as reciprocal titers for each of 8 mice per group, with the reciprocal titer measured as the dilution of serum giving an optical density reading of 1.

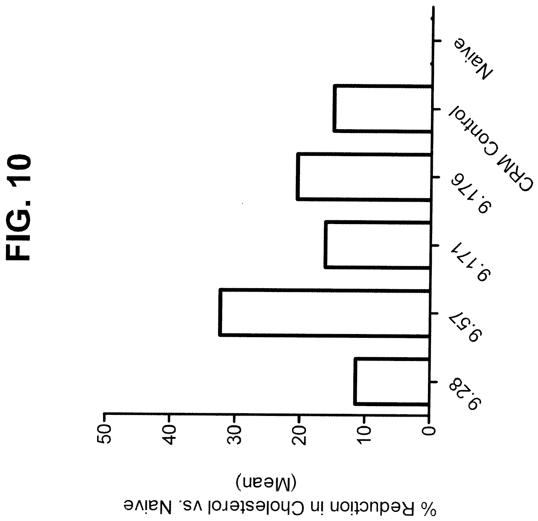

**Figure 10****:** Mice were immunized with 9.28, 9.57, 9.171 or 9.176 conjugated to CRM₁₉₇ with Alum plus CpG as adjuvant. Cholesterol levels were measured in the sera of vaccinated mice. Percentage reduction of cholesterol compared to control (naive, unvaccinated mice) is shown.

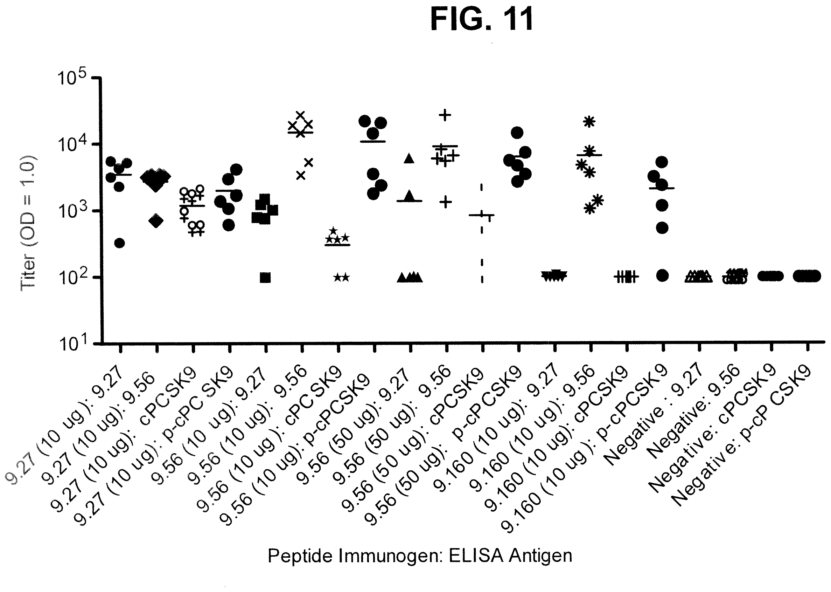

**Figure 11****:** Cynomolgus macaques were immunized with peptides conjugated to CRM₁₉₇ with Alum plus CpG as adjuvant. Antibody responses to peptides, full-length recombinant Cynomolgus PCSK9 and in vitro phosphorylated full-length recombinant Cynomolgus PCSK9 were measured by titrating sera in an ELISA assay. Results for serum 42 days post prime are shown as reciprocal titers for each of 6 animals per group, with the reciprocal titer measured as the dilution of serum giving an optical density reading of 1. Negative samples are shown with a reciprocal titer of 100.

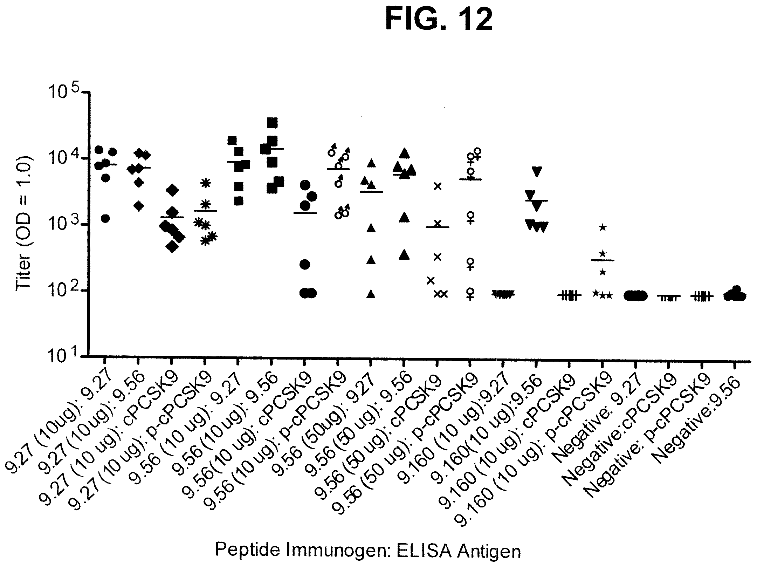

**Figure 12****:** Cynomolgus macaques were immunized with peptides conjugated to CRM₁₉₇ with Alum plus CpG as adjuvant. Antibody responses to peptides, full-length recombinant Cynomolgus PCSK9 and in vitro phosphorylated full-length recombinant Cynomolgus PCSK9 were measured by titrating sera in an ELISA assay. Results for serum 99 days post prime are shown as reciprocal titers for each of 6 animals per group, with the reciprocal titer measured as the dilution of serum giving an optical density reading of 1. Negative samples are shown with a reciprocal titer of 100.

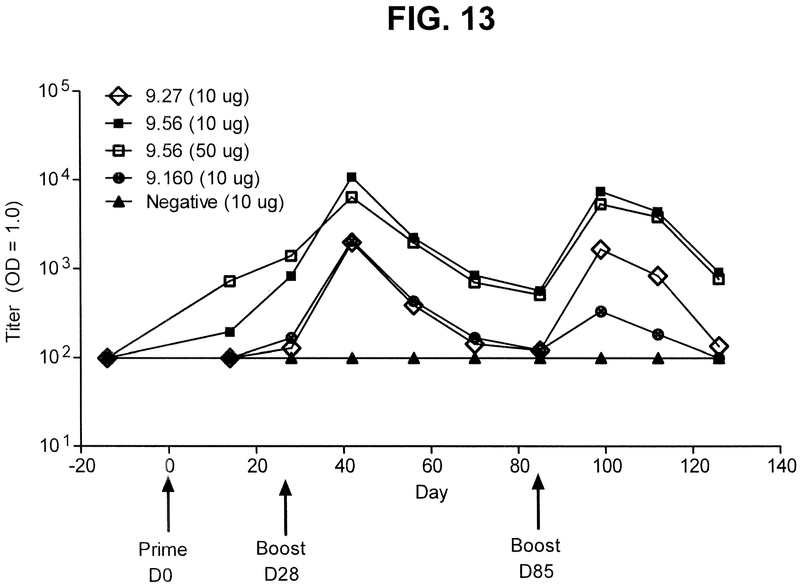

**Figure 13****:** Cynomolgus macaques were immunized with peptides conjugated to CRM₁₉₇ with Alum plus CpG as adjuvant. Antibody responses to in vitro phosphorylated full-length recombinant cynomolgus PCSK9 were measured by titrating sera in an ELISA assay. Results for days -14, 14, 28, 42, 56, 70, 85, 99, 112 and 126 post prime are shown as the average reciprocal titers of the 6 animals per group, with the reciprocal titer measured as the dilution of serum giving an optical density reading of 1. A reciprocal titer of 100 was used for negative samples.

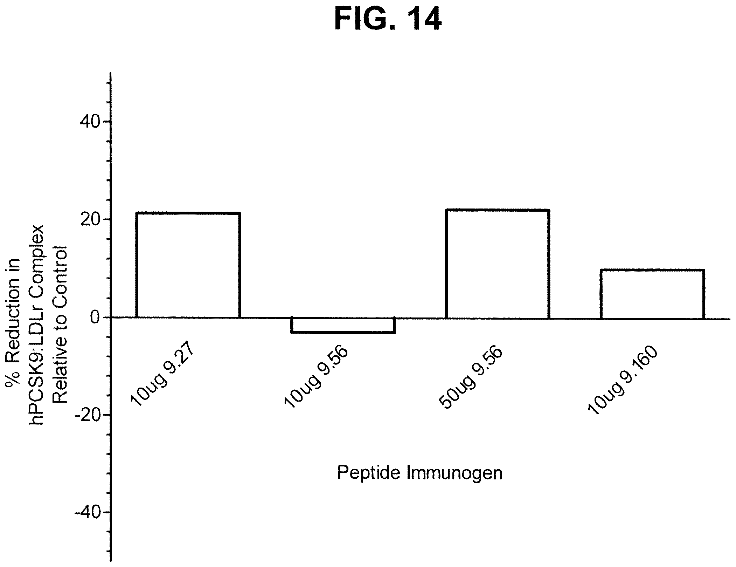

**Figure 14****:** Cynomolgus macaques were immunized with peptides conjugated to CRM₁₉₇ with Alum plus CpG as adjuvant. After the third vaccination (D99) with 9.27, 9.56, 9.160 peptides or CRM₁₉₇ control, the ability of anti-PCSK9 antibodies in serum to modulate unphosphorylated human PCSK9 binding to the human LDLr was measured. Results are shown as mean+SEM of 6 animals per group.

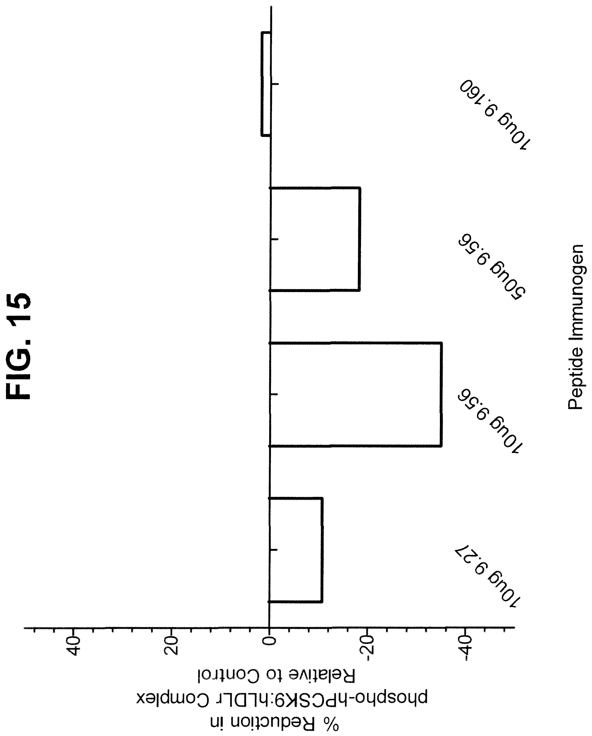

**Figure 15****:** Cynomolgus macaques were immunized with peptides conjugated to CRM₁₉₇ with Alum plus CpG as adjuvant. After the third vaccination (D99) with 9.27, 9.56, 9.160 peptides or CRM₁₉₇ control, the ability of anti-PCSK9 antibodies in serum to affect in vitro phosphorylated human PCSK9 binding to the human LDLr was measured. Results are shown as mean+SEM of 6 animals per group.

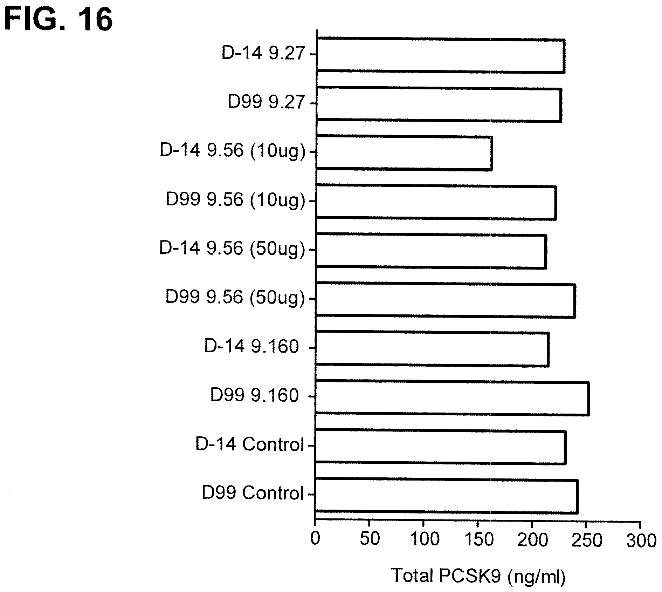

**Figure 16****:** Cynomolgus macaques were immunized with peptides conjugated to CRM₁₉₇ with Alum plus CpG as adjuvant. Total PCSK9 levels in serum were measured at prebleed (D-14) and 2 weeks after the third vaccination (D99) with 9.27, 9.56, 9.160 peptides or CRM₁₉₇ control. Results are shown as mean±SEM of 6 animals per group.

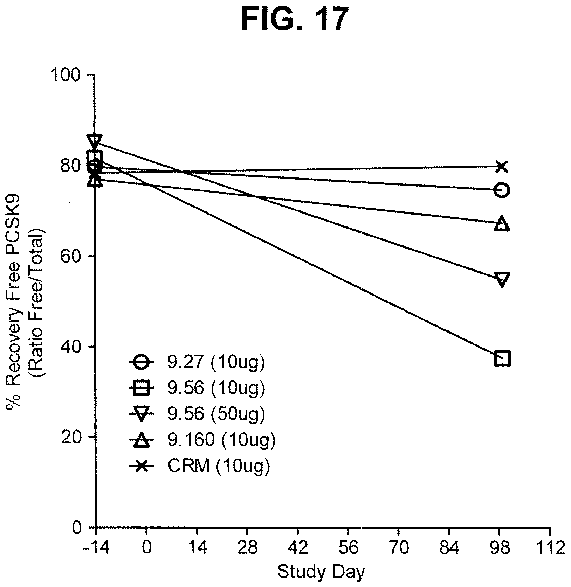

**Figure 17****:** Cynomolgus macaques were immunized with peptides conjugated to CRM₁₉₇ with Alum plus CpG as adjuvant. Free and total PCSK9 levels in serum were measured at prebleed (D-14) and 2 weeks after the third vaccination (D99) with 9.27, 9.56, 9.160 peptides or CRM₁₉₇ control. Results are shown as % recovery of free PCSK9, mean±SEM of 6 animals per group.

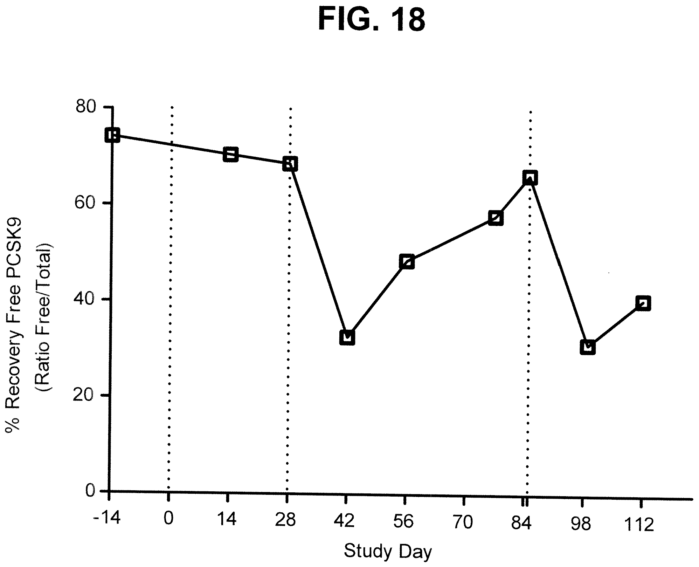

**Figure 18****:** Cynomolgus macaques were immunized with peptides conjugated to CRM₁₉₇ with Alum plus CpG as adjuvant. Free and total PCSK9 levels in serum were measured at prebleed (D-14) and every 2 weeks during the course of the study following vaccination (D99) 10µg peptide 9.56. Results are shown as % recovery of free PCSK9, mean±SEM of 6 animals per group.

### DETAILED DESCRIPTION OF THE INVENTION

### Antigenic PCSK9 Peptide of the Disclosure

The present disclosure relates to an immunogen comprising an antigenic PCSK9 peptide optionally linked to an immunogenic carrier.

In one embodiment, the antigenic PCSK9 peptide is a portion of PCSK9 comprising from 4 to 20 amino acids and, when administered to a subject, is able to lower the LDL-cholesterol level in blood of said subject. Preferably, said subject is a mammal, preferably a human. Preferably, said antigenic PCSK9 peptide is able to lower the LDL-cholesterol level by at least 2%, 5%, 10%, 20%, 30% or 50%.

In one embodiment, the antigenic PCSK9 peptide is a portion of PCSK9 which participates in the interaction of PCSK9 with the LDL receptor.

In another embodiment, the antigenic PCSK9 peptide is a portion of PCSK9 which participates in the interaction of PCSK9 with the LDL receptor, comprising from 4 to 20 amino acids and, when administered to a subject, is able to lower the LDL-cholesterol level in blood of said subject. Preferably, said subject is a mammal, preferably a human. Preferably, said antigenic PCSK9 peptide is able to lower the LDL-cholesterol level by at least 2%, 5%, 10%, 20%, 30% or 50%.

In an embodiment, the antigenic PCSK9 peptide is selected from the group consisting of SEQ ID Nos 1 to 581.

In another embodiment, the antigenic PCSK9 peptide is a portion of PCSK9 which participates in the interaction of PCSK9 with the EGF-A domain of the LDL receptor.

In a further embodiment, the antigenic PCSK9 peptide is a portion of PCSK9 which may participate in the interaction with the domain EGF-A of the LDL receptor, comprising from 4 to 20 amino acids and, when administered to a subject, is able to lower the LDL-cholesterol level in blood of said subject. Preferably, said subject is a mammal, preferably a human. Preferably, said antigenic PCSK9 peptide is able to lower the LDL-cholesterol level by at least 2%, 5%, 10%, 20%, 30% or 50%.

In one embodiment, the antigenic PCSK9 peptide is a portion of PCSK9 which may participate in the interaction with a region of the LDL receptor other than the EGF-A domain, comprising from 4 to 20 amino acids and, when administered to a subject, is able to lower the LDL-cholesterol level in blood of said subject. Preferably, said subject is a mammal, preferably a human. Preferably, said antigenic PCSK9 peptide is able to lower the LDL-cholesterol level by at least 2%, 5%, 10%, 20%, 30% or 50%.

In an embodiment, the antigenic PCSK9 peptide is selected in a region of PCSK9 pro-domain (SEQ ID NOs. 1, 3 to 286).

In one embodiment, the antigenic PCSK9 peptide is a portion of PCSK9 pro-domain, comprising from 4 to 20 amino acids and, when administered to a subject, is able to lower the LDL-cholesterol level in blood of said subject. Preferably, said subject is a mammal, preferably a human. Preferably, said antigenic PCSK9 peptide is able to lower the LDL-cholesterol level by at least 2%, 5%, 10%, 20%, 30% or 50%.

In an embodiment, the antigenic PCSK9 peptide is selected in a region of PCSK9 pro-domain and may contain potential phosphorylation sites and amino acids that may be modified by phosphorylation (SEQ ID NOs. 1, 3 to 286).

In one embodiment, the antigenic PCSK9 peptide is a portion of PCSK9 pro-domain, and contains potential phosphorylation sites and amino acids that may be modified by phosphorylation, comprising from 4 to 20 amino acids and, when administered to a subject, is able to lower the LDL-cholesterol level in blood of said subject. Preferably, said subject is a mammal, preferably a human. Preferably, said antigenic PCSK9 peptide is able to lower the LDL-cholesterol level by at least 2%, 5%, 10%, 20%, 30% or 50%.

In an embodiment, the antigenic PCSK9 peptide is selected in a region of PCSK9 C-terminal domain (SEQ ID NOs. 1 to 3, 287 to 581).

In one embodiment, the antigenic PCSK9 peptide is a portion of PCSK9 C-terminal domain, comprising from 4 to 20 amino acids and, when administered to a subject, is able to lower the LDL-cholesterol level in blood of said subject. Preferably, said subject is a mammal, preferably a human. Preferably, said antigenic PCSK9 peptide is able to lower the LDL-cholesterol level by at least 2%, 5%, 10%, 20%, 30% or 50%.

In an embodiment, the antigenic PCSK9 peptide is selected in a region of PCSK9 C-terminal domain and may contain potential phosphorylation sites and amino acids that may be modified by phosphorylation (SEQ ID NOs. 1 to 3, 287 to 581).

In one embodiment, the antigenic PCSK9 peptide is a portion of PCSK9 C-terminal domain, and contains a serine residue modified by phosphorylation, comprising from 4 to 20 amino acids and, when administered to a subject, is able to lower the LDL-cholesterol level in blood of said subject. Preferably, said subject is a mammal, preferably a human. Preferably, said antigenic PCSK9 peptide is able to lower the LDL-cholesterol level by at least 2%, 5%, 10%, 20%, 30% or 50%.

Such antigenic PCSK9 peptides may be used alone or in combination, preferably when conjugated to an immunogenic carrier, to induce auto anti-PCSK9 antibodies in a subject in order to treat, prevent or ameliorate PCSK9-related disorders.

It will be apparent to one skilled in the art which techniques may be used to confirm whether a specific construct falls within the scope of the present disclosure. Such techniques include, but are not restricted to, the techniques described in the Example section of the present application, and also to the following.

The ability of the antigenic PCSK9 peptide of the disclosure to induce auto anti-PCSK9 antibodies may be measured in mice, using the test disclosed in Example 4 of the present application. The ability of auto-antibodies induced by the antigenic PCSK9 peptide of the disclosure to decrease the level of circulating plasma cholesterol may be measured in mice, using the test disclosed in Example 4. The ability of auto-antibodies induced by the antigenic PCSK9 peptide of the disclosure to affect the interaction between PCSK9 and LDL receptors may be measured directly using a method similar to that disclosed in Examples 4 and 7 (PCSK9:LDLr interaction ELISA) or indirectly by measuring the upregulation of cell surface LDL receptors or increase in LDL uptake which is a consequence of blocking PCSK9-mediated down-regulation using a method similar to that disclosed in Example 7 (as well described in the relevant literature, either using cell lines in vitro or by measuring LDL receptor levels in liver biopsies of antibody expressing animals (e.g., by Western blotting)). The ability of auto-antibodies induced by the antigenic PCSK9 peptide of the disclosure to affect circulating levels of PCSK9 may be measured using a method similar to that disclosed in Example 7.

The term "antigenic PCSK9 peptide biological activity", when used herein, refers to the ability of the antigenic PCSK9 peptides of the disclosure to induce auto anti-PCSK9 antibodies in a patient.

In an embodiment, the antigenic PCSK9 peptides of the present disclosure are of a size such that they mimic a region selected from the whole PCSK9 domain in which the native epitope is found. In a particular embodiment, the antigenic PCSK9 peptides of the disclosure are less than 100 amino acids in length, preferably shorter than 75 amino acids, more preferably less than 50 amino acids, even more preferably less than 40 amino acids. The antigenic PCSK9 peptides of the disclosure are typically 4, 5, 6, 7, 8, 9, 10, 11, 12, 13, 14, 15, 16, 17, 18, 19, 20, 21, 22, 23, 24, 25, 26, 27, 28, 29 or 30 amino acids in length, preferably from 4 to 20 amino acids, for example 6 to 12, 6 to 8 or 9 to 12 amino acids.

Specific examples of antigenic PCSK9 peptides of the disclosure are provided in the sequence listing and include peptides ranging from 6 to 16 amino acids in length.

The antigenic peptides of the disclosure include an amino acid sequence derived from a portion of a mammalian PCSK9, preferably a human PCSK9 (SEQ ID NO. 1) or mouse PCSK9 (SEQ ID NO. 2), more preferably human PCSK9, such derived portion of PCSK9 either corresponding to the amino acid sequence of naturally occurring PCSK9 or corresponding to variant PCSK9, i.e., the amino acid sequence of naturally occurring PCSK9 in which a small number of amino acids have been substituted, added or deleted but which retains essentially the same immunological properties. In addition, such derived PCSK9 portion may contain potential or known phosphorylation sites suggestive of functional relevance that can be modified (or not) by inclusion of modified amino acids, for example phosphorylated residues, to mimic post-translational modifications of PCSK9 that may occur in vivo. Peptides containing such potential phosphorylation sites, whether phosphorylated at these residues or not, are expected to present the antigenic PCSK9 epitope in a manner similar to their functionally relevant native conformation, thereby inducing anti-PCSK9 antibodies more susceptible to recognize intact, native self PCSK9 molecules or with an increased affinity to recognize self PCSK9 molecules. In addition, such derived PCSK9 portion can be further modified by amino acids, especially at the N- and C-terminal ends to allow the antigenic PCSK9 peptide to be conformationally constrained and/or to allow coupling (such as linking) of the antigenic PCSK9 peptide to an immunogenic carrier after appropriate chemistry has been carried out.

The antigenic PCSK9 peptides disclosed herein encompass functionally active variant peptides derived from the amino acid sequence of PCSK9 in which amino acids have been deleted, inserted, modified by phosphorylation or substituted without essentially detracting from the immunological properties thereof, i.e., such functionally active variant peptides retain or enhance a substantial antigenic PCSK9 peptide biological activity. Typically, such functionally variant peptides have an amino acid sequence homologous, preferably highly homologous, to an amino acid sequence selected from the group consisting of SEQ ID NOs.: 1 to 581.

In one embodiment, such functionally active variant peptides exhibit at least 60%, 65%, 70%, 75%, 80%, 85%, 90% or 95% identity to an amino acid sequence selected from the group consisting of SEQ ID NOs.: 1 to 581.

The term "phosphopeptide" refers to a peptide that incorporates one or more phosphate groups, and is typically associated with protein phosphorylation.

As used herein, the term "phosphorylated" in reference to an amino acid residue refers to the presence of a phosphate group on the side chain of the residue where a hydroxyl group is otherwise normally present. Such phosphorylation typically occurs as a substitution of the hydrogen atom from a hydroxyl group for a phosphate group (-PO₃H₂). As recognized by those of skill in the art, depending on the pH of the local environment, this phosphate group can exist as an uncharged, neutral group (-PO₃H₂), or with a single (-PO₃H⁻), or double (-PO₃²⁻) negative charge. Amino acid residues that can typically be phosphorylated include the side chains of serine, threonine, and tyrosine. Throughout the present disclosure, an amino acid residue that is phosphorylated is indicated by a "p" preceding the phosphorylated residue, or by bold text and underlined.

Sequence similarity for polypeptides, which is also referred to as sequence identity, is typically measured using sequence analysis software. Protein analysis software matches similar sequences using measures of similarity assigned to various substitutions, deletions and other modifications, including conservative amino acid substitutions. For instance, GCG contains programs such as "Gap" and "Bestfit" which can be used with default parameters to determine sequence homology or sequence identity between closely related polypeptides, such as homologous polypeptides from different species of organisms or between a wild type protein and a mutein thereof. See, e.g., GCG Version 6.1. Polypeptide sequences also can be compared using FASTA using default or recommended parameters, a program in GCG Version 6.1. FASTA (e.g., FASTA2 and FASTA3) provides alignments and percent sequence identity of the regions of the best overlap between the query and search sequences (Pearson, Methods Enzymol. 183:63-98 (1990); Pearson, Methods Mol. Biol. 132:185-219 (2000)). An alternative algorithm when comparing a sequence of the disclosure to a database containing a large number of sequences from different organisms is the computer program BLAST, especially blastp or tblastn, using default parameters. *See, e.g.,* Altschul et al., J. Mol. Biol. 215:403-410 (1990); Altschul et al., Nucleic Acids Res. 25:3389-402 (1997).

Functionally active variants comprise naturally occurring functionally active variants such as allelic variants, and species variants and non-naturally occurring functionally active variants that can be produced by, for example, mutagenesis techniques or by direct synthesis.

A functionally active variant differs by about, for example, 1, 2, 3, 4 or 5 amino acid residues from any of the peptide shown at SEQ ID NOs.: 1 to 581, and yet retain an antigenic PCSK9 biological activity. Where this comparison requires alignment, the sequences are aligned for maximum homology. The site of variation can occur anywhere in the peptide, as long as the biological activity is substantially similar to a peptide shown in SEQ ID NOs.: 1 to 581.

Guidance concerning how to make phenotypically silent amino acid substitutions is provided in Bowie et al., Science, 247: 1306-1310 (1990), which teaches that there are two main strategies for studying the tolerance of an amino acid sequence to change.

The first strategy exploits the tolerance of amino acid substitutions by natural selection during the process of evolution. By comparing amino acid sequences in different species, the amino acid positions which have been conserved between species can be identified. These conserved amino acids are likely important for protein function. In contrast, the amino acid positions in which substitutions have been tolerated by natural selection indicate positions which are not critical for protein function. Thus, positions tolerating amino acid substitution can be modified while still maintaining specific immunogenic activity of the modified peptide.

The second strategy uses genetic engineering to introduce amino acid changes at specific positions of a cloned gene to identify regions critical for protein function. For example, site-directed mutagenesis or alanine-scanning mutagenesis can be used (Cunningham et al., Science, 244: 1081-1085 (1989)). The resulting variant peptides can then be tested for specific antigenic PCSK9 biological activity.

According to Bowie et al., these two strategies have revealed that proteins are surprisingly tolerant of amino acid substitutions. The authors further indicate which amino acid changes are likely to be permissive at certain amino acid positions in the protein. For example, the most buried or interior (within the tertiary structure of the protein) amino acid residues require nonpolar side chains, whereas few features of surface or exterior side chains are generally conserved.

Methods of introducing a mutation into amino acids of a protein is well known to those skilled in the art. See, e. g., Ausubel (ed.), Current Protocols in Molecular Biology, John Wiley and Sons, Inc. (1994); T. Maniatis, E. F. Fritsch and J. Sambrook, Molecular Cloning: A Laboratory Manual, Cold Spring Harbor laboratory, Cold Spring Harbor, N. Y. (1989)).

Mutations can also be introduced using commercially available kits such as "QuikChangeTM Site-Directed Mutagenesis Kit" (Stratagene) or directly by peptide synthesis. The generation of a functionally active variant to an antigenic PCSK9 peptide by replacing an amino acid which does not significantly influence the function of said antigenic PCSK9 peptide can be accomplished by one skilled in the art.

A type of amino acid substitution that may be made in one of the peptides according to the disclosure is a conservative amino acid substitution. A "conservative amino acid substitution" is one in which an amino acid residue is substituted by another amino acid residue having a side chain R group with similar chemical properties (e.g., charge or hydrophobicity). In general, a conservative amino acid substitution will not substantially change the functional properties of a protein. In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. See e.g. Pearson, Methods Mol. Biol. 243:307-31 (1994).

Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine.

Alternatively, a conservative replacement is any change having a positive value in the PAM250 log-likelihood matrix disclosed in Gonnet et al., Science 256:1443-45 (1992). A "moderately conservative" replacement is any change having a nonnegative value in the PAM250 log-likelihood matrix.

A functionally active variant peptide can also be isolated using a hybridization technique. Briefly, DNA having a high homology to the whole or part of a nucleic acid sequence encoding the peptide of interest, e.g. SEQ ID NOs.: 1 to 581, is used to prepare a functionally active peptide. Therefore, an antigenic PCSK9 peptide of the disclosure also includes peptides which are functionally equivalent to one or more of the peptide of SEQ ID NOs.: 1 to 581 and which are encoded by a nucleic acid molecule which hybridizes with a nucleic acid encoding any one of SEQ ID NOs.: 1 to 581, or a complement thereof. One of skill in the art can easily determine nucleic acid sequences that encode peptides of the invention using readily available codon tables. As such, these nucleic acid sequences are not presented herein.

The stringency of hybridization for a nucleic acid encoding a peptide, polypeptide or protein that is a functionally active variant is, for example, 10% formamide, 5 x SSPE, 1 x Denhart's solution, and 1 x salmon sperm DNA (low stringency conditions). More preferable conditions are, 25% formamide, 5 x SSPE, 1 x Denhart's solution, and 1 x salmon sperm DNA (moderate stringency conditions), and even more preferable conditions are, 50% formamide, 5 x SSPE, 1 x Denhart's solution, and 1 x salmon sperm DNA (high stringency conditions). However, several factors influence the stringency of hybridization other than the above-described formamide concentration, and one skilled in the art can suitably select these factors to accomplish a similar stringency.

Nucleic acid molecules encoding a functionally active variant can also be isolated by a gene amplification method such as PCR using a portion of a nucleic acid molecule DNA encoding a peptide, polypeptide or protein of interest, e.g. any one of the peptides shown SEQ ID NOs.: 1 to 581, as the probe.

In one embodiment of the disclosure a peptide is derived from a natural source and isolated from a mammal, such as a human, a primate, a cat, a dog, a horse, a mouse, or a rat, preferably from a human source. A peptide of the invention can thus be isolated from cells or tissue sources using standard protein purification techniques.

Alternatively, peptides can be synthesized chemically or produced using recombinant DNA techniques.

For example, a peptide can be synthesized by solid phase procedures well known in the art. Suitable syntheses may be performed by utilising "T-boc" or "F-moc" procedures. Cyclic peptides can be synthesised by the solid phase procedure employing the well-known "F-moc"procedure and polyamide resin in the fully automated apparatus. Alternatively, those skilled in the art will know the necessary laboratory procedures to perform the process manually. Techniques and procedures for solid phase synthesis are described in 'Solid Phase Peptide Synthesis: A Practical Approach' by E. Atherton and R. C. Sheppard, published by IRL at Oxford University Press (1989) and 'Methods in Molecular Biology, Vol. 35: Peptide Synthesis Protocols (ed. M. W.Pennington and B. M. Dunn), chapter 7, pp91-171 by D. Andreau et al.

Alternatively, a polynucleotide encoding a peptide can be introduced into an expression vector that can be expressed in a suitable expression system using techniques well known in the art, followed by isolation or purification of the expressed peptide, polypeptide, or protein of interest. A variety of bacterial, yeast, plant, mammalian, and insect expression systems are available in the art and any such expression system can be used. Optionally, a polynucleotide encoding a peptide of the invention can be translated in a cell-free translation system.

Antigenic PCSK9 peptides can also comprise those that arise as a result of the existence of multiple genes, alternative transcription events, alternative RNA splicing events, and alternative translational and postranslational events. A peptide can be expressed in systems, e.g., cultured cells, which result in substantially the same postranslational modifications present as when the peptide is expressed in a native cell, or in systems that result in the alteration or omission of postranslational modifications, e.g. glycosylation or cleavage, present when expressed in a native cell.

An antigenic PCSK9 peptide can be produced as a fusion protein that contains other non- PCSK9 or non- PCSK9-derived amino acid sequences, such as amino acid linkers or signal sequences or immunogenic carriers as defined herein, as well as ligands useful in protein purification, such as glutathione-S-transferase, histidine tag, and staphylococcal protein A. More than one antigenic PCSK9 peptide can be present in a fusion protein. The heterologous polypeptide can be fused, for example, to the N- terminus or C-terminus of the peptide. A peptide can also be produced as fusion proteins comprising homologous amino acid sequences, i. e., other PCSK9 or PCSK9-derived sequences.

The antigenic PCSK9 peptides might be linear or conformationally constrained. As used herein in reference to a peptide, the term "conformationally constrained" means a peptide, in which the three-dimensional structure is maintained substantially in one spatial arrangement over time. Conformationally constrained molecules can have improved properties such as increased affinity, metabolic stability, membrane permeability or solubility.

In addition, such conformationally constrained peptides are expected to present the antigenic PCSK9 epitope in a conformation similar to their native loop conformation, thereby inducing anti-PCSK9 antibodies more susceptible to recognize intact, native self PCSK9 molecules or with an increased affinity to recognize self PCSK9 molecules. Methods of conformational constraint are well known in the art and include, without limitation, bridging and cyclization.

There are several approaches known in the prior art to introduce conformational constraints into a linear peptide. For example, bridging between two neighbouring amino acids in a peptide leads to a local conformational modification, the flexibility of which is limited in comparison with that of regular peptides. Some possibilities for forming such bridges include incorporation of lactams and piperazinones (for review see Giannis and. Kolter, Angew. Chem. Int. Ed., 1993,32:1244).

As used herein in reference to a peptide, the term "cyclic" or "cyclised" refers to a structure including an intramolecular bond between two non-adjacent amino acids or amino acid analogs. The cyclization can be effected through a covalent or non-covalent bond. Intramolecular bonds include, but are not limited to, backbone to backbone, side-chain to backbone, side-chain to side-chain, side chain to end-group, end-to-end bonds. Methods of cyclization include, without limitation, formation of an amide bond between the N-term residue and the C-term residue of a peptide, formation of a disulfide bond between the side-chains of non-adjacent amino acids or amino acid analogs; formation of an amide bond between the side-chains of Lys and Asp/Glu residues; formation of an ester bond between serine residues and Asp/Glu residues; formation of a lactam bond, for example, between a side-chain group of one amino acid or analog thereof to the N-terminal amine of the amino-terminal residue; and formation of lysinonorleucine and dityrosine bonds. Carbon versions of a disulfide linkage, for example an ethenyl or ethyl linkage, could also be used (J. Peptide Sc., 2008, 14, 898-902) as well as alkylation reactions with an appropriately polysubstituted electrophilic reagent such as a di-, tri- or tetrahaloalkane (PNAS, 2008, 105(40), 15293-15298; ChemBioChem, 2005, 6, 821-824). Various modified proline analogs can also be used to incorporate conformational constraints into peptides (Zhang et al., J. Med Chem., 1996,39: 2738-2744; Pfeifer and Robinson, Chem. Comm., 1998,1977-1978). Chemistries that may be used to cyclise peptides of the invention result in peptides cyclised with a bond including, but not limiting to the following: lactam, hydrazone, oxime, thiazolidine, thioether or sulfonium bonds.

Yet another approach in the design of conformationally constrained peptides, which is described in USSN 10/114918, is to attach a short amino acid sequence of interest to a template, to generate a cyclic constrained peptide. Such cyclic peptides are not only structurally stabilized by their templates, and thereby offer three-dimensional conformations that may imitate conformational epitopes on native proteins such as on viruses and parasites or on self proteins (autologous mammalian proteins such as PCSK9), but they are also more resistant than linear peptides to proteolytic degradation in serum. USSN 10/114918 further discloses the synthesis of conformationally constrained cross-linked peptides by preparation of synthetic amino acids for backbone coupling to appropriately positioned amino acids in order to stabilize the supersecondary structure of peptides. Cross-linking can be achieved by amide coupling of the primary amino group of an orthogonally protected (2S, 3R)-3-aminoproline residue to a suitably positioned side chain carboxyl group of glutamate. This approach has been followed in the preparation of conformationally constrained tetrapeptide repeats of the CS protein wherein at least one proline has been replaced by (2S, 3R)-3-aminoproline and, in order to introduce a side chain carboxyl group, glutamate has been incorporated as a replacement for alanine.

Cross-linking strategies also include the application of the Grubbs ring-closing metathesis reaction to form 'stapled' peptides designed to mimic alpha-helical conformations (Angew. Int. Ed. Engl., 1998, 37, 3281 ; JACS, 2000, 122, 5891); use of poly-functionalised saccharides ; use of a tryptathionine linkage (Chemistry Eu. J., 2008, 24, 3404-3409); use of 'click' reaction of azides and alkynes which could be incorporated as either a side chain amino acid residues or located within the backbone of the peptide sequence (Drug Disc. Today, 2003, 8(24), 1128-1137). It is also known in the literature that metal ions can stabilise constrained conformations of linear peptides through sequestering specific residues e.g. histidine, which co-ordinate to metal cations (Angew. Int. Ed. Engl., 2003, 42, 421). Similarly, functionalising a linear peptide sequence with non-natural acid and amine functionality, or polyamine and polyacid functionality can be used to allow access to cyclised structures following activation and amide bond formation.

According to one embodiment, the antigenic PCSK9 peptide is conformationally constrained by intramolecular covalent bonding of two non-adjacent amino acids of the antigenic PCSK9 peptide to each other, e.g. the N- and C- terminal amino acids. According to another embodiment, the antigenic PCSK9 peptide is conformationally constrained by covalent binding to a scaffold molecule. According to a further embodiment, the antigenic PCSK9 peptide is simply constrained, i.e., coupled either at one end, (C or N terminus) or through another amino acid not located at either end, to the scaffold molecule. According to another embodiment, the antigenic PCSK9 peptide is doubly constrained, i.e., coupled at both C and N termini to the scaffold molecule. According to another embodiment, the antigenic peptide is constrained by cyclising via the templating effect of a heterochiral Diproline unit (D-Pro-L-Pro) (Spath et al, 1998, Helvetica Chimica Acta 81, p1726-1738).

The scaffold (also called 'platform') can be any molecule which is capable of reducing, through covalent bonding, the number of conformations which the antigenic PCSK9 peptide can assume. Examples of conformation-constraining scaffolds include proteins and peptides, for example lipocalin-related molecules such as beta-barrel containing thioredoxin and thioredoxin-like proteins, nucleases (e.g. RNaseA), proteases (e.g., trypsin), protease inhibitors (e.g., eglin C), antibodies or structurally-rigid fragments thereof, fluorescent proteins such as GFP or YFP, conotoxins, loop regions of fibronectin type III domain, CTL-A4, and virus-like particles (VLPs).

Other suitable platform molecules include carbohydrates such as sepharose. The platform may be a linear or circular molecule, for example, closed to form a loop. The platform is generally heterologous with respect to the antigenic PCSK9 peptide. Such conformationally constrained peptides linked to a platform are thought to be more resistant to proteolytic degradation than linear peptide.

According to an embodiment, the scaffold is an immunogenic carrier as defined in the present application. In a further embodiment, the antigenic PCSK9 peptide is simply constrained onto the immunogenic carrier. In another further embodiment, the antigenic PCSK9 peptide is doubly constrained onto the immunogenic carrier. In this manner, the antigenic PCSK9 peptide forms a conformationally constrained loop structure which has proven to be a particularly suitable structure as an intracellular recognition molecule.

The antigenic PCSK9 peptides may be modified for the ease of conjugation to a platform, for example by the addition of a terminal cysteine at one or both ends and/or by the addition of a linker sequence, such a double glycine head or tail plus a terminal cysteine, a linker terminating with a lysine residue or any other linker known to those skilled in the art to perform such function. Details of such linkers are disclosed hereafter. Bioorthogonal chemistry (such as the click reaction described above) to couple the full peptide sequence to the carrier, thus avoiding any regiochemical and chemoselectivity issues, might also be used. Rigidified linkers such as the one described in Jones et al. Angew. Chem. Int. Ed. 2002, 41:4241-4244 are known to elicit an improved immunological response and might also be used.

In a further embodiment, the antigenic PCSK9 peptide is attached to a multivalent template, which itself is coupled to the carrier, thus increasing the density of the antigen (see below). The multivalent template could be an appropriately functionalised polymer or oligomer such as (but not limited to) oligoglutamate or oligochitosan.

### Immunogenic Carrier

The antigenic PCSK9 peptide is linked to an immunogenic carrier molecule to form immunogens for vaccination protocols, preferably wherein the carrier molecule is not related to the native PCSK9 molecule.

The term "immunogenic carrier" herein includes those materials which have the property of independently eliciting an immunogenic response in a host animal and which can be covalently coupled to a peptide, polypeptide or protein either directly via formation of peptide or ester bonds between free carboxyl, amino or hydroxyl groups in the peptide, polypeptide or protein and corresponding groups on the immunogenic carrier material, or alternatively by bonding through a conventional bifunctional linking group, or as a fusion protein.

The types of carriers used in the immunogens of the present disclosure will be readily known to the person skilled in the art. Examples of such immunogenic carriers are: serum albumins such as bovine serum albumin (BSA); globulins; thyroglobulins; hemoglobins; hemocyanins (particularly Keyhole Limpet Hemocyanin [KLH]); polylysin; polyglutamic acid; lysine-glutamic acid copolymers; copolymers containing lysine or ornithine; liposome carriers; the purified protein derivative of tuberculin (PPD); inactivated bacterial toxins or toxoids such as tetanus or diptheria toxins (TT and DT) or fragment C of TT, CRM197 (a nontoxic but antigenically identical variant of diphtheria toxin) other DT point mutants, such as CRM176, CRM228, CRM 45 (Uchida et al J. Biol. Chem. 218; 3838-3844, 1973); CRM 9, CRM 45, CRM102, CRM 103 and CRM107 and other mutations described by Nicholls and Youle in Genetically Engineered Toxins, Ed: Frankel, Maecel Dekker Inc, 1992; deletion or mutation of Glu-148 to Asp, Gln or Ser and/or Ala 158 to Gly and other mutations disclosed in US 4709017 or US 4950740; mutation of at least one or more residues Lys 516, Lys 526, Phe 530 and/or Lys 534 and other mutations disclosed in US 5917017 or US 6455673; or fragment disclosed in US 5843711, pneumococcal pneumolysin (Kuo et al (1995) Infect Immun 63; 2706-13) including ply detoxified in some fashion for example dPLY-GMBS (WO04081515, PCT/EP2005/010258) or dPLY-formol, PhtX, including PhtA, PhtB, PhtD, PhtE (sequences of PhtA, PhtB, PhtD or PhtE are disclosed in WO00/37105 or WO 00/39299) and fusions of Pht proteins for example PhtDE fusions, PhtBE fusions, Pht A-E (WO01/98334, WO03/54007, WO2009/000826), OMPC (meningococcal outer membrane protein - usually extracted from N. meningitidis serogroup B - EP0372501), PorB (from N. meningitidis), PD (Haemophilus influenzae protein D - see, e.g., EP0594610B), or immunologically functional equivalents thereof, synthetic peptides (EP0378881, EP0427347), heat shock proteins (WO93/17712, WO94/03208), pertussis proteins (WO98/58668, EP0471 177), cytokines, lymphokines, growth factors or hormones (WO91/01146), artificial proteins comprising multiple human CD4+ T cell epitopes from various pathogen derived antigens (Falugi et al (2001) Eur J Immunol 31; 3816-3824) such as N19 protein (Baraldoi et al (2004) Infect Immun 72; 4884-7) pneumococcal surface protein PspA (WO02/091998), iron uptake proteins (WO01/72337), toxin A or B of C. difficile (WO00/61761).

In an embodiment, the immunogenic carrier is CRM197.

In another embodiment, the immunogenic carrier is a virus-like particle (VLPs), preferably a recombinant virus-like particle.

As used herein, the term "virus-like particle" refers to a structure resembling a virus particle but which has been demonstrated to be non pathogenic. In general, virus-like particles lack at least part of the viral genome. Also, virus-like particles can often be produced in large quantities by heterologous expression and can be easily purified. A virus-like particle in accordance with the invention may contain nucleic acid distinct from their genome. A typical embodiment of a virus-like particle in accordance with the present disclosure is a viral capsid such as the viral capsid of the corresponding virus, bacteriophage, or RNA-phage.

As used herein, the term "virus- like particle of a bacteriophage" refers to a virus-like particle resembling the structure of a bacteriophage, being non replicative and noninfectious, and lacking at least the gene or genes encoding for the replication machinery of the bacteriophage, and typically also lacking the gene or genes encoding the protein or proteins responsible for viral attachment to or entry into the host. This definition should, however, also encompass virus-like particles of bacteriophages, in which the aforementioned gene or genes are still present but inactive, and, therefore, also leading to non-replicative and noninfectious virus-like particles of a bacteriophage.

The capsid structure formed from the self-assembly of 180 subunits of RNA phage coat protein and optionally containing host RNA is herein referred to as a "VLP of RNA phage coat protein". Specific examples are the VLP of Qbeta, MS2, PP7 or AP205 coat proteins. In the specific case of Qbeta coat protein, for example, the VLP may either be assembled exclusively from Qbeta CP subunits (generated by expression of a Qbeta CP gene containing, for example, a TAA stop codon precluding any expression of the longer A1 protein through suppression, see Kozlovska, T. M., et al., Intervirology 39: 9-15 (1996)), or additionally contain A1 protein subunits in the capsid assembly. Generally, the percentage of Qbeta A1 protein relative to Qbeta CP in the capsid assembly will be limited, in order to ensure capsid formation.

Examples of VLPs suitable as immunogenic carriers in the context of the present disclosure include, but are not limited to, VLPs of Qbeta, MS2, PP7, AP205 and other bacteriophage coat proteins, the capsid and core proteins of Hepatitis B virus (Ulrich, et al., Virus Res. 50: 141-182 (1998)), measles virus (Warnes, et al., Gene 160: 173-178 (1995)), Sindbis virus, rotavirus (US Patent Nos. 5,071, 651 and 5,374, 426), foot-and-mouth-disease virus (Twomey, et al., Vaccine 13: 1603-1610, (1995)), Norwalk virus (Jiang, X., et al., Science 250: 1580-1583 (1990); Matsui, S. M., et al., J Clin. Invest. 87: 1456-1461 (1991)), the retroviral GAG protein (PCT Patent Appl. No. WO96/30523), the retrotransposon Ty protein pl, the surface protein of Hepatitis B virus (WO92/11291), human papilloma virus (WO98/15631), human polyoma virus (Sasnauskas K., et al., Biol. Chem. 380 (3): 381-386 (1999); Sasnauskas K., et al., Generation of recombinant virus-like particles of different polyomaviruses in yeast. 3rd Interational Workshop"Virus-like particles as vaccines. "Berlin, September 26-29 (2001)), RNA phages, Ty, frphage, GA-phage, AP 205-phage and, in particular, Qbeta-phage, Cowpea chlorotic mottle virus, cowpea mosaic virus, human papilloma viruses (HPV), bovine papilloma viruses, porcine parvovirus, parvoviruses such as B19, porcine (PPV) and canine (CPV) parvovirues, caliciviruses (e.g. Norwalk virus,rabbit hemorrhagic disease virus [RHDV]), animal hepadnavirus core Antigen VLPs, filamentous/rod-shaped plant viruses, including but not limited to Tobacco Mosaic Virus (TMV), Potato Virus X (PVX), Papaya Mosaic Virus (PapMV), Alfalfa Mosaic Virus (AIMV), and Johnson Grass Mosaic Virus (JGMV), insect viruses *such* as flock house virus (FHV) and tetraviruses, polyomaviruses such as Murine Polyomavirus (MPyV), Murine Pneumotropic Virus (MPtV), BK virus (BKV), and JC virus (JCV).

As will be readily apparent to those skilled in the art, the VLP to be used as an immunogenic carrier is not limited to any specific form. The particle can be synthesized chemically or through a biological process, which can be natural or nonnatural. By way of example, this type of embodiment includes a virus-like particle or a recombinant form thereof. In another embodiment, the VLP can comprise, or alternatively consist of, recombinant polypeptides of any of the virus known to form a VLP. The virus-like particle can further comprise, or alternatively consist of, one or more fragments of such polypeptides, as well as variants of such polypeptides. Variants of polypeptides can share, for example, at least 80%, 85%, 90%, 95%, 97%, or 99% identity at the amino acid level with their wild-type counterparts. Variant VLPs suitable for use in the present invention can be derived from any organism so long as they are able to form a "virus-like particle" and can be used as an "immunogenic carrier" as defined herein.

Preferred VLPs include the capsid protein or surface antigen of HBV (HBcAg and HBsAg respectively) or recombinant proteins or fragments thereof, and the coat proteins of RNA-phages or recombinant proteins or fragments thereof, more preferably the coat protein of Qbeta or recombinant proteins or fragments thereof.

In one embodiment, the immunogic carrier used in combination with an antigenic PCSK9 peptide is an HBcAg protein. Examples of HBcAg proteins that can be used in the context of the present invention can be readily determined by one skilled in the art. Examples include, but are limited to, HBV core proteins described in Yuan et al., (J. Virol. 73: 10122-10128 (1999)), and in WO00/198333, WO00/177158, WO00/214478, WO00/32227, WO01/85208, WO02/056905, WO03/024480, and WO03/024481. HBcAgs suitable for use can be derived from any organism so long as they are able to form a "virus-like particle" and can be used as an "immunogenic carrier" as defined herein.

HBcAg variants of particular interest that could be used are those variants in which one or more naturally resident cysteine residues have been either deleted or substituted. It is well known in the art that free cysteine residues can be involved in a number of chemical side reactions including disulfide exchanges, reaction with chemical substances or metabolites that are, for example, injected or formed in a combination therapy with other substances, or direct oxidation and reaction with nucleotides upon exposure to UV light. Toxic adducts could thus be generated, especially considering the fact that HBcAgs have a strong tendency to bind nucleic acids. The toxic adducts would thus be distributed between a multiplicity of species, which individually may each be present at low concentration, but reach toxic levels when together. In view of the above, one advantage to the use of HBcAgs in vaccine compositions which have been modified to remove naturally resident cysteine residues is that sites to which toxic species can bind when antigens or antigenic determinants are attached would be reduced in number or eliminated altogether.

In addition, the processed form of HBcAg lacking the N-terminal leader sequence of the Hepatitis B core antigen precursor protein can also be used especially when HBcAg is produced under conditions where processing will not occur (e.g., expression in bacterial systems).

Other HBcAg variants include (i) polypeptide sequence having at least 80%, 85%, 90%, 95%, 97% or 99% identical to one of the wild-type HBcAg amino acid sequences, or a subportion thereof, using conventionally using known computer programs, (ii) C-terminal truncation mutants including mutants where 1, 5, 10, 15, 20, 25, 30, 34 or 35, amino acids have been removed from the C-terminus, (iii) N-terminal truncation mutants including mutants where 1, 2, 5, 7, 9, 10, 12, 14, 15, or 17 amino acids have been removed from the N-terminus, (iv) mutants truncated in both N-terminal and C-terminal include HBcAgs where 1, 2 ,5, 7, 9, 10, 12, 14, 15 or 17 amino acids have been removed from the N-terminus and 1, 5, 10, 15, 20, 25, 30, 34 or 35 amino acids have been removed from the C-terminus.

Still other HBcAg variant proteins are those variants modified in order to enhance immunogenic presentation of a foreign epitope wherein one or more of the four arginine repeats has been deleted, but in which the C-terminal cysteine is retained (see e.g. WO01/98333), and chimeric C-terminally truncated HBcAg such as those described in WO02/14478, WO03/102165 and WO04/053091.

In another embodiment, the immunogenic carrier used in combination with an antigenic PCSK9 peptide is an HBsAg protein. HBsAg proteins that could be used in the context of the present invention can be readily determined by one skilled in the art. Examples include, but are limited to, HBV surface proteins described in US5792463, WO02/10416, and WO08/020331. HBsAgs suitable for use can be derived from any organism so long as they are able to form a "virus-like particle" and can be used as an "immunogenic carrier" as defined herein.

In still another embodiment, the immunogic carrier used in combination with an antigenic PCSK9 peptide or polypeptide is a Qbeta coat protein.

Qbeta coat protein was found to self-assemble into capsids when expressed in E. coli (Kozlovska TM. et al., GENE 137:133-137 (1993)). The obtained capsids or virus-like particles showed an icosahedral phage-like capsid structure with a diameter of 25 nm and T=3 quasi symmetry. Further, the crystal structure of phage Qss has been solved. The capsid contains 180 copies of the coat protein, which are linked in covalent pentamers and hexamers by disulfide bridges (Golmohammadi, R. et al., Structure 4: 5435554 (1996)) leading to a remarkable stability of the capsid of Qbeta coat protein. Qbeta capsid protein also shows unusual resistance to organic solvents and denaturing agents. The high stability of the capsid of Qbeta coat protein is an advantageous feature, in particular, for its use in immunization and vaccination of mammals and humans in accordance of the present invention.

Examples of Qbeta coat proteins that can be used can be readily determined by one skilled in the art. Examples have been extensively described in WO02/056905, WO03/024480, WO03/024481 and include, but are not limited to, amino acid sequences disclosed in PIR database, accession No. VCBPQbeta referring to Qbeta CP; Accession No. AAA16663 referring to Qbeta Al protein; and variants thereof including variants proteins in which the N-terminal methionine is cleaved; C-terminal truncated forms of Qbeta A1 missing as much as 100, 150 or 180 amino acids; variant proteins which have been modified by the removal of a lysine residue by deletion or substitution or by the addition of a lysine residue by substitution or insertion (see for example Qbeta-240, Qbeta-243, Qbeta-250, Qbeta-251 and Qbeta-259 disclosed in WO03/024481), and variants exhibiting at least 80%, 85%, 90%, 95%, 97%, or 99% identity to any of the Qbeta core proteins described above. Variant Qbeta coat proteins suitable for use can be derived from any organism so long as they are able to form a "virus-like particle" and can be used as "immunogenic carriers" as defined herein.

The antigenic PCSK9 peptides may be coupled to immunogenic carriers via chemical conjugation or by expression of genetically engineered fusion partners. The coupling does not necessarily need to be direct, but can occur through linker sequences. More generally, in the case that antigenic peptides either fused, conjugated or otherwise attached to an immunogenic carrier, spacer or linker sequences are typically added at one or both ends of the antigenic peptides. Such linker sequences generally comprise sequences recognized by the proteasome, proteases of the endosomes or other vesicular compartment of the cell.

In one embodiment, the peptides are expressed as fusion proteins with the immunogenic carrier. Fusion of the peptide can be effected by insertion into the immunogenic carrier primary sequence, or by fusion to either the N-or C-terminus of the immunogenic carrier. Hereinafter, when referring to fusion proteins of a peptide to an immunogenic carrier, the fusion to either ends of the subunit sequence or internal insertion of the peptide within the carrier sequence are encompassed. Fusion, as referred to hereinafter, may be effected by insertion of the antigenic peptide into the sequence of carrier, by substitution of part of the sequence of the carrier with the antigenic peptide, or by a combination of deletion, substitution or insertions.

When the immunogenic carrier is a VLP, the chimeric antigenic peptide-VLP subunit will be in general capable of self-assembly into a VLP. VLP displaying epitopes fused to their subunits are also herein referred to as chimeric VLPs. For example, EP0421 635 B describes the use of chimaeric hepadnavirus core antigen particles to present foreign peptide sequences in a virus-like particle.

Flanking amino acid residues may be added to either end of the sequence of the antigenic peptide to be fused to either end of the sequence of the subunit of a VLP, or for internal insertion of such peptidic sequence into the sequence of the subunit of a VLP. Glycine and serine residues are particularly favored amino acids to be used in the flanking sequences added to the peptide to be fused. Glycine residues confer additional flexibility, which may diminish the potentially destabilizing effect of fusing a foreign sequence into the sequence of a VLP subunit.

In an embodiment of the invention, the immunogenic carrier is a HBcAg VLP. Fusion proteins of the antigenic peptide to either the N-terminus of a HBcAg (Neyrinck, S. et al., Nature Med. 5: 11571163 (1999)) or insertions in the so called major immunodominant region (MIR) have been described (Pumpens, P. and Grens, E., Intervirology 44:98114 (2001)), WO01/98333), and are embodiments of the invention. Naturally occurring variants of HBcAg with deletions in the MIR have also been described (Pumpens, P. and Grens, E., Intervirology 44:98-114 (2001)), and fusions to the N-or C-terminus, as well as insertions at the position of the MIR corresponding to the site of deletion as compared to a wt HBcAg are further embodiments of the disclosure. Fusions to the C-terminus have also been described (Pumpens, P. and Grens, E., Intervirology 44:98-114 (2001)). One skilled in the art will easily find guidance on how to construct fusion proteins using classical molecular biology techniques. Vectors and plasmids encoding HBcAg and HBcAg fusion proteins and useful for the expression of a HBcAg and HBcAg fusion proteins have been described (Pumpens, P. and #38; Grens, E. Intervirology 44:98-114 (2001), Neyrinck, S. et al., Nature Med. 5: 1157-1163 (1999)) and can be used in the practice of the invention. An important factor for the optimization of the efficiency of self-assembly and of the display of the epitope to be inserted in the MIR of HBcAg is the choice of the insertion site, as well as the number of amino acids to be deleted from the HBcAg sequence within the MIR (Pumpens, P. and Grens, E., Intervirology 44:98-114 (2001); EP0421635; US Patent No. 6,231,864) upon insertion, or in other words, which amino acids form HBcAg are to be substituted with the new epitope. For example, substitution of HBcAg amino acids 76-80, 79-81, 79-80, 75-85 or 80-81 with foreign epitopes has been described (Pumpens, P. and Grens, E., Intervirology 44: 98-114 (2001); EP0421635; US 6,231,864, WO00/26385). HBcAg contains a long arginine tail (Pumpens, P. and Grens, E., Intervirology 44: 98-114 (2001)) which is dispensable for capsid assembly and capable of binding nucleic acids (Pumpens, P. and Grens, E., Intervirology 44: 98-114 (2001)). HBcAg either comprising or lacking this arginine tail are both embodiments of the disclosure. In another embodiment the immunogenic carrier is a VLP of an RNA phage, preferably Qbeta. The major coat proteins of RNA phages spontaneously assemble into VLPs upon expression in bacteria, and in particular in E. coli. Fusion protein constructs wherein antigenic peptides have been fused to the C-terminus of a truncated form of the A1 protein of Qbeta, or inserted within the A1 protein have been described (Kozlovska, T. M., et al., Intervirology, 39:9-15 (1996)). The A1 protein is generated by suppression at the UGA stop codon and has a length of 329 aa, or 328 aa, if the cleavage of the N-terminal methionine is taken into account. Cleavage of the N-terminal methionine before an alanine (the second amino acid encoded by the Qbeta CP gene) usually takes place in E. coli, and such is the case for N-termini of the Qbeta coat proteins. The part of the A1 gene, 3' of the UGA amber codon encodes the CP extension, which has a length of 195 amino acids. Insertion of the antigenic peptide between position 72 and 73 of the CP extension leads to further embodiments of the invention (Kozlovska, T. M., et al., Intervirology 39:9-15 (1996)). Fusion of an antigenic peptide at the C-terminus of a C-terminally truncated Qbeta A1 protein leads to further embodiments of the disclosure. For example, Kozlovska et al., (Intervirology, 39:9-15 (1996)) describe Qbeta A1 protein fusions where the epitope is fused at the C-terminus of the Qbeta CP extension truncated at position 19.

As described by Kozlovska et al. (Intervirology, 39:9-15 (1996)), assembly of the particles displaying the fused epitopes typically requires the presence of both the Al protein-antigen fusion and the wt CP to form a mosaic particle. However, embodiments comprising virus-like particles, and hereby in particular the VLPs of the RNA phage Qbeta coat protein, which are exclusively composed of VLP subunits having an antigenic peptide fused thereto, are also within the scope of the present disclosure. The production of mosaic particles may be effected in a number of ways. Kozlovska et al., Intervirology, 39:9-15 (1996), describe three methods, which all can be used in the practice of the disclosure. In the first approach, efficient display of the fused epitope on the VLPs is mediated by the expression of the plasmid encoding the Qbeta A1l protein fusion having a UGA stop codon between CP and CP extension in a E. coli strain harboring a plasmid encoding a cloned UGA suppressor tRNA which leads to translation of the UGA codon into Trp (pISM3001 plasmid (Smiley B. K., et al., Gene 134:33-40 (1993))). In another approach, the CP gene stop codon is modified into UAA, and a second plasmid expressing the A1 protein-antigen fusion is cotransformed. The second plasmid encodes a different antibiotic resistance and the origin of replication is compatible with the first plasmid. In a third approach, CP and the A1 protein-antigen fusion are encoded in a bicistronic manner, operatively linked to a promoter such as the Trp promoter, as described in Figure 1 of Kozlovska et al., Intervirology, 39:9-15 (1996).

Further VLPs suitable for fusion of antigens or antigenic determinants are described in WO03/024481 and include bacteriophage fr, RNA phase MS-2, capsid proteine of papillomavirus, retrotransposon Ty, yeast and also Retrovirus-like-particles, HIV2 Gag, Cowpea Mosaic Virus, parvovirus VP2 VLP, HBsAg (US Patent 4,722,840; EP0020416B1). Examples of chimeric VLPs suitable for the practice of the disclosure are also those described in Intervirology 39:1(1996). Further examples of VLPs contemplated for use are: HPV-1, HPV-6, HPV-11, HPV-16, HPV-18, HPV-33, HPV-45, CRPV, COPV, HIV GAG, Tobacco Mosaic Virus. Virus-like particles of SV-40, Polyomavirus, Adenovirus, Herpes Simplex Virus, Rotavirus and Norwalk virus.

For any recombinantly expressed antigenic PCSK9 peptide according to the disclosure coupled or not to an immunogenic carrier, the nucleic acid which encodes said peptide or protein also forms an aspect of the present disclosure, as does an expression vector comprising the nucleic acid, and a host cell containing the expression vector (autonomously or chromosomally inserted). A method of recombinantly producing the peptide or protein by expressing it in the above host cell and isolating the immunogen therefrom is a further aspect of the disclosure. The full-length native PCSK9 molecule or the full-length native DNA sequence encoding it are not covered by the present invention.

In another embodiment, the peptide of the invention is chemically coupled to an immunogenic carrier, using techniques well known in the art. Conjugation can occur to allow free movement of peptides via single point conjugation (e.g. either N-terminal or C-terminal point) or as locked down structure where both ends of peptides are conjugated to either a immunogenic carrier protein or to a scaffold structure such as a VLP. Conjugation occurs via conjugation chemistry known to those skilled in the art such as via cysteine residues, lysine residues or other carboxy moiety's commonly known as conjugation points such as glutamic acid or aspartic acid. Thus, for example, for direct covalent coupling it is possible to utilise a carbodiimide, glutaraldehyde or (N-[y- malcimidobutyryloxy] succinimide ester, utilising common commercially available heterobifunctional linkers such as CDAP and SPDP (using manufacturers instructions). Examples of conjugation of peptides, particularly cyclised peptides, to a protein carrier via acylhydrazine peptide derivatives are described in WO03/092714. After the coupling reaction, the immunogen can easily be isolated and purified by means of a dialysis method, a gel filtration method, a fractionation method etc. Peptides terminating with a cysteine residue (preferably with a linker outside the cyclised region) may be conveniently conjugated to a carrier protein via maleimide chemistry.

When the immunogenic carrier is a VLP, several antigenic peptide, either having an identical amino acid sequence or a different amino acid sequence, may be coupled to a single VLP molecule, leading preferably to a repetitive and ordered structure presenting several antigenic determinants in an oriented manner as described in WO00/32227, WO03/024481, WO02/056905 and WO04/007538.

In an embodiment, the antigenic PCSK9 peptide is bound to the VLP by way of chemical cross-linking, typically and preferably by using a heterobifunctional cross-linker. Several hetero-bifunctional cross-linkers are known to the art. In some embodiments, the hetero-bifunctional crosslinker contains a functional group which can react with first attachment sites, i. e. with the side-chain amino group of lysine residues of the VLP or VLP subunit, and a further functional group which can react with a preferred second attachment site, i. e. a cysteine residue fused to the antigenic peptide and optionally also made available for reaction by reduction. The first step of the procedure, typically called the derivatization, is the reaction of the VLP with the cross-linker. The product of this reaction is an activated VLP, also called activated carrier. In the second step, unreacted cross-linker is removed using usual methods such as gel filtration or dialysis. In the third step, the antigenic peptide is reacted with the activated VLP, and this step is typically called the coupling step. Unreacted antigenic peptide may be optionally removed in a fourth step, for example by dialysis. Several hetero-bifunctional crosslinkers are known to the art. These include the preferred cross-linkers SMPH (Pierce), Sulfo-MBS, Sulfo-EMCS, Sulfo-GMBS, Sulfo-SIAB, Sulfo-SMPB, Sulfo-SMCC, SVSB, SIA and other cross-linkers available for example from the Pierce Chemical Company (Rockford, IL, USA), and having one functional group reactive towards amino groups and one functional group reactive towards cysteine residues. The above mentioned cross-linkers all lead to formation of a thioether linkage.

Another class of cross-linkers suitable in the practice of the invention is characterized by the introduction of a disulfide linkage between the antigenic peptide and the VLP upon coupling. Preferred cross-linkers belonging to this class include for example SPDP and Sulfo-LC-SPDP (Pierce). The extent of derivatization of the VLP with cross-linker can be influenced by varying experimental conditions such as the concentration of each of the reaction partners, the excess of one reagent over the other, the pH, the temperature and the ionic strength. The degree of coupling, i. e. the amount of antigenic peptide per subunits of the VLP can be adjusted by varying the experimental conditions described above to match the requirements of the vaccine.

Another method of binding of antigenic peptides to the VLP, is the linking of a lysine residue on the surface of the VLP with a cysteine residue on the antigenic peptide. In some embodiments, fusion of an amino acid linker containing a cysteine residue, as a second attachment site or as a part thereof, to the antigenic peptide for coupling to the VLP may be required. In general, flexible amino acid linkers are favored. Examples of the amino acid linker are selected from the group consisting of: (a) CGG; (b) N-terminal gamma 1-linker; (c) N-terminal gamma 3-linker; (d) Ig hinge regions; (e) N-terminal glycine linkers; (f) (G) kC (G) n with n=0-12 and k=0-5; (g) N-terminal glycine-serine linkers; (h) (G) kC (G) m (S) i (GGGGS) n with n=0-3, k=0-5, m=0-10, i=0-2; (i) GGC; (k) GGC-NH2; (1) C-terminal gamma 1-linker; (m) C-terminal gamma 3-linker; (n) C-terminal glycine linkers; (o) (G) nC (G) k with n=0-12 and k=0-5; (p) C-terminal glycine-serine linkers; (q) (G) m (S) t (GGGGS) n (G) oC (G) k with n=0-3, k=0-5, m=0-10, 1=0-2, and o=0-8. Further examples of amino acid linkers are the hinge region of immunoglobulins, glycine serine linkers (GGGGS) n, and glycine linkers (G) n all further containing a cysteine residue as second attachment site and optionally further glycine residues. Typically preferred examples of said amino acid linkers are N-terminal gamma 1: CGDKTHTSPP; C-terminal gamma 1: DKTHTSPPCG; N-terminal gamma 3: CGGPKPSTPPGSSGGAP; C-terminal gamma 3: PKPSTPPGSSGGAPGGCG; N-terminal glycine linker: GCGGGG and C-terminal glycine linker: GGGGCG.

Other amino acid linkers particularly suitable in the practice of the invention, when a hydrophobic antigenic peptide is bound to a VLP, are CGKKGG, or CGDEGG for N-terminal linkers, or GGKKGC and GGEDGC, for the C-terminal linkers. For the C-terminal linkers, the terminal cysteine is optionally C-terminally amidated.

In some embodiments of the present invention, GGCG, GGC or GGC-NH2 ("NH2"stands for amidation) linkers at the C-terminus of the peptide or CGG at its N-terminus are preferred as amino acid linkers. In general, glycine residues will be inserted between bulky amino acids and the cysteine to be used as second attachment site, to avoid potential steric hindrance of the bulkier amino acid in the coupling reaction. In a further embodiment of the invention, the amino acid linker GGC-NH2 is fused to the C-terminus of the antigenic peptide.

The cysteine residue present on the antigenic peptide has to be in its reduced state to react with the hetero-bifunctional cross-linker on the activated VLP, that is a free cysteine or a cysteine residue with a free sulfhydryl group has to be available. In the instance where the cysteine residue to function as binding site is in an oxidized form, for example if it is forming a disulfide bridge, reduction of this disulfide bridge with, e. g., DTT, TCEP or p- mercaptoethanol is required. Low concentrations of reducing agent are compatible with coupling as described in WO02/05690, higher concentrations inhibit the coupling reaction, as a skilled artisan would know, in which case the reductand has to be removed or its concentration decreased prior to coupling, e. g., by dialysis, gel filtration or reverse phase HPLC.

Binding of the antigenic peptide to the VLP by using a hetero-bifunctional cross-linker according to the methods described above, allows coupling of the antigenic peptide to the VLP in an oriented fashion. Other methods of binding the antigenic peptide to the VLP include methods wherein the antigenic peptide is cross-linked to the VLP using the carbodiimide EDC, and NHS.

In other methods, the antigenic peptide is attached to the VLP using a homo-bifunctional cross-linker such as glutaraldehyde, DSGBM [PEO] 4, BS3, (Pierce Chemical Company, Rockford, IL, USA) or other known homo-bifunctional cross-linkers with functional groups reactive towards amine groups or carboxyl groups of the VLP.

Other methods of binding the VLP to an antigenic peptide include methods where the VLP is biotinylated, and the antigenic peptide expressed as a streptavidin-fusion protein, or methods wherein both the antigenic peptide and the VLP are biotinylated, for example as described in WO00/23955. In this case, the antigenic peptide may be first bound to streptavidin or avidin by adjusting the ratio of antigenic peptide to streptavidin such that free binding sites are still available for binding of the VLP, which is added in the next step. Alternatively, all components may be mixed in a "one pot" reaction. Other ligand-receptor pairs, where a soluble form of the receptor and of the ligand is available, and are capable of being cross-linked to the VLP or the antigenic peptide, may be used as binding agents for binding antigenic peptide to the VLP. Alternatively, either the ligand or the receptor may be fused to the antigenic peptide, and so mediate binding to the VLP chemically bound or fused either to the receptor, or the ligand respectively. Fusion may also be effected by insertion or substitution.

One or several antigen molecules can be attached to one subunit of the capsid or VLP of RNA phages coat proteins, preferably through the exposed lysine residues of the VLP of RNA phages, if sterically allowable. A specific feature of the VLP of the coat protein of RNA phages and in particular of the QP coat protein VLP is thus the possibility to couple several antigens per subunit. This allows for the generation of a dense antigen array.

VLPs or capsids of Q coat protein display a defined number of lysine residues on their surface, with a defined topology with three lysine residues pointing towards the interior of the capsid and interacting with the RNA, and four other lysine residues exposed to the exterior of the capsid. These defined properties favor the attachment of antigens to the exterior of the particle, rather than to the interior of the particle where the lysine residues interact with RNA. VLPs of other RNA phage coat proteins also have a defined number of lysine residues on their surface and a defined topology of these lysine residues.

In a further embodiment of the present disclosure the first attachment site is a lysine residue and/or the second attachment comprises sulfhydryl group or a cysteine residue. In an even further embodiment of the present disclosure the first attachment site is a lysine residue and the second attachment is a cysteine residue. In further embodiments of the disclosure the antigen or antigenic determinant is bound via a cysteine residue, to lysine residues of the VLP of RNA phage coat protein, and in particular to the VLP of Qbeta coat protein.

Another advantage of the VLPs derived from RNA phages is their high expression yield in bacteria that allows production of large quantities of material at affordable cost. Moreover, the use of the VLPs as carriers allow the formation of robust antigen arrays and conjugates, respectively, with variable antigen density. In particular, the use of VLPs of RNA phages, and hereby in particular the use of the VLP of RNA phage Qbeta coat protein allows a very high epitope density to be achieved.

According to an embodiment of the present disclosure the antigenic PCSK9 peptide disclosed herein are linked, preferably chemically cross linked, to CRM197, either directly or *via* one of the peptide linker disclosed herein, to generate an immunogen. In an embodiment, the antigenic PCSK9 peptide disclosed herein is linked to CRM197, by way of chemical cross-linking as described herein and preferably by using a heterobifunctional cross-linker, as disclosed above.

Preferred heterobifunctional cross-linkers for use with CRM197 are BAANS (bromoacetic acid N-hydroxysuccinimide ester), SMPH (Succinimidyl-6-[β-maleimidopropionamido]hexanoate), Sulfo-MBS, Sulfo-EMCS, Sulfo-GMBS, Sulfo-SIAB, Sulfo-SMPB, Sulfo-SMCC, SVSB, SIA, SMPEG(*n*) and other cross-linkers available for example from the Pierce Chemical Company (Rockford, IL, USA). In an embodiment of the present invention, the hetero-bifunctional crosslinker is BAANS or SMPH.

Alternatively, cross-linkers suitable allowing the introduction of a disulfide linkage between the antigenic peptide and CRM197 could also be used in the context of the invention. Preferred cross-linkers belonging to this class include for example SPDP and Sulfo-LC-SPDP (Pierce).

In a particular embodiment, when the sequence of the antigenic PCSK9 peptide disclosed herein comprises a cysteine, said antigenic PCSK9 peptide may be covalently linked to CRM197 directly via said cysteine.

In some embodiments immunogenic compositions of the invention may comprise mixtures of immunogenic conjugates, i.e. immunogenic carriers coupled to one or several antigenic PCSK9 peptides. Thus, these immunogenic compositions may be composed of immunogenic carriers which differ in amino acid sequence. For example, vaccine compositions could be prepared comprising a "wild-type" VLP and a modified VLP protein in which one or more amino acid residues have been altered (e. g., deleted, inserted or substituted). Alternatively, the same immunogenic carrier might be used but coupled to antigenic PCSK9 peptides of different amino acid sequences.

The disclosure therefore also relates to method for producing an immunogen according to the disclosure comprising (i) providing an antigenic PCSK9 peptide according to the disclosure, (ii) providing an immunogenic carrier according to the disclosure, preferably a VLP, and (iii) combining said antigenic PCSK9 peptide and said immunogenic carrier. In one embodiment, said combining step occurs through chemical cross-linking, preferably through an heterobifunctional cross-linker.

In an embodiment of the present disclosure, the antigenic PCSK9 peptide disclosed herein is linked to an immunogenic carrier molecule. In an embodiment, said immunogenic carrier is selected from the group consisting of any of the immunogenic carrier described herein. In another embodiment said immunogenic carrier is selected from the group consisting of: serum albumins such as bovine serum albumin (BSA); globulins; thyroglobulins; hemoglobins; hemocyanins (particularly Keyhole Limpet Hemocyanin [KLH]) and virus-like particle (VLPs). In an embodiment, said immunogenic carrier is Diphtheria Toxoid, CRM197 mutant of diphtheria toxin, Tetanus Toxoid, Keyhole Limpet Hemocyanin or virus-like particle (VLPs). In another embodiment, said immunogenic carrier is DT, CRM197 or a VLP selected from the group consisting of HBcAg VLP, HBsAg VLP, Qbeta VLP, PP7 VLP, PPV VLP, Norwalk Virus VLP or any variant disclosed herein. In an even further embodiment, said immunogenic carrier is a bacteriophage VLP such as Qbeta VLP selected from the group consisting of Qbeta CP; Qbeta A1, Qbeta-240, Qbeta-243, Qbeta-250, Qbeta-251 and Qbeta-259 (disclosed in WO03/024481) or PP7.

In another embodiment, said immunogenic carrier is CRM197.

In an embodiment, said immunogenic carrier is covalently linked to the antigenic PCSK9 peptide disclosed herein either directly or via a linker. In an embodiment, said immunogenic carrier is linked to the antigenic PCSK9 peptide disclosed herein by expression of a fusion protein as described herein. In another embodiment, the antigenic PCSK9 peptide disclosed herein is linked to the immunogenic carrier, preferably a VLP, by way of chemical cross-linking as described herein and preferably by using a heterobifunctional cross-linker. Several hetero-bifunctional cross-linkers are known to the art. In some embodiments, the hetero-bifunctional crosslinker contains a functional group which can react with first attachment sites, i.e., with the side-chain amino group of lysine residues of the VLP or VLP subunit, and a further functional group which can react with a preferred second attachment site, i.e. a cysteine residue fused to the antigenic peptide made available for reaction by reduction.

### Antigenic PCSK9 Peptide Comprising a Linker

In an embodiment of the present disclosure the antigenic PCSK9 peptide further comprises either at its N-terminus, or at its C-terminus or at both the N-terminus and C-terminus a linker which is able to react with an attachment site of the immunogenic carrier in a chemical cross-linking reaction. In an embodiment, the antigenic PCSK9 peptide further comprises at its C-terminus a linker having the formula (G)ₙC, (G)ₙSC or (G)ₙK, preferably (G)ₙC, wherein n is an integer chosen from the group consisting of 0, 1, 2, 3, 4, 5, 6, 7, 8, 9 and 10, preferably in the group consisting of 0, 1, 2, 3, 4 and 5, more preferably in the groups consisting of 0, 1, 2 and 3, most preferably n is 0 or 1 (where n is equal to 0 said formula represents a cysteine). Preferably the antigenic PCSK9 peptide further comprises at its C-terminus a linker having the formula GGGC, GGC, GC or C.

In another embodiment the antigenic PCSK9 peptide further comprises at its N-terminus a linker having the formula C(G)ₙ, CS(G)ₙ or K(G)ₙ, preferably C(G)ₙ wherein n is an integer chosen from the group consisting of 0, 1, 2, 3, 4, 5, 6, 7, 8, 9 and 10, preferably in the group consisting of 0, 1, 2, 3, 4 and 5, more preferably in the groups consisting of 0, 1, 2 and 3, most preferably n is 0 or 1 (where n is equal to 0, the formula represents a cysteine). Preferably the antigenic PCSK9 peptide further comprise at its N-terminus a linker having the formula CGGG, CGG, CG or C.

In another embodiment, the antigenic PCSK9 peptide further comprises at its C-terminus a linker having the formula (G)ₙC, (G)ₙSC or (G)ₙK, preferably (G)ₙC wherein n is an integer chosen from the group consisting of 0, 1, 2, 3, 4, 5, 6, 7, 8, 9 and 10, preferably in the group consisting of 0, 1, 2, 3, 4 and 5, more preferably in the groups consisting of 0, 1, 2 and 3, most preferably n 0 or 1 (where n is equal to 0 said formula represents a cysteine) and at its N-terminus a linker having the formula C(G)ₙ, CS(G)ₙ or K(G)ₙ, preferably C(G)ₙ wherein n is an integer chosen from the group consisting of 0, 1, 2, 3, 4, 5, 6, 7, 8, 9 and 10, preferably in the group consisting of 0, 1, 2, 3, 4 and 5, more preferably in the groups consisting of 0, 1, 2 and 3, most preferably n is 0 or 1 (where n is equal to 0, the formula represents a cysteine). Preferably, the antigenic PCSK9 peptide further comprises at its N-terminus a linker having the formula CGGG, CGG, CG or C and at its C-terminus a linker having the formula GGGC, GGC, GC or C. Preferably, the antigenic PCSK9 peptide further comprises at its N-terminus a cysteine and at its C-terminus a cysteine.

Representative examples of said antigenic PCSK9 peptides further comprising such a linker are disclosed at SEQ ID NOs. 55 to 148, 199 to 286, 321 to 378, 400 to 433.

In one embodiment, the antigenic PCSK9 peptide is cyclised. In one embodiment, the cyclised antigenic PCSK9 peptide is attached to an immunogenic carrier. In one embodiment, said cyclised antigenic PCSK9 peptide is attached to an immunogenic carrier by covalent binding. In one embodiment, said cyclised antigenic PCSK9 peptide is attached to an immunogenic carrier by covalent binding of one of the side chain of its amino acids to the carrier. In one embodiment, a cysteine, a GC or a CC fragment comprising a variable number of glycine residues and one cysteine residue is added to the cyclised PCSK9 peptides to enable the covalent binding to the immunogenic carrier through the added cysteine.

In one embodiment, the antigenic PCSK9 peptide is cyclised and comprises a cysteine, a (G)ₙC or a C(G)ₙ fragment, wherein n is an integer chosen from the group consisting of 0, 1, 2, 3, 4, 5, 6, 7, 8, 9 and 10, preferably from the group consisting of 0, 1, 2, 3, 4 and 5, more preferably from the groups consisting of 0, 1, 2 and 3, most preferably n is 0 or 1 (where n is equal to 0, the formula represents a cysteine).

Examples of conjugations of antigenic PCSK9 peptides with carrier or scaffolds described above, all within the scope of the present disclosure and constituting various embodiments, using various linkers are provided below:
Peptide - GGGGGC - scaffold, peptide - GGGGC - scaffold, peptide - GGGC - scaffold, peptide - GGC - scaffold, peptide - GC - scaffold, peptide - C - scaffold, peptide - GGGGGK - scaffold, peptide - GGGGK - scaffold, Peptide - GGGK - scaffold, Peptide - GGK - scaffold, Peptide - GK - scaffold, Peptide - K- scaffold, Peptide - GGGGSC - scaffold, Peptide - GGGSC - scaffold, Peptide - GGSC - scaffold, Peptide - GSC - scaffold, Peptide - SC - scaffold, Scaffold - CSGGGG - Peptide, Scaffold - CSGGG- Peptide, Scaffold - CSGG- Peptide, Scaffold - CSG-Peptide, Scaffold - CS - Peptide, Scaffold - KGGGG - Peptide, Scaffold - KGGG-Peptide, Scaffold - KGG- Peptide, Scaffold - KG- Peptide, Scaffold - K - Peptide.

In an embodiment, the peptide consists of any of the antigenic PCSK9 peptide disclosed herein and the scaffold consists of any of the immunogenic carrier disclosed herein, preferably a VLP.

Exemplary combinations of conjugations using various linkers and doubly constrained peptides are provided below, where the carrier can be the identical monomer of a carrier or a differential monomer of a carrier. (In the example below, the GC linker can be substituted by any of the GK linker or GSC linker exemplified above or any other known to those skilled in the art):
Carrier - CGGGGG - Peptide - GGGGGC - carrier, Carrier - CGGGG - Peptide - GGGGC - carrier, Carrier - CGGGG - Peptide - GGGGC - carrier, Carrier - CGGG - Peptide - GGGC - carrier, Carrier - CG - Peptide - GC - carrier, Carrier - CG - Peptide - C - carrier, Carrier - C - Peptide - C - carrier.

In an embodiment, the peptide consists of any of the antigenic PCSK9 peptide disclosed herein and the carrier consists of any of the immunogenic carrier disclosed herein, preferably a VLP.

In an embodiment, the disclosure relates to an immunogen comprising an antigenic PCSK9 peptide consisting of, or consisting essentially of, an amino acid sequence selected from the group consisting of SEQ ID NOs.: 1 to 54, 149 to 198, 287 to 320, 379 to 399, 434 to 468, 527 to 547, wherein said antigenic PCSK9 peptide further comprises at its C-terminus or at its N-terminus a cysteine which is chemically cross linked to an immunogenic carrier via a thioether linkage. In another embodiment, said immunogenic carrier is selected from the group consisting of DT (Diphtheria toxin), TT (tetanus toxid) or fragment C of TT, PD (Haemophilus influenzae protein D), CRM197, other DT point mutants, such as CRM176, CRM228, CRM 45, CRM 9, CRM102, CRM 103 and CRM107. Preferably said immunogenic carrier is CRM197.

In an embodiment, the disclosure relates to an immunogen comprising an antigenic PCSK9 peptide consisting of, or consisting essentially of, an amino acid sequence selected from the group consisting of SEQ ID NOs.: 1 to 54, 149 to 198, 287 to 320, 379 to 399, 434 to 468, and 527 to 547, wherein said antigenic PCSK9 peptide further comprises at its C-terminus or at its N-terminus a cysteine which is chemically cross linked to an immunogenic carrier *via* a thioether linkage using SMPH (Succinimidyl-6-[β-maleimidopropionamido]hexanoate) or BAANS (bromoacetic acid N-hydroxysuccinimide ester) as cross linker. In an embodiment, said immunogenic carrier is selected from the group consisting of DT (Diphtheria toxin), TT (tetanus toxid) or fragment C of TT, PD (Haemophilus influenzae protein D, CRM197, other DT point mutants, such as CRM176, CRM228, CRM 45, CRM 9, CRM102, CRM 103 and CRM107. Preferably said immunogenic carrier is CRM197.

In an embodiment, the disclosure relates to an immunogen comprising an antigenic PCSK9 peptide consisting of, or consisting essentially of, an amino acid sequence selected from the group consisting of SEQ ID NOs.: 1 to 54, 149 to 198, 287 to 320, 379 to 399, 434 to 468, 527 to 547, wherein said antigenic PCSK9 peptide further comprises at its C-terminus a cysteine which is chemically cross linked to an immunogenic carrier *via* a thioether linkage using SMPH (Succinimidyl-6-[β-maleimidopropionamido]hexanoate) or BAANS (bromoacetic acid N-hydroxysuccinimide ester) as cross linker, said linkage being between a lysine residue of CRM197 and the cysteine residue of said antigenic peptide.

### Compositions Comprising an Antigenic PCSK9 Peptide

The present disclosure further relates to compositions, particularly immunogenic compositions also referred to as "subject immunogenic compositions", comprising an antigenic PCSK9 peptide linked to an immunogenic carrier, and optionally at least one adjuvant. Such immunogenic compositions, particularly when formulated as pharmaceutical compositions, are deemed useful to prevent, treat or alleviate PCSK9-related disorders.

In some embodiments, a subject immunogenic composition according to the invention comprises an antigenic PCSK9 peptide, optionally comprising a linker, comprising an amino acid sequence corresponding to SEQ ID NO 12, said antigenic PCSK9 peptide is linked to an immunogenic carrier, wherein said immunogenic carrier is selected from a DT, CRM197 or a VLP selected from HBcAg, HBsAg, Qbeta, PP7, PPV or Norwalk Virus VLP.

In another embodiment, a subject immunogenic composition comprises an antigenic PCSK9 peptide, optionally comprising a linker, comprising an amino acid sequence selected from SEQ ID NOs. 5 and 12, linked to a Qbeta VLP.

In a further embodiment, a subject immunogenic composition comprises an antigenic PCSK9 peptide optionally comprising a linker, comprising an amino acid sequence selected from SEQ ID NOs. 5 and 12 linked to CRM197.

A subject immunogenic composition comprising an antigenic PCSK9 peptide can be formulated in a number of ways, as described in more detail below.

In some embodiments, a subject immunogenic composition comprises single species of antigenic PCSK9 peptide, e.g., the immunogenic composition comprises a population of antigenic PCSK9 peptides, substantially all of which have the same amino acid sequence. In other embodiments, a subject immunogenic composition comprises two or more different antigenic PCSK9 peptides, e.g., the immunogenic composition comprises a population of antigenic PCSK9 peptides, the members of which population can differ in amino acid sequence. A subject immunogenic composition can comprise from two to about 20 different antigenic PCSK9 peptides, e.g., a subject immunogenic composition can comprise 2, 3, 4, 5, 6, 7, 8, 9, 10, 11-15, or 15-20 different antigenic PCSK9 peptides, each having an amino acid sequence that differs from the amino acid sequences of the other antigenic PCSK9 peptides.

In other embodiments, a subject immunogenic composition comprises a multimerized antigenic PCSK9 polypeptide, as described above. As used herein, the terms "immunogenic composition comprising an antigenic PCSK9 peptide" or "subject immunogenic composition" refers to an immunogenic composition comprising either single species (multimerized or not) or multiple species of antigenic PCSK9 peptide(s) coupled or not to an immunogenic carrier. Where two or more peptides are used coupled to a carrier, the peptide may be coupled to the same carrier molecule or individually coupled to carrier molecules and then combined to produce an immunogenic composition.

Another aspect of the disclosure relates to methods for producing an immunogen according to the disclosure, said method comprising coupling an antigenic PCSK9 peptide to an immunogenic carrier. In one embodiment, said coupling is chemical.

### Adjuvants

In some embodiments, a subject immunogenic composition comprises at least one adjuvant. Suitable adjuvants include those suitable for use in mammals, preferably in humans. Examples of known suitable adjuvants that can be used in humans include, but are not necessarily limited to, alum, aluminum phosphate, aluminum hydroxide, MF59 (4.3% w/v squalene, 0.5% w/v polysorbate 80 (Tween 80), 0.5% w/v sorbitan trioleate (Span 85)), CpG-containing nucleic acid (where the cytosine is unmethylated), QS21 (saponin adjuvant), MPL (Monophosphoryl Lipid A), 3DMPL (3-O-deacylated MPL), extracts from Aquilla, ISCOMS (see, e.g., Sjölander et al. (1998) J. Leukocyte Biol. 64:713; WO90/03184, WO96/11711, WO 00/48630, WO98/36772, WO00/41720, WO06/134423 and WO07/026190), LT/CT mutants, poly(D,L-lactide-co-glycolide) (PLG) microparticles, Quil A, TiterMax classic, TiterMax Gold, interleukins, and the like. For veterinary applications including but not limited to animal experimentation, one can use Freund's adjuvant, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion.

Further exemplary adjuvants to enhance effectiveness of the composition include, but are not limited to: (1) oil-in-water emulsion formulations (with or without other specific immunostimulating agents such as muramyl peptides (see below) or bacterial cell wall components), such as for example (a) MF59™ (WO90/14837; Chapter 10 in Vaccine design: the subunit and adjuvant approach, eds. Powell & Newman, Plenum Press 1995), containing 5% Squalene, 0.5% Tween 80 (polyoxyethylene sorbitan mono-oleate), and 0.5% Span 85 (sorbitan trioleate) (optionally containing muramyl tri-peptide covalently linked to dipalmitoyl phosphatidylethanolamine (MTP-PE)) formulated into submicron particles using a microfluidizer, (b) SAF, containing 10% Squalane, 0.4% Tween 80, 5% pluronic-blocked polymer L121, and thr-MDP either microfluidized into a submicron emulsion or vortexed to generate a larger particle size emulsion, and (c) RIBI™ adjuvant system (RAS), (Ribi Immunochem, Hamilton, MT) containing 2% Squalene, 0.2% Tween 80, and one or more bacterial cell wall components such as monophosphorylipid A (MPL), trehalose dimycolate (TDM), and cell wall skeleton (CWS), preferably MPL + CWS (DETOX™); (2) saponin adjuvants, such as QS21, STIMULON™ (Cambridge Bioscience, Worcester, MA), Abisco® (Isconova, Sweden), or Iscomatrix® (Commonwealth Serum Laboratories, Australia), may be used or particles generated therefrom such as ISCOMs (immunostimulating complexes), which ISCOMS may be devoid of additional detergent e.g. WO00/07621; (3) Complete Freund's Adjuvant (CFA) and Incomplete Freund's Adjuvant (IFA); (4) cytokines, such as interleukins *(e.g.* IL-1, IL-2, IL-4, IL-5, IL-6, IL-7, IL-12 (WO99/44636), *etc.),* interferons *(e.g.* gamma interferon), macrophage colony stimulating factor (M-CSF), tumor necrosis factor (TNF), *etc.;* (5) monophosphoryl lipid A (MPL) or 3-O-deacylated MPL (3dMPL) *e.g.* GB-2220221, EP-A-0689454, optionally in the substantial absence of alum when used with pneumococcal saccharides *e.g.* WO00/56358; (6) combinations of 3dMPL with, for example, QS21 and/or oil-in-water emulsions *e.g.* EP-A-0835318, EP-A-0735898, EP-A-0761231; (7) oligonucleotides comprising CpG motifs [Krieg Vaccine 2000, 19, 618-622; Krieg Curr opin Mol Ther2001 3:15-24; Roman et al., Nat. Med., 1997, 3, 849-854; Weiner et αl., PNAS USA, 1997, 94, 10833-10837; Davis et al, J. Immunol, 1998, 160, 870-876; Chu et αι., J. Exp.Med, 1997, 186, 1623-1631; Lipford et al, Ear. J. Immunol., 1997, 27, 2340-2344; Moldoveami e/ al., Vaccine, 1988, 16, 1216-1224, Krieg etal., Nature, 1995, 374, 546-549; Klinman et al., PNAS USA, 1996, 93, 2879-2883; Ballas et al, J. Immunol, 1996, 157, 1840-1845; Cowdery et al, J. Immunol, 1996, 156, 4570-4575; Halpern et al, Cell Immunol, 1996, 167, 72-78; Yamamoto et al, Jpn. J. Cancer Res., 1988, 79, 866-873; Stacey et al, J. Immunol., 1996, 157,2116-2122; Messina et al, J. Immunol, 1991, 147, 1759-1764; Yi et al, J. Immunol, 1996, 157,4918-4925; Yi et al, J. Immunol, 1996, 157, 5394-5402; Yi et al, J. Immunol, 1998, 160, 4755-4761; and Yi et al, J. Immunol, 1998, 160, 5898-5906; International patent applications WO96/02555, WO98/16247, WO98/18810, WO98/40100, WO98/55495, WO98/37919 and WO98/52581] *i.e.* containing at least one CG dinucleotide, where the cytosine is unmethylated; (8) a polyoxyethylene ether or a polyoxyethylene ester *e.g.* WO99/52549; (9) a polyoxyethylene sorbitan ester surfactant in combination with an octoxynol (WO01/21207) or a polyoxyethylene alkyl ether or ester surfactant in combination with at least one additional non-ionic surfactant such as an octoxynol (WO01/21152); (10) a saponin and an immunostimulatory oligonucleotide *(e.g.* a CpG oligonucleotide) (WO00/62800); (11) an immunostimulant and a particle of metal salt *e.g.* WO00/23105; (12) a saponin and an oil-in-water emulsion *e.g.* WO99/11241; (13) a saponin *(e.g.* QS21) + 3dMPL + IM2 (optionally + a sterol) *e.g.* WO98/57659; (14) other substances that act as immunostimulating agents to enhance the efficacy of the composition, such as Muramyl peptides include N-acetyl-muramyl-L-threonyl-D-isoglutamine (thr-MDP), N-25 acetyl-normuramyl-L-alanyl-D-isoglutamine (nor-MDP), N-acetylmuramyl-L-alanyl-D-isoglutarninyl-L-alanine-2-(1'-2'-dipalmitoyl-*sn*-glycero-3-hydroxyphosphoryloxy)-ethylamine MTP-PE), (15) ligands for toll-like receptors (TLR), natural or synthesized (e.g. as described in Kanzler et al 2007, Nature Medicine 13, p1552-9), including TLR3 ligands such as polyl:C and similar compounds such as Hiltonol and Ampligen.

In a particular embodiment, said adjuvant is an immunostimulatory oligonucleotide and more preferably a CpG oligonucleotide. A CpG oligonucleotide as used herein refers to an immunostimulatory CpG oligodeoxynucleotide (CpG ODN), and accordingly these terms are used interchangeably unless otherwise indicated. Immunostimulatory CpG oligodeoxynucleotides contain one or more immunostimulatory CpG motifs that are unmethylated cytosine-guanine dinucleotides, optionally within certain preferred base contexts. The methylation status of the CpG immunostimulatory motif generally refers to the cytosine residue in the dinucleotide. An immunostimulatory oligonucleotide containing at least one unmethylated CpG dinucleotide is an oligonucleotide which contains a 5' unmethylated cytosine linked by a phosphate bond to a 3' guanine, and which activates the immune system through binding to Toll-like receptor 9 (TLR-9). In another embodiment the immunostimulatory oligonucleotide may contain one or more methylated CpG dinucleotides, which will activate the immune system through TLR9 but not as strongly as if the CpG motif(s) was/were unmethylated. CpG immunostimulatory oligonucleotides may comprise one or more palindromes that in turn may encompass the CpG dinucleotide. CpG oligonucleotides have been described in a number of issued patents, published patent applications, and other publications, including US Patent Nos. 6,194,388; 6,207,646; 6,214,806; 6,218,371; 6,239,116; and 6,339,068.

Different classes of CpG immunostimulatory oligonucleotides have been identified. These are referred to as A, B, C and P class, and are described in greater detail below. Methods of the disclosure embrace the use of these different classes of CpG immunostimulatory oligonucleotides.

Any of the classes may be subjugated to an E modification which enhances its potency. An E modification may be a halogen substitution for the 5' terminal nucleotide; examples of such substitutions include but are not limited to bromo-uridine or iodo-uridine substitutions. An E modification can also include an ethyl-uridine substituation for the 5' terminal nucleotide.

The "A class" CpG immunostimulatory oligonucleotides are characterized functionally by the ability to induce high levels of interferon-alpha (IFN-α) from plasmacytoid dendritic cells (pDC) and inducing NK cell activation while having minimal effects on B cell activation. Structurally, this class typically has stabilized poly-G sequences at 5' and 3' ends. It also has a palindromic phosphodiester CpG dinucleotide-containing sequence of at least 6 nucleotides, for example but not necessarily, it contains one of the following hexamer palindromes: GACGTC, AGCGCT, or AACGTT described by Yamamoto and colleagues. Yamamoto S et al. J. Immunol 148:4072-6 (1992). A class CpG immunostimulatory oligonucleotides and exemplary sequences of this class have been described in U.S. Non-Provisional Patent Application Serial No. 09/672,126 and published PCT application PCT/USOO/26527 (WO01/22990), both filed on September 27, 2000.

In an embodiment, the "A class" CpG oligonucleotide of the invention has the following nucleic acid sequence: 5' GGGGACGACGTCGTGGGGGGG 3'

Some non-limiting examples of A-Class oligonucleotides include:
5' G*G*G_G_A_C_G_A_C_G_T_C_G_T_G_G*G*G*G*G*G 3'; wherein * refers to a phosphorothioate bond and_refers to a phosphodiester bond.

The B class CpG oligonucleotide sequences of the invention are those broadly described above as well as disclosed in published PCT Patent Applications PCT/US95/01570 and PCT/US97/19791, and in US Patents 6,194,388; 6,207,646; 6,214,806; 6,218,371; 6,239,116 and 6,339,068. Exemplary sequences include but are not limited to those disclosed in these latter applications and patents.

In an embodiment, the "B class" CpG oligonucleotide of the invention has the following nucleic acid sequence:
5' TCGTCGTTTTTCGGTGCTTTT 3' (SEQ ID NO. 582), or
5' TCGTCGTTTTTCGGTCGTTTT 3'(SEQ ID NO. 583)or
5' TCGTCGTTTTGTCGTTTTGTCGTT 3' (SEQ ID NO. 584) or
5' TCGTCGTTTCGTCGTTTTGTCGTT 3' (SEQ ID NO. 585), or
5' TCGTCGTTTTGTCGTTTTTTTCGA 3' (SEQ ID NO. 586).

In any of these sequences, all of the linkages may be all phosphorothioate bonds. In another embodiment, in any of these sequences, one or more of the linkages may be phosphodiester, preferably between the "C" and the "G" of the CpG motif making a semi-soft CpG oligonucleotide. In any of these sequences, an ethyl-uridine or a halogen may substitute for the 5' T; examples of halogen substitutions include but are not limited to bromo-uridine or iodo-uridine substitutions.

Some non-limiting examples of B-Class oligonucleotides include:
5' T*C*G*T*C*G*T*T*T*T*T*C*G*G*T*G*C*T*T*T*T 3', or
5' T*C*G*T*C*G*T*T*T*T*T*C*G*G*T*C*G*T*T*T*T 3' or
5' T*C*G*T*C*G*T*T*T*T*G*T*C*G*T*T*T*T*G*T*C*G*T*T 3', or
5' T*C*G*T*C*G*T*T*T*C*G*T*C*G*T*T*T*T*G*T*C*G*T*T 3' , or
5' T*C*G*T*C*G*T*T*T*T*G*T*C*G*T*T*T*T*T*T*T*C*G*A 3'.
wherein * refers to a phosphorothioate bond.

The "C class" of CpG immunostimulatory oligonucleotides is characterized functionally by the ability to activate B cells and NK cells and induce IFN-α. Structurally, this class typically includes a region with one or more B class-type immunostimulatory CpG motifs, and a GC -rich palindrome or near-palindrome region that allows the molecules to form secondary (e.g., stem-loop) or tertiary (e.g., dimer) type structures. Some of these oligonucleotides have both a traditional "stimulatory" CpG sequence and a "GC-rich" or "B-cell neutralizing" motif. These combination motif oligonucleotides have immune stimulating effects that fall somewhere between the effects associated with traditional B class CpG oligonucleotides (i.e., strong induction of B cell activation and dendritic cell (DC) activation), and the effects associated with A class CpG ODN (i.e., strong induction of IFN-α and NK cell activation but relatively poor induction of B cell and DC activation). Krieg AM et al. (1995) Nature 374:546-9; Ballas ZK et al. (1996) J Immunol 157:1840-5; Yamamoto S et al. (1992) J Immunol 148:4072-6.

The C class of combination motif immune stimulatory oligonucleotides may have either completely stabilized, (e.g., all phosphorothioate), chimeric (phosphodiester central region), or semi-soft (e.g., phosphodiester within CpG motif) backbones. This class has been described in U.S. patent application US 10/224,523 filed on August 19, 2002.

One stimulatory domain or motif of the C class CpG oligonucleotide is defined by the formula: 5' X₁DCGHX₂ 3'. D is a nucleotide other than C. C is cytosine. G is guanine. H is a nucleotide other than G. X₁ and X₂ are any nucleic acid sequence 0 to 10 nucleotides long. X₁ may include a CG, in which case there is preferably a T immediately preceding this CG. In some embodiments, DCG is TCG. X₁ is preferably from 0 to 6 nucleotides in length. In some embodiments, X₂ does not contain any poly G or poly A motifs. In other embodiments, the immunostimulatory oligonucleotide has a poly-T sequence at the 5' end or at the 3' end. As used herein, "poly- A" or "poly-T" shall refer to a stretch of four or more consecutive A's or T's respectively, e.g., 5' AAAA 3' or 5' TTTT 3'. As used herein, "poly-G end" shall refer to a stretch of four or more consecutive G's, e.g., 5' GGGG 3', occurring at the 5' end or the 3' end of a nucleic acid. As used herein, "poly-G oligonucleotide" shall refer to an oligonucleotide having the formula 5' X₁X₂GGGX₃X₄ 3' wherein X₁, X₂, X₃, and X₄ are nucleotides and preferably at least one of X₃ and X₄ is a G. Some preferred designs for the B cell stimulatory domain under this formula comprise TTTTTCG, TCG, TTCG, TTTCG, TTTTCG, TCGT, TTCGT, TTTCGT, TCGTCGT.

The second motif of the C class CpG oligonucleotide is referred to as either P or N and is positioned immediately 5' to X₁ or immediately 3' to X₂.

N is a B cell neutralizing sequence that begins with a CGG trinucleotide and is at least 10 nucleotides long. A B cell neutralizing motif includes at least one CpG sequence in which the CG is preceded by a C or followed by a G (Krieg AM et al. (1998) Proc Natl Acad Sd USA 95:12631-12636) or is a CG containing DNA sequence in which the C of the CG is methylated. Neutralizing motifs or sequences have some degree of immunostimulatory capability when present in an otherwise non- stimulatory motif, but when present in the context of other immunostimulatory motifs serve to reduce the immunostimulatory potential of the other motifs.

P is a GC-rich palindrome containing sequence at least 10 nucleotides long.

As used herein, "palindrome" and equivalently "palindromic sequence" shall refer to an inverted repeat, i.e., a sequence such as ABCDEE'D'C'B'A' in which A and A', B and B', etc., are bases capable of forming the usual Watson-Crick base pairs.

As used herein, "GC-rich palindrome" shall refer to a palindrome having a base composition of at least two-thirds G's and Cs. In some embodiments the GC- rich domain is preferably 3' to the "B cell stimulatory domain". In the case of a 10- base long GC-rich palindrome, the palindrome thus contains at least 8 G's and Cs. In the case of a 12-base long GC-rich palindrome, the palindrome also contains at least 8 G's and Cs. In the case of a 14-mer GC-rich palindrome, at least ten bases of the palindrome are G's and Cs. In some embodiments the GC-rich palindrome is made up exclusively of G's and Cs.

In some embodiments the GC-rich palindrome has a base composition of at least 81 % G's and Cs. In the case of such a 10-base long GC-rich palindrome, the palindrome thus is made exclusively of G's and Cs. In the case of such a 12-base long GC-rich palindrome, it is preferred that at least ten bases (83 %) of the palindrome are G's and Cs. In some embodiments, a 12-base long GC-rich palindrome is made exclusively of G's and Cs. In the case of a 14-mer GC-rich palindrome, at least twelve bases (86 %) of the palindrome are G's and Cs. In some embodiments, a 14-base long GC-rich palindrome is made exclusively of G's and Cs. The Cs of a GC-rich palindrome can be unmethylated or they can be methylated.

In general this domain has at least 3 Cs and Gs, more preferably 4 of each, and most preferably 5 or more of each. The number of Cs and Gs in this domain need not be identical. It is preferred that the Cs and Gs are arranged so that they are able to form a self-complementary duplex, or palindrome, such as CCGCGCGG. This may be interrupted by As or Ts, but it is preferred that the self-complementarity is at least partially preserved as for example in the motifs CGACGTTCGTCG or CGGCGCCGTGCCG. When complementarity is not preserved, it is preferred that the non-complementary base pairs be TG. In an embodiment there are no more than 3 consecutive bases that are not part of the palindrome, preferably no more than 2, and most preferably only 1. In some embodiments, the GC-rich palindrome includes at least one CGG trimer, at least one CCG trimer, or at least one CGCG tetramer. In other embodiments, the GC-rich palindrome is not CCCCCCGGGGGG or GGGGGGCCCCCC, CCCCCGGGGG or GGGGGCCCCC.

At least one of the G's of the GC rich region may be substituted with an inosine (I). In some embodiments, P includes more than one I.

In certain embodiments, the immunostimulatory oligonucleotide has one of the following formulas 5' NX₁DCGHX₂ 3', 5' X₁DCGHX₂N 3', 5' PX₁DCGHX₂ 3', 5' X₁DCGHX₂P 3', 5' X₁DCGHX₂PX₃ 3', 5' X₁DCGHPX₃ 3', 5' DCGHX₂PX₃ 3', 5' TCGHX₂PX3 3', 5' DCGHPX₃ 3' or 5'DCGHP 3'.

The invention provides other immune stimulatory oligonucleotides defined by a formula 5' N₁PyGN₂P 3'. N₁ is any sequence 1 to 6 nucleotides long. Py is a pyrimidine. G is guanine. N₂ is any sequence 0 to 30 nucleotides long. P is a GC- rich palindrome containing a sequence at least 10 nucleotides long.

N₁ and N₂ may contain more than 50% pyrimidines, and more preferably more than 50% T. N₁ may include a CG, in which case there is preferably a T immediately preceding this CG. hi some embodiments, N1PyG is TCG, and most preferably a TCGN₂, where N₂ is not G.

N₁PyGN₂P may include one or more inosine (I) nucleotides. Either the C or the G in N₁ may be replaced by inosine, but the Cpl is preferred to the IpG. For inosine substitutions such as IpG, the optimal activity may be achieved with the use of a "semi-soft" or chimeric backbone, where the linkage between the IG or the CI is phosphodiester. N1 may include at least one CI, TCI, IG or TIG motif.

In certain embodiments N₁PyGN₂ is a sequence selected from the group consisting of TTTTTCG, TCG, TTCG , TTTCG, TTTTCG, TCGT, TTCGT, TTTCGT, and TCGTCGT.

In an embodiment, the "C class" CpG oligonucleotide of the invention has the following nucleic acid sequence:
5' TCGCGTCGTTCGGCGCGCGCCG 3' (SEQ ID NO. 587), or
5' TCGTCGACGTTCGGCGCGCGCCG 3' (SEQ ID NO. 588), or
5' TCGGACGTTCGGCGCGCGCCG 3' (SEQ ID NO. 589), or
5' TCGGACGTTCGGCGCGCCG 3' (SEQ ID NO. 590), or
5' TCGCGTCGTTCGGCGCGCCG 3' (SEQ ID NO. 591), or
5' TCGACGTTCGGCGCGCGCCG 3' (SEQ ID NO. 592), or
5' TCGACGTTCGGCGCGCCG 3' (SEQ ID NO. 593), or
5' TCGCGTCGTTCGGCGCCG 3' (SEQ ID NO. 594), or
5' TCGCGACGTTCGGCGCGCGCCG 3' (SEQ ID NO. 595), or
5' TCGTCGTTTTCGGCGCGCGCCG 3' (SEQ ID NO. 596), or
5' TCGTCGTTTTCGGCGGCCGCCG 3' (SEQ ID NO. 597), or
5' TCGTCGTTTTACGGCGCCGTGCCG 3' (SEQ ID NO. 598, or
5' TCGTCGTTTTCGGCGCGCGCCGT 3' (SEQ ID NO. 599.

In any of these sequences, all of the linkages may be all phosphorothioate bonds. In another embodiment, in any of these sequences, one or more of the linkages may be phosphodiester, preferably between the "C" and the "G" of the CpG motif making a semi-soft CpG oligonucleotide.

Some non-limiting examples of C-Class oligonucleotides include:
5' T*C_G*C_G*T*C_G*T*T*C_G*G*C*G*C_G*C*G*C*C*G 3', or
5' T*C_G*T*C_G*A*C_G*T*T*C_G*G*C*G*C_G*C*G*C*C*G 3', or
5' T*C_G*G*A*C_G*T*T*C_G*G*C*G*C_G*C*G*C*C*G 3', or
5' T*C_G*G*A*C_G*T*T*C_G*G*C*G*C*G*C*C*G 3', or
5' T*C_G*C_G*T*C_G*T*T*C_G*G*C*G*C*G*C*C*G 3', or
5' T*C_G*A*C_G*T*T*C_G*G*C*G*C_G*C*G*C*C*G 3', or
5' T*C_G*A*C_G*T*T*C_G*G*C*G*C*G*C*C*G 3', or
5' T*C_G*C_G*T*C_G*T*T*C_G*G*C*G*C*C*G 3', or
5' T*C_G*C_G*A*C_G*T*T*C_G*G*C*G*C_G*C*G*C*C*G 3', or
5' T*C*G*T*C*G*T*T*T*T*C*G*G*C*G*C*G*C*G*C*C*G 3', or
5' T*C*G*T*C*G*T*T*T*T*C*G*G*C*G*G*C*C*G*C*C*G 3', or
5' T*C*G*T*C_G*T*T*T*T*A*C_G*G*C*G*C*C_G*T*G*C*C*G 3', or
5' T*C_G*T*C*G*T*T*T*T*C*G*G*C*G*C*G*C*G*C*C*G*T 3'
wherein * refers to a phosphorothioate bond and _ refers to a phosphodiester bond.

In any of these sequences, an ethyl-uridine or a halogen may substitute for the 5' T; examples of halogen substitutions include but are not limited to bromo-uridine or iodo-uridine substitutions.

The "P class" CpG immunostimulatory oligonucleotides have been described in WO2007/095316 and are characterized by the fact that they contain duplex forming regions such as, for example, perfect or imperfect palindromes at or near both the 5' and 3' ends, giving them the potential to form higher ordered structures such as concatamers. These oligonucleotides referred to as P-Class oligonucleotides have the ability in some instances to induce much high levels of IFN-α secretion than the C-Class. The P-Class oligonucleotides have the ability to spontaneously self-assemble into concatamers either in vitro and/or in vivo. Without being bound by any particular theory for the method of action of these molecules, one potential hypothesis is that this property endows the P-Class oligonucleotides with the ability to more highly crosslink TLR9 inside certain immune cells, inducing a distinct pattern of immune activation compared to the previously described classes of CpG oligonucleotides.

In an embodiment, the CpG oligonucleotide for use in the present invention is a P class CpG oligonucleotide containing a 5' TLR activation domain and at least two palindromic regions, one palindromic region being a 5' palindromic region of at least 6 nucleotides in length and connected to a 3' palindromic region of at least 8 nucleotides in length either directly or through a spacer, wherein the oligonucleotide includes at least one YpR dinucleotide. In an embodiment, said oligoonucleotide is not T*C_G*T*C_G*A*C_G*T*T*C_G*G*C*G*C_G*C*G*C*C*G. In one embodiment the P class CpG oligonucleotide includes at least one unmethylated CpG dinucleotide. In another embodiment the TLR activation domain is TCG, TTCG, TTTCG, TYpR, TTYpR, TTTYpR, UCG, UUCG, UUUCG, TTT, or TTTT. In yet another embodiment the TLR activation domain is within the 5' palindromic region. In another embodiment the TLR activation domain is immediately 5' to the 5' palindromic region. In still another embodiment the 5' palindromic region is at least 8 nucleotides in length. In another embodiment the 3' palindromic region is at least 10 nucleotides in length. In another embodiment the 5' palindromic region is at least 10 nucleotides in length. In yet another embodiment the 3' palindromic region includes an unmethylated CpG dinucleotide. In another embodiment the 3' palindromic region includes two unmethylated CpG dinucleotides. In another embodiment the 5' palindromic region includes an unmethylated CpG dinucleotide. In yet another embodiment the 5' palindromic region includes two unmethylated CpG dinucleotides. In another embodiment the 5' and 3' palindromic regions have a duplex stability value of at least 25. In another embodiment the 5' and 3' palindromic regions have a duplex stability value of at least 30. In another embodiment the 5' and 3' palindromic regions have a duplex stability value of at least 35. In another embodiment the 5' and 3' palindromic regions have a duplex stability value of at least 40. In another embodiment the 5' and 3' palindromic regions have a duplex stability value of at least 45. In another embodiment the 5' and 3' palindromic regions have a duplex stability value of at least 50. In another embodiment the 5' and 3' palindromic regions have a duplex stability value of at least 55. In another embodiment the 5' and 3' palindromic regions have a duplex stability value of at least 60. In another embodiment the 5' and 3' palindromic regions have a duplex stability value of at least 65.

In one embodiment the two palindromic regions are connected directly. In another embodiment the two palindromic regions are connected via a 3 '-3' linkage. In another embodiment the two palindromic regions overlap by one nucleotide. In yet another embodiment the two palindromic regions overlap by two nucleotides. In another embodiment the two palindromic regions do not overlap. In another embodiment the two palindromic regions are connected by a spacer. In one embodiment the spacer is a nucleic acid having a length of 1-50 nucleotides. In another embodiment the spacer is a nucleic acid having a length of 1 nucleotide. In another embodiment the spacer is a non-nucleotide spacer. In one embodiment the non-nucleotide spacer is a D-spacer. In another embodiment the non-nucleotide spacer is a linker. In one embodiment the oligonucleotide has the formula 5' XP₁SP₂T 3', wherein X is the TLR activation domain, P₁ is a palindrome, S is a spacer, P₂ is a palindrome, and T is a 3' tail of 0-100 nucleotides in length. In one embodiment X is TCG, TTCG, or TTTCG. In another embodiment T is 5-50 nucleotides in length. In yet another embodiment T is 5-10 nucleotides in length. In one embodiment S is a nucleic acid having a length of 1-50 nucleotides. In another embodiment S is a nucleic acid having a length of 1 nucleotide. In another embodiment S is a non-nucleotide spacer. In one embodiment the non-nucleotide spacer is a D-spacer. In another embodiment the non-nucleotide spacer is a linker. In another embodiment the oligonucleotide is not an antisense oligonucleotide or a ribozyme. In one embodiment P₁ is A and T rich. In another embodiment P₁ includes at least 4 Ts. In another embodiment P₂ is a perfect palindrome. In another embodiment P2 is G-C rich. In still another embodiment P₂ is CGGCGCX₁GCGCCG, where X₁ is T or nothing.

In one embodiment the oligonucleotide includes at least one phosphorothioate linkage. In another embodiment all internucleotide linkages of the oligonucleotide are phosphorothioate linkages. In another embodiment the oligonucleotide includes at least one phosphodiester-like linkage. In another embodiment the phosphodiester-like linkage is a phosphodiester linkage. In another embodiment a lipophilic group is conjugated to the oligonucleotide. In one embodiment the lipophilic group is cholesterol.

In an embodiment, the TLR-9 agonist is a P class CpG oligonucleotide with a 5' TLR activation domain and at least two complementarity-containing regions, a 5' and a 3' complementarity-containing region, each complementarity-containing region being at least 8 nucleotides in length and connected to one another either directly or through a spacer, wherein the oligonucleotide includes at least one pyrimidine-purine (YpR) dinucleotide, and wherein at least one of the complementarity-containing regions is not a perfect palindrome. In one embodiment the oligonucleotide includes at least one unmethylated CpG dinucleotide. In another embodiment the TLR activation domain is TCG, TTCG, TTTCG, TYpR, TTYpR, TTTYpR, UCG, UUCG, UUUCG, TTT, or TTTT. In another embodiment the TLR activation domain is within the 5' complementarity-containing region. In another embodiment the TLR activation domain is immediately 5' to the 5' complementarity-containing region. In another embodiment the 3' complementarity-containing region is at least 10 nucleotides in length. In yet another embodiment the 5' complementarity-containing region is at least 10 nucleotides in length. In one embodiment the 3' complementarity- containing region includes an unmethylated CpG dinucleotide. In another embodiment the 3' complementarity-containing region includes two unmethylated CpG dinucleotides. In yet another embodiment the 5' complementarity-containing region includes an unmethylated CpG dinucleotide. In another embodiment the 5' complementarity-containing region includes two unmethylated CpG dinucleotides. In another embodiment the complementarity- containing regions include at least one nucleotide analog. In another embodiment the complementarity-containing regions form an intramolecular duplex. In one embodiment the intramolecular duplex includes at least one non- Watson Crick base pair. In another embodiment the non- Watson Crick base pair is G-T, G-A, G-G, or C-A. In one embodiment the complementarity-containing regions form intermolecular duplexes. In another embodiment at least one of the intermolecular duplexes includes at least one non- Watson Crick base pair. In another embodiment the non- Watson Crick base pair is G-T, G- A, G-G, or C-A. In yet another embodiment the complementarity-containing regions contain a mismatch. In still another embodiment the complementarity-containing regions contain two mismatches. In another embodiment the complementarity-containing regions contain an intervening nucleotide. In another embodiment the complementarity-containing regions contain two intervening nucleotides.

In one embodiment the 5' and 3' complementarity-containing regions have a duplex stability value of at least 25. In another embodiment the 5' and 3' complementarity- containing regions have a duplex stability value of at least 30. In another embodiment the 5' and 3' complementarity-containing regions have a duplex stability value of at least 35. In another embodiment the complementarity-containing regions have a duplex stability value of at least 40. In another embodiment the complementarity-containing regions have a duplex stability value of at least 45. In another embodiment the complementarity-containing regions have a duplex stability value of at least 50. In another embodiment the complementarity- containing regions have a duplex stability value of at least 55. In another embodiment the complementarity-containing regions have a duplex stability value of at least 60. In another embodiment the complementarity-containing regions have a duplex stability value of at least 65.

In another embodiment the two complementarity-containing regions are connected directly. In another embodiment the two palindromic regions are connected via a 3 '-3' linkage. In yet another embodiment the two complementarity-containing regions overlap by one nucleotide. In another embodiment the two complementarity-containing regions overlap by two nucleotides. In another embodiment the two complementarity-containing regions do not overlap. In another embodiment the two complementarity-containing regions are connected by a spacer. In another embodiment the spacer is a nucleic acid having a length of 1 -50 nucleotides. In another embodiment the spacer is a nucleic acid having a length of 1 nucleotide. In one embodiment the spacer is a non-nucleotide spacer. In another embodiment the non-nucleotide spacer is a D-spacer. In yet another embodiment the non- nucleotide spacer is a linker.

In one embodiment the P-class oligonucleotide has the formula 5' XNSPT 3', wherein X is the TLR activation domain, N is a non-perfect palindrome, P is a palindrome, S is a spacer, and T is a 3' tail of 0-100 nucleotides in length. In another embodiment X is TCG, TTCG, or TTTCG. In another embodiment T is 5-50 nucleotides in length. In another embodiment T is 5-10 nucleotides in length. In another embodiment S is a nucleic acid having a length of 1-50 nucleotides. In another embodiment S is a nucleic acid having a length of 1 nucleotide. In another embodiment S is a non-nucleotide spacer. In another embodiment the non-nucleotide spacer is a D-spacer. In another embodiment the non-nucleotide spacer is a linker. In another embodiment the oligonucleotide is not an antisense oligonucleotide or a ribozyme. In another embodiment N is A and T rich. In another embodiment N is includes at least 4 Ts. In another embodiment P is a perfect palindrome. In another embodiment P is G-C rich. In another embodiment P is CGGCGCX₁GCGCCG, wherein X₁ is T or nothing. In another embodiment the oligonucleotide includes at least one phosphorothioate linkage. In another embodiment all interaucleotide linkages of the oligonucleotide are phosphorothioate linkages. In another embodiment the oligonucleotide includes at least one phosphodiester-like linkage. In another embodiment the phosphodiester-like linkage is a phosphodiester linkage. In another embodiment a lipophilic group is conjugated to the oligonucleotide. In one embodiment the lipophilic group is cholesterol.

In an embodiment, the "P class" CpG oligonucleotides have the following nucleic acid sequence: 5' TCGTCGACGATCGGCGCGCGCCG 3' (SEQ ID NO. 600).

In said sequences, all of the linkages may be all phosphorothioate bonds. In another embodiment, one or more of the linkages may be phosphodiester, preferably between the "C" and the "G" of the CpG motif making a semi-soft CpG oligonucleotide. In any of these sequences, an ethyl-uridine or a halogen may substitute for the 5' T; examples of halogen substitutions include but are not limited to bromo-uridine or iodo-uridine substitutions.

A non-limiting example of P-Class oligonucleotides include:
5' T*C_G*T*C_G*A*C_G*A*T*C_G*G*C*G*C_G*C*G*C*C*G 3'
wherein * refers to a phosphorothioate bond and _ refers to a phosphodiester bond.

In an embodiment, all the internucleotide linkage of the CpG oligonucleotides disclosed herein are phosphodiester bonds ("soft" oligonucleotides, as described in the PCT application WO2007/026190). In another embodiment, CpG oligonucleotides of the invention are rendered resistant to degradation (e.g., are stabilized). A "stabilized oligonucleotide" refers to an oligonucleotide that is relatively resistant to *in vivo* degradation (e.g. via an exo- or endo-nuclease). Nucleic acid stabilization can be accomplished via backbone modifications. Oligonucleotides having phosphorothioate linkages provide maximal activity and protect the oligonucleotide from degradation by intracellular exo- and endo-nucleases.

The immunostimulatory oligonucleotides may have a chimeric backbone, which have combinations of phosphodiester and phosphorothioate linkages. For purposes of the instant invention, a chimeric backbone refers to a partially stabilized backbone, wherein at least one internucleotide linkage is phosphodiester or phosphodiester-like, and wherein at least one other internucleotide linkage is a stabilized internucleotide linkage, wherein the at least one phosphodiester or phosphodiester-like linkage and the at least one stabilized linkage are different. When the phosphodiester linkage is preferentially located within the CpG motif such molecules are called "semi-soft" as described in PCT application WO2007/026190.

Other modified oligonucleotides include combinations of phosphodiester, phosphorothioate, methylphosphonate, methylphosphorothioate, phosphorodithioate, and/or p-ethoxy linkages.

Since boranophosphonate linkages have been reported to be stabilized relative to phosphodiester linkages, for purposes of the chimeric nature of the backbone, boranophosphonate linkages can be classified either as phosphodiester-like or as stabilized, depending on the context. For example, a chimeric backbone according to the instant invention could, in some embodiments, includes at least one phosphodiester (phosphodiester or phosphodiester-like) linkage and at least one boranophosphonate (stabilized) linkage. In other embodiments, a chimeric backbone according to the instant invention could include boranophosphonate (phosphodiester or phosphodiester-like) and phosphorothioate (stabilized) linkages. A "stabilized internucleotide linkage" shall mean an internucleotide linkage that is relatively resistant to in vivo degradation (e.g., via an exo- or endo-nuclease), compared to a phosphodiester internucleotide linkage. Preferred stabilized internucleotide linkages include, without limitation, phosphorothioate, phosphorodithioate, methylphosphonate, and methylphosphorothioate. Other stabilized internucleotide linkages include, without limitation, peptide, alkyl, dephospho, and others as described above.

Modified backbones such as phosphorothioates may be synthesized using automated techniques employing either phosphoramidate or H-phosphonate chemistries. Aryl- and alkyl-phosphonates can be made, e.g., as described in US Patent No. 4,469,863; and alkylphosphotriesters (in which the charged oxygen moiety is alkylated as described in US Patent No. 5,023,243 and European Patent No. 092,574) can be prepared by automated solid phase synthesis using commercially available reagents. Methods for making other DNA backbone modifications and substitutions have been described. Uhlmann E et al. (1990) Chem Rev 90:544; Goodchild J (1990) Bioconjugate Chem 1:165. Methods for preparing chimeric oligonucleotides are also known. For instance, patents issued to Uhlmann et al have described such techniques.

Mixed backbone modified ODN may be synthesized as described in PCT application WO2007/026190.

The oligonucleotides can also include other modifications. These include nonionic DNA analogs, such as alkyl- and aryl-phosphates (in which the charged phosphonate oxygen is replaced by an alkyl or aryl group), phosphodiester and alkylphosphotriesters, in which the charged oxygen moiety is alkylated. Nucleic acids which contain diol, such as tetraethyleneglycol or hexaethyleneglycol, at either or both termini have also been shown to be substantially resistant to nuclease degradation.

The size of the CpG oligonucleotide (i.e., the number of nucleotide residues along the length of the oligonucleotide) also may contribute to the stimulatory activity of the oligonucleotide. For facilitating uptake into cells, CpG oligonucleotides of the invention preferably have a minimum length of 6 nucleotide residues. Oligonucleotides of any size greater than 6 nucleotides (even many kb long) are capable of inducing an immune response if sufficient immunostimulatory motifs are present, because larger oligonucleotides are degraded inside cells. In certain embodiments, the CpG oligonucleotides are 6 to 100 nucleotides long, preferentially 8 to 30 nucleotides long. In important embodiments, nucleic acids and oligonucleotides of the invention are not plasmids or expression vectors.

In an embodiment, the CpG oligonucleotides disclosed herein comprise substitutions or modifications, such as in the bases and/or sugars as described at paragraph 134 to 147 of WO2007/026190.

In an embodiment, the CpG oligonucleotide is chemically modified. Examples of chemical modifications are known to the skilled person and are described, for example in Uhlmann E. et al. (1990), Chem. Rev. 90:543, S. Agrawal, Ed., Humana Press, Totowa, USA 1993; Crooke, S.T. et al. (1996) Annu. Rev. Pharmacol. Toxicol. 36:107-129; and Hunziker J. et al., (1995), Mod. Synth. Methods 7:331-417. An oligonucleotide may have one or more modifications, wherein each modification is located at a particular phosphodiester internucleoside bridge and/or at a particular β-D-ribose unit and/or at a particular natural nucleoside base position in comparison to an oligonucleotide of the same sequence which is composed of natural DNA or RNA.

In some embodiments of the invention, CpG-containing nucleic acids might be simply mixed with immunogenic carriers according to methods known to those skilled in the art (see, e.g., WO03/024480).

In a particular embodiment any of the vaccine disclosed herein comprises from 20µg to 20mg of CpG oligonucleotide, preferably from 0.1 mg to 10 mg CpG oligonucleotide, preferably from 0.2 mg to 5 mg CpG oligonucleotide, preferably from 0.3 mg to 3 mg CpG oligonucleotide, even preferably from 0.4 to 2 mg CpG oligonucleotide, even preferably from 0.5 to 1.5 mg CpG oligonucleotide. In an embodiement, any of the vaccine disclosed herein comprises approximately 0.5 to 1mg CpG oligonucleotide.

Preferred adjuvants for use in the present invention are alum, QS21, CpG ODN, alum in combination with CpG ODN, Iscomatrix and Iscomatrix in combination with CpG ODN.

### Pharmaceutical Compositions

The disclosure also provides pharmaceutical compositions comprising an antigenic PCSK9 peptide or an immunogenic composition thereof, in a formulation in association with one or more pharmaceutically acceptable excipient(s) and optionally combined with one or more adjuvants (as adjuvant described above). The term 'excipient' is used herein to describe any ingredient other than the active ingredient, i.e. the antigenic PCSK9 peptide coupled to an immunogenic carrier and optionally combined with one or more adjuvants. The choice of excipient(s) will to a large extent depend on factors such as the particular mode of administration, the effect of the excipient on solubility and stability, and the nature of the dosage form. As used herein, "pharmaceutically acceptable excipient" includes any and all solvents, dispersion media, coatings, antibacterial and antifungal agents, isotonic and absorption delaying agents, and the like that are physiologically compatible. Some examples of pharmaceutically acceptable excipients are water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Additional examples of pharmaceutically acceptable substances are wetting agents or minor amounts of auxiliary substances such as wetting or emulsifying agents, preservatives or buffers, which enhance the shelf life or effectiveness of the active ingredient.

Pharmaceutical compositions and methods for their preparation will be readily apparent to those skilled in the art. Such compositions and methods for their preparation may be found, for example, in Remington's Pharmaceutical Sciences, 19th Edition (Mack Publishing Company, 1995). Pharmaceutical compositions are preferably manufactured under GMP conditions.

A pharmaceutical composition of the invention may be prepared, packaged, or sold in bulk, as a single unit dose, or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

Any method for administering peptides, or proteins accepted in the art may suitably be employed for the peptides or proteins of the invention.

The pharmaceutical compositions of the invention are typically suitable for parenteral administration. As used herein, "parenteral administration" of a pharmaceutical composition includes any route of administration characterized by physical breaching of a tissue of a subject and administration of the pharmaceutical composition through the breach in the tissue, thus generally resulting in the direct administration into the blood stream, into muscle, or into an internal organ. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intrasternal, intravenous, intraarterial, intrathecal, intraventricular, intraurethral, intracranial, intrasynovial injection or infusions; and kidney dialytic infusion techniques. Embodiments include the intravenous, subcutaneous, intradermal and intramuscular routes.

Formulations of a pharmaceutical composition suitable for parenteral administration typically generally comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampoules or in multi-dose containers containing a preservative. Formulations for parenteral administration include, but are not limited to, suspensions, solutions, emulsions in oily or aqueous vehicles, pastes, and the like. Such formulations may further comprise one or more additional ingredients including, but not limited to, suspending, stabilizing, or dispersing agents. In one embodiment of a formulation for parenteral administration, the active ingredient is provided in dry (i.e. powder or granular) form for reconstitution with a suitable vehicle (e.g. sterile pyrogen-free water) prior to parenteral administration of the reconstituted composition. Parenteral formulations also include aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water. Exemplary parenteral administration forms include solutions or suspensions in sterile aqueous solutions, for example, aqueous propylene glycol or dextrose solutions. Such dosage forms can be suitably buffered, if desired. Other parentally-administrable formulations which are useful include those which comprise the active ingredient in microcrystalline form, microparticles, or in a liposomal preparation. Formulations for parenteral administration may be formulated to be immediate and/or modified release. Modified release formulations include delayed-, sustained-, pulsed-, controlled-, targeted and programmed release.

For example, in one aspect, sterile injectable solutions can be prepared by incorporating the anti-PCSK9 peptide, preferably coupled to an immunogenic carrier, optionally in combination with one or more adjuvants, in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

An exemplary, non-limiting pharmaceutical composition of the invention is a formulation as a sterile aqueous solution having a pH that ranges from about 5.0 to about 6.5 and comprising from about 0.1 mg/mL to about 20 mg/mL of a peptide of the invention, from about 1 millimolar to about 100 millimolar of histidine buffer, from about 0.01 mg/mL to about 10 mg/mL of polysorbate 80, from about 100 millimolar to about 400 millimolar of trehalose, and from about 0.01 millimolar to about 1.0 millimolar of disodium EDTA dihydrate.

The antigenic PCSK9 peptides can also be administered intranasally or by inhalation, typically in the form of a dry powder (either alone, as a mixture, or as a mixed component particle, for example, mixed with a suitable pharmaceutically acceptable excipient) from a dry powder inhaler, as an aerosol spray from a pressurised container, pump, spray, atomiser (preferably an atomiser using electrohydrodynamics to produce a fine mist), or nebuliser, with or without the use of a suitable propellant, or as nasal drops.

The pressurised container, pump, spray, atomizer, or nebuliser generally contains a solution or suspension of an antibody of the disclosure comprising, for example, a suitable agent for dispersing, solubilising, or extending release of the active, a propellant(s) as solvent.

Prior to use in a dry powder or suspension formulation, the drug product is generally micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules, blisters and cartridges for use in an inhaler or insufflator may be formulated to contain a powder mix of the compound of the invention, a suitable powder base and a performance modifier.

A suitable solution formulation for use in an atomiser using electrohydrodynamics to produce a fine mist may contain a suitable dose of the antigenic PCSK9 peptide per actuation and the actuation volume may for example vary from 1µL to 100µL.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" of an antibody of the disclosure. The overall daily dose will typically be administered in a single dose or, more usually, as divided doses throughout the day.

A pharmaceutical composition comprising an antigenic PCSK9 peptide may also be formulated for an oral route administration. Oral administration may involve swallowing, so that the compound enters the gastrointestinal tract, and/or buccal, lingual, or sublingual administration by which the compound enters the blood stream directly from the mouth.

Formulations suitable for oral administration include solid, semi-solid and liquid systems such as tablets; soft or hard capsules containing multi- or nano-particulates, liquids, or powders; lozenges (including liquid-filled); chews; gels; fast dispersing dosage forms; films; ovules; sprays; and buccal/mucoadhesive patches.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compositions of the invention can be used to treat, alleviate or prevent PCSK9-mediated disorders or symptoms in a subject at risk or suffering from such disorder or symptom by stimulating an immune response in said subject by immunotherapy. Immunotherapy can comprise an initial immunization followed by additional, e. g. one, two, three, or more boosters.

An "immunologically effective amount" of an antigenic PCSK9 peptide or composition thereof, is an amount that is delivered to a mammalian subject, either in a single dose or as part of a series, which is effective for inducing an immune response against PCSK9 in said subject. This amount varies depending upon the health and physical condition of the individual to be treated, the taxonomic group of individual to be treated, the capacity of the individual's immune system to synthesize antibodies, the formulation of the vaccine, and other relevant factors. It is expected that the amount will fall in a relatively broad range that can be determined through routine trials.

A "pharmaceutically effective dose" or "therapeutically effective dose" is that dose required to treat or prevent, or alleviate one or more PCSK9-related disorder or symptom in a subject. The pharmaceutically effective dose depends on *inter alia* the specific compound to administer, the severity of the symptoms, the susceptibility of the subject to side effects, the type of disease, the composition used, the route of administration, the type of mammal being treated, the physical characteristics of the specific mammal under consideration such as health and physical condition, concurrent medication, the capacity of the individual's immune system to synthesize antibodies, the degree of protection desired, and other factors that those skilled in the medical arts will recognize. For prophylaxis purposes, the amount of peptide in each dose is selected as an amount which induces an immunoprotective response without significant adverse side effects in typical vaccinees. Following an initial vaccination, subjects may receive one or several booster immunisations adequately spaced.

It is understood that the specific dose level for any particular patient depends upon a variety of factors including the activity of the specific compound employed, the age, body weight, general health, sex, diet, time of administration, route of administration, and rate of excretion, drug combination and the severity of the particular disease undergoing therapy.

For example, antigenic PCSK9 peptides or pharmaceutical composition can be administered to a subject at a dose of about 0.1 µg to about 5 mg, e.g., from about 0.1 µg to about 5 µg, from about 5 µg to about 10 µg, from about 10 µg to about 25 µg, from about 25 µg to about 50 µg, from about 50 µg to about 100 µg, from about 100 µg to about 500 µg, from about 500 µg to about 1 mg, from about 1 mg to about 2 mg, with optional boosters given at, for example, 1 week, 2 weeks, 3 weeks, 4 weeks, two months, three months, 6 months and/or a year later.

In some embodiments, a single dose of an antigenic PCSK9 peptide or pharmaceutical composition is administered. In other embodiments, multiple doses of an antigenic PCSK9 peptide or pharmaceutical composition according to the invention are administered. The frequency of administration can vary depending on any of a variety of factors, e.g., severity of the symptoms, degree of immunoprotection desired, whether the composition is used for prophylactic or curative purposes, etc. For example, in some embodiments, an antigenic PCSK9 peptide or pharmaceutical composition according to the invention is administered once per month, twice per month, three times per month, every other week (qow), once per week (qw), twice per week (biw), three times per week (tiw), four times per week, five times per week, six times per week, every other day (qod), daily (qd), twice a day (qid), or three times a day (tid). When the composition of the invention is used for prophylaxis purposes, they will be generally administered for both priming and boosting doses. It is expected that the boosting doses will be adequately spaced, or preferably given yearly or at such times where the levels of circulating antibody fall below a desired level. Boosting doses may consist of the antigenic PCSK9 peptide in the absence of the original immunogenic carrier molecule. Such booster constructs may comprise an alternative immunogenic carrier or may be in the absence of any carrier. Such booster compositions may be formulated either with or without adjuvant.

The duration of administration of an antigenic PCSK9 peptide e.g., the period of time over which an antigenic PCSK9 peptide is administered, can vary, depending on any of a variety of factors, e.g., patient response, etc. For example, an antigenic PCSK9 peptide can be administered over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

A variety of treatment methods are also contemplated by the present disclosure, which methods comprise administering an antigenic PCSK9 peptide according to the invention. Subject treatment methods include methods of inducing an immune response in an individual to self-PCSK9, and methods of preventing, alleviating or treating a PCSK9-related disorder or symptom in an individual.

In one aspect, the present disclosure provides a method for treating, preventing or alleviating a PCSK9-related disorder or symptom in a subject, comprising administering a therapeutically effective amount of an antigenic PCSK9 peptide of the invention, or immunogenic or pharmaceutical composition thereof, to said subject.

In another aspect, the present disclosure provides a method for inducing an immune response against self-PCSK9 in a subject, comprising administering a therapeutically or immunogenically effective amount of an antigenic PCSK9 peptide of the invention, or immunogenic or pharmaceutical composition thereof, to said subject.

A PCSK9 related disease or a PCSK9 mediated disease is, for example, a disease where the inhibition of PCSK9 activity or the inhibition of the interaction of PCSK9 with the LDL receptor could be beneficial.

"Treat", "treating" and "treatment" refer to a method of alleviating or abrogating a biological disorder and/or at least one of its attendant symptoms. As used herein, to "alleviate" a disease, disorder or condition means reducing the severity and/or occurrence frequency of the symptoms of the disease, disorder, or condition. Further, references herein to "treatment" include references to curative, palliative and prophylactic treatment. Said subject is preferably human, and may be either male or female, of any age.

Other aspects of the disclosure relate to an antigenic PCSK9 peptide or of an immunogenic composition or a pharmaceutical composition thereof, for use as a medicament, preferably in treatment, alleviation or prophylaxis of PCSK9-related disorders.

In yet another aspect, the present disclosure provides the use of an antigenic PCSK9 peptide or of an immunogenic composition or a pharmaceutical composition thereof, in the manufacture of a medicament, preferably for treating a PCSK9-related disorder.

In particular, the disclosure relates to an antigenic PCSK9 peptide or an immunogenic or pharmaceutical composition thereof, for use as a medicament preferably in treatment, alleviation or prophylaxis of diseases associated with an elevated level of cholesterol.

In yet another aspect, the present disclosure provides the use of an antigenic PCSK9 peptide or of an immunogenic composition or a pharmaceutical composition thereof, in the manufacture of a medicament, preferably for lowering the LDL-cholesterol level in blood in a subject in need thereof.

In some aspects of the uses or methods of the disclosure said PCSK9-related disorder is selected from the group consisting of elevated cholesterol, a condition associated with elevated LDL-cholesterol, e.g., a lipid disorder (e.g., hyperlipidemia, type I, type II, type III, type IV, or type V hyperlipidemia, secondary hypertriglyceridemia, hypercholesterolemia, familial hypercholesterolemia, xanthomatosis, cholesterol acetyltransferase deficiency), arteriosclerotic conditions (e.g., atherosclerosis), coronary artery disease, and cardiovascular disease.

In yet another aspect, the present disclosure provides the use of an antigenic PCSK9 peptide or of an immunogenic composition or a pharmaceutical composition thereof, in the manufacture of a medicament for treating or alleviating diseases where an up-regulation of the LDL receptor or an inhibition of the interaction between PCSK9 and the LDL receptor is beneficial.

In yet another aspect, the present disclosure provides the use of an antigenic PCSK9 peptide or of an immunogenic composition or a pharmaceutical composition thereof, in the manufacture of a medicament for the treatment of Alzheimer's disease.

In other aspects of the uses or methods said subject is a mammal, preferably a human subject.

In still other aspects of the uses or methods said subject suffers from said PSCK9-related disorder. Alternatively, said subject is at risk of suffering from said PCSK9-related disorder, e.g., due to the presence of one or more risk factors (e.g., hypertension, cigarette smoking, diabetes, obesity, or hyperhomocysteinemia).

The antigenic PCSK9 peptide or an immunogenic composition or a pharmaceutical composition thereof are useful for subjects who are intolerant to therapy with another cholesterol-reducing agent, or for whom therapy with another cholesterol-reducing agent has produced inadequate results (e.g., subjects who experience insufficient LDL-c reduction on statin therapy). The antigenic PCSK9 peptide described herein can be administered to a subject with elevated LDL-cholesterol.

Preferably a subject with elevated cholesterol is a human subject with total plasma cholesterol levels of 200 mg/dl or greater. Preferably a subject with elevated cholesterol is a human subject with LDL-cholesterol levels of 120 mg/dl or greater.

Total plasma cholesterol levels and LDL-cholesterol levels are measured using standard methods on blood samples obtained after an appropriate fast. Protocols to measure total plasma cholesterol levels and LDL-cholesterol levels are well-known to the man skilled in the art.

In one embodiment the antigenic PCSK9 peptide or an immunogenic composition or a pharmaceutical composition thereof is administered together with another agent, the two can be administered sequentially in either order or simultaneously. In some embodiments, an antigenic PCSK9 peptide or an immunogenic composition or a pharmaceutical composition thereof is administered to a subject who is also receiving therapy with a second agent (e.g., a second cholesterol-reducing agent). Cholesterol reducing agents include statins, bile acid sequestrants, niacin, fibric acid derivatives, and long chain alpha, omego-dicarboxylic acids. Statins inhibit cholesterol synthesis by blocking HMGCoA, a key enzyme in cholesterol biosynthesis. Examples of statins are lovastatin, pravastatin, atorvastatin, cerivastatin, fluvastatin, and simvastatin. Bile acid sequestrants interrupt the recycling of bile acids from the intestine to the liver. Examples of these agents are cholestyramine and colestipol hydrochloride. Examples of fibric acid derivatives are clofibrate and gemfibrozil. Long chain alpha, omego-dicarboxylic acids are described, e.g., by Bisgaier et al., 1998, J. Lipid Res. 39:17-30; WO 98/30530; US Patent No. 4,689,344; WO99/00116; US Patent Nos. 5,756,344; 3,773,946; 4,689,344; 4,689,344; 4,689,344; and 3,930,024); ethers (see, e.g., US Patent Nos. 4,711,896; 5,756,544; and 6,506,799). Phosphates of dolichol (US Patent No. 4,613,593), and azolidinedione derivatives (US Patent No. 4,287,200) can also be used to reduce cholesterol levels. A combination therapy regimen may be additive, or it may produce synergistic results (e.g., reductions in cholesterol greater than expected for the combined use of the two agents). In some embodiments, combination therapy with an antigenic PCSK9 peptide or an immunogenic composition or a pharmaceutical composition thereof and a statin produces synergistic results (e.g., synergistic reductions in cholesterol). In some subjects, this can allow reduction in statin dosage to achieve the desired cholesterol levels.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g., amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

### Example 1. Selection of PCSK9 peptides within the Pro- and C-terminal domains

While, at present, there is no direct evidence of functional interaction between the LDLR and either the pro- or C-terminal domains of PCSK9, there are numerous identified gain and loss of function mutations within these regions (Lambert et al, Atherosclerosis, 2031-7, 2009) and increasing evidence indicating a critical role for the pro- and/ or C-terminal domains in PCSK9 secretion function (Du et al. JBC doi/10.1074/jbc. m111.273474). In addition, both domains contain phosphorylation sites suggesting regulation of function (Dewpura et al, FEBS Journal, 275, 3480-3493 2008). Therefore, peptides from within these domains including the identified phosphorylation sites were designed for conjugation (Table 1). The designed peptides included both the phosphorylated and non phosphorylated forms of the peptides. Such peptides including the phosphorylation site (with or without phosphorylation of the residue) were hypothesised to represent the epitopes in a manner similar to that found in native forms of PCSK9, thereby inducing anti-PCSK9 antibodies more able to bind to intact, native self PCSK9 molecules or to bind with an increased affinity to self PCSK9 molecules. In addition, where sequence identity was not conserved, both the human and murine sequences were generated for assessment as vaccine candidates (Table 1). Cys or Gly-Gly-Cys sequences were added for the purpose of conjugation to CRM₁₉₇.

**Table 1 - Example 1 Peptide Summary:**

| **Peptide ID** | **Species** | **Sequence** |
|---|---|---|
| 9.24 (Sequence ID NO.379) | Mouse | CRSRPSAKASWVQ |
| 9.58 (Sequence ID NO.527) | Mouse | CRSRPSAKApSWVQ |
| 9.27 (Sequence ID NO. 86) | Human | LVLALRSEEDG**GC** |
| 9.56 (Sequence ID NO. 224) | Human | LVLALRpSEEDG**GC** |
| 9.28 (Sequence ID NO. 99) | Mouse | LMLALPSQED**GGC** |
| 9.57 (Sequence ID NO. 243) | Mouse | LMLALPpSQED**GGC** |

Residues in bold indicate amino acids added for conjugation purposes. Underlined residues represent phosphoserine.

### Example 2: Synthesis of peptides for evaluation as vaccine candidates:

**Peptides:** The peptides were synthesised using a standard Fmoc protocol on CLEAR amide resin using a Symphony peptide synthesizer (Protein Technologies, Inc). The amino acid coupling reactions were carried out using 5 fold excess of Fmoc-protected amino acid activated with 1 eq of HBTU (2-(1H-Benzotriazole-1-yl)-1,1,3,3-tetramethyluroniumhexafluorophosphate) in the presence of HOBt (hydroxybenzotriazole) and NMM (N-methylmorpholine). The deprotection of Fmoc group was achieved with 20%piperidine/DMF. Resin-bound peptide was then cleaved and side chain protecting groups removed simultaneously with Reagent D (TFA/H2O/DODT: 89/3/8). The peptides were made with a free N-terminus and amidated C-terminus. The crude peptides were purified to homogeneity by HPLC using a BEH 130 C18 column and a water/ acetonitrile gradient in the presence of 0.1% TFA. The purified peptides were vacuum-dried using a lyophilizer. The peptides were analyzed using mass-spectrometry (LCMS).

**Phosphoserine peptides:** The peptides were synthesized on Rink amide MBHA resin with Fmoc chemistry using a Symphony peptide synthesizer (Protein Technologies, Inc). The mono-protected amino acid Fmoc-Ser[PO(O-Bzl)OH]-OH (EMD Chemicals, Inc) was used for incorporating phosphoserine into the sequences. The amino acid coupling reactions were carried out using 5 fold excess of Fmoc-protected amino acid, activated with 1 eq of HBTU/HOBt in the presence of 2 eq of DIPEA, except that 4 eq of DIPEA was used for the coupling of Fmoc-Ser[PO(O-Bzl)OH]-OH. All amino acids were double coupled and a coupling time of 60 min was used for each coupling step. The deprotection of Fmoc group was carried out with 20% piperidine in DMF for 2 x 6 minutes. Resin-bound peptides were cleaved and side chain protecting groups removed simultaneously with TFA/H2O/TIPS/Thioanisole (92.5/2.5/2.5/2.5). The crude peptides were purified to homogeneity by reverse phase HPLC on a BEH 130 Preparative C18 column with TFA system (solvent A: 0.1%TFA/water; solvent B: 0.1%TFA/ acetontrile) for gradient elution. The purified peptides were analyzed by LC/MS.

### Example 3: Preparation of Peptide-CRM₁₉₇ conjugates for evaluation as vaccine candidates:

***CRM₁₉₇:*** The peptides used in this example were conjugated to CRM₁₉₇, a genetically detoxified form of Diphtheria toxin, which is inactivated by a single point mutation of glycine to glutamic acid at position 52 of the protein. The material used in this study was produced by Pfizer's global manufacturing plant in Sanford, North Carolina, USA. The material was stored frozen at -80C, in PBS + 15% sucrose.

***Activation of CRM₁₉₇:*** A 21 mL sample (124mg) of CRM₁₉₇ was thawed at room temperature for approximately 2 hours. Meanwhile, seven 10DG desalting columns (Bio-Rad) were each equilibrated with 25 mL of Dulbecco's PBS (Gibco). Once equilibrated, 3 mL of the CRM₁₉₇ sample was loaded into each of the desalting columns. The samples were eluted from the desalting columns in 4 mL of Dulbecco's PBS then pooled into one reaction vessel. At this stage, the reaction vessel was equipped with a sterile magnetic stirrer, and placed on a magnetic stirring platform.

In order to conjugate the peptide, the CRM₁₉₇ carrier protein was first activated by reaction with the NHS ester group of the cross-linker with surface available lysines present on the CRM₁₉₇ molecule. In this study, conjugation was performed using the hetero-bifunctional cross-linker, SMPH (Succinimidyl-6-[(β-maleimidopropionamido)hexanoate]; Pierce), which contains a N-hydroxysuccinimide (NHS) ester (amine reactive) group at one end, and a maleimide (thiol reactive) group at the other end.

To activate the de-salted CRM₁₉₇, 25mg of SMPH was solubilised in DMSO to prepare a 50 mM stock solution. The SMPH was added to the desalted CRM₁₉₇ in 30 times molar excess, slowly and drop-wise with gentle stirring agitation. The activated CRM₁₉₇ was then transferred to a rocking platform, and reacted for 60 minutes at room temperature. Ten 10DG desalting columns were equilibrated with 25 mL of Dulbecco's PBS. The activated CRM₁₉₇ sample was diluted to 30 mL with Dulbecco's PBS and 3 mL of the activated CRM₁₉₇ was added into each of the ten 10DG columns. The samples were eluted from each de-salting columns in 4 mL of Dulbecco's PBS, then pooled into one reaction vessel. The activated CRM was then divided into 2.7 mL aliquots in sterile bijous, each equipped with micro-magnetic fleas.

***Conjugation of peptides to activated CRM₁₉₇:*** Peptides were conjugated to CRM₁₉₇ via an N-terminal or C-terminal cysteine residue which may or may not adjoin two non native glycine residues to aid flexibility of the peptide, and better presentation of the epitope(s) (shown in bold in Table 1).

Approximately 3 mg of each peptide was solubilised with DMSO to a final concentration of 10 mg/ mL. Subsequently, 300 µL of the peptide was added with gentle agitation on a stirring platform, into a bijou containing 2.7 mL CRM₁₉₇. All conjugation reactions were then transferred to a rocking platform for 3 hrs at room temperature. Following conjugation, each 3 mL reaction was desalted using a 10DG desalting column (pre-equilibrated with 25 mL of Dulbecco's PBS) and eluted in 4 mL of Dulbecco's PBS to the column.

***Final sample preparation and characterisation:*** Each sample was sterile filtered using 0.22 µm sterile Millex-GV syringe filters. Protein concentration was determined by BCA, in duplicate, using 1 in 4 dilutions. An SDS-PAGE was performed against standard CRM₁₉₇, to monitor shift in conjugate compared to standard. The endotoxin level was measured using the Charles River Endosafe reader with the samples loaded at a 1 in 40 dilution, using endo-free water as the sample diluent. The remaining 3.5 mL of each conjugate was stored at 2-8C until *in vivo* administration.

**Table 2- CRM₁₉₇-Peptide Conjugates:**

| **Conjugate** | **Peptide ID** | **Approx. Recovery of Input CRM₁₉₇** | **Average No. Peptides/ Molecule** |
|---|---|---|---|
| **CRM-9.24** | 9.24 (Sequence ID NO.379) | 70% | 11.0 |
| **CRM-9.58** | 9.58 (Sequence ID NO.527) | 75% | 12.0 |
| **CRM-9.27** | 9.27 (Sequence ID NO. 86) | 90% | 11.5 |
| **CRM-9.56** | 9.56 (Sequence ID NO. 224) | 75% | 11.9 |
| **CRM-9.28** | 9.28 (Sequence ID NO. 99) | 75% | 11.4 |
| **CRM-9.57** | 9.57 (Sequence ID NO. 243) | 75% | 12.1 |

### Example 4: Mouse & human specific PCSK9 peptide immunogenicity

This study aimed to evaluate how effective peptides conjugated to CRM₁₉₇ (as detailed in Example 3, above) were in inducing an antibody response that can bind to human and/ or mouse PCSK9 and reduce serum cholesterol in the mouse. Female C57BI/6 (6-8 weeks) were injected by the intramuscular route (50 µL volume injected into each Tibialis anterior muscle) on days 0, 28 and 56 with CRM₁₉₇-peptide conjugates formulated in Alum with CpG of formula 5' TCGTCGTTTTTCGGTGCTTTT 3'. One group of control mice was immunized with unconjugated CRM₁₉₇ following the same protocol. Necropsy took place on day 63. At necropsy 400 - 600 µL blood was sampled from euthanised mice by cardiac puncture using an anti-coagulant. Blood was centrifuged to separate the serum, which was stored frozen until testing.

IgG antibody responses to full length human and mouse recombinant PCSK9 protein, human and mouse PCSK9 peptides and human and mouse PCSK9 phospho-peptides were measured using a colorimetric ELISA method. Serial dilutions were prepared from sera samples and tested in the assay.

**ELISA method:** 384-well high bind assay plates (VWR-Greiner bio-one Cat#82051-264) were coated with 25µL/ well of 2.5ug/ mL mouse PCSK9 protein, 1.0ug/ mL human PCSK9 protein or 0.3ug/ mL peptide (9.24, 9.27, 9.28, 9.56, 9.57 or 9.58), as appropriate, and incubated overnight at 4°C. The protein and peptides were diluted to the final concentration with 0.01M PBS pH 7.4. Plates were blocked using 25µL/ well of 1%BSA/ 1xPBS-Tween (0.01M PBS pH 7.4/0.05% Tween 20) and incubated shaking at 600 rpm RT for 1 hour. An 8 point ½ log serial dilution of each sample was prepared starting at 1:100 dilution (1xPBS-Tween diluent), 25µL/ well of the serial dilution transferred in duplicate into the peptide coated plate then incubated shaking at 600 rpm RT for 1 hour. After washing x 3-5 with 1xPBS-Tween, 25µL/ well of Total IgG detection antibody (Goat anti-mouse pAb IgG HRP, Cat# ab20043 Abcam) at a 1:30,000 dilution with 1xPBS-Tween was added, then incubated shaking at 600 rpm RT for 1 hour. After washing x3-5 with 1xPBS-Tween, 25µL/ well of TMB Peroxidase EIA-Substrate (solution A+B) (Bio-Rad Cat#172-1067) was added and the plates were incubated at RT for 30mins. The colorimetric reaction was stopped by addition of 25µL/ well 1N Sulfuric acid and the absorbance then read at 450nm. Titration curves were plotted for each test sample (sample dilution vs absorbance). The sample titer (subsequently transformed into reciprocal titer) was then taken as the serum dilution achieving an optical density (O.D.) values of 1.0. Negative samples were assigned a reciprocal titer of 100.

**Measurement of serum cholesterol level:** Cholesterol levels in serum samples were measured using a WAKO Cholesterol E Assay kit (Cat# 439-17501) following the manufacturers' instructions. Dilutions of cholesterol standard or test serum samples (4µL volume) were added to wells of a 96-well plate and 196µL of prepared cholesterol reagent added. The plate was incubated for 5 minutes at 37°C and the absorbance of the developed colour read at 600nm within 30 minutes.

**Measurement of serum murine PCSK9 level:** Murine PCSK9 protein levels in mouse serum samples were measured using a R&D Quantikine Mouse PCSK9 serum level kit (Cat# MPC900) following the manufacturers' instructions. 50 µL/ well of Assay Diluent RD1-21 was added, then 50 µL of either standard, control, or sample was added to each well. Mouse serum was diluted to 1:200 in Calibrator Diluant (RD5-26). The plates were incubated for 2 hours at room temperature, then washed x 5 with 250 µL wash buffer. 100 µL/ well of Mouse PCSK9 Conjugate was added and the plates were incubated for 2 hours at room temperature, followed by washing x 5 with 250 µL wash buffer. 100 µL/ well of Substrate Solution was added and the plates were incubated at room temperature for 30min while protected from light. The colorimetric reaction was stopped by addition of 100 µL/ well of Stop Solution. The absorbance was read at 450 nm and 540 nm within 30 minutes. For each well, the 540 nm optical density was subtracted from the 450 nm optical density.

**Results:** As shown in Figure 1 and Table 3, human specific pro-domain peptide immunogens were able to induce antibody responses to the recombinant full-length human PCSK9 protein that were also cross reactive with phosphorylated and non-phosphorylated versions of the immunising peptide, some inducing higher responses than others. Similarly, mouse specific pro-domain (Figure 2 and Table 3) and C-terminal domain peptide immunogens (Figure 3) were able to induce antibody responses to the recombinant full-length mouse PCSK9 protein that were also cross reactive with phosphorylated and non-phosphorylated versions of the immunising peptide, some inducing higher responses than others. Figure 4 shows that peptide 9.57 immunization also leads to a reduction in total cholesterol levels in serum. Figure 5 shows that peptide immunogens differentially modulate serum PCSK9 levels in the mouse.

**Table 3: Serum Phospho-Selectivity:**

| | **Peptide 9.27** | | **Peptide 9.28** | | **Peptide 9.56** | | **Peptide 9.57** | | **9.56/9.27** | | **9.57/9.28** | |
|---|---|---|---|---|---|---|---|---|---|---|---|---|
| **Treatment** | **Avg** | **StDev** | **Avg** | **StDev** | **Avg** | **StDev** | **Avg** | **StDev** | **Avg** | **StDev** | **Avg** | **StDev** |
| **9.56** | 3.49E+02 | 5.75E+02 | 4.80E+02 | 1.21E+03 | 4.18E+04 | 3.20E+04 | 4.02E+02 | 6.43E+02 | 292.30 | 290.47 | 2.64 | 5.41 |
| **9.57** | 8.27E+02 | 2.27E+03 | 4.28E+03 | 6.59E+03 | 1.35E+03 | 2.75E+03 | 6.56E+04 | 2.35E+04 | 5.07 | 12.23 | 65.37 | 60.61 |

| | **Peptide 9.24** | | | | **Peptide 9.58** | | | | **9.58/9.24** | | | |
|---|---|---|---|---|---|---|---|---|---|---|---|---|
| **Treatment** | **Avg** | | **StDev** | | **Avg** | | **StDev** | | **Avg** | | **StDev** | |
| **9.58** | 7.38E+03 | | 8.62E+03 | | 7.55E+04 | | 2.52E+04 | | 17.29 | | 12.06 | |

### Example 5: Serum recognition of in vitro phosphorylated recombinant human PCSK9

The recombinant human PCSK9 used in ELISA experiments described in Example 4 is predominantly unphosphorylated. To investigate the ability of vaccine induced IgG antibodies to recognise human PCSK9 in its phosphorylated state, recombinant full-length human PCSK9 (with C-terminal FLAG) was treated with 5000 units of casein kinase II (New England Bioscience) in the presence of 200uM ATP and 1x CK2 reaction buffer (20mM Tris-HCl, 50mM KCl, 10mM MgCl2, pH 7.5) for 1 hour at RT. The reaction was performed with mild agitation on a rocking platform. After 1 hour, a 100µL sample was desalted using a 0.5ml Zeba spin desalt column and eluted in 100µL Dulbecco's PBS. Expected function of the in vitro phosphorylated PCSK9 was confirmed by ELISA measurement of PCSK9 binding to LDL receptor. Serum binding to in vitro phosphorylated human PCSK9 was determined using MSD assay format.

**Human PCSK9:LDLr Interaction ELISA:** 96-well assay plate (Costar EIA, Bio-Rad, Cat# 224-0096) was coated with 50 µL/well of 6 µg/mL of anti-Flag mAb (Sigma, cat# F1804) in PBS pH 7.4 overnight at 4°C. The plate was washed with PBST (PBS-0.05%Tween-20) and blocked with 300 µL/ well of 1%BSA-PBS for 1 hour at room temperature. After washing three times with PBST, 50µL/ well of 5 µg/ml Flag-PCSK9 (either untreated or in vitro phosphorylated) in 1% BSA-PBS was added to the plate and incubated with gentle shaking for 1 hour at room temperature. The plate was washed three times with PBST and 50µL/ well of His-LDLrECD (R&D Sytems, cat # 2148 LD/CF)) two fold serially diluted in interaction buffer (20 mM Hepes, 100 mM NaCl, 0.1 mM CaCl2, 0.2% (w/v) BSA, pH 7.1) with starting concentration at 8 µg/ mL was added to the plate in duplicate and incubated with gentle shaking for 1 hour at room temperature. The plate was washed three times with PBST and 50µL/ well of anti-His-HRP detection antibody (Invitrogen, cat# R931-25, diluted 1:1,000 in 1% BSA-PBS) was added to the plate followed by 1 hour incubation with gentle shaking at room temperature. After washing three times with PBST, 100 µL/ well of TMB substrate (Sigma, cat # T-4444) was added and the plate was incubated at RT for 15 minutes. The colorimetric reaction was stopped by addition of 2N Sulfuric acid (100µL/ well) and the absorbance was read at 450nm. Dose response curves were plotted for untreated and in vitro-phosphorylated PCSK9 (LDLr concentration vs absorbance).

**Serum binding to untreated and in vitro phosphorylated human PCSK9:** A 96-well MSD plate was incubated overnight at 4°C with 25uL/ well of untreated or in vitro phosphorylated human PCSK9 at 2.5µg/ mL in PBS. Plate was washed three times with PBS (200 uL/ well), then incubated with Pierce Starting Blocking Buffer (100 uL/ well) for 1 hour at room temperature on a shaking platform. Plate was washed three times with MSD Wash Buffer (100 uL/ well). Four serum pools from 10 mice vaccinated with 9.27, 9.28, 9.56 and 9.57 were prepared. A 10 point ½ log serial dilution of each pool was prepared starting at 1:100 dilution and 25µL/ well of each dilution was added to the plate (in duplicate). Plate was sealed and incubated at room temperature, shaking for 2 hours then washed three times with PBS/ 0.05%Tween20 (200µL/ well). SULFO-tagged goat anti-mouse IgG secondary Ab was diluted 1 in 4000µL in PBS/ 0.05%Tween20 and 25µL/ well was added to the 96-well assay plate. Plate was sealed and transferred onto a shaking platform for 1 hour at room temperature, then washed three times with PBS/ 0.05%Tween20 (200µL/ well). MSD Read buffer (x4) was diluted 1 in 2 with ultrapure water and 150µL/ well was added to the plate before reading on an MSD 6000.

**Results:** As shown in Figure 6, in vitro phosphorylated recombinant human PCSK9 binds LDLr extracellular domain in a similar manner to untreated recombinant human PCSK9. Human specific prodomain peptide immunogens induced antibody responses capable of binding the recombinant full-length human PCSK9 protein (Figure 7). Antibodies induced by peptide 9.27 and 9.56 were cross reactive with untreated and in vitro phosphorylated recombinant human PCSK9. Peptide 9.27 induced antibodies that preferentially bound untreated recombinant human PCSK9. Peptide 9.56 induced antibodies that preferentially bound in vitro phosphorylated recombinant human PCSK9.

### Example 6: Immunogenicity of human and mouse specific phosphorylated prodomain peptides

This study aimed to evaluate how effective shorter versions of peptides 9.56 and 9.57, when conjugated to CRM₁₉₇ (as detailed in Example 3, above), were in inducing an antibody response that can bind to human and/ or mouse PCSK9 as well as human or mouse PCSK9 peptides and reduce serum cholesterol in the mouse. Unconjugated CRM₁₉₇ was used as a control. Female C57BI/6 (8-10 weeks) were injected by the intramuscular route (50 µL volume injected into each Tibialis anterior muscle) on days 0, 28 and 56 with CRM₁₉₇-peptide conjugates formulated in Alum with CpG of formula 5' TCGTCGTTTTTCGGTGCTTTT 3'. One group of control mice was immunized with unconjugated CRM₁₉₇ following the same protocol. One group of control mice received no vaccination. Necropsy took place on day 70. At necropsy 400 - 600 µL blood was sampled from euthanised mice by cardiac puncture using an anti-coagulant. Blood was centrifuged to separate the serum, which was stored frozen until testing.

For each peptide, IgG antibody responses to some of the following were measured using a colorimetric ELISA method: full length human recombinant phospho-PCSK9 protein, full length mouse recombinant phospho-PCSK9 protein, human PCSK9 peptide 9.27, human PCSK9 phospho-peptide 9.56, mouse PCSK9 peptide 9.28 and mouse PCSK9 phospho-peptide 9.57. Serial dilutions were prepared from sera samples and tested in the assay.

**ELISA method:** The ELISAs were performed as per example 4 with the following changes: the high bind assay plates were coated with 25µL/ well of 2.5ug/ mL mouse phospho-PCSK9 protein, 1.0ug/ mL human phospho-PCSK9 protein or 0.3ug/ mL of a peptide (9.27, 9.28, 9.56, 9.57), as appropriate. Mouse and human PCSK9 were phosphorylated as per example 5.

**Measurement of serum cholesterol level:** Cholesterol levels in serum samples were measured as per example 4.

**Results:** As shown in Figure 8 (part 1 and 2) and Table 4, phosphorylated human pro-domain peptide immunogens were able to induce antibody responses to the phosphorylated recombinant full-length human PCSK9 protein that were also cross reactive with the 9.56 phosphorylated peptide but, for most immunogens, not to the non-phosphorylated 9.27 peptide. As shown in Figure 9 and Table 5, phosphorylated mouse specific pro-domain peptide immunogens were able to induce antibody responses to the phosphorylated recombinant full-length mouse PCSK9 protein that were also cross reactive with the 9.57 phosphorylated peptide. Some were cross reactive with the non-phosphorylated 9.28 peptide. Some immunogens induced higher responses than others. These results indicate that some peptides, when used as immunogens, elicit an antibody response with greater phospho-selectivity than do others. These results also indicate that the position of the CGG linker on the N- or C- terminus of the peptide can influence the resulting antibody response when these peptides are used as immunogens. Figure 10 shows post immunization reduction in serum cholesterol levels compared with unvaccinated control animals. Some immunogens induced greater reductions in cholesterol than others.

**Table 4: Serum Phospho-Selectivity**

| | **Peptide 9.27** | | **Peptide 9.56** | | **9.56/9.27** | |
|---|---|---|---|---|---|---|
| **Treatment** | **Avg** | **StDev** | **Avg** | **StDev** | **Avg** | **StDev** |
| **9.157** | < 1.00E+02 | 0.00E+00 | 2.62E+04 | 4.27E+04 | > 265.1 | 431.4 |
| **9.158** | < 1.01E+02 | 4.38E+00 | < 9.12E+02 | 9.31E+02 | > 8.8 | 8.6 |
| **9.159** | < 1.00E+02 | 0.00E+00 | < 1.00E+02 | 0.00E+00 | 1.0 | 0.0 |
| **9.160** | < 1.00E+02 | 0.00E+00 | < 5.79E+03 | 6.59E+03 | > 58.4 | 66.6 |
| **9.161** | < 1.00E+02 | 0.00E+00 | < 2.21E+03 | 2.29E+03 | > 22.4 | 23.1 |
| **9.162** | < 1.00E+02 | 0.00E+00 | < 6.14E+02 | 6.02E+02 | > 6.2 | 6.1 |
| **9.163** | < 1.00E+02 | 0.00E+00 | < 1.62E+03 | 1.72E+03 | > 16.4 | 17.4 |
| **9.164** | < 1.00E+02 | 0.00E+00 | < 1.24E+02 | 6.15E+01 | > 1.25 | 0.6 |
| **9.165** | < 1.00E+02 | 0.00E+00 | < 1.43E+02 | 6.06E+01 | > 1.44 | 0.6 |
| **9.166** | < 1.00E+02 | 0.00E+00 | < 4.68E+02 | 5.82E+02 | > 4.73 | 5.9 |
| **9.167** | < 1.00E+02 | 0.00E+00 | < 6.81E+02 | 1.43E+03 | > 6.88 | 14.4 |
| **9.181** | < 1.00E+02 | 0.00E+00 | 1.86E+03 | 2.08E+03 | > 18.8 | 21.0 |
| **9.182** | < 1.00E+02 | 0.00E+00 | < 1.94E+02 | 2.28E+02 | > 1.96 | 2.3 |
| **9.183** | < 1.00E+02 | 0.00E+00 | < 4.62E+03 | 9.77E+03 | > 46.7 | 98.7 |
| **9.56** | < 2.58E+03 | 2.45E+03 | 4.60E+04 | 5.62E+04 | > 69.7 | 98.0 |
| **9.27** | 2.08E+04 | 4.74E+04 | < 5.09E+03 | 7.09E+03 | 0.7 | 0.5 |
| **CRM** | < 1.00E+02 | 0.00E+00 | < 1.00E+02 | 0.00E+00 | 1.0 | 0.0 |

**Table 5: Serum Phospho-Selectivity**

| | **Peptide 9.28** | | **Peptide 9.57** | | **9.57/9.28** | |
|---|---|---|---|---|---|---|
| **Treatment** | **Avg** | **StDev** | **Avg** | **StDev** | **Avg** | **StDev** |
| **9.28** | 1.41E+04 | 1.30E+04 | 7.02E+03 | 7.56E+03 | 0.5 | 0.4 |
| **9.57** | < 2.10E+03 | 3.20E+03 | 5.14E+04 | 2.09E+04 | > 204.7 | 200.0 |
| **9.171** | < 1.04E+02 | 1.43E+01 | 3.12E+04 | 4.26E+04 | > 309.3 | 433.4 |
| **9.176** | < 1.00E+02 | 0.00E+00 | 1.07E+04 | 1.70E+04 | > 108.3 | 171.9 |
| **CRM** | < 1.00E+02 | 0.00E+00 | < 1.00E+02 | 0.00E+00 | 1.0 | 0.0 |

### Example 7: Immunogenicity of human specific prodomain peptides in Cynomologus macaque

This study aimed to evaluate the ability of human/ cynomolgus PCSK9 peptides (100% homologous between these two species) conjugated to CRM₁₉₇ (as detailed in Example 2 above) to induce antibody responses that can bind to human and/or cynomologus PCSK9 and affect its function in this species . Male and female animals were injected by the intramuscular route on days 0, 28 and 84 of the study with CRM₁₉₇-peptide conjugates (prepared as described in example 3) formulated in Alum with CpG. One group of control animals was immunized with CRM₁₉₇ conjugated to a non-PCSK9 peptide following the same protocol. Blood was sampled from animals at regular intervals before, during and after the vaccination schedule by venous puncture using an anti-coagulant. Blood was centrifuged to separate the serum, which was stored frozen until testing.

IgG antibody responses to full length recombinant Cynomologus PCSK9 protein were measured using a colorimetric ELISA method. Serum PCSK9 protein levels are measured using ELISA methods.

**ELISA method:** The ELISAs were performed as per example 4 with the following changes: the high bind assay plates were coated with 25 µL/ well of 1.0 ug/ mL in vitro phosphorylated Cynomolgus phospho-PCSK9 protein, 1.0 ug/ mL Cynomolgus PCSK9 protein or 0.3 ug/ mL of a peptide (9.27 9.56), as appropriate; 25 µL/ well of mouse antiHuman IgG HRP, Cat# 9042-05, Southern Biotech) at a 1:5,000 dilution with 1xPBS-Tween was used as the secondary antibody. Cynomolgus PCSK9 were phosphorylated as per example 5.

### hPCSK9:LDLr interaction assay:

96-well assay plates (Costar EIA, Bio-Rad, Cat# 224-0096) were coated with 50 µL/well of 6 µg/mL of anti-Flag mAb (Sigma, cat# F1804) in PBS pH 7.4 overnight at 4oC. Plates were washed with PBST (PBS-0.01%Tween-20) and blocked with 300 µL/well of 1% BSA-PBS for 1 hour at room temperature. After washing three times with PBST, 50 µl/well of 5 µg/ml human Flag-PCSK9 (either phosphorylated or non-phosphorylated as described in Example5) in 1% BSA-PBS was added to the plates and incubated with gentle shaking for 1 hour at room temperature. After this incubation plates were washed three times with PBST. A 1:4 dilution of each Cynomolgus serum sample or a pooled group sample was prepared in 1% BSA-PBS; 50 µL/well of the sample was transferred in duplicate into the plates with flag-captured PCSK9. Any unbound serum was removed by washing the plates three times with PBST. Then 50 µL/well of 0.8 µg/ml human His-LDLrECD (R&D Sytems, cat # 2148 LD/CF)) diluted in interaction buffer (20 mM Hepes, 100 mM NaCl, 0.1 mM CaCl2, 0.2% (w/v) BSA, pH 7.1) was added to the plates and incubated with gentle shaking for 1 hour at room temperature. Plates were washed three times with PBST and 50 µL/well of anti-His-HRP detection antibody (Invitrogen, cat# R931-25, diluted 1:1,000 in 1% BSA-PBS) was added to the plates followed by 1 hour incubation with gentle shaking at room temperature. After washing three times with PBST, 100 µL/well of TMB substrate (Sigma, cat # T-4444) was added and the plates were incubated at room temperature for 15 minutes. The colorimetric reaction was stopped by addition of 2N Sulfuric acid (100µL/well) and the absorbance was read at 450nm. Data was plotted with GraphPad Prism software.

### Measurement of Total and Free PCSK9 in Cynomolgus serum:

Levels of free Cynomolgus PCSK9 in serum were assessed by separating the anti-PCSK9 Ab: PCSK9 complexes from unbound free PCSK9 using Protein A. Serum samples diluted 1:5 in PBS were incubated with Protein A in a 96-well plate (GE Healthcare Protein A HP MultiTrap cat# 28-9031-33) overnight at 4°C. The following day, the plate was centrifuged at 100g for 2min and the flow-through was collected. The amount of free PCSK9 in the flow-through was determined as described below.

Cynomolgus PCSK9 protein levels in serum samples were measured using a R&D Quantikine Human PCSK9 serum level kit (Cat# DPC900) following the manufacturers' instructions. 100 µL/ well of Assay Diluent RD1-9 was added, then 50 µL of either standard, control, or sample was added to each well. Cynomolgus serum diluted in PBS and flow-through from Protein A plate were diluted to 1:5 in Calibrator Diluent (RD5P) to reach a 1:20 final dilution. The plates were incubated for 2h at RT, then washed x 4 with 400 µL wash buffer. 200 µL/ well of Human PCSK9 Conjugate was added and the plates were incubated for 2h at RT, followed by washing x 4 with 400 µL wash buffer. 200 µL/ well of Substrate Solution was added and the plates were incubated for 30min at RT while protected from light. The colorimetric reaction was stopped by addition of 50 µL/ well of Stop Solution. The absorbance was read at 450 nm and 540 nm within 30 minutes. For each well, the 540 nm optical density was subtracted from the 450 nm optical density. PCSK9 levels were determined, the ratio post protein A (free)/ pre-protein A (total) was determined and expressed as % recovery free PCSK9. **Results:** As shown in Figures 11 (Day 42) and 12 (Day 99) and Tables 6 and 7 (Day 99), human specific pro-domain peptide immunogens were able to induce antibody responses to in vitro phosphorylated recombinant full-length Cynomolgus PCSK9 protein with cross reactivity to peptide the phosphorylated peptide 9.56. Some Antibody responses were cross reactive with recombinant full-length Cynomolgus PCSK9 protein and the non-phosphorylated 9.28 peptide. Some immunogens induced higher responses than others. The negative control, CRM₁₉₇ conjugated to a non-PCSK9 peptide, did not induce titers. The responses over time of 10 and 50 ug doses of 9.27, 9.56 and 9.160-CRM₁₉₇ conjugates are shown in Figure 13. These results indicate that some peptides, when used as immunogens, elicit an antibody response with greater phospho-selectivity than do others. As shown in Figures 14 and 15 human specific pro-domain peptide immunogens were able to induce antibody responses capable of affecting the ability of human PCSK9 to bind the human LDLr. Dependent upon the phosphorylation state of the protein, antibodies were able to inhibit or enhance PCSK9 binding to the LDLr as measured in this assay. Total serum PCSK9 levels at pre-bleed and 2 weeks following third vaccination with PCSK9 derived peptides are shown in Figure 16. As shown in Figure 17, the level of free PCSK9 in serum was decreased in animals vaccinated with pro-domain peptides to varying levels depending on the immunogen. Free PCSK9 levels did not change following immunization with the control vaccine. Figure 17 shows the level of free PCSK9 over the course of the study in animals vaccinated with a 10ug dose of 9.56 peptide. Free PCSK9 levels in serum decreased following the first and second boosts in the immunization schedule.

**Table 6: Day 99 Serum Phospho-Selectivity**

| | **Peptide 9.27** | | **Peptide 9.56** | | **9.56/9.27** | |
|---|---|---|---|---|---|---|
| **Treatment** | **Avq** | **StDev** | **Avq** | **StDev** | **Avg** | **StDev** |
| **9.27** | 8.14E+03 | 4.60E+03 | 7.37E+03 | 3.96E+03 | 1.0 | 0.3 |
| **9.56 (10 ug)** | 9.20E+03 | 6.27E+03 | 1.47E+04 | 1.23E+04 | 1.5 | 0.3 |
| **9.56 (50 ug)** | < 3.37E+03 | 3.62E+03 | 6.23E+03 | 4.77E+03 | > 3.3 | 2.0 |
| **9.16** | < 1.00E+02 | 0.00E+00 | 2.59E+03 | 2.36E+03 | > 26.2 | 23.8 |
| **Negative** | < 1.00E+02 | 0.00E+00 | < 1.05E+02 | 1.10E+01 | > 1.1 | 0.1 |

**Table 7: Day 99 Serum Phospho-Selectivity**

| | **cPCSK9** | | **p-cPCKS9** | | **cPCSP9/p-cPCKS9** | |
|---|---|---|---|---|---|---|
| **Treatment** | **Avg** | **StDev** | **Avg** | **StDev** | **Avg** | **StDev** |
| **9.27** | 1.32E+03 | 1.08E+03 | < 1.65E+03 | 1.46E+03 | 2.0 | 1.9 |
| **9.56 (10 ug)** | < 1.59E+03 | 1.72E+03 | 7.40E+03 | 5.48E+03 | > 11.1 | 7.4 |
| **9.56 (50 ug)** | < 1.01E+03 | 1.62E+03 | < 5.33E+03 | 5.62E+03 | > 10 | 13.4 |
| **9.16** | < 1.00E+02 | 0.00E+00 | < 3.30E+02 | 3.68E+02 | > 3.3 | 3.7 |
| **Negative** | < 1.00E+02 | 0.00E+00 | < 1.00E+02 | 0.00E+00 | 1.0 | 0.0 |

**Table 8: Sequence Listing**

| **ID** | **Species** | **Description** | **Modification** | **Linker** | Sequence |
|---|---|---|---|---|---|
| SEQ ID No: 1 | Human | PCSK9 | N/A | | |
| SEQ ID No: 2 | Mouse | PCSK9 | N/A | | |
| SEQ ID No: 3 | Human | PCSK9 ProDomain | N/A | | |
| SEQ ID No: 4 | Human | ProDomain Peptide | N/A | | GDYEELVLALRSEEDG |
| SEQ ID No: 5 | Human | ProDomain Peptide | N/A | | LVLALRSEEDG |
| SEQ ID No: 6 | Human | ProDomain Peptide | N/A | | VLALRSEEDG |
| SEQ ID No: 7 | Human | ProDomain Peptide | N/A | | LALRSEEDG |
| SEQ ID No: 8 | Human | ProDomain Peptide | N/A | | ALRSEEDG |
| SEQ ID No: 9 | Human | ProDomain Peptide | N/A | | LRSEEDG |
| SEQ ID No: 10 | Human | ProDomain Peptide | N/A | | RSEEDG |
| SEQ ID No: 11 | Human | ProDomain Peptide | N/A | | SEEDG |
| SEQ ID No: 12 | Human | ProDomain Peptide | N/A | | LVLALRSEED |
| SEQ ID No: 13 | Human | ProDomain Peptide | N/A | | VLALRSEED |
| SEQ ID No: 14 | Human | ProDomain Peptide | N/A | | LALRSEED |
| SEQ ID No: 15 | Human | ProDomain Peptide | N/A | | ALRSEED |
| SEQ ID No: 16 | Human | ProDomain Peptide | N/A | | LRSEED |
| SEQ ID No: 17 | Human | ProDomain Peptide | N/A | | RSEED |
| SEQ ID No: 18 | Human | ProDomain Peptide | N/A | | LVLALRSEE |
| SEQ ID No: 19 | Human | ProDomain Peptide | N/A | | VLALRSEE |
| SEQ ID No: 20 | Human | ProDomain Peptide | N/A | | LALRSEE |
| SEQ ID No: 21 | Human | ProDomain Peptide | N/A | | ALRSEE |
| SEQ ID No: 22 | Human | ProDomain Peptide | N/A | | LRSEE |
| SEQ ID No: 23 | Human | ProDomain Peptide | N/A | | LVLALRSE |
| SEQ ID No: 24 | Human | ProDomain Peptide | N/A | | VLALRSE |
| SEQ ID No: 25 | Human | ProDomain Peptide | N/A | | LALRSE |
| SEQ ID No: 26 | Human | ProDomain Peptide | N/A | | ALRSE |
| SEQ ID No: 27 | Human | ProDomain Peptide | N/A | | LVLALRS |
| SEQ ID No: 28 | Human | ProDomain Peptide | N/A | | VLALRS |
| SEQ ID No: 29 | Human | ProDomain Peptide | N/A | | LALRS |
| SEQ ID No: 30 | Mouse | ProDomain Peptide | N/A | | LMLALPSQEDG |
| SEQ ID No: 31 | Mouse | ProDomain Peptide | N/A | | MLALPSQEDG |
| SEQ ID No: 32 | Mouse | ProDomain Peptide | N/A | | LALPSQEDG |
| SEQ ID No: 33 | Mouse | ProDomain Peptide | N/A | | ALPSQEDG |
| SEQ ID No: 34 | Mouse | ProDomain Peptide | N/A | | LPSQEDG |
| SEQ ID No: 35 | Mouse | ProDomain Peptide | N/A | | PSQEDG |
| SEQ ID No: 36 | Mouse | ProDomain Peptide | N/A | | SQEDG |
| SEQ ID No: 37 | Mouse | ProDomain Peptide | N/A | | LMLALPSQED |
| SEQ ID No: 38 | Mouse | ProDomain Peptide | N/A | | MLALPSQED |
| SEQ ID No: 39 | Mouse | ProDomain Peptide | N/A | | LALPSQED |
| SEQ ID No: 40 | Mouse | ProDomain Peptide | N/A | | ALPSQED |
| SEQ ID No: 41 | Mouse | ProDomain Peptide | N/A | | LPSQED |
| SEQ ID No: 42 | Mouse | ProDomain Peptide | N/A | | PSQED |
| SEQ ID No: 43 | Mouse | ProDomain Peptide | N/A | | LMLALPSQE |
| SEQ ID No: 44 | Mouse | ProDomain Peptide | N/A | | MLALPSQE |
| SEQ ID No: 45 | Mouse | ProDomain Peptide | N/A | | LALPSQE |
| SEQ ID No: 46 | Mouse | ProDomain Peptide | N/A | | ALPSQE |
| SEQ ID No: 47 | Mouse | ProDomain Peptide | N/A | | LPSQE |
| SEQ ID No: 48 | Mouse | ProDomain Peptide | N/A | | LMLALPSQ |
| SEQ ID No: 49 | Mouse | ProDomain Peptide | N/A | | MLALPSQ |
| SEQ ID No: 50 | Mouse | ProDomain Peptide | N/A | | LALPSQ |
| SEQ ID No: 51 | Mouse | ProDomain Peptide | N/A | | ALPSQ |
| SEQ ID No: 52 | Mouse | ProDomain Peptide | N/A | | LMLALPS |
| SEQ ID No: 53 | Mouse | ProDomain Peptide | N/A | | MLALPS |
| SEQ ID No: 54 | Mouse | ProDomain Peptide | N/A | | LALPS |
| SEQ ID No: 55 | Human | ProDomain Peptide + Linker | N/A | N: CGG | CGGLVLALRSEEDG |
| SEQ ID No: 56 | Human | ProDomain Peptide + Linker | N/A | N: CGG | CGGVLALRSEEDG |
| SEQ ID No: 57 | Human | ProDomain Peptide + Linker | N/A | N: CGG | CGGLALRSEEDG |
| SEQ ID No: 58 | Human | ProDomain Peptide + Linker | N/A | N: CGG | CGGALRSEEDG |
| SEQ ID No: 59 | Human | ProDomain Peptide + Linker | N/A | N: CGG | CGGLRSEEDG |
| SEQ ID No: 60 | Human | ProDomain Peptide + Linker | N/A | N: CGG | CGGRSEEDG |
| SEQ ID No: 61 | Human | ProDomain Peptide + Linker | N/A | N: CGG | CGGSEEDG |
| SEQ ID No: 62 | Human | ProDomain Peptide + Linker | N/A | N: CGG | CGG LVLALRSEED |
| SEQ ID No: 63 | Human | ProDomain Peptide + Linker | N/A | N: CGG | CGGVLALRSEED |
| SEQ ID No: 64 | Human | ProDomain Peptide + Linker | N/A | N: CGG | CGG LALRSEED |
| SEQ ID No: 65 | Human | ProDomain Peptide + Linker | N/A | N: CGG | CGG ALRSEED |
| SEQ ID No: 66 | Human | ProDomain Peptide + Linker | N/A | N: CGG | CGGLRSEED |
| SEQ ID No: 67 | Human | ProDomain Peptide + Linker | N/A | N: CGG | CGG RSEED |
| SEQ ID No: 68 | Human | ProDomain Peptide + Linker | N/A | N: CGG | CGGLVLALRSEE |
| SEQ ID No: 69 | Human | ProDomain Peptide + Linker | N/A | N: CGG | CGGVLALRSEE |
| SEQ ID No: 70 | Human | ProDomain Peptide + Linker | N/A | N: CGG | CGGLALRSEE |
| SEQ ID No: 71 | Human | ProDomain Peptide + Linker | N/A | N: CGG | CGGALRSEE |
| SEQ ID No: 72 | Human | ProDomain Peptide + Linker | N/A | N: CGG | CGGLRSEE |
| SEQ ID No: 73 | Human | ProDomain Peptide + Linker | N/A | N: CGG | CGGLVLALRSE |
| SEQ ID No: 74 | Human | ProDomain Peptide + Linker | N/A | N: CGG | CGG VLALRSE |
| SEQ ID No: 75 | Human | ProDomain Peptide + Linker | N/A | N: CGG | CGG LALRSE |
| SEQ ID No: 76 | Human | ProDomain Peptide + Linker | N/A | N: CGG | CGGALRSE |
| SEQ ID No: 77 | Human | ProDomain Peptide + Linker | N/A | N: CGG | CGGLVLALRS |
| SEQ ID No: 78 | Human | ProDomain Peptide + Linker | N/A | N: CGG | CGGVLALRS |
| SEQ ID No: 79 | Human | ProDomain Peptide + Linker | N/A | N: CGG | CGGLALRS |
| SEQ ID No: 80 | Human | ProDomain Peptide + Linker | N/A | C: GC/ GGC | VLALRSEEDGGC |
| SEQ ID No: 81 | Human | ProDomain Peptide + Linker | N/A | C: GC/ GGC | LALRSEEDGGC |
| SEQ ID No: 82 | Human | ProDomain Peptide + Linker | N/A | C: GC/ GGC | ALRSEEDGGC |
| SEQ ID No: 83 | Human | ProDomain Peptide + Linker | N/A | C: GC/ GGC | LRSEEDGGC |
| SEQ ID No: 84 | Human | ProDomain Peptide + Linker | N/A | C: GC/ GGC | RSEEDGGC |
| SEQ ID No: 85 | Human | ProDomain Peptide + Linker | N/A | C: GC/ GGC | SEEDGGC |
| SEQ ID No: 86 | **Human** | **ProDomain Peptide + Linker (9.27)** | **N/A** | **C: GC/ GGC** | **LVLALRSEEDGGC** |
| SEQ ID No: 87 | Human | ProDomain Peptide + Linker | N/A | C: GGC | LVLALRSEEGGC |
| SEQ ID No: 88 | Human | ProDomain Peptide + Linker | N/A | C: GGC | VLALRSEEGGC |
| SEQ ID No: 89 | Human | ProDomain Peptide + Linker | N/A | C: GGC | LALRSEEGGC |
| SEQ ID No: 90 | Human | ProDomain Peptide + Linker | N/A | C: GGC | ALRSEEGGC |
| SEQ ID No: 91 | Human | ProDomain Peptide + Linker | N/A | C: GGC | LRSEEGGC |
| SEQ ID No: 92 | Human | ProDomain Peptide + Linker | N/A | C: GGC | LVLALRSEGGC |
| SEQ ID No: 93 | Human | ProDomain Peptide + Linker | N/A | C: GGC | VLALRSEGGC |
| SEQ ID No: 94 | Human | ProDomain Peptide + Linker | N/A | C: GGC | LALRSEGGC |
| SEQ ID No: 95 | Human | ProDomain Peptide + Linker | N/A | C: GGC | ALRSEGGC |
| SEQ ID No: 96 | Human | ProDomain Peptide + Linker | N/A | C: GGC | LVLALRSGGC |
| SEQ ID No: 97 | Human | ProDomain Peptide + Linker | N/A | C: GGC | VLALRSGGC |
| SEQ ID No: 98 | Human | ProDomain Peptide + Linker | N/A | C: GGC | LALRSGGC |
| SEQ ID No: 99 | **Mouse** | **ProDomain Peptide + Linker (9.28)** | **N/A** | **C: GC/ GGC** | **LMLALPSQEDGGC** |
| SEQ ID No: 100 | Mouse | ProDomain Peptide + Linker | N/A | C: GC/ GGC | MLALPSQEDGGC |
| SEQ ID No: 101 | Mouse | ProDomain Peptide + Linker | N/A | C: GC/ GGC | LALPSQEDGGC |
| SEQ ID No: 102 | Mouse | ProDomain Peptide + Linker | N/A | C: GC/ GGC | ALPSQEDGGC |
| SEQ ID No: 103 | Mouse | ProDomain Peptide + Linker | N/A | C: GC/ GGC | LPSQEDGGC |
| SEQ ID No: 104 | Mouse | ProDomain Peptide + Linker | N/A | C: GC/ GGC | PSQEDGGC |
| SEQ ID No: 105 | Mouse | ProDomain Peptide + Linker | N/A | C: GC/ GGC | SQEDGGC |
| SEQ ID No: 106 | Mouse | ProDomain Peptide + Linker | N/A | C: GGC | LMLALPSQEDGGC |
| SEQ ID No: 107 | Mouse | ProDomain Peptide + Linker | N/A | C: GGC | MLALPSQEDGGC |
| SEQ ID No: 108 | Mouse | ProDomain Peptide + Linker | N/A | C: GGC | LALPSQEDGGC |
| SEQ ID No: 109 | Mouse | ProDomain Peptide + Linker | N/A | C: GGC | ALPSQEDGGC |
| SEQ ID No: 110 | Mouse | ProDomain Peptide + Linker | N/A | C: GGC | LPSQEDGGC |
| SEQ ID No: 111 | Mouse | ProDomain Peptide + Linker | N/A | C: GGC | PSQEDGGC |
| SEQ ID No: 112 | Mouse | ProDomain Peptide + Linker | N/A | C: GGC | LMLALPSQEGGC |
| SEQ ID No: 113 | Mouse | ProDomain Peptide + Linker | N/A | C: GGC | MLALPSQEGGC |
| SEQ ID No: 114 | Mouse | ProDomain Peptide + Linker | N/A | C: GGC | LALPSQEGGC |
| SEQ ID No: 115 | Mouse | ProDomain Peptide + Linker | N/A | C: GGC | ALPSQEGGC |
| SEQ ID No: 116 | Mouse | ProDomain Peptide + Linker | N/A | C: GGC | LPSQEGGC |
| SEQ ID No: 117 | Mouse | ProDomain Peptide + Linker | N/A | C: GGC | LMLALPSQGGC |
| SEQ ID No: 118 | Mouse | ProDomain Peptide + Linker | N/A | C: GGC | MLALPSQGGC |
| SEQ ID No: 119 | Mouse | ProDomain Peptide + Linker | N/A | C: GGC | LALPSQGGC |
| SEQ ID No: 120 | Mouse | ProDomain Peptide + Linker | N/A | C: GGC | ALPSQGGC |
| SEQ ID No: 121 | Mouse | ProDomain Peptide + Linker | N/A | C: GGC | LMLALPSGGC |
| SEQ ID No: 122 | Mouse | ProDomain Peptide + Linker | N/A | C: GGC | MLALPSGGC |
| SEQ ID No: 123 | Mouse | ProDomain Peptide + Linker | N/A | C: GGC | LALPSGGC |
| SEQ ID No: 124 | Mouse | ProDomain Peptide + Linker | N/A | N: CGG | CGGLMLALPSQEDG |
| SEQ ID No: 125 | Mouse | ProDomain Peptide + Linker | N/A | N: CGG | CGGMLALPSQEDG |
| SEQ ID No: 126 | Mouse | ProDomain Peptide + Linker | N/A | N: CGG | CGGLALPSQEDG |
| SEQ ID No: 127 | Mouse | ProDomain Peptide + Linker | N/A | N: CGG | CGGALPSQEDG |
| SEQ ID No: 128 | Mouse | ProDomain Peptide + Linker | N/A | N: CGG | CGGLPSQEDG |
| SEQ ID No: 129 | Mouse | ProDomain Peptide + Linker | N/A | N: CGG | CGGPSQEDG |
| SEQ ID No: 130 | Mouse | ProDomain Peptide + Linker | N/A | N: CGG | CGGSQEDG |
| SEQ ID No: 131 | Mouse | ProDomain Peptide + Linker | N/A | N: CGG | CGG LMLALPSQED |
| SEQ ID No: 132 | Mouse | ProDomain Peptide + Linker | N/A | N: CGG | CGGMLALPSQED |
| SEQ ID No: 133 | Mouse | ProDomain Peptide + Linker | N/A | N: CGG | CGGLALPSQED |
| SEQ ID No: 134 | Mouse | ProDomain Peptide + Linker | N/A | N: CGG | CGGALPSQED |
| SEQ ID No: 135 | Mouse | ProDomain Peptide + Linker | N/A | N: CGG | CGG LPSQED |
| SEQ ID No: 136 | Mouse | ProDomain Peptide + Linker | N/A | N: CGG | CGGPSQED |
| SEQ ID No: 137 | Mouse | ProDomain Peptide + Linker | N/A | N: CGG | CGGLMLALPSQE |
| SEQ ID No: 138 | Mouse | ProDomain Peptide + Linker | N/A | N: CGG | CGGMLALPSQE |
| SEQ ID No: 139 | Mouse | ProDomain Peptide + Linker | N/A | N: CGG | CGG LALPSQE |
| SEQ ID No: 140 | Mouse | ProDomain Peptide + Linker | N/A | N: CGG | CGGALPSQE |
| SEQ ID No: 141 | Mouse | ProDomain Peptide + Linker | N/A | N: CGG | CGGLPSQE |
| SEQ ID No: 142 | Mouse | ProDomain Peptide + Linker | N/A | N: CGG | CGGLMLALPSQ |
| SEQ ID No: 143 | Mouse | ProDomain Peptide + Linker | N/A | N: CGG | CGG MLALPSQ |
| SEQ ID No: 144 | Mouse | ProDomain Peptide + Linker | N/A | N: CGG | CGGLALPSQ |
| SEQ ID No: 145 | Mouse | ProDomain Peptide + Linker | N/A | N: CGG | CGGALPSQ |
| SEQ ID No: 146 | Mouse | ProDomain Peptide + Linker | N/A | N: CGG | CGGLMLALPS |
| SEQ ID No: 147 | Mouse | ProDomain Peptide + Linker | N/A | N: CGG | CGG MLALPS |
| SEQ ID No: 148 | Mouse | ProDomain Peptide + Linker | N/A | N: CGG | CGG LALPS |
| SEQ ID No: 149 | Human | ProDomain PhosphoPeptide | pSer47 | | LVLALRpSEEDG |
| SEQ ID No: 150 | Human | ProDomain PhosphoPeptide | pSer47 | | VLALRpSEEDG |
| SEQ ID No: 151 | Human | ProDomain PhosphoPeptide | pSer47 | | LALRpSEEDG |
| SEQ ID No: 152 | Human | ProDomain PhosphoPeptide | pSer47 | | ALRpSEEDG |
| SEQ ID No: 153 | Human | ProDomain PhosphoPeptide | pSer47 | | LRpSEEDG |
| SEQ ID No: 154 | Human | ProDomain PhosphoPeptide | pSer47 | | RpSEEDG |
| SEQ ID No: 155 | Human | ProDomain PhosphoPeptide | pSer47 | | pSEEDG |
| SEQ ID No: 156 | Human | ProDomain PhosphoPeptide | pSer47 | | LVLALRpSEED |
| SEQ ID No: 157 | Human | ProDomain PhosphoPeptide | pSer47 | | VLALRpSEED |
| SEQ ID No: 158 | Human | ProDomain PhosphoPeptide | pSer47 | | LALRpSEED |
| SEQ ID No: 159 | Human | ProDomain PhosphoPeptide | pSer47 | | ALRpSEED |
| SEQ ID No: 160 | Human | ProDomain PhosphoPeptide | pSer47 | | LRpSEED |
| SEQ ID No: 161 | Human | ProDomain PhosphoPeptide | pSer47 | | RpSEED |
| SEQ ID No: 162 | Human | ProDomain PhosphoPeptide | pSer47 | | LVLALRpSEE |
| SEQ ID No: 163 | Human | ProDomain PhosphoPeptide | pSer47 | | VLALRpSEE |
| SEQ ID No: 164 | Human | ProDomain PhosphoPeptide | pSer47 | | LALRpSEE |
| SEQ ID No: 165 | Human | ProDomain PhosphoPeptide | pSer47 | | ALRpSEE |
| SEQ ID No: 166 | Human | ProDomain PhosphoPeptide | pSer47 | | LRpSEE |
| SEQ ID No: 167 | Human | ProDomain PhosphoPeptide | pSer47 | | LVLALRpSE |
| SEQ ID No: 168 | Human | ProDomain PhosphoPeptide | pSer47 | | VLALRpSE |
| SEQ ID No: 169 | Human | ProDomain PhosphoPeptide | pSer47 | | LALRpSE |
| SEQ ID No: 170 | Human | ProDomain PhosphoPeptide | pSer47 | | ALRpSE |
| SEQ ID No: 171 | Human | ProDomain PhosphoPeptide | pSer47 | | LVLALRpS |
| SEQ ID No: 172 | Human | ProDomain PhosphoPeptide | pSer47 | | VLALRpS |
| SEQ ID No: 173 | Mouse | ProDomain PhosphoPeptide | pSer47 | | LALRpS |
| SEQ ID No: 174 | Mouse | ProDomain PhosphoPeptide | pSer48 | | LMLALPpSQEDG |
| SEQ ID No: 175 | Mouse | ProDomain PhosphoPeptide | pSer49 | | MLALPpSQEDG |
| SEQ ID No: 176 | Mouse | ProDomain PhosphoPeptide | pSer50 | | LALPpSQEDG |
| SEQ ID No: 177 | Mouse | ProDomain PhosphoPeptide | pSer51 | | ALPpSQEDG |
| SEQ ID No: 178 | Mouse | ProDomain PhosphoPeptide | pSer52 | | LPpSQEDG |
| SEQ ID No: 179 | Mouse | ProDomain PhosphoPeptide | pSer53 | | PpSQEDG |
| SEQ ID No: 180 | Mouse | ProDomain PhosphoPeptide | pSer54 | | pSQEDG |
| SEQ ID No: 181 | Mouse | ProDomain PhosphoPeptide | pSer55 | | LMLALPpSQED |
| SEQ ID No: 182 | Mouse | ProDomain PhosphoPeptide | pSer56 | | MLALPpSQED |
| SEQ ID No: 183 | Mouse | ProDomain PhosphoPeptide | pSer57 | | LALPpSQED |
| SEQ ID No: 184 | Mouse | ProDomain PhosphoPeptide | pSer58 | | ALPpSQED |
| SEQ ID No: 185 | Mouse | ProDomain PhosphoPeptide | pSer59 | | LPpSQED |
| SEQ ID No: 186 | Mouse | ProDomain PhosphoPeptide | pSer60 | | PpSQED |
| SEQ ID No: 187 | Mouse | ProDomain PhosphoPeptide | pSer61 | | LMLALPpSQE |
| SEQ ID No: 188 | Mouse | ProDomain PhosphoPeptide | pSer62 | | MLALPpSQE |
| SEQ ID No: 189 | Mouse | ProDomain PhosphoPeptide | pSer63 | | LALPpSQE |
| SEQ ID No: 190 | Mouse | ProDomain PhosphoPeptide | pSer64 | | ALPpSQE |
| SEQ ID No: 191 | Mouse | ProDomain PhosphoPeptide | pSer65 | | LPpSQE |
| SEQ ID No: 192 | Mouse | ProDomain PhosphoPeptide | pSer66 | | LMLALPpSQ |
| SEQ ID No: 193 | Mouse | ProDomain PhosphoPeptide | pSer67 | | MLALPpSQ |
| SEQ ID No: 194 | Mouse | ProDomain PhosphoPeptide | pSer68 | | LALPpSQ |
| SEQ ID No: 195 | Mouse | ProDomain PhosphoPeptide | pSer69 | | ALPpSQ |
| SEQ ID No: 196 | Mouse | ProDomain PhosphoPeptide | pSer70 | | LMLALPpS |
| SEQ ID No: 197 | Mouse | ProDomain PhosphoPeptide | pSer71 | | MLALPpS |
| SEQ ID No: 198 | Mouse | ProDomain PhosphoPeptide | pSer72 | | LALPpS |
| SEQ ID No: 199 | Human | ProDomain PhosphoPeptide + Linker | pSer47 | N: CGG | CGGLVLALRpSEEDG |
| SEQ ID No: 200 | Human | ProDomain PhosphoPeptide + Linker | pSer47 | N: CGG | CGGVLALRpSEEDG |
| SEQ ID No: 201 | Human | ProDomain PhosphoPeptide + Linker | pSer47 | N: CGG | CGGLALRpSEEDG |
| SEQ ID No: 202 | Human | ProDomain PhosphoPeptide + Linker | pSer47 | N: CGG | CGGALRpSEEDG |
| SEQ ID No: 203 | Human | ProDomain PhosphoPeptide + Linker | pSer47 | N: CGG | CGGLRpSEEDG |
| SEQ ID No: 204 | Human | ProDomain PhosphoPeptide + Linker | pSer47 | N: CGG | CGGRpSEEDG |
| SEQ ID No: 205 | Human | ProDomain PhosphoPeptide + Linker | pSer47 | N: CGG | CGGpSEEDG |
| SEQ ID No: 206 | Human | ProDomain PhosphoPeptide + Linker | pSer47 | N: CGG | CGG LVLALRpSEED |
| SEQ ID No: 207 | Human | ProDomain PhosphoPeptide + Linker | pSer47 | N: CGG | CGGVLALRpSEED |
| SEQ ID No: 208 | Human | ProDomain PhosphoPeptide + Linker | pSer47 | N: CGG | CGG LALRpSEED |
| SEQ ID No: 209 | Human | ProDomain PhosphoPeptide + Linker | pSer47 | N: CGG | CGG ALRpSEED |
| SEQ ID No: 210 | Human | ProDomain PhosphoPeptide + Linker | pSer47 | N: CGG | CGGLRpSEED |
| SEQ ID No: 211 | Human | ProDomain PhosphoPeptide + Linker | pSer47 | N: CGG | CGG RpSEED |
| SEQ ID No: 212 | Human | ProDomain PhosphoPeptide + Linker | pSer47 | N: CGG | CGGLVLALRpSEE |
| SEQ ID No: 213 | Human | ProDomain PhosphoPeptide + Linker | pSer47 | N: CGG | CGGVLALRpSEE |
| SEQ ID No: 214 | Human | ProDomain PhosphoPeptide + Linker | pSer47 | N: CGG | CGGLALRpSEE |
| SEQ ID No: 215 | Human | ProDomain PhosphoPeptide + Linker | pSer47 | N: CGG | CGGALRpSEE |
| SEQ ID No: 216 | Human | ProDomain PhosphoPeptide + Linker | pSer47 | N: CGG | CGGLRpSEE |
| SEQ ID No: 217 | Human | ProDomain PhosphoPeptide + Linker | pSer47 | N: CGG | CGGLVLALRpSE |
| SEQ ID No: 218 | Human | ProDomain PhosphoPeptide + Linker | pSer47 | N: CGG | CGG VLALRpSE |
| SEQ ID No: 219 | Human | ProDomain PhosphoPeptide + Linker | pSer47 | N: CGG | CGG LALRpSE |
| SEQ ID No: 220 | Human | ProDomain PhosphoPeptide + Linker | pSer47 | N: CGG | CGGALRpSE |
| SEQ ID No: 221 | Human | ProDomain PhosphoPeptide + Linker | pSer47 | N: CGG | CGGLVLALRpS |
| SEQ ID No: 222 | Human | ProDomain PhosphoPeptide + Linker | pSer47 | N: CGG | CGGVLALRpS |
| SEQ ID No: 223 | Human | ProDomain PhosphoPeptide + Linker | pSer47 | N: CGG | CGGLALRpS |
| SEQ ID No: 224 | **Human** | **ProDomain PhosphoPeptid e + Linker (9.56)** | **pSer47** | **C: GC/GGC** | LVLALRpSEEDGGC |
| SEQ ID No: 225 | Human | ProDomain PhosphoPeptide + Linker | pSer47 | C: GC/GGC | VLALRpSEEDGGC |
| SEQ ID No: 226 | Human | ProDomain PhosphoPeptide + Linker | pSer47 | C: GC/GGC | LALRpSEEDGGC |
| SEQ ID No: 227 | Human | ProDomain PhosphoPeptide + Linker | pSer47 | C: GC/GGC | ALRpSEEDGGC |
| SEQ ID No: 228 | Human | ProDomain PhosphoPeptide + Linker | pSer47 | C: GC/GGC | LRpSEEDGGC |
| SEQ ID No: 229 | Human | ProDomain PhosphoPeptide + Linker | pSer47 | C: GC/GGC | RpSEEDGGC |
| SEQ ID No: 230 | Human | ProDomain PhosphoPeptide + Linker | pSer47 | C: GC/GGC | pSEEDGGC |
| SEQ ID No: 231 | Human | ProDomain PhosphoPeptide + Linker | pSer47 | C: GGC | LVLALRpSEEGGC |
| SEQ ID No: 232 | Human | ProDomain PhosphoPeptide + Linker | pSer47 | C: GGC | VLALRpSEEGGC |
| SEQ ID No: 233 | Human | ProDomain PhosphoPeptide + Linker | pSer47 | C: GGC | LALRpSEEGGC |
| SEQ ID No: 234 | Human | ProDomain PhosphoPeptide + Linker | pSer47 | C: GGC | ALRpSEEGGC |
| SEQ ID No: 235 | Human | ProDomain PhosphoPeptide + Linker | pSer47 | C: GGC | LRpSEEGGC |
| SEQ ID No: 236 | Human | ProDomain PhosphoPeptide + Linker | pSer47 | C: GGC | LVLALRpSEGGC |
| SEQ ID No: 237 | Human | ProDomain PhosphoPeptide + Linker | pSer47 | C: GGC | VLALRpSEGGC |
| SEQ ID No: 238 | Human | ProDomain PhosphoPeptide + Linker | pSer47 | C: GGC | LALRpSEGGC |
| SEQ ID No: 239 | Human | ProDomain PhosphoPeptide + Linker | pSer47 | C: GGC | ALRpSEGGC |
| SEQ ID No: 240 | Human | ProDomain PhosphoPeptide + Linker | pSer47 | C: GGC | LVLALRpSGGC |
| SEQ ID No: 241 | Human | ProDomain PhosphoPeptide + Linker | pSer47 | C: GGC | VLALRpSGGC |
| SEQ ID No: 242 | Human | ProDomain PhosphoPeptide + Linker | pSer47 | C: GGC | LALRpSGGC |
| SEQ ID No: 243 | **Mouse** | **ProDomain PhosphoPeptid e + Linker (9.57)** | **pSer50** | **C: GGC** | LMLALPpSQEDGGC |
| SEQ ID No: 244 | Mouse | ProDomain PhosphoPeptide + Linker | pSer50 | C: GC/GGC | MLALPpSQEDGGC |
| SEQ ID No: 245 | Mouse | ProDomain PhosphoPeptide + Linker | pSer50 | C: GC/GGC | LALPpSQEDGGC |
| SEQ ID No: 246 | Mouse | ProDomain PhosphoPeptide + Linker | pSer50 | C: GC/GGC | ALPpSQEDGGC |
| SEQ ID No: 247 | Mouse | ProDomain PhosphoPeptide + Linker | pSer50 | C: GC/GGC | LPpSQEDGGC |
| SEQ ID No: 248 | Mouse | ProDomain PhosphoPeptide + Linker | pSer50 | C: GC/GGC | PpSQEDGGC |
| SEQ ID No: 249 | Mouse | ProDomain PhosphoPeptide + Linker | pSer50 | C: GC/GGC | pSQEDGGC |
| SEQ ID No: 250 | Mouse | ProDomain PhosphoPeptide + Linker | pSer50 | C: GGC | LMLALPpSQEGGC |
| SEQ ID No: 251 | Mouse | ProDomain PhosphoPeptide + Linker | pSer50 | C: GGC | MLALPpSQEGGC |
| SEQ ID No: 252 | Mouse | ProDomain PhosphoPeptide + Linker | pSer50 | C: GGC | LALPpSQEGGC |
| SEQ ID No: 253 | Mouse | ProDomain PhosphoPeptide + Linker | pSer50 | C: GGC | ALPpSQEGGC |
| SEQ ID No: 254 | Mouse | ProDomain PhosphoPeptide + Linker | pSer50 | C: GGC | LPpSQEGGC |
| SEQ ID No: 255 | Mouse | ProDomain PhosphoPeptide + Linker | pSer50 | C: GGC | LMLALPpSQGGC |
| SEQ ID No: 256 | Mouse | ProDomain PhosphoPeptide + Linker | pSer50 | C: GGC | MLALPpSQGGC |
| SEQ ID No: 257 | Mouse | ProDomain PhosphoPeptide + Linker | pSer50 | C: GGC | LALPpSQGGC |
| SEQ ID No: 258 | Mouse | ProDomain PhosphoPeptide + Linker | pSer50 | C: GGC | ALPpSQGGC |
| SEQ ID No: 259 | Mouse | ProDomain PhosphoPeptide + Linker | pSer50 | C: GGC | LMLALPpSGGC |
| SEQ ID No: 260 | Mouse | ProDomain PhosphoPeptide + Linker | pSer50 | C: GGC | MLALPpSGGC |
| SEQ ID No: 261 | Mouse | ProDomain PhosphoPeptide + Linker | pSer50 | C: GGC | LALPpSGGC |
| SEQ ID No: 262 | Mouse | ProDomain PhosphoPeptide + Linker | pSer50 | N: CGG | CGGLMLALPpSQEDG |
| SEQ ID No: 263 | Mouse | ProDomain PhosphoPeptide + Linker | pSer50 | N: CGG | CGGMLALPpSQEDG |
| SEQ ID No: 264 | Mouse | ProDomain PhosphoPeptide + Linker | pSer50 | N: CGG | CGGLALPpSQEDG |
| SEQ ID No: 265 | Mouse | ProDomain PhosphoPeptide + Linker | pSer50 | N: CGG | CGGALPpSQEDG |
| SEQ ID No: 266 | Mouse | ProDomain PhosphoPeptide + Linker | pSer50 | N: CGG | CGGLPpSQEDG |
| SEQ ID No: 267 | Mouse | ProDomain PhosphoPeptide + Linker | pSer50 | N: CGG | CGGPpSQEDG |
| SEQ ID No: 268 | Mouse | ProDomain PhosphoPeptide + Linker | pSer50 | N: CGG | CGGpSQEDG |
| SEQ ID No: 269 | Mouse | ProDomain PhosphoPeptide + Linker | pSer50 | N: CGG | CGG LMLALPpSQED |
| SEQ ID No: 270 | Mouse | ProDomain PhosphoPeptide + Linker | pSer50 | N: CGG | CGGMLALPpSQED |
| SEQ ID No: 271 | Mouse | ProDomain PhosphoPeptide + Linker | pSer50 | N: CGG | CGGLALPpSQED |
| SEQ ID No: 272 | Mouse | ProDomain PhosphoPeptide + Linker | pSer50 | N: CGG | CGGALPpSQED |
| SEQ ID No: 273 | Mouse | ProDomain PhosphoPeptide + Linker | pSer50 | N: CGG | CGG LPpSQED |
| SEQ ID No: 274 | Mouse | ProDomain PhosphoPeptide + Linker | pSer50 | N: CGG | CGGPpSQED |
| SEQ ID No: 275 | Mouse | ProDomain PhosphoPeptide + Linker | pSer50 | N: CGG | CGGLMLALPpSQE |
| SEQ ID No: 276 | Mouse | ProDomain PhosphoPeptide + Linker | pSer50 | N: CGG | CGGMLALPpSQE |
| SEQ ID No: 277 | Mouse | ProDomain PhosphoPeptide + Linker | pSer50 | N: CGG | CGG LALPpSQE |
| SEQ ID No: 278 | Mouse | ProDomain PhosphoPeptide + Linker | pSer50 | N: CGG | CGGALPpSQE |
| SEQ ID No: 279 | Mouse | ProDomain PhosphoPeptide + Linker | pSer50 | N: CGG | CGGLPpSQE |
| SEQ ID No: 280 | Mouse | ProDomain PhosphoPeptide + Linker | pSer50 | N: CGG | CGGLMLALPpSQ |
| SEQ ID No: 281 | Mouse | ProDomain PhosphoPeptide + Linker | pSer50 | N: CGG | CGG MLALPpSQ |
| SEQ ID No: 282 | Mouse | ProDomain Phospho Peptide + Linker | pSer50 | N: CGG | CGGLALPpSQ |
| SEQ ID No: 283 | Mouse | ProDomain PhosphoPeptide + Linker | pSer50 | N: CGG | CGGALPpSQ |
| SEQ ID No: 284 | Mouse | ProDomain PhosphoPeptide + Linker | pSer50 | N: CGG | CGGLMLALPpS |
| SEQ ID No: 285 | Mouse | ProDomain PhosphoPeptide + Linker | pSer50 | N: CGG | CGG MLALPpS |
| SEQ ID No: 286 | Mouse | ProDomain PhosphoPeptide + Linker | pSer50 | N: CGG | CGG LALPpS |
| **SEQ ID No: 287** | **Human** | **C-Terminal Domain Peptide (9.23)** | **N/A** | **Native C** | CSRHLAQASQELQ |
| SEQ ID No: 288 | Human | C-Terminal Domain Peptide | N/A | Native C | CSRHLAQASQEL |
| SEQ ID No: 289 | Human | C-Terminal Domain Peptide | N/A | Native C | CSRHLAQASQE |
| SEQ ID No: 290 | Human | C-Terminal Domain Peptide | N/A | Native C | CSRHLAQASQ |
| SEQ ID No: 291 | Human | C-Terminal Domain Peptide | N/A | Native C | CSRHLAQAS |
| SEQ ID No: 292 | Human | C-Terminal Domain Peptide | N/A | | SRHLAQASQELQ |
| SEQ ID No: 293 | Human | C-Terminal Domain Peptide | N/A | | SRHLAQASQEL |
| SEQ ID No: 294 | Human | C-Terminal Domain Peptide | N/A | | SRHLAQASQE |
| SEQ ID No: 295 | Human | C-Terminal Domain Peptide | N/A | | SRHLAQASQ |
| SEQ ID No: 296 | Human | C-Terminal Domain Peptide | N/A | | SRHLAQAS |
| SEQ ID No: 297 | Human | C-Terminal Domain Peptide | N/A | | RHLAQASQELQ |
| SEQ ID No: 298 | Human | C-Terminal Domain Peptide | N/A | | RHLAQASQEL |
| SEQ ID No: 299 | Human | C-Terminal Domain Peptide | N/A | | RHLAQASQE |
| SEQ ID No: 300 | Human | C-Terminal Domain Peptide | N/A | | RHLAQASQ |
| SEQ ID No: 301 | Human | C-Terminal Domain Peptide | N/A | | RHLAQAS |
| SEQ ID No: 302 | Human | C-Terminal Domain Peptide | N/A | | HLAQASQELQ |
| SEQ ID No: 303 | Human | C-Terminal Domain Peptide | N/A | | HLAQASQEL |
| SEQ ID No: 304 | Human | C-Terminal Domain Peptide | N/A | | HLAQASQ |
| SEQ ID No: 305 | Human | C-Terminal Domain Peptide | N/A | | HLAQAS |
| SEQ ID No: 306 | Human | C-Terminal Domain Peptide | N/A | | LAQASQELQ |
| SEQ ID No: 307 | Human | C-Terminal Domain Peptide | N/A | | LAQASQEL |
| SEQ ID No: 308 | Human | C-Terminal Domain Peptide | N/A | | LAQASQE |
| SEQ ID No: 309 | Human | C-Terminal Domain Peptide | N/A | | LAQASQ |
| SEQ ID No: 310 | Human | C-Terminal Domain Peptide | N/A | | LAQAS |
| SEQ ID No: 311 | Human | C-Terminal Domain Peptide | N/A | | AQASQELQ |
| SEQ ID No: 312 | Human | C-Terminal Domain Peptide | N/A | | AQASQEL |
| SEQ ID No: 313 | Human | C-Terminal Domain Peptide | N/A | | AQASQE |
| SEQ ID No: 314 | Human | C-Terminal Domain Peptide | N/A | | AQASQ |
| SEQ ID No: 315 | Human | C-Terminal Domain Peptide | N/A | | QASQELQ |
| SEQ ID No: 316 | Human | C-Terminal Domain Peptide | N/A | | QASQEL |
| SEQ ID No: 317 | Human | C-Terminal Domain Peptide | N/A | | QASQE |
| SEQ ID No: 318 | Human | C-Terminal Domain Peptide | N/A | | ASQELQ |
| SEQ ID No: 319 | Human | C-Terminal Domain Peptide | N/A | | ASQEL |
| SEQ ID No: 320 | Human | C-Terminal Domain Peptide | N/A | | SQELQ |
| SEQ ID No: 321 | Human | C-Terminal Domain Peptide + Linker | N/A | N: CGG | CGGSRHLAQASQELQ |
| SEQ ID No: 322 | Human | C-Terminal Domain Peptide + Linker | N/A | N: CGG | CGGSRHLAQASQEL |
| SEQ ID No: 323 | Human | C-Terminal Domain Peptide + Linker | N/A | N: CGG | CGGSRHLAQASQE |
| SEQ ID No: 324 | Human | C-Terminal Domain Peptide + Linker | N/A | N: CGG | CGGSRHLAQASQ |
| SEQ ID No: 325 | Human | C-Terminal Domain Peptide + Linker | N/A | N: CGG | CGGSRHLAQAS |
| SEQ ID No: 326 | Human | C-Terminal Domain Peptide + Linker | N/A | N: CGG | CGGRHLAQASQELQ |
| SEQ ID No: 327 | Human | C-Terminal Domain Peptide + Linker | N/A | N: CGG | CGGRHLAQASQEL |
| SEQ ID No: 328 | Human | C-Terminal Domain Peptide + Linker | N/A | N: CGG | CGGRHLAQASQE |
| SEQ ID No: 329 | Human | C-Terminal Domain Peptide + Linker | N/A | N: CGG | CGGRHLAQASQ |
| SEQ ID No: 330 | Human | C-Terminal Domain Peptide + Linker | N/A | N: CGG | CGGRHLAQAS |
| SEQ ID No: 331 | Human | C-Terminal Domain Peptide + Linker | N/A | N: CGG | CGGHLAQASQELQ |
| SEQ ID No: 332 | Human | C-Terminal Domain Peptide + Linker | N/A | N: CGG | CGGHLAQASQEL |
| SEQ ID No: 333 | Human | C-Terminal Domain Peptide + Linker | N/A | N: CGG | CGGHLAQASQ |
| SEQ ID No: 334 | Human | C-Terminal Domain Peptide + Linker | N/A | N: CGG | CGGHLAQAS |
| SEQ ID No: 335 | Human | C-Terminal Domain Peptide + Linker | N/A | N: CGG | CGGLAQASQELQ |
| SEQ ID No: 336 | Human | C-Terminal Domain Peptide + Linker | N/A | N: CGG | CGGLAQASQEL |
| SEQ ID No: 337 | Human | C-Terminal Domain Peptide + Linker | N/A | N: CGG | CGGLAQASQE |
| SEQ ID No: 338 | Human | C-Terminal Domain Peptide + Linker | N/A | N: CGG | CGGLAQASQ |
| SEQ ID No: 339 | Human | C-Terminal Domain Peptide + Linker | N/A | N: CGG | CGGLAQAS |
| SEQ ID No: 340 | Human | C-Terminal Domain Peptide + Linker | N/A | N: CGG | CGGAQASQELQ |
| SEQ ID No: 341 | Human | C-Terminal Domain Peptide + Linker | N/A | N: CGG | CGGAQASQEL |
| SEQ ID No: 342 | Human | C-Terminal Domain Peptide + Linker | N/A | N: CGG | CGGAQASQE |
| SEQ ID No: 343 | Human | C-Terminal Domain Peptide + Linker | N/A | N: CGG | CGGAQASQ |
| SEQ ID No: 344 | Human | C-Terminal Domain Peptide + Linker | N/A | N: CGG | CGGQASQELQ |
| SEQ ID No: 345 | Human | C-Terminal Domain Peptide + Linker | N/A | N: CGG | CGGQASQEL |
| SEQ ID No: 346 | Human | C-Terminal Domain Peptide + Linker | N/A | N: CGG | CGGQASQE |
| SEQ ID No: 347 | Human | C-Terminal Domain Peptide + Linker | N/A | N: CGG | CGGASQELQ |
| SEQ ID No: 348 | Human | C-Terminal Domain Peptide + Linker | N/A | N: CGG | CGGASQEL |
| SEQ ID No: 349 | Human | C-Terminal Domain Peptide + Linker | N/A | N: CGG | CGGSQELQ |
| SEQ ID No: 350 | Human | C-Terminal Domain Peptide + Linker | N/A | C: GGC | SRHLAQASQELQGGC |
| SEQ ID No: 351 | Human | C-Terminal Domain Peptide + Linker | N/A | | SRHLAQASQELGGC |
| SEQ ID No: 352 | Huma"n | C-Terminal Domain Peptide + Linker | N/A | | SRHLAQASQEGGC |
| SEQ ID No: 353 | Human | C-Terminal Domain Peptide + Linker | N/A | | SRHLAQASQGGC |
| SEQ ID No: 354 | Human | C-Terminal Domain Peptide + Linker | N/A | | SRHLAQASGGC |
| SEQ ID No: 355 | Human | C-Terminal Domain Peptide + Linker | N/A | | RHLAQASQELQGGC |
| SEQ ID No: 356 | Human | C-Terminal Domain Peptide + Linker | N/A | | RHLAQASQELGGC |
| SEQ ID No: 357 | Human | C-Terminal Domain Peptide + Linker | N/A | | RHLAQASQEGGC |
| SEQ ID No: 358 | Human | C-Terminal Domain Peptide + Linker | N/A | | RHLAQASQGGC |
| SEQ ID No: 359 | Human | C-Terminal Domain Peptide + Linker | N/A | | RHLAQASGGC |
| SEQ ID No: 360 | Human | C-Terminal Domain Peptide + Linker | N/A | | HLAQASQELQGGC |
| SEQ ID No: 361 | Human | C-Terminal Domain Peptide + Linker | N/A | | HLAQASQELGGC |
| SEQ ID No: 362 | Human | C-Terminal Domain Peptide + Linker | N/A | | HLAQASQGGC |
| SEQ ID No: 363 | Human | C-Terminal Domain Peptide + Linker | N/A | | HLAQASGGC |
| SEQ ID No: 364 | Human | C-Terminal Domain Peptide + Linker | N/A | | LAQASQELQGGC |
| SEQ ID No: 365 | Human | C-Terminal Domain Peptide + Linker | N/A | | LAQASQELGGC |
| SEQ ID No: 366 | Human | C-Terminal Domain Peptide + Linker | N/A | | LAQASQEGGC |
| SEQ ID No: 367 | Human | C-Terminal Domain Peptide + Linker | N/A | | LAQASQGGC |
| SEQ ID No: 368 | Human | C-Terminal Domain Peptide + Linker | N/A | | LAQASGGC |
| SEQ ID No: 369 | Human | C-Terminal Domain Peptide + Linker | N/A | | AQASQELQGGC |
| SEQ ID No: 370 | Human | C-Terminal Domain Peptide + Linker | N/A | | AQASQELGGC |
| SEQ ID No: 371 | Human | C-Terminal Domain Peptide + Linker | N/A | | AQASQEGGC |
| SEQ ID No: 372 | Human | C-Terminal Domain Peptide + Linker | N/A | | AQASQGGC |
| SEQ ID No: 373 | Human | C-Terminal Domain Peptide + Linker | N/A | | QASQELQGGC |
| SEQ ID No: 374 | Human | C-Terminal Domain Peptide + Linker | N/A | | QASQELGGC |
| SEQ ID No: 375 | Human | C-Terminal Domain Peptide + Linker | N/A | | QASQEGGC |
| SEQ ID No: 376 | Human | C-Terminal Domain Peptide + Linker | N/A | | ASQELQGGC |
| SEQ ID No: 377 | Human | C-Terminal Domain Peptide + Linker | N/A | | ASQELGGC |
| SEQ ID No: 378 | Human | C-Terminal Domain Peptide + Linker | N/A | | SQELQGGC |
| **SEQ ID No: 379** | **Mouse** | **C-Terminal Domain Peptide (9.24)** | **N/A** | **Native C** | CRSRPSAKASWVQ |
| SEQ ID No: 380 | Mouse | C-Terminal Domain Peptide | N/A | Native C | CRSRPSAKASWV |
| SEQ ID No: 381 | Mouse | C-Terminal Domain Peptide | N/A | Native C | CRSRPSAKASW |
| SEQ ID No: 382 | Mouse | C-Terminal Domain Peptide | N/A | Native C | CRSRPSAKAS |
| SEQ ID No: 383 | Mouse | C-Terminal Domain Peptide | N/A | | RSRPSAKASWVQ |
| SEQ ID No: 384 | Mouse | C-Terminal Domain Peptide | N/A | | RSRPSAKASWV |
| SEQ ID No: 385 | Mouse | C-Terminal Domain Peptide | N/A | | RSRPSAKASW |
| SEQ ID No: 386 | Mouse | C-Terminal Domain Peptide | N/A | | RSRPSAKAS |
| SEQ ID No: 387 | Mouse | C-Terminal Domain Peptide | N/A | | SRPSAKASWVQ |
| SEQ ID No: 388 | Mouse | C-Terminal Domain Peptide | N/A | | SRPSAKASWV |
| SEQ ID No: 389 | Mouse | C-Terminal Domain Peptide | N/A | | SRPSAKASW |
| SEQ ID No: 390 | Mouse | C-Terminal Domain Peptide | N/A | | SRPSAKAS |
| SEQ ID No: 391 | Mouse | C-Terminal Domain Peptide | N/A | | RPSAKASWVQ |
| SEQ ID No: 392 | Mouse | C-Terminal Domain Peptide | N/A | | RPSAKASWV |
| SEQ ID No: 393 | Mouse | C-Terminal Domain Peptide | N/A | | RPSAKASW |
| SEQ ID No: 394 | Mouse | C-Terminal Domain Peptide | N/A | | RPSAKAS |
| SEQ ID No: 395 | Mouse | C-Terminal Domain Peptide | N/A | | PSAKASWVQ |
| SEQ ID No: 396 | Mouse | C-Terminal Domain Peptide | N/A | | PSAKASWV |
| SEQ ID No: 397 | Mouse | C-Terminal Domain Peptide | N/A | | PSAKASW |
| SEQ ID No: 398 | Mouse | C-Terminal Domain Peptide | N/A | | PSAKAS |
| SEQ ID No: 399 | Mouse | C-Terminal Domain Peptide | N/A | | SAKAS |
| SEQ ID No: 400 | Mouse | C-Terminal Domain Peptide + Linker | N/A | N: CGG | CGGRSRPSAKASWVQ |
| SEQ ID No: 401 | Mouse | C-Terminal Domain Peptide + Linker | N/A | N: CGG | CGGRSRPSAKASWV |
| SEQ ID No: 402 | Mouse | C-Terminal Domain Peptide + Linker | N/A | N: CGG | CGGRSRPSAKASW |
| SEQ ID No: 403 | Mouse | C-Terminal Domain Peptide + Linker | N/A | N: CGG | CGGRSRPSAKAS |
| SEQ ID No: 404 | Mouse | C-Terminal Domain Peptide + Linker | N/A | N: CGG | CGGSRPSAKASWVQ |
| SEQ ID No: 405 | Mouse | C-Terminal Domain Peptide + Linker | N/A | N: CGG | CGGSRPSAKASWV |
| SEQ ID No: 406 | Mouse | C-Terminal Domain Peptide + Linker | N/A | N: CGG | CGGSRPSAKASW |
| SEQ ID No: 407 | Mouse | C-Terminal Domain Peptide + Linker | N/A | N: CGG | CGGSRPSAKAS |
| SEQ ID No: 408 | Mouse | C-Terminal Domain Peptide + Linker | N/A | N: CGG | CGGRPSAKASWVQ |
| SEQ ID No: 409 | Mouse | C-Terminal Domain Peptide + Linker | N/A | N: CGG | CGGRPSAKASWV |
| SEQ ID No: 410 | Mouse | C-Terminal Domain Peptide + Linker | N/A | N: CGG | CGGRPSAKASW |
| SEQ ID No: 411 | Mouse | C-Terminal Domain Peptide + Linker | N/A | N: CGG | CGGRPSAKAS |
| SEQ ID No: 412 | Mouse | C-Terminal Domain Peptide + Linker | N/A | N: CGG | CGGPSAKASWVQ |
| SEQ ID No: 413 | Mouse | C-Terminal Domain Peptide + Linker | N/A | N: CGG | CGGPSAKASWV |
| SEQ ID No: 414 | Mouse | C-Terminal Domain Peptide + Linker | N/A | N: CGG | CGGPSAKASW |
| SEQ ID No: 415 | Mouse | C-Terminal Domain Peptide + Linker | N/A | N: CGG | CGGPSAKAS |
| SEQ ID No: 416 | Mouse | C-Terminal Domain Peptide + Linker | N/A | N: CGG | CGGSAKAS |
| SEQ ID No: 417 | Mouse | C-Terminal Domain Peptide + Linker | N/A | C: GGC | RSRPSAKASWVQGGC |
| SEQ ID No: 418 | Mouse | C-Terminal Domain Peptide + Linker | N/A | C: GGC | RSRPSAKASWVGGC |
| SEQ ID No: 419 | Mouse | C-Terminal Domain Peptide + Linker | N/A | C: GGC | RSRPSAKASWGGC |
| SEQ ID No: 420 | Mouse | C-Terminal Domain Peptide + Linker | N/A | C: GGC | RSRPSAKASGGC |
| SEQ ID No: 421 | Mouse | C-Terminal Domain Peptide + Linker | N/A | C: GGC | SRPSAKASWVQGGC |
| SEQ ID No: 422 | Mouse | C-Terminal Domain Peptide + Linker | N/A | C: GGC | SRPSAKASWVGGC |
| SEQ ID No: 423 | Mouse | C-Terminal Domain Peptide + Linker | N/A | C: GGC | SRPSAKASWGGC |
| SEQ ID No: 424 | Mouse | C-Terminal Domain Peptide + Linker | N/A | C: GGC | SRPSAKASGGC |
| SEQ ID No: 425 | Mouse | C-Terminal Domain Peptide + Linker | N/A | C: GGC | RPSAKASWVQGGC |
| SEQ ID No: 426 | Mouse | C-Terminal Domain Peptide + Linker | N/A | C: GGC | RPSAKASWVGGC |
| SEQ ID No: 427 | Mouse | C-Terminal Domain Peptide + Linker | N/A | C: GGC | RPSAKASWGGC |
| SEQ ID No: 428 | Mouse | C-Terminal Domain Peptide + Linker | N/A | C: GGC | RPSAKASGGC |
| SEQ ID No: 429 | Mouse | C-Terminal Domain Peptide + Linker | N/A | C: GGC | PSAKASWVQGGC |
| SEQ ID No: 430 | Mouse | C-Terminal Domain Peptide + Linker | N/A | C: GGC | PSAKASWVGGC |
| SEQ ID No: 431 | Mouse | C-Terminal Domain Peptide + Linker | N/A | C: GGC | PSAKASWGGC |
| SEQ ID No: 432 | Mouse | C-Terminal Domain Peptide + Linker | N/A | C: GGC | PSAKASGGC |
| SEQ ID No: 433 | Mouse | C-Terminal Domain Peptide + Linker | N/A | C: GGC | SAKASGGC |
| SEQ ID No: 434 | Human | C-Terminal Domain PhosphoPeptide | pSer688 | Native C | CSRHLAQApSQELQ |
| SEQ ID No: 435 | Human | C-Terminal Domain PhosphoPeptide | pSer688 | Native C | CSRHLAQApSQELQ |
| SEQ ID No: 436 | Human | C-Terminal Domain PhosphoPeptide | pSer688 | Native C | CSRHLAQApSQEL |
| SEQ ID No: 437 | Human | C-Terminal Domain PhosphoPeptide | pSer688 | Native C | CSRHLAQApSQE |
| SEQ ID No: 438 | Human | C-Terminal Domain PhosphoPeptide | pSer688 | Native C | CSRHLAQApSQ |
| SEQ ID No: 439 | Human | C-Terminal Domain PhosphoPeptide | pSer688 | Native C | CSRHLAQApS |
| SEQ ID No: 440 | Human | C-Terminal Domain PhosphoPeptide | pSer688 | | SRHLAQApSQELQ |
| SEQ ID No: 441 | Human | C-Terminal Domain PhosphoPeptide | pSer688 | | SRHLAQApSQEL |
| SEQ ID No: 442 | Human | C-Terminal Domain PhosphoPeptide | pSer688 | | SRHLAQApSQE |
| SEQ ID No: 443 | Human | C-Terminal Domain PhosphoPeptide | pSer688 | | SRHLAQApSQ |
| SEQ ID No: 444 | Human | C-Terminal Domain PhosphoPeptide | pSer688 | | SRHLAQApS |
| SEQ ID No: 445 | Human | C-Terminal Domain PhosphoPeptide | pSer688 | | RHLAQApSQELQ |
| SEQ ID No: 446 | Human | C-Terminal Domain PhosphoPeptide | pSer688 | | RHLAQApSQEL |
| SEQ ID No: 447 | Human | C-Terminal Domain PhosphoPeptide | pSer688 | | RHLAQApSQE |
| SEQ ID No: 448 | Human | C-Terminal Domain PhosphoPeptide | pSer688 | | RHLAQApSQ |
| SEQ ID No: 449 | Human | C-Terminal Domain PhosphoPeptide | pSer688 | | RHLAQApS |
| SEQ ID No: 450 | Human | C-Terminal Domain PhosphoPeptide | pSer688 | | HLAQApSQELQ |
| SEQ ID No: 451 | Human | C-Terminal Domain PhosphoPeptide | pSer688 | | HLAQApSQEL |
| SEQ ID No: 452 | Human | C-Terminal Domain PhosphoPeptide | pSer688 | | HLAQApSQ |
| SEQ ID No: 453 | Human | C-Terminal Domain PhosphoPeptide | pSer688 | | HLAQApS |
| SEQ ID No: 454 | Human | C-Terminal Domain PhosphoPeptide | pSer688 | | LAQApSQELQ |
| SEQ ID No: 455 | Human | C-Terminal Domain PhosphoPeptide | pSer688 | | LAQApSQEL |
| SEQ ID No: 456 | Human | C-Terminal Domain PhosphoPeptide | pSer688 | | LAQApSQE |
| SEQ ID No: 457 | Human | C-Terminal Domain PhosphoPeptide | pSer688 | | LAQApSQ |
| SEQ ID No: 458 | Human | C-Terminal Domain PhosphoPeptide | pSer688 | | LAQApS |
| SEQ ID No: 459 | Human | C-Terminal Domain PhosphoPeptide | pSer688 | | AQApSQELQ |
| SEQ ID No: 460 | Human | C-Terminal Domain PhosphoPeptide | pSer688 | | AQApSQEL |
| SEQ ID No: 461 | Human | C-Terminal Domain PhosphoPeptide | pSer688 | | AQApSQE |
| SEQ ID No: 462 | Human | C-Terminal Domain PhosphoPeptide | pSer688 | | AQApSQ |
| SEQ ID No: 463 | Human | C-Terminal Domain PhosphoPeptide | pSer688 | | QApSQELQ |
| SEQ ID No: 464 | Human | C-Terminal Domain PhosphoPeptide | pSer688 | | QApSQEL |
| SEQ ID No: 465 | Human | C-Terminal Domain PhosphoPeptide | pSer688 | | QApSQE |
| SEQ ID No: 466 | Human | C-Terminal Domain PhosphoPeptide | pSer688 | | ApSQELQ |
| SEQ ID No: 467 | Human | C-Terminal Domain PhosphoPeptide | pSer688 | | ApSQEL |
| SEQ ID No: 468 | Human | C-Terminal Domain PhosphoPeptide | pSer688 | | pSQELQ |
| SEQ ID No: 469 | Human | C-Terminal Domain PhosphoPeptide + Linker | pSer688 | N: CGG | CGGSRHLAQApSQELQ |
| SEQ ID No: 470 | Human | C-Terminal Domain PhosphoPeptide + Linker | pSer688 | N: CGG | CGGSRHLAQApSQEL |
| SEQ ID No: 471 | Human | C-Terminal Domain PhosphoPeptide + Linker | pSer688 | N: CGG | CGGSRHLAQApSQE |
| SEQ ID No: 472 | Human | C-Terminal Domain PhosphoPeptide + Linker | pSer688 | N: CGG | CGGSRHLAQApSQ |
| SEQ ID No: 473 | Human | C-Terminal Domain PhosphoPeptide + Linker | pSer688 | N: CGG | CGGSRHLAQApS |
| SEQ ID No: 474 | Human | C-Terminal Domain PhosphoPeptide + Linker | pSer688 | N: CGG | CGGRHLAQApSQELQ |
| SEQ ID No: 475 | Human | C-Terminal Domain PhosphoPeptide + Linker | pSer688 | N: CGG | CGGRHLAQApSQEL |
| SEQ ID No: 476 | Human | C-Terminal Domain PhosphoPeptide + Linker | pSer688 | N: CGG | CGGRHLAQApSQE |
| SEQ ID No: 477 | Human | C-Terminal Domain PhosphoPeptide + Linker | pSer688 | N: CGG | CGGRHLAQApSQ |
| SEQ ID No: 478 | Human | C-Terminal Domain PhosphoPeptide + Linker | pSer688 | N: CGG | CGGRHLAQApS |
| SEQ ID No: 479 | Human | C-Terminal Domain PhosphoPeptide + Linker | pSer688 | N: CGG | CGGHLAQApSQELQ |
| SEQ ID No: 480 | Human | C-Terminal Domain PhosphoPeptide + Linker | pSer688 | N: CGG | CGGHLAQApSQEL |
| SEQ ID No: 481 | Human | C-Terminal Domain PhosphoPeptide + Linker | pSer688 | N: CGG | CGGHLAQApSQ |
| SEQ ID No: 482 | Human | C-Terminal Domain PhosphoPeptide + Linker | pSer688 | N: CGG | CGGHLAQApS |
| SEQ ID No: 483 | Human | C-Terminal Domain PhosphoPeptide + Linker | pSer688 | N: CGG | CGGLAQApSQELQ |
| SEQ ID No: 484 | Human | C-Terminal Domain PhosphoPeptide + Linker | pSer688 | N: CGG | CGGLAQApSQEL |
| SEQ ID No: 485 | Human | C-Terminal Domain PhosphoPeptide + Linker | pSer688 | N: CGG | CGGLAQApSQE |
| SEQ ID No: 486 | Human | C-Terminal Domain PhosphoPeptide + Linker | pSer688 | N: CGG | CGGLAQApSQ |
| SEQ ID No: 487 | Human | C-Terminal Domain PhosphoPeptide + Linker | pSer688 | N: CGG | CGGLAQApS |
| SEQ ID No: 488 | Human | C-Terminal Domain PhosphoPeptide + Linker | pSer688 | N: CGG | CGGAQApSQELQ |
| SEQ ID No: 489 | Human | C-Terminal Domain PhosphoPeptide + Linker | pSer688 | N: CGG | CGGAQApSQEL |
| SEQ ID No: 490 | Human | C-Terminal Domain PhosphoPeptide + Linker | pSer688 | N: CGG | CGGAQApSQE |
| SEQ ID No: 491 | Human | C-Terminal Domain PhosphoPeptide + Linker | pSer688 | N: CGG | CGGAQApSQ |
| SEQ ID No: 492 | Human | C-Terminal Domain PhosphoPeptide + Linker | pSer688 | N: CGG | CGGQApSQELQ |
| SEQ ID No: 493 | Human | C-Terminal Domain PhosphoPeptide + Linker | pSer688 | N: CGG | CGGQApSQEL |
| SEQ ID No: 494 | Human | C-Terminal Domain PhosphoPeptide + Linker | pSer688 | N: CGG | CGGQApSQE |
| SEQ ID No: 495 | Human | C-Terminal Domain PhosphoPeptide + Linker | pSer688 | N: CGG | CGGApSQELQ |
| SEQ ID No: 496 | Human | C-Terminal Domain PhosphoPeptide + Linker | pSer688 | N: CGG | CGGApSQEL |
| SEQ ID No: 497 | Human | C-Terminal Domain PhosphoPeptide + Linker | pSer688 | N: CGG | CGGpSQELQ |
| SEQ ID No: 498 | Human | C-Terminal Domain PhosphoPeptide + Linker | pSer688 | C: GGC | SRHLAQApSQELQGGC |
| SEQ ID No: 499 | Human | C-Terminal Domain PhosphoPeptide + Linker | pSer688 | | SRHLAQApSQELGGC |
| SEQ ID No: 500 | Human | C-Terminal Domain PhosphoPeptide + Linker | pSer688 | | SRHLAQApSQEGGC |
| SEQ ID No: 501 | Human | C-Terminal Domain PhosphoPeptide + Linker | pSer688 | | SRHLAQApSQGGC |
| SEQ ID No: 502 | Human | C-Terminal Domain PhosphoPeptide + Linker | pSer688 | | SRHLAQApSGGC |
| SEQ ID No: 503 | Human | C-Terminal Domain PhosphoPeptide + Linker | pSer688 | | RHLAQApSQELQGGC |
| SEQ ID No: 504 | Human | C-Terminal Domain PhosphoPeptide + Linker | pSer688 | | RHLAQApSQELGGC |
| SEQ ID No: 505 | Human | C-Terminal Domain PhosphoPeptide + Linker | pSer688 | | RHLAQApSQEGGC |
| SEQ ID No: 506 | Human | C-Terminal Domain PhosphoPeptide + Linker | pSer688 | | RHLAQApSQGGC |
| SEQ ID No: 507 | Human | C-Terminal Domain PhosphoPeptide + Linker | pSer688 | | RHLAQApSGGC |
| SEQ ID No: 508 | Human | C-Terminal Domain PhosphoPeptide + Linker | pSer688 | | HLAQApSQELQGGC |
| SEQ ID No: 509 | Human | C-Terminal Domain PhosphoPeptide + Linker | pSer688 | | HLAQApSQELGGC |
| SEQ ID No: 510 | Human | C-Terminal Domain PhosphoPeptide + Linker | pSer688 | | HLAQApSQGGC |
| SEQ ID No: 511 | Human | C-Terminal Domain PhosphoPeptide + Linker | pSer688 | | HLAQApSGGC |
| SEQ ID No: 512 | Human | C-Terminal Domain PhosphoPeptide + Linker | pSer688 | | LAQApSQELQGGC |
| SEQ ID No: 513 | Human | C-Terminal Domain PhosphoPeptide + Linker | pSer688 | | LAQApSQELGGC |
| SEQ ID No: 514 | Human | C-Terminal Domain PhosphoPeptide + Linker | pSer688 | | LAQApSQEGGC |
| SEQ ID No: 515 | Human | C-Terminal Domain PhosphoPeptide + Linker | pSer688 | | LAQApSQGGC |
| SEQ ID No: 516 | Human | C-Terminal Domain PhosphoPeptide + Linker | pSer688 | | LAQApSGGC |
| SEQ ID No: 517 | Human | C-Terminal Domain PhosphoPeptide + Linker | pSer688 | | AQApSQELQGGC |
| SEQ ID No: 518 | Human | C-Terminal Domain PhosphoPeptide + Linker | pSer688 | | AQApSQELGGC |
| SEQ ID No: 519 | Human | C-Terminal Domain PhosphoPeptide + Linker | pSer688 | | AQApSQEGGC |
| SEQ ID No: 520 | Human | C-Terminal Domain PhosphoPeptide + Linker | pSer688 | | AQApSQGGC |
| SEQ ID No: 521 | Human | C-Terminal Domain PhosphoPeptide + Linker | pSer688 | | QApSQELQGGC |
| SEQ ID No: 522 | Human | C-Terminal Domain PhosphoPeptide + Linker | pSer688 | | QApSQELGGC |
| SEQ ID No: 523 | Human | C-Terminal Domain PhosphoPeptide + Linker | pSer688 | | QApSQEGGC |
| SEQ ID No: 524 | Human | C-Terminal Domain PhosphoPeptide + Linker | pSer688 | | ApSQELQGGC |
| SEQ ID No: 525 | Human | C-Terminal Domain PhosphoPeptide + Linker | pSer688 | | ApSQELGGC |
| SEQ ID No: 526 | Human | C-Terminal Domain PhosphoPeptide + Linker | pSer688 | | pSQELQGGC |
| **SEQ ID No: 527** | **Mouse** | **C-Terminal Domain PhosphoPeptid e (9.58)** | **pSer691** | | CRSRPSAKApSWVQ |
| SEQ ID No: 528 | Mouse | C-Terminal Domain PhosphoPeptide | pSer691 | Native C | CRSRPSAKApSWV |
| SEQ ID No: 529 | Mouse | C-Terminal Domain PhosphoPeptide | pSer691 | Native C | CRSRPSAKApSW |
| SEQ ID No: 530 | Mouse | C-Terminal Domain PhosphoPeptide | pSer691 | Native C | CRSRPSAKApS |
| SEQ ID No: 531 | Mouse | C-Terminal Domain PhosphoPeptide | pSer691 | | RSRPSAKApSWVQ |
| SEQ ID No: 532 | Mouse | C-Terminal Domain PhosphoPeptide | pSer691 | | RSRPSAKApSWV |
| SEQ ID No: 533 | Mouse | C-Terminal Domain PhosphoPeptide | pSer691 | | RSRPSAKApSW |
| SEQ ID No: 534 | Mouse | C-Terminal Domain PhosphoPeptide | pSer691 | | RSRPSAKApS |
| SEQ ID No: 535 | Mouse | C-Terminal Domain PhosphoPeptide | pSer691 | | SRPSAKApSWVQ |
| SEQ ID No: 536 | Mouse | C-Terminal Domain PhosphoPeptide | pSer691 | | SRPSAKApSWV |
| SEQ ID No: 537 | Mouse | C-Terminal Domain PhosphoPeptide | pSer691 | | SRPSAKApSW |
| SEQ ID No: 538 | Mouse | C-Terminal Domain PhosphoPeptide | pSer691 | | SRPSAKApS |
| SEQ ID No: 539 | Mouse | C-Terminal Domain PhosphoPeptide | pSer691 | | RPSAKApSWVQ |
| SEQ ID No: 540 | Mouse | C-Terminal Domain PhosphoPeptide | pSer691 | | RPSAKApSWV |
| SEQ ID No: 541 | Mouse | C-Terminal Domain PhosphoPeptide | pSer691 | | RPSAKApSW |
| SEQ ID No: 542 | Mouse | C-Terminal Domain PhosphoPeptide | pSer691 | | RPSAKApS |
| SEQ ID No: 543 | Mouse | C-Terminal Domain PhosphoPeptide | pSer691 | | PSAKApSWVQ |
| SEQ ID No: 544 | Mouse | C-Terminal Domain PhosphoPeptide | pSer691 | | PSAKApSWV |
| SEQ ID No: 545 | Mouse | C-Terminal Domain PhosphoPeptide | pSer691 | | PSAKApSW |
| SEQ ID No: 546 | Mouse | C-Terminal Domain PhosphoPeptide | pSer691 | | PSAKApS |
| SEQ ID No: 547 | Mouse | C-Terminal Domain PhosphoPeptide | pSer691 | | SAKApS |
| SEQ ID No: 548 | Mouse | C-Terminal Domain PhosphoPeptide + Linker | pSer691 | N: CGG | CGGRSRPSAKApSWVQ |
| SEQ ID No: 549 | Mouse | C-Terminal Domain PhosphoPeptide + Linker | pSer691 | N: CGG | CGGRSRPSAKApSWV |
| SEQ ID No: 550 | Mouse | C-Terminal Domain PhosphoPeptide + Linker | pSer691 | N: CGG | CGGRSRPSAKApSW |
| SEQ ID No: 551 | Mouse | C-Terminal Domain PhosphoPeptide + Linker | pSer691 | N: CGG | CGGRSRPSAKApS |
| SEQ ID No: 552 | Mouse | C-Terminal Domain PhosphoPeptide + Linker | pSer691 | N: CGG | CGGSRPSAKApSWVQ |
| SEQ ID No: 553 | Mouse | C-Terminal Domain PhosphoPeptide + Linker | pSer691 | N: CGG | CGGSRPSAKApSWV |
| SEQ ID No: 554 | Mouse | C-Terminal Domain PhosphoPeptide + Linker | pSer691 | N: CGG | CGGSRPSAKApSW |
| SEQ ID No: 555 | Mouse | C-Terminal Domain PhosphoPeptide + Linker | pSer691 | N: CGG | CGGSRPSAKApS |
| SEQ ID No: 556 | Mouse | C-Terminal Domain PhosphoPeptide + Linker | pSer691 | N: CGG | CGGRPSAKApSWVQ |
| SEQ ID No: 557 | Mouse | C-Terminal Domain PhosphoPeptide + Linker | pSer691 | N: CGG | CGGRPSAKApSWV |
| SEQ ID No: 558 | Mouse | C-Terminal Domain PhosphoPeptide + Linker | pSer691 | N: CGG | CGGRPSAKApSW |
| SEQ ID No: 559 | Mouse | C-Terminal Domain PhosphoPeptide + Linker | pSer691 | N: CGG | CGGRPSAKApS |
| SEQ ID No: 560 | Mouse | C-Terminal Domain PhosphoPeptide + Linker | pSer691 | N: CGG | CGGPSAKApSWVQ |
| SEQ ID No: 561 | Mouse | C-Terminal Domain PhosphoPeptide + Linker | pSer691 | N: CGG | CGGPSAKApSWV |
| SEQ ID No: 562 | Mouse | C-Terminal Domain PhosphoPeptide + Linker | pSer691 | N: CGG | CGGPSAKApSW |
| SEQ ID No: 563 | Mouse | C-Terminal Domain PhosphoPeptide + Linker | pSer691 | N: CGG | CGGPSAKApS |
| SEQ ID No: 564 | Mouse | C-Terminal Domain PhosphoPeptide + Linker | pSer691 | N: CGG | CGGSAKApS |
| SEQ ID No: 565 | Mouse | C-Terminal Domain PhosphoPeptide + Linker | pSer691 | C: GGC | RSRPSAKApSWVQGGC |
| SEQ ID No: 566 | Mouse | C-Terminal Domain PhosphoPeptide + Linker | pSer691 | C: GGC | RSRPSAKApSWVGGC |
| SEQ ID No: 567 | Mouse | C-Terminal Domain PhosphoPeptide + Linker | pSer691 | C: GGC | RSRPSAKApSWGGC |
| SEQ ID No: 568 | Mouse | C-Terminal Domain PhosphoPeptide + Linker | pSer691 | C: GGC | RSRPSAKApSGGC |
| SEQ ID No: 569 | Mouse | C-Terminal Domain PhosphoPeptide + Linker | pSer691 | C: GGC | SRPSAKApSWVQGGC |
| SEQ ID No: 570 | Mouse | C-Terminal Domain PhosphoPeptide + Linker | pSer691 | C: GGC | SRPSAKApSWVGGC |
| SEQ ID No: 571 | Mouse | C-Terminal Domain PhosphoPeptide + Linker | pSer691 | C: GGC | SRPSAKApSWGGC |
| SEQ ID No: 572 | Mouse | C-Terminal Domain PhosphoPeptide + Linker | pSer691 | C: GGC | SRPSAKApSGGC |
| SEQ ID No: 573 | Mouse | C-Terminal Domain PhosphoPeptide + Linker | pSer691 | C: GGC | RPSAKApSWVQGGC |
| SEQ ID No: 574 | Mouse | C-Terminal Domain PhosphoPeptide + Linker | pSer691 | C: GGC | RPSAKApSWVGGC |
| SEQ ID No: 575 | Mouse | C-Terminal Domain PhosphoPeptide + Linker | pSer691 | C: GGC | RPSAKApSWGGC |
| SEQ ID No: 576 | Mouse | C-Terminal Domain PhosphoPeptide + Linker | pSer691 | C: GGC | RPSAKApSGGC |
| SEQ ID No: 577 | Mouse | C-Terminal Domain PhosphoPeptide + Linker | pSer691 | C: GGC | PSAKApSWVQGGC |
| SEQ ID No: 578 | Mouse | C-Terminal Domain PhosphoPeptide + Linker | pSer691 | C: GGC | PSAKApSWVGGC |
| SEQ ID No: 579 | Mouse | C-Terminal Domain PhosphoPeptide + Linker | pSer691 | C: GGC | PSAKApSWGGC |
| SEQ ID No: 580 | Mouse | C-Terminal Domain PhosphoPeptide + Linker | pSer691 | C: GGC | PSAKApSGGC |
| SEQ ID No: 581 | Mouse | C-Terminal Domain PhosphoPeptide + Linker | pSer691 | C: GGC | SAKApSGGC |
| SEQ ID No: 582 | | CpG ODN | | | TCGTCGTTTTTCGGTGCTTTT |
| SEQ ID No: 583 | | CpG ODN | | | TCGTCGTTTTTCGGTCGTTTT |
| SEQ ID No: 584 | | CpG ODN | | | TCGTCGTTTTGTCGTTTTGTCGTT |
| SEQ ID No: 585 | | CpG ODN | | | TCGTCGTTTCGTCGTTTTGTCGTT |
| SEQ ID No: 586 | | CpG ODN | | | TCGTCGTTTTGTCGTTTTTTTCGA |
| SEQ ID No: 587 | | CpG ODN | | | TCGCGTCGTTCGGCGCGCGCCG |
| SEQ ID No: 588 | | CpG ODN | | | TCGTCGACGTTCGGCGCGCGCCG |
| SEQ ID No: 589 | | CpG ODN | | | TCGGACGTTCGGCGCGCGCCG |
| SEQ ID No: 590 | | CpG ODN | | | TCGGACGTTCGGCGCGCCG |
| SEQ ID No: 591 | | CpG ODN | | | TCGCGTCGTTCGGCGCGCCG |
| SEQ ID No: 592 | | CpG ODN | | | TCGACGTTCGGCGCGCGCCG |
| SEQ ID No: 593 | | CpG ODN | | | TCGACGTTCGGCGCGCCG |
| SEQ ID No: 594 | | CpG ODN | | | TCGCGTCGTTCGGCGCCG |
| SEQ ID No: 595 | | CpG ODN | | | TCGCGACGTTCGGCGCGCGCCG |
| SEQ ID No: 596 | | CpG ODN | | | TCGTCGTTTTCGGCGCGCGCCG |
| SEQ ID No: 597 | | CpG ODN | | | TCGTCGTTTTCGGCGGCCGCCG |
| SEQ ID No: 598 | | CpG ODN | | | TCGTCGTTTTACGGCGCCGTGCCG |
| SEQ ID No: 599 | | CpG ODN | | | TCGTCGTTTTCGGCGCGCGCCGT |
| SEQ ID No: 600 | | CpG ODN | | | TCGTCGACGATCGGCGCGCGCCG |

### Key to Sequence Listing (from Table 8):

| **Description** | **Sequence ID No.** |
|---|---|
| Human PCSK9 | **1** |
| Mouse PCSK9 | **2** |
| Human ProDomain | **3** |
| | |
| Human ProDomain Peptide | **4 to 29** |
| Mouse ProDomain Peptide | **30-54** |
| Human ProDomain Peptide + Linkers | **55-98** |
| Mouse ProDomain Peptide + Linkers | **99-148** |
| | |
| Human ProDomain PhosphoPeptide | **149-172** |
| Mouse ProDomain PhosphoPeptide | **173-198** |
| Human ProDomain PhosphoPeptide + Linkers | **199-242** |
| Mouse ProDomain PhosphoPeptide + Linkers | **243-286** |
| | |
| Human CTD Peptide | **287-320** |
| Mouse CTD Peptide | **379-399** |
| Human CTD Peptide + Linkers | **321-378** |
| Mouse CTD Peptide + Linkers | **400-433** |
| | |
| Human CTD PhosphoPeptide | **434-468** |
| Mouse CTD PhosphoPeptide | **527-547** |
| Human CTD PhosphoPeptide + Linkers | **469-526** |
| Mouse CTD PhosphoPeptide + Linkers | **548-581** |
| | |
| CpG ODN | **582-600** |

NB: The designation of a "p" before a listed amino acid indicates phosporylation at that amino acid residue

## Claims

1. An immunogen comprising at least one antigenic PCSK9 peptide containing a phosphorylation site linked to an immunogenic carrier, wherein said antigenic PCSK9 peptide containing a phosphorylation site is SEQ ID No. 12 and wherein said immunogenic carrier is selected from Diphtheria Toxoid, CRM197 or a VLP selected from HBcAg, HBsAg, Qbeta, PP7, PPV or Norwalk Virus VLP.

2. The immunogen according to claim 1 wherein:
(i) said antigenic PCSK9 peptide further comprises at its C-terminus a linker having the formula (G)ₙC, (G)ₙSC or (G)ₙK wherein n is an integer chosen from the group consisting of 0, 1, 2, 3, 4, 5, 6, 7, 8, 9 and 10; or
(ii) said antigenic PCSK9 peptide containing a phosphorylation site further comprises at its N-terminus a linker having the formula C(G)ₙ, CS(G)ₙ or K(G)ₙ; wherein n is an integer chosen from the group consisting of 0, 1, 2, 3, 4, 5 ,6, 7, 8, 9 or 10.

3. The immunogen according to claim 1 wherein said antigenic PCSK9 peptide containing a phosphorylation site further comprises GGC at its C-terminus.

4. The immunogen according to claim 1 wherein said antigenic PCSK9 peptide containing a phosphorylation site further comprises KG or KGG at its N-terminus.

5. An immunogen according to claim 3 wherein said antigenic PCSK9 peptide containing a phosphorylation site and further comprising GGC at its C-terminus is selected from the group consisting of SEQ ID Nos. 86 and 224.

6. The immunogen according to any one of claims 1 to 5 wherein said immunogen is able, when administered to a subject, to lower the LDL-cholesterol level in blood of a subject by at least 2%, 5%, 10%, 20% or 30%.

7. A pharmaceutical composition comprising the immunogen of any one of claims 1 to 6, and a pharmaceutically acceptable excipient.

8. The immunogen of any one of claims 1 to 6, or the pharmaceutical composition of claim 7, for use as a medicament.

9. The immunogen of any one of claims 1 to 6, or the pharmaceutical composition of claim 7, for use in a method of preventing, alleviating or treating elevated LDL-cholesterol.

10. The immunogen of any one of claims 1 to 6, or the pharmaceutical composition of claim 7, for use in a method of preventing, alleviating or treating a lipid disorder selected from hyperlipidemia, type I, type II, type III, type IV, or type V hyperlipidemia, secondary hypertriglyceridemia, hypercholesterolemia, familial hypercholesterolemia, xanthomatosis, and cholesterol acetyltransferase deficiency; an arteriosclerotic condition (e.g., atherosclerosis), a coronary artery disease, and a cardiovascular disease.

## Patentansprüche

1. Immunogen, umfassend mindestens ein antigenes PCSK9-Peptid, das eine Phosphorylierungsstelle enthält, gebunden an einen immunogenen Carrier, wobei besagtes antigenes PCSK9-Peptid, das eine Phosphorylierungsstelle enthält die SEQ ID NO:12 ist, und wobei besagter immunogener Carrier selektiert wird aus Diphtherietoxoid, CRM197 oder einem VLP, selektiert aus HBcAg, HBsAg, Qbeta, PP7, PPV oder Norwalk-Virus-VLP.

2. Immunogen gemäß Anspruch 1, wobei:
(i) besagtes antigenes PCSK9-Peptid ferner an seinem C-Terminus einen Linker mit der Formel (G)ₙC, (G)ₙSC oder (G)ₙK umfasst, wobei n eine ganze Zahl ist, ausgewählt aus der Gruppe, bestehend aus 0, 1, 2, 3, 4, 5, 6, 7, 8, 9 und 10, oder
(ii) besagtes antigenes PCSK9-Peptid, das eine Phosporylierungsstelle enthält, ferner an seinem N-Terminus einen Linker mit der Formel C(G)ₙ, CS(G)ₙ oder K(G)ₙ umfasst; wobei n eine ganze Zahl ist, ausgewählt aus der Gruppe, bestehend aus 0, 1, 2, 3, 4, 5, 6, 7, 8, 9 oder 10.

3. Immunogen gemäß Anspruch 1, wobei besagtes antigenes PCSK9-Peptid, das eine Phosphorylierungsstelle enthält, ferner GGC an seinem C-Terminus umfasst.

4. Immunogen gemäß Anspruch 1, wobei besagtes antigenes PCSK9-Peptid, das eine Phosphorylierungsstelle enthält, ferner KG oder KGG an seinem N-Terminus umfasst.

5. Immunogen gemäß Anspruch 3, wobei besagtes antigenes PCSK9-Peptid, das eine Phosphorylierungsstelle enthält und ferner GGC an seinem C-Terminus umfasst, selektiert wird aus einer Gruppe, bestehend aus SEQ ID NOS:86 und 224.

6. Immunogen gemäß einem der Ansprüche 1 bis 5, wobei besagtes Immunogen bei Verabreichung an ein Subjekt in der Lage ist, den LDL-Cholesterinspiegel im Blut des Subjekts um mindestens 2%, 5%, 10%, 20% oder 30% zu senken.

7. Pharmazeutische Zusammensetzung, umfassend das Immunogen gemäß einem der Ansprüche 1 bis 6 und einen pharmazeutisch annehmbaren Exzipienten.

8. Immunogen gemäß einem der Ansprüche 1 bis 6 oder pharmazeutische Zusammensetzung gemäß Anspruch 7 zur Verwendung als Medikament.

9. Immunogen gemäß einem der Ansprüche 1 bis 6 oder pharmazeutische Zusammensetzung gemäß Anspruch 7 zur Verwendung in einem Verfahren zur Vorbeugung, Linderung oder Behandlung von erhöhtem LDL-Cholesterin.

10. Immunogen gemäß einem der Ansprüche 1 bis 6 oder pharmazeutische Zusammensetzung gemäß Anspruch 7 zur Verwendung in einem Verfahren zur Vorbeugung, Linderung oder Behandlung einer Störung im Lipidstoffwechsel, selektiert aus Hyperlipidämie, Hyperlipidämie vom Typ I, Typ II, Typ III, Typ IV oder Typ V, sekundärer Hypertriglyceridämie, Hypercholesterinämie, familiärer Hypercholesterinämie, Xanthomatose und Cholesterin-Acetyltransferasedefizienz; einem Arterioskleroseleiden (z.B. Atherosklerose), einer koronaren Herzkrankheit und einer kardiovaskulären Krankheit.

## Revendications

1. Immunogène comprenant au moins un peptide PCSK9 antigénique contenant un site de phosphorylation lié à un support immunogène, dans lequel ledit peptide PCSK9 antigénique contenant un site de phosphorylation est SEQ ID NO : 12 et dans lequel ledit support immunogène est choisi parmi l'anatoxine diphtérique, la CRM197 ou une VLP choisie parmi des VLP de l'Ag HBc, l'Ag HBs, de Qbêta, de PP7, de PPV ou de virus de Norwalk.

2. Immunogène selon la revendication 1 dans lequel :
(i) ledit peptide PCSK9 antigénique comprend en outre à son extrémité C-terminale un lieur ayant la formule (G)ₙC, (G)ₙSC ou (G)ₙK dans laquelle n est un nombre entier choisi dans le groupe constitué de 0, 1, 2, 3, 4, 5, 6, 7, 8, 9 et 10 ; ou
(ii) ledit peptide PCSK9 antigénique contenant un site de phosphorylation comprend en outre à son extrémité N-terminale un lieur ayant la formule C(G)ₙ, CS(G)ₙ ou K(G)ₙ ; dans laquelle n est un nombre entier choisi dans le groupe constitué de 0, 1, 2, 3, 4, 5, 6, 7, 8, 9 et 10.

3. Immunogène selon la revendication 1 dans lequel ledit peptide PCSK9 antigénique contenant un site de phosphorylation comprend en outre GGC à son extrémité C-terminale.

4. Immunogène selon la revendication 1 dans lequel ledit peptide PCSK9 antigénique contenant un site de phosphorylation comprend en outre KG ou KGG à son extrémité N-terminale.

5. Immunogène selon la revendication 3 dans lequel ledit peptide PCSK9 antigénique contenant un site de phosphorylation et comprenant en outre GGC à son extrémité C-terminale est choisi dans le groupe constitué de SEQ ID NO : 86 et 224.

6. Immunogène selon l'une quelconque des revendications 1 à 5 dans lequel ledit immunogène est capable, lorsqu'il est administré à un sujet, d'abaisser le taux de LDL-cholestérol dans le sang d'un sujet d'au moins 2 %, 5 %, 10 %, 20 % ou 30 %.

7. Composition pharmaceutique comprenant l'immunogène selon l'une quelconque des revendications 1 à 6, et un excipient pharmaceutiquement acceptable.

8. Immunogène selon l'une quelconque des revendications 1 à 6, ou composition pharmaceutique selon la revendication 7, pour une utilisation en tant que médicament.

9. Immunogène selon l'une quelconque des revendications 1 à 6, ou composition pharmaceutique selon la revendication 7, pour une utilisation dans un procédé de prévention, de soulagement ou de traitement de LDL-cholestérol élevé.

10. Immunogène selon l'une quelconque des revendications 1 à 6, ou composition pharmaceutique selon la revendication 7, pour une utilisation dans un procédé de prévention, de soulagement ou de traitement d'un trouble lipidique choisi parmi une hyperlipidémie, l'hyperlipidémie de type I, de type II, de type III, de type IV, ou de type V, une hypertriglycéridémie secondaire, une hypercholestérolémie, une hypercholestérolémie familiale, une xanthomatose, et un déficit en cholestérol acétyltransférase ; une affection artérioscléreuse (par exemple, l'athérosclérose), une maladie coronarienne, et une maladie cardiovasculaire.


### SEQUENCE LISTING

<110> Pfizer Inc.
<120> PCSK9 VACCINE
<130> PC71776A
<150> US 61/448,398
   <151> 2011-03-02
<160> 600
<170> PatentIn version 3.5
<210> 1
   <211> 692
   <212> PRT
   <213> Homo sapiens
<400> 1
<210> 2
   <211> 694
   <212> PRT
   <213> Mus musculus
<400> 2
   Met Gly Thr His Cys Ser Ala Trp Leu Arg Trp Pro Leu Leu Pro Leu
   1 5 10 15
   Leu Pro Pro Leu Leu Leu Leu Leu Leu Leu Leu Cys Pro Thr Gly Ala
   20 25 30
   Gly Ala Gln Asp Glu Asp Gly Asp Tyr Glu Glu Leu Met Leu Ala Leu
   35 40 45
   Pro Ser Gln Glu Asp Gly Leu Ala Asp Glu Ala Ala His Val Ala Thr
   50 55 60
   Ala Thr Phe Arg Arg Cys Ser Lys Glu Ala Trp Arg Leu Pro Gly Thr
   65 70 75 80
   Tyr Ile Val Val Leu Met Glu Glu Thr Gln Arg Leu Gln Ile Glu Gln
   85 90 95
   Thr Ala His Arg Leu Gln Thr Arg Ala Ala Arg Arg Gly Tyr Val Ile
   100 105 110
   Lys Val Leu His Ile Phe Tyr Asp Leu Phe Pro Gly Phe Leu Val Lys
   115 120 125
   Met Ser Ser Asp Leu Leu Gly Leu Ala Leu Lys Leu Pro His Val Glu
   130 135 140
   Tyr Ile Glu Glu Asp Ser Phe Val Phe Ala Gln Ser Ile Pro Trp Asn
   145 150 155 160
   Leu Glu Arg Ile Ile Pro Ala Trp His Gln Thr Glu Glu Asp Arg Ser
   165 170 175
   Pro Asp Gly Ser Ser Gln Val Glu Val Tyr Leu Leu Asp Thr Ser Ile
   180 185 190
   Gln Gly Ala His Arg Glu Ile Glu Gly Arg Val Thr Ile Thr Asp Phe
           195                 200                 205
   Asn Ser Val Pro Glu Glu Asp Gly Thr Arg Phe His Arg Gln Ala Ser
       210                 215                 220
   Lys Cys Asp Ser His Gly Thr His Leu Ala Gly Val Val Ser Gly Arg
   225                 230                 235                 240
   Asp Ala Gly Val Ala Lys Gly Thr Ser Leu His Ser Leu Arg Val Leu
                   245                 250                 255
   Asn Cys Gln Gly Lys Gly Thr Val Ser Gly Thr Leu Ile Gly Leu Glu
               260                 265                 270
   Phe Ile Arg Lys Ser Gln Leu Ile Gln Pro Ser Gly Pro Leu Val Val
           275                 280                 285
   Leu Leu Pro Leu Ala Gly Gly Tyr Ser Arg Ile Leu Asn Ala Ala Cys
       290                 295                 300
   Arg His Leu Ala Arg Thr Gly Val Val Leu Val Ala Ala Ala Gly Asn
   305                 310                 315                 320
   Phe Arg Asp Asp Ala Cys Leu Tyr Ser Pro Ala Ser Ala Pro Glu Val
                   325                 330                 335
   Ile Thr Val Gly Ala Thr Asn Ala Gln Asp Gln Pro Val Thr Leu Gly
               340                 345                 350
   Thr Leu Gly Thr Asn Phe Gly Arg Cys Val Asp Leu Phe Ala Pro Gly
           355                 360                 365
   Lys Asp Ile Ile Gly Ala Ser Ser Asp Cys Ser Thr Cys Phe Met Ser
       370                 375                 380
   Gln Ser Gly Thr Ser Gln Ala Ala Ala His Val Ala Gly Ile Val Ala
   385                 390                 395                 400
   Arg Met Leu Ser Arg Glu Pro Thr Leu Thr Leu Ala Glu Leu Arg Gln
                   405                 410                 415
   Arg Leu Ile His Phe Ser Thr Lys Asp Val Ile Asn Met Ala Trp Phe
               420                 425                 430
   Pro Glu Asp Gln Gln Val Leu Thr Pro Asn Leu Val Ala Thr Leu Pro
           435                 440                 445
   Pro Ser Thr His Glu Thr Gly Gly Gln Leu Leu Cys Arg Thr Val Trp
   450 455 460
   Ser Ala His Ser Gly Pro Thr Arg Thr Ala Thr Ala Thr Ala Arg Cys
   465 470 475 480
   Ala Pro Glu Glu Glu Leu Leu Ser Cys Ser Ser Phe Ser Arg Ser Gly
   485 490 495
   Arg Arg Arg Gly Asp Trp Ile Glu Ala Ile Gly Gly Gln Gln Val Cys
   500 505 510
   Lys Ala Leu Asn Ala Phe Gly Gly Glu Gly Val Tyr Ala Val Ala Arg
   515 520 525
   Cys Cys Leu Val Pro Arg Ala Asn Cys Ser Ile His Asn Thr Pro Ala
   530 535 540
   Ala Arg Ala Gly Leu Glu Thr His Val His Cys His Gln Lys Asp His
   545 550 555 560
   Val Leu Thr Gly Cys Ser Phe His Trp Glu Val Glu Asp Leu Ser Val
   565 570 575
   Arg Arg Gln Pro Ala Leu Arg Ser Arg Arg Gln Pro Gly Gln Cys Val
   580 585 590
   Gly His Gln Ala Ala Ser Val Tyr Ala Ser Cys Cys His Ala Pro Gly
   595 600 605
   Leu Glu Cys Lys Ile Lys Glu His Gly Ile Ser Gly Pro Ser Glu Gln
   610 615 620
   Val Thr Val Ala Cys Glu Ala Gly Trp Thr Leu Thr Gly Cys Asn Val
   625 630 635 640
   Leu Pro Gly Ala Ser Leu Thr Leu Gly Ala Tyr Ser Val Asp Asn Leu
   645 650 655
   Cys Val Ala Arg Val His Asp Thr Ala Arg Ala Asp Arg Thr Ser Gly
   660 665 670
   Glu Ala Thr Val Ala Ala Ala Ile Cys Cys Arg Ser Arg Pro Ser Ala
   675 680 685
   Lys Ala Ser Trp Val Gln
   690
<210> 3
   <211> 152
   <212> PRT
   <213> Homo sapiens
<400> 3
   Met Gly Thr Val Ser Ser Arg Arg Ser Trp Trp Pro Leu Pro Leu Leu
   1               5                   10                  15
   Leu Leu Leu Leu Leu Leu Leu Gly Pro Ala Gly Ala Arg Ala Gln Glu
               20                  25                  30
   Asp Glu Asp Gly Asp Tyr Glu Glu Leu Val Leu Ala Leu Arg Ser Glu
           35                  40                  45
   Glu Asp Gly Leu Ala Glu Ala Pro Glu His Gly Thr Thr Ala Thr Phe
       50                  55                  60
   His Arg Cys Ala Lys Asp Pro Trp Arg Leu Pro Gly Thr Tyr Val Val
   65                  70                  75                  80
   Val Leu Lys Glu Glu Thr His Leu Ser Gln Ser Glu Arg Thr Ala Arg
                   85                  90                  95
   Arg Leu Gln Ala Gln Ala Ala Arg Arg Gly Tyr Leu Thr Lys Ile Leu
               100                 105                 110
   His Val Phe His Gly Leu Leu Pro Gly Phe Leu Val Lys Met Ser Gly
           115                 120                 125
   Asp Leu Leu Glu Leu Ala Leu Lys Leu Pro His Val Asp Tyr Ile Glu
       130                 135                 140
   Glu Asp Ser Ser Val Phe Ala Gln
   145                 150
<210> 4
   <211> 16
   <212> PRT
   <213> Homo sapiens
<400> 4
   Gly Asp Tyr Glu Glu Leu Val Leu Ala Leu Arg Ser Glu Glu Asp Gly
   1               5                   10                  15
<210> 5
   <211> 11
   <212> PRT
   <213> Homo sapiens
<400> 5
   Leu Val Leu Ala Leu Arg Ser Glu Glu Asp Gly
   1 5 10
<210> 6
   <211> 10
   <212> PRT
   <213> Homo sapiens
<400> 6
   Val Leu Ala Leu Arg Ser Glu Glu Asp Gly
   1 5 10
<210> 7
   <211> 9
   <212> PRT
   <213> Homo sapiens
<400> 7
   Leu Ala Leu Arg Ser Glu Glu Asp Gly
   1 5
<210> 8
   <211> 8
   <212> PRT
   <213> Homo sapiens
<400> 8
   Ala Leu Arg Ser Glu Glu Asp Gly
   1 5
<210> 9
   <211> 7
   <212> PRT
   <213> Homo sapiens
<400> 9
   Leu Arg Ser Glu Glu Asp Gly
   1 5
<210> 10
   <211> 6
   <212> PRT
   <213> Homo sapiens
<400> 10
   Arg Ser Glu Glu Asp Gly
   1 5
<210> 11
   <211> 5
   <212> PRT
   <213> Homo sapiens
<400> 11
   Ser Glu Glu Asp Gly
   1 5
<210> 12
   <211> 10
   <212> PRT
   <213> Homo sapiens
<400> 12
   Leu Val Leu Ala Leu Arg Ser Glu Glu Asp
   1 5 10
<210> 13
   <211> 9
   <212> PRT
   <213> Homo sapiens
<400> 13
   Val Leu Ala Leu Arg Ser Glu Glu Asp
   1 5
<210> 14
   <211> 8
   <212> PRT
   <213> Homo sapiens
<400> 14
   Leu Ala Leu Arg Ser Glu Glu Asp
   1 5
<210> 15
   <211> 7
   <212> PRT
   <213> Homo sapiens
<400> 15
   Ala Leu Arg Ser Glu Glu Asp
   1 5
<210> 16
   <211> 6
   <212> PRT
   <213> Homo sapiens
<400> 16
   Leu Arg Ser Glu Glu Asp
   1 5
<210> 17
   <211> 5
   <212> PRT
   <213> Homo sapiens
<400> 17
   Arg Ser Glu Glu Asp
   1 5
<210> 18
   <211> 9
   <212> PRT
   <213> Homo sapiens
<400> 18
   Leu Val Leu Ala Leu Arg Ser Glu Glu
   1 5
<210> 19
   <211> 8
   <212> PRT
   <213> Homo sapiens
<400> 19
   Val Leu Ala Leu Arg Ser Glu Glu
   1 5
<210> 20
   <211> 7
   <212> PRT
   <213> Homo sapiens
<400> 20
   Leu Ala Leu Arg Ser Glu Glu
   1 5
<210> 21
   <211> 6
   <212> PRT
   <213> Homo sapiens
<400> 21
   Ala Leu Arg Ser Glu Glu
   1 5
<210> 22
   <211> 5
   <212> PRT
   <213> Homo sapiens
<400> 22
   Leu Arg Ser Glu Glu
   1               5
<210> 23
   <211> 8
   <212> PRT
   <213> Homo sapiens
<400> 23
   Leu Val Leu Ala Leu Arg Ser Glu
   1               5
<210> 24
   <211> 7
   <212> PRT
   <213> Homo sapiens
<400> 24
   Val Leu Ala Leu Arg Ser Glu
   1               5
<210> 25
   <211> 6
   <212> PRT
   <213> Homo sapiens
<400> 25
   Leu Ala Leu Arg Ser Glu
   1               5
<210> 26
   <211> 5
   <212> PRT
   <213> Homo sapiens
<400> 26
   Ala Leu Arg Ser Glu
   1               5
<210> 27
   <211> 7
   <212> PRT
   <213> Homo sapiens
<400> 27
   Leu Val Leu Ala Leu Arg Ser
   1               5
<210> 28
   <211> 6
   <212> PRT
   <213> Homo sapiens
<400> 28
   Val Leu Ala Leu Arg Ser
   1 5
<210> 29
   <211> 5
   <212> PRT
   <213> Homo sapiens
<400> 29
   Leu Ala Leu Arg Ser
   1 5
<210> 30
   <211> 11
   <212> PRT
   <213> Mus musculus
<400> 30
   Leu Met Leu Ala Leu Pro Ser Gln Glu Asp Gly
   1 5 10
<210> 31
   <211> 10
   <212> PRT
   <213> Mus musculus
<400> 31
   Met Leu Ala Leu Pro Ser Gln Glu Asp Gly
   1 5 10
<210> 32
   <211> 9
   <212> PRT
   <213> Mus musculus
<400> 32
   Leu Ala Leu Pro Ser Gln Glu Asp Gly
   1 5
<210> 33
   <211> 8
   <212> PRT
   <213> Mus musculus
<400> 33
   Ala Leu Pro Ser Gln Glu Asp Gly
   1               5
<210> 34
   <211> 7
   <212> PRT
   <213> Mus musculus
<400> 34
   Leu Pro Ser Gln Glu Asp Gly
   1               5
<210> 35
   <211> 6
   <212> PRT
   <213> Mus musculus
<400> 35
   Pro Ser Gln Glu Asp Gly
   1               5
<210> 36
   <211> 5
   <212> PRT
   <213> Mus musculus
<400> 36
   Ser Gln Glu Asp Gly
   1               5
<210> 37
   <211> 10
   <212> PRT
   <213> Mus musculus
<400> 37
   Leu Met Leu Ala Leu Pro Ser Gln Glu Asp
   1               5                   10
<210> 38
   <211> 9
   <212> PRT
   <213> Mus musculus
<400> 38
   Met Leu Ala Leu Pro Ser Gln Glu Asp
   1               5
<210> 39
   <211> 8
   <212> PRT
   <213> Mus musculus
<400> 39
   Leu Ala Leu Pro Ser Gln Glu Asp
   1 5
<210> 40
   <211> 7
   <212> PRT
   <213> Mus musculus
<400> 40
   Ala Leu Pro Ser Gln Glu Asp
   1 5
<210> 41
   <211> 6
   <212> PRT
   <213> Mus musculus
<400> 41
   Leu Pro Ser Gln Glu Asp
   1 5
<210> 42
   <211> 5
   <212> PRT
   <213> Mus musculus
<400> 42
   Pro Ser Gln Glu Asp
   1 5
<210> 43
   <211> 9
   <212> PRT
   <213> Mus musculus
<400> 43
   Leu Met Leu Ala Leu Pro Ser Gln Glu
   1 5
<210> 44
   <211> 8
   <212> PRT
   <213> Mus musculus
<400> 44
   Met Leu Ala Leu Pro Ser Gln Glu
   1 5
<210> 45
   <211> 7
   <212> PRT
   <213> Mus musculus
<400> 45
   Leu Ala Leu Pro Ser Gln Glu
   1 5
<210> 46
   <211> 6
   <212> PRT
   <213> Mus musculus
<400> 46
   Ala Leu Pro Ser Gln Glu
   1 5
<210> 47
   <211> 5
   <212> PRT
   <213> Mus musculus
<400> 47
   Leu Pro Ser Gln Glu
   1 5
<210> 48
   <211> 8
   <212> PRT
   <213> Mus musculus
<400> 48
   Leu Met Leu Ala Leu Pro Ser Gln
   1 5
<210> 49
   <211> 7
   <212> PRT
   <213> Mus musculus
<400> 49
   Met Leu Ala Leu Pro Ser Gln
   1 5
<210> 50
   <211> 6
   <212> PRT
   <213> Mus musculus
<400> 50
   Leu Ala Leu Pro Ser Gln
   1 5
<210> 51
   <211> 5
   <212> PRT
   <213> Mus musculus
<400> 51
   Ala Leu Pro Ser Gln
   1               5
<210> 52
   <211> 7
   <212> PRT
   <213> Mus musculus
<400> 52
   Leu Met Leu Ala Leu Pro Ser
   1               5
<210> 53
   <211> 6
   <212> PRT
   <213> Mus musculus
<400> 53
   Met Leu Ala Leu Pro Ser
   1               5
<210> 54
   <211> 5
   <212> PRT
   <213> Mus musculus
<400> 54
   Leu Ala Leu Pro Ser
   1               5
<210> 55
   <211> 14
   <212> PRT
   <213> Homo sapiens
<400> 55
   Cys Gly Gly Leu Val Leu Ala Leu Arg Ser Glu Glu Asp Gly
   1               5                   10
<210> 56
   <211> 13
   <212> PRT
   <213> Homo sapiens
<400> 56
   Cys Gly Gly Val Leu Ala Leu Arg Ser Glu Glu Asp Gly
   1 5 10
<210> 57
   <211> 12
   <212> PRT
   <213> Homo sapiens
<400> 57
   Cys Gly Gly Leu Ala Leu Arg Ser Glu Glu Asp Gly
   1 5 10
<210> 58
   <211> 11
   <212> PRT
   <213> Homo sapiens
<400> 58
   Cys Gly Gly Ala Leu Arg Ser Glu Glu Asp Gly
   1 5 10
<210> 59
   <211> 10
   <212> PRT
   <213> Homo sapiens
<400> 59
   Cys Gly Gly Leu Arg Ser Glu Glu Asp Gly
   1 5 10
<210> 60
   <211> 9
   <212> PRT
   <213> Homo sapiens
<400> 60
   Cys Gly Gly Arg Ser Glu Glu Asp Gly
   1 5
<210> 61
   <211> 8
   <212> PRT
   <213> Homo sapiens
<400> 61
   Cys Gly Gly Ser Glu Glu Asp Gly
   1 5
<210> 62
   <211> 13
   <212> PRT
   <213> Homo sapiens
<400> 62
   Cys Gly Gly Leu Val Leu Ala Leu Arg Ser Glu Glu Asp
   1 5 10
<210> 63
   <211> 12
   <212> PRT
   <213> Homo sapiens
<400> 63
   Cys Gly Gly Val Leu Ala Leu Arg Ser Glu Glu Asp
   1 5 10
<210> 64
   <211> 11
   <212> PRT
   <213> Homo sapiens
<400> 64
   Cys Gly Gly Leu Ala Leu Arg Ser Glu Glu Asp
   1 5 10
<210> 65
   <211> 10
   <212> PRT
   <213> Homo sapiens
<400> 65
   Cys Gly Gly Ala Leu Arg Ser Glu Glu Asp
   1 5 10
<210> 66
   <211> 9
   <212> PRT
   <213> Homo sapiens
<400> 66
   Cys Gly Gly Leu Arg Ser Glu Glu Asp
   1 5
<210> 67
   <211> 8
   <212> PRT
   <213> Homo sapiens
<400> 67
   Cys Gly Gly Arg Ser Glu Glu Asp
   1 5
<210> 68
   <211> 12
   <212> PRT
   <213> Homo sapiens
<400> 68
   Cys Gly Gly Leu Val Leu Ala Leu Arg Ser Glu Glu
   1 5 10
<210> 69
   <211> 11
   <212> PRT
   <213> Homo sapiens
<400> 69
   Cys Gly Gly Val Leu Ala Leu Arg Ser Glu Glu
   1 5 10
<210> 70
   <211> 10
   <212> PRT
   <213> Homo sapiens
<400> 70
   Cys Gly Gly Leu Ala Leu Arg Ser Glu Glu
   1 5 10
<210> 71
   <211> 9
   <212> PRT
   <213> Homo sapiens
<400> 71
   Cys Gly Gly Ala Leu Arg Ser Glu Glu
   1 5
<210> 72
   <211> 8
   <212> PRT
   <213> Homo sapiens
<400> 72
   Cys Gly Gly Leu Arg Ser Glu Glu
   1 5
<210> 73
   <211> 11
   <212> PRT
   <213> Homo sapiens
<400> 73
   Cys Gly Gly Leu Val Leu Ala Leu Arg Ser Glu
   1 5 10
<210> 74
   <211> 10
   <212> PRT
   <213> Homo sapiens
<400> 74
   Cys Gly Gly Val Leu Ala Leu Arg Ser Glu
   1               5                   10
<210> 75
   <211> 9
   <212> PRT
   <213> Homo sapiens
<400> 75
   Cys Gly Gly Leu Ala Leu Arg Ser Glu
   1               5
<210> 76
   <211> 8
   <212> PRT
   <213> Homo sapiens
<400> 76
   Cys Gly Gly Ala Leu Arg Ser Glu
   1               5
<210> 77
   <211> 10
   <212> PRT
   <213> Homo sapiens
<400> 77
   Cys Gly Gly Leu Val Leu Ala Leu Arg Ser
   1               5                   10
<210> 78
   <211> 9
   <212> PRT
   <213> Homo sapiens
<400> 78
   Cys Gly Gly Val Leu Ala Leu Arg Ser
   1               5
<210> 79
   <211> 8
   <212> PRT
   <213> Homo sapiens
<400> 79
   Cys Gly Gly Leu Ala Leu Arg Ser
   1               5
<210> 80
   <211> 12
   <212> PRT
   <213> Homo sapiens
<400> 80
   Val Leu Ala Leu Arg Ser Glu Glu Asp Gly Gly Cys
   1 5 10
<210> 81
   <211> 11
   <212> PRT
   <213> Homo sapiens
<400> 81
   Leu Ala Leu Arg Ser Glu Glu Asp Gly Gly Cys
   1 5 10
<210> 82
   <211> 10
   <212> PRT
   <213> Homo sapiens
<400> 82
   Ala Leu Arg Ser Glu Glu Asp Gly Gly Cys
   1 5 10
<210> 83
   <211> 9
   <212> PRT
   <213> Homo sapiens
<400> 83
   Leu Arg Ser Glu Glu Asp Gly Gly Cys
   1 5
<210> 84
   <211> 8
   <212> PRT
   <213> Homo sapiens
<400> 84
   Arg Ser Glu Glu Asp Gly Gly Cys
   1 5
<210> 85
   <211> 7
   <212> PRT
   <213> Homo sapiens
<400> 85
   Ser Glu Glu Asp Gly Gly Cys
   1 5
<210> 86
   <211> 13
   <212> PRT
   <213> Homo sapiens
<400> 86
   Leu Val Leu Ala Leu Arg Ser Glu Glu Asp Gly Gly Cys
   1 5 10
<210> 87
   <211> 12
   <212> PRT
   <213> Homo sapiens
<400> 87
   Leu Val Leu Ala Leu Arg Ser Glu Glu Gly Gly Cys
   1 5 10
<210> 88
   <211> 11
   <212> PRT
   <213> Homo sapiens
<400> 88
   Val Leu Ala Leu Arg Ser Glu Glu Gly Gly Cys
   1 5 10
<210> 89
   <211> 10
   <212> PRT
   <213> Homo sapiens
<400> 89
   Leu Ala Leu Arg Ser Glu Glu Gly Gly Cys
   1 5 10
<210> 90
   <211> 9
   <212> PRT
   <213> Homo sapiens
<400> 90
   Ala Leu Arg Ser Glu Glu Gly Gly Cys
   1 5
<210> 91
   <211> 8
   <212> PRT
   <213> Homo sapiens
<400> 91
   Leu Arg Ser Glu Glu Gly Gly Cys
   1 5
<210> 92
   <211> 11
   <212> PRT
   <213> Homo sapiens
<400> 92
   Leu Val Leu Ala Leu Arg Ser Glu Gly Gly Cys
   1 5 10
<210> 93
   <211> 10
   <212> PRT
   <213> Homo sapiens
<400> 93
   Val Leu Ala Leu Arg Ser Glu Gly Gly Cys
   1 5 10
<210> 94
   <211> 9
   <212> PRT
   <213> Homo sapiens
<400> 94
   Leu Ala Leu Arg Ser Glu Gly Gly Cys
   1 5
<210> 95
   <211> 8
   <212> PRT
   <213> Homo sapiens
<400> 95
   Ala Leu Arg Ser Glu Gly Gly Cys
   1 5
<210> 96
   <211> 10
   <212> PRT
   <213> Homo sapiens
<400> 96
   Leu Val Leu Ala Leu Arg Ser Gly Gly Cys
   1 5 10
<210> 97
   <211> 9
   <212> PRT
   <213> Homo sapiens
<400> 97
   Val Leu Ala Leu Arg Ser Gly Gly Cys
   1 5
<210> 98
   <211> 8
   <212> PRT
   <213> Homo sapiens
<400> 98
   Leu Ala Leu Arg Ser Gly Gly Cys
   1 5
<210> 99
   <211> 13
   <212> PRT
   <213> Mus musculus
<400> 99
   Leu Met Leu Ala Leu Pro Ser Gln Glu Asp Gly Gly Cys
   1 5 10
<210> 100
   <211> 12
   <212> PRT
   <213> Mus musculus
<400> 100
   Met Leu Ala Leu Pro Ser Gln Glu Asp Gly Gly Cys
   1 5 10
<210> 101
   <211> 11
   <212> PRT
   <213> Mus musculus
<400> 101
   Leu Ala Leu Pro Ser Gln Glu Asp Gly Gly Cys
   1 5 10
<210> 102
   <211> 10
   <212> PRT
   <213> Mus musculus
<400> 102
   Ala Leu Pro Ser Gln Glu Asp Gly Gly Cys
   1               5                   10
<210> 103
   <211> 9
   <212> PRT
   <213> Mus musculus
<400> 103
   Leu Pro Ser Gln Glu Asp Gly Gly Cys
   1               5
<210> 104
   <211> 8
   <212> PRT
   <213> Mus musculus
<400> 104
   Pro Ser Gln Glu Asp Gly Gly Cys
   1               5
<210> 105
   <211> 7
   <212> PRT
   <213> Mus musculus
<400> 105
   Ser Gln Glu Asp Gly Gly Cys
   1               5
<210> 106
   <211> 13
   <212> PRT
   <213> Mus musculus
<400> 106
   Leu Met Leu Ala Leu Pro Ser Gln Glu Asp Gly Gly Cys
   1               5                   10
<210> 107
   <211> 12
   <212> PRT
   <213> Mus musculus
<400> 107
   Met Leu Ala Leu Pro Ser Gln Glu Asp Gly Gly Cys
   1 5 10
<210> 108
   <211> 11
   <212> PRT
   <213> Mus musculus
<400> 108
   Leu Ala Leu Pro Ser Gln Glu Asp Gly Gly Cys
   1 5 10
<210> 109
   <211> 10
   <212> PRT
   <213> Mus musculus
<400> 109
   Ala Leu Pro Ser Gln Glu Asp Gly Gly Cys
   1 5 10
<210> 110
   <211> 9
   <212> PRT
   <213> Mus musculus
<400> 110
   Leu Pro Ser Gln Glu Asp Gly Gly Cys
   1 5
<210> 111
   <211> 8
   <212> PRT
   <213> Mus musculus
<400> 111
   Pro Ser Gln Glu Asp Gly Gly Cys
   1 5
<210> 112
   <211> 12
   <212> PRT
   <213> Mus musculus
<400> 112
   Leu Met Leu Ala Leu Pro Ser Gln Glu Gly Gly Cys
   1 5 10
<210> 113
   <211> 11
   <212> PRT
   <213> Mus musculus
<400> 113
   Met Leu Ala Leu Pro Ser Gln Glu Gly Gly Cys
   1 5 10
<210> 114
   <211> 10
   <212> PRT
   <213> Mus musculus
<400> 114
   Leu Ala Leu Pro Ser Gln Glu Gly Gly Cys
   1 5 10
<210> 115
   <211> 9
   <212> PRT
   <213> Mus musculus
<400> 115
   Ala Leu Pro Ser Gln Glu Gly Gly Cys
   1 5
<210> 116
   <211> 8
   <212> PRT
   <213> Mus musculus
<400> 116
   Leu Pro Ser Gln Glu Gly Gly Cys
   1 5
<210> 117
   <211> 11
   <212> PRT
   <213> Mus musculus
<400> 117
   Leu Met Leu Ala Leu Pro Ser Gln Gly Gly Cys
   1 5 10
<210> 118
   <211> 10
   <212> PRT
   <213> Mus musculus
<400> 118
   Met Leu Ala Leu Pro Ser Gln Gly Gly Cys
   1 5 10
<210> 119
   <211> 9
   <212> PRT
   <213> Mus musculus
<400> 119
   Leu Ala Leu Pro Ser Gln Gly Gly Cys
   1 5
<210> 120
   <211> 8
   <212> PRT
   <213> Mus musculus
<400> 120
   Ala Leu Pro Ser Gln Gly Gly Cys
   1 5
<210> 121
   <211> 10
   <212> PRT
   <213> Mus musculus
<400> 121
   Leu Met Leu Ala Leu Pro Ser Gly Gly Cys
   1 5 10
<210> 122
   <211> 9
   <212> PRT
   <213> Mus musculus
<400> 122
   Met Leu Ala Leu Pro Ser Gly Gly Cys
   1 5
<210> 123
   <211> 8
   <212> PRT
   <213> Mus musculus
<400> 123
   Leu Ala Leu Pro Ser Gly Gly Cys
   1 5
<210> 124
   <211> 14
   <212> PRT
   <213> Mus musculus
<400> 124
   Cys Gly Gly Leu Met Leu Ala Leu Pro Ser Gln Glu Asp Gly
   1 5 10
<210> 125
   <211> 13
   <212> PRT
   <213> Mus musculus
<400> 125
   Cys Gly Gly Met Leu Ala Leu Pro Ser Gln Glu Asp Gly
   1 5 10
<210> 126
   <211> 12
   <212> PRT
   <213> Mus musculus
<400> 126
   Cys Gly Gly Leu Ala Leu Pro Ser Gln Glu Asp Gly
   1 5 10
<210> 127
   <211> 11
   <212> PRT
   <213> Mus musculus
<400> 127
   Cys Gly Gly Ala Leu Pro Ser Gln Glu Asp Gly
   1 5 10
<210> 128
   <211> 10
   <212> PRT
   <213> Mus musculus
<400> 128
   Cys Gly Gly Leu Pro Ser Gln Glu Asp Gly
   1 5 10
<210> 129
   <211> 9
   <212> PRT
   <213> Mus musculus
<400> 129
   Cys Gly Gly Pro Ser Gln Glu Asp Gly
   1 5
<210> 130
   <211> 8
   <212> PRT
   <213> Mus musculus
<400> 130
   Cys Gly Gly Ser Gln Glu Asp Gly
   1 5
<210> 131
   <211> 13
   <212> PRT
   <213> Mus musculus
<400> 131
   Cys Gly Gly Leu Met Leu Ala Leu Pro Ser Gln Glu Asp
   1 5 10
<210> 132
   <211> 12
   <212> PRT
   <213> Mus musculus
<400> 132
   Cys Gly Gly Met Leu Ala Leu Pro Ser Gln Glu Asp
   1 5 10
<210> 133
   <211> 11
   <212> PRT
   <213> Mus musculus
<400> 133
   Cys Gly Gly Leu Ala Leu Pro Ser Gln Glu Asp
   1 5 10
<210> 134
   <211> 10
   <212> PRT
   <213> Mus musculus
<400> 134
   Cys Gly Gly Ala Leu Pro Ser Gln Glu Asp
   1 5 10
<210> 135
   <211> 9
   <212> PRT
   <213> Mus musculus
<400> 135
   Cys Gly Gly Leu Pro Ser Gln Glu Asp
   1 5
<210> 136
   <211> 8
   <212> PRT
   <213> Mus musculus
<400> 136
   Cys Gly Gly Pro Ser Gln Glu Asp
   1 5
<210> 137
   <211> 12
   <212> PRT
   <213> Mus musculus
<400> 137
   Cys Gly Gly Leu Met Leu Ala Leu Pro Ser Gln Glu
   1 5 10
<210> 138
   <211> 11
   <212> PRT
   <213> Mus musculus
<400> 138
   Cys Gly Gly Met Leu Ala Leu Pro Ser Gln Glu
   1 5 10
<210> 139
   <211> 10
   <212> PRT
   <213> Mus musculus
<400> 139
   Cys Gly Gly Leu Ala Leu Pro Ser Gln Glu
   1 5 10
<210> 140
   <211> 9
   <212> PRT
   <213> Mus musculus
<400> 140
   Cys Gly Gly Ala Leu Pro Ser Gln Glu
   1               5
<210> 141
   <211> 8
   <212> PRT
   <213> Mus musculus
<400> 141
   Cys Gly Gly Leu Pro Ser Gln Glu
   1               5
<210> 142
   <211> 11
   <212> PRT
   <213> Mus musculus
<400> 142
   Cys Gly Gly Leu Met Leu Ala Leu Pro Ser Gln
   1               5                   10
<210> 143
   <211> 10
   <212> PRT
   <213> Mus musculus
<400> 143
   Cys Gly Gly Met Leu Ala Leu Pro Ser Gln
   1               5                   10
<210> 144
   <211> 9
   <212> PRT
   <213> Mus musculus
<400> 144
   Cys Gly Gly Leu Ala Leu Pro Ser Gln
   1               5
<210> 145
   <211> 8
   <212> PRT
   <213> Mus musculus
<400> 145
   Cys Gly Gly Ala Leu Pro Ser Gln
   1               5
<210> 146
   <211> 10
   <212> PRT
   <213> Mus musculus
<400> 146
   Cys Gly Gly Leu Met Leu Ala Leu Pro Ser
   1 5 10
<210> 147
   <211> 9
   <212> PRT
   <213> Mus musculus
<400> 147
   Cys Gly Gly Met Leu Ala Leu Pro Ser
   1 5
<210> 148
   <211> 8
   <212> PRT
   <213> Mus musculus
<400> 148
   Cys Gly Gly Leu Ala Leu Pro Ser
   1 5
<210> 149
   <211> 11
   <212> PRT
   <213> Homo sapiens
<400> 149
   Leu Val Leu Ala Leu Arg Ser Glu Glu Asp Gly
   1 5 10
<210> 150
   <211> 10
   <212> PRT
   <213> Homo sapiens
<400> 150
   Val Leu Ala Leu Arg Ser Glu Glu Asp Gly
   1 5 10
<210> 151
   <211> 9
   <212> PRT
   <213> Homo sapiens
<400> 151
   Leu Ala Leu Arg Ser Glu Glu Asp Gly
   1 5
<210> 152
   <211> 8
   <212> PRT
   <213> Homo sapiens
<400> 152
   Ala Leu Arg Ser Glu Glu Asp Gly
   1 5
<210> 153
   <211> 7
   <212> PRT
   <213> Homo sapiens
<400> 153
   Leu Arg Ser Glu Glu Asp Gly
   1 5
<210> 154
   <211> 6
   <212> PRT
   <213> Homo sapiens
<400> 154
   Arg Ser Glu Glu Asp Gly
   1 5
<210> 155
   <211> 5
   <212> PRT
   <213> Homo sapiens
<400> 155
   Ser Glu Glu Asp Gly
   1 5
<210> 156
   <211> 10
   <212> PRT
   <213> Homo sapiens
<400> 156
   Leu Val Leu Ala Leu Arg Ser Glu Glu Asp
   1 5 10
<210> 157
   <211> 9
   <212> PRT
   <213> Homo sapiens
<400> 157
   Val Leu Ala Leu Arg Ser Glu Glu Asp
   1 5
<210> 158
   <211> 8
   <212> PRT
   <213> Homo sapiens
<400> 158
   Leu Ala Leu Arg Ser Glu Glu Asp
   1 5
<210> 159
   <211> 7
   <212> PRT
   <213> Homo sapiens
<400> 159
   Ala Leu Arg Ser Glu Glu Asp
   1 5
<210> 160
   <211> 6
   <212> PRT
   <213> Homo sapiens
<400> 160
   Leu Arg Ser Glu Glu Asp
   1 5
<210> 161
   <211> 5
   <212> PRT
   <213> Homo sapiens
<400> 161
   Arg Ser Glu Glu Asp
   1 5
<210> 162
   <211> 9
   <212> PRT
   <213> Homo sapiens
<400> 162
   Leu Val Leu Ala Leu Arg Ser Glu Glu
   1 5
<210> 163
   <211> 8
   <212> PRT
   <213> Homo sapiens
<400> 163
   Val Leu Ala Leu Arg Ser Glu Glu
   1 5
<210> 164
   <211> 7
   <212> PRT
   <213> Homo sapiens
<400> 164
   Leu Ala Leu Arg Ser Glu Glu
   1 5
<210> 165
   <211> 6
   <212> PRT
   <213> Homo sapiens
<400> 165
   Ala Leu Arg Ser Glu Glu
   1 5
<210> 166
   <211> 5
   <212> PRT
   <213> Homo sapiens
<400> 166
   Leu Arg Ser Glu Glu
   1 5
<210> 167
   <211> 8
   <212> PRT
   <213> Homo sapiens
<400> 167
   Leu Val Leu Ala Leu Arg Ser Glu
   1 5
<210> 168
   <211> 7
   <212> PRT
   <213> Homo sapiens
<400> 168
   Val Leu Ala Leu Arg Ser Glu
   1 5
<210> 169
   <211> 6
   <212> PRT
   <213> Homo sapiens
<400> 169
   Leu Ala Leu Arg Ser Glu
   1 5
<210> 170
   <211> 5
   <212> PRT
   <213> Homo sapiens
<400> 170
   Ala Leu Arg Ser Glu
   1 5
<210> 171
   <211> 7
   <212> PRT
   <213> Homo sapiens
<400> 171
   Leu Val Leu Ala Leu Arg Ser
   1 5
<210> 172
   <211> 6
   <212> PRT
   <213> Homo sapiens
<400> 172
   Val Leu Ala Leu Arg Ser
   1 5
<210> 173
   <211> 5
   <212> PRT
   <213> Mus musculus
<400> 173
   Leu Ala Leu Arg Ser
   1 5
<210> 174
   <211> 11
   <212> PRT
   <213> Mus musculus
<400> 174
   Leu Met Leu Ala Leu Pro Ser Gln Glu Asp Gly
   1               5                   10
<210> 175
   <211> 10
   <212> PRT
   <213> Mus musculus
<400> 175
   Met Leu Ala Leu Pro Ser Gln Glu Asp Gly
   1               5                   10
<210> 176
   <211> 9
   <212> PRT
   <213> Mus musculus
<400> 176
   Leu Ala Leu Pro Ser Gln Glu Asp Gly
   1               5
<210> 177
   <211> 8
   <212> PRT
   <213> Mus musculus
<400> 177
   Ala Leu Pro Ser Gln Glu Asp Gly
   1               5
<210> 178
   <211> 7
   <212> PRT
   <213> Mus musculus
<400> 178
   Leu Pro Ser Gln Glu Asp Gly
   1               5
<210> 179
   <211> 6
   <212> PRT
   <213> Mus musculus
<400> 179
   Pro Ser Gln Glu Asp Gly
   1               5
<210> 180
   <211> 5
   <212> PRT
   <213> Mus musculus
<400> 180
   Ser Gln Glu Asp Gly
   1 5
<210> 181
   <211> 10
   <212> PRT
   <213> Mus musculus
<400> 181
   Leu Met Leu Ala Leu Pro Ser Gln Glu Asp
   1 5 10
<210> 182
   <211> 9
   <212> PRT
   <213> Mus musculus
<400> 182
   Met Leu Ala Leu Pro Ser Gln Glu Asp
   1 5
<210> 183
   <211> 8
   <212> PRT
   <213> Mus musculus
<400> 183
   Leu Ala Leu Pro Ser Gln Glu Asp
   1 5
<210> 184
   <211> 7
   <212> PRT
   <213> Mus musculus
<400> 184
   Ala Leu Pro Ser Gln Glu Asp
   1 5
<210> 185
   <211> 6
   <212> PRT
   <213> Mus musculus
<400> 185
   Leu Pro Ser Gln Glu Asp
   1 5
<210> 186
   <211> 5
   <212> PRT
   <213> Mus musculus
<400> 186
   Pro Ser Gln Glu Asp
   1 5
<210> 187
   <211> 9
   <212> PRT
   <213> Mus musculus
<400> 187
   Leu Met Leu Ala Leu Pro Ser Gln Glu
   1 5
<210> 188
   <211> 8
   <212> PRT
   <213> Mus musculus
<400> 188
   Met Leu Ala Leu Pro Ser Gln Glu
   1 5
<210> 189
   <211> 7
   <212> PRT
   <213> Mus musculus
<400> 189
   Leu Ala Leu Pro Ser Gln Glu
   1 5
<210> 190
   <211> 6
   <212> PRT
   <213> Mus musculus
<400> 190
   Ala Leu Pro Ser Gln Glu
   1 5
<210> 191
   <211> 5
   <212> PRT
   <213> Mus musculus
<400> 191
   Leu Pro Ser Gln Glu
   1 5
<210> 192
   <211> 8
   <212> PRT
   <213> Mus musculus
<400> 192
   Leu Met Leu Ala Leu Pro Ser Gln
   1 5
<210> 193
   <211> 7
   <212> PRT
   <213> Mus musculus
<400> 193
   Met Leu Ala Leu Pro Ser Gln
   1 5
<210> 194
   <211> 6
   <212> PRT
   <213> Mus musculus
<400> 194
   Leu Ala Leu Pro Ser Gln
   1 5
<210> 195
   <211> 5
   <212> PRT
   <213> Mus musculus
<400> 195
   Ala Leu Pro Ser Gln
   1 5
<210> 196
   <211> 7
   <212> PRT
   <213> Mus musculus
<400> 196
   Leu Met Leu Ala Leu Pro Ser
   1 5
<210> 197
   <211> 6
   <212> PRT
   <213> Mus musculus
<400> 197
   Met Leu Ala Leu Pro Ser
   1 5
<210> 198
   <211> 5
   <212> PRT
   <213> Mus musculus
<400> 198
   Leu Ala Leu Pro Ser
   1 5
<210> 199
   <211> 14
   <212> PRT
   <213> Homo sapiens
<400> 199
   Cys Gly Gly Leu Val Leu Ala Leu Arg Ser Glu Glu Asp Gly
   1 5 10
<210> 200
   <211> 13
   <212> PRT
   <213> Homo sapiens
<400> 200
   Cys Gly Gly Val Leu Ala Leu Arg Ser Glu Glu Asp Gly
   1 5 10
<210> 201
   <211> 12
   <212> PRT
   <213> Homo sapiens
<400> 201
   Cys Gly Gly Leu Ala Leu Arg Ser Glu Glu Asp Gly
   1 5 10
<210> 202
   <211> 11
   <212> PRT
   <213> Homo sapiens
<400> 202
   Cys Gly Gly Ala Leu Arg Ser Glu Glu Asp Gly
   1 5 10
<210> 203
   <211> 10
   <212> PRT
   <213> Homo sapiens
<400> 203
   Cys Gly Gly Leu Arg Ser Glu Glu Asp Gly
   1 5 10
<210> 204
   <211> 9
   <212> PRT
   <213> Homo sapiens
<400> 204
   Cys Gly Gly Arg Ser Glu Glu Asp Gly
   1 5
<210> 205
   <211> 8
   <212> PRT
   <213> Homo sapiens
<400> 205
   Cys Gly Gly Ser Glu Glu Asp Gly
   1 5
<210> 206
   <211> 13
   <212> PRT
   <213> Homo sapiens
<400> 206
   Cys Gly Gly Leu Val Leu Ala Leu Arg Ser Glu Glu Asp
   1 5 10
<210> 207
   <211> 12
   <212> PRT
   <213> Homo sapiens
<400> 207
   Cys Gly Gly Val Leu Ala Leu Arg Ser Glu Glu Asp
   1 5 10
<210> 208
   <211> 11
   <212> PRT
   <213> Homo sapiens
<400> 208
   Cys Gly Gly Leu Ala Leu Arg Ser Glu Glu Asp
   1 5 10
<210> 209
   <211> 10
   <212> PRT
   <213> Homo sapiens
<400> 209
   Cys Gly Gly Ala Leu Arg Ser Glu Glu Asp
   1 5 10
<210> 210
   <211> 9
   <212> PRT
   <213> Homo sapiens
<400> 210
   Cys Gly Gly Leu Arg Ser Glu Glu Asp
   1 5
<210> 211
   <211> 8
   <212> PRT
   <213> Homo sapiens
<400> 211
   Cys Gly Gly Arg Ser Glu Glu Asp
   1 5
<210> 212
   <211> 12
   <212> PRT
   <213> Homo sapiens
<400> 212
   Cys Gly Gly Leu Val Leu Ala Leu Arg Ser Glu Glu
   1 5 10
<210> 213
   <211> 11
   <212> PRT
   <213> Homo sapiens
<400> 213
   Cys Gly Gly Val Leu Ala Leu Arg Ser Glu Glu
   1 5 10
<210> 214
   <211> 10
   <212> PRT
   <213> Homo sapiens
<400> 214
   Cys Gly Gly Leu Ala Leu Arg Ser Glu Glu
   1 5 10
<210> 215
   <211> 9
   <212> PRT
   <213> Homo sapiens
<400> 215
   Cys Gly Gly Ala Leu Arg Ser Glu Glu
   1 5
<210> 216
   <211> 8
   <212> PRT
   <213> Homo sapiens
<400> 216
   Cys Gly Gly Leu Arg Ser Glu Glu
   1 5
<210> 217
   <211> 11
   <212> PRT
   <213> Homo sapiens
<400> 217
   Cys Gly Gly Leu Val Leu Ala Leu Arg Ser Glu
   1 5 10
<210> 218
   <211> 10
   <212> PRT
   <213> Homo sapiens
<400> 218
   Cys Gly Gly Val Leu Ala Leu Arg Ser Glu
   1 5 10
<210> 219
   <211> 9
   <212> PRT
   <213> Homo sapiens
<400> 219
   Cys Gly Gly Leu Ala Leu Arg Ser Glu
   1 5
<210> 220
   <211> 8
   <212> PRT
   <213> Homo sapiens
<400> 220
   Cys Gly Gly Ala Leu Arg Ser Glu
   1 5
<210> 221
   <211> 10
   <212> PRT
   <213> Homo sapiens
<400> 221
   Cys Gly Gly Leu Val Leu Ala Leu Arg Ser
   1 5 10
<210> 222
   <211> 9
   <212> PRT
   <213> Homo sapiens
<400> 222
   Cys Gly Gly Val Leu Ala Leu Arg Ser
   1 5
<210> 223
   <211> 8
   <212> PRT
   <213> Homo sapiens
<400> 223
   Cys Gly Gly Leu Ala Leu Arg Ser
   1 5
<210> 224
   <211> 13
   <212> PRT
   <213> Homo sapiens
<400> 224
   Leu Val Leu Ala Leu Arg Ser Glu Glu Asp Gly Gly Cys
   1 5 10
<210> 225
   <211> 12
   <212> PRT
   <213> Homo sapiens
<400> 225
   Val Leu Ala Leu Arg Ser Glu Glu Asp Gly Gly Cys
   1 5 10
<210> 226
   <211> 11
   <212> PRT
   <213> Homo sapiens
<400> 226
   Leu Ala Leu Arg Ser Glu Glu Asp Gly Gly Cys
   1               5                   10
<210> 227
   <211> 10
   <212> PRT
   <213> Homo sapiens
<400> 227
   Ala Leu Arg Ser Glu Glu Asp Gly Gly Cys
   1               5                   10
<210> 228
   <211> 9
   <212> PRT
   <213> Homo sapiens
<400> 228
   Leu Arg Ser Glu Glu Asp Gly Gly Cys
   1               5
<210> 229
   <211> 8
   <212> PRT
   <213> Homo sapiens
<400> 229
   Arg Ser Glu Glu Asp Gly Gly Cys
   1               5
<210> 230
   <211> 7
   <212> PRT
   <213> Homo sapiens
<400> 230
   Ser Glu Glu Asp Gly Gly Cys
   1               5
<210> 231
   <211> 12
   <212> PRT
   <213> Homo sapiens
<400> 231
   Leu Val Leu Ala Leu Arg Ser Glu Glu Gly Gly Cys
   1               5                   10
<210> 232
   <211> 11
   <212> PRT
   <213> Homo sapiens
<400> 232
   Val Leu Ala Leu Arg Ser Glu Glu Gly Gly Cys
   1 5 10
<210> 233
   <211> 10
   <212> PRT
   <213> Homo sapiens
<400> 233
   Leu Ala Leu Arg Ser Glu Glu Gly Gly Cys
   1 5 10
<210> 234
   <211> 9
   <212> PRT
   <213> Homo sapiens
<400> 234
   Ala Leu Arg Ser Glu Glu Gly Gly Cys
   1 5
<210> 235
   <211> 8
   <212> PRT
   <213> Homo sapiens
<400> 235
   Leu Arg Ser Glu Glu Gly Gly Cys
   1 5
<210> 236
   <211> 11
   <212> PRT
   <213> Homo sapiens
<400> 236
   Leu Val Leu Ala Leu Arg Ser Glu Gly Gly Cys
   1 5 10
<210> 237
   <211> 10
   <212> PRT
   <213> Homo sapiens
<400> 237
   Val Leu Ala Leu Arg Ser Glu Gly Gly Cys
   1 5 10
<210> 238
   <211> 9
   <212> PRT
   <213> Homo sapiens
<400> 238
   Leu Ala Leu Arg Ser Glu Gly Gly Cys
   1 5
<210> 239
   <211> 8
   <212> PRT
   <213> Homo sapiens
<400> 239
   Ala Leu Arg Ser Glu Gly Gly Cys
   1 5
<210> 240
   <211> 10
   <212> PRT
   <213> Homo sapiens
<400> 240
   Leu Val Leu Ala Leu Arg Ser Gly Gly Cys
   1 5 10
<210> 241
   <211> 9
   <212> PRT
   <213> Homo sapiens
<400> 241
   Val Leu Ala Leu Arg Ser Gly Gly Cys
   1 5
<210> 242
   <211> 8
   <212> PRT
   <213> Homo sapiens
<400> 242
   Leu Ala Leu Arg Ser Gly Gly Cys
   1 5
<210> 243
   <211> 13
   <212> PRT
   <213> Mus musculus
<400> 243
   Leu Met Leu Ala Leu Pro Ser Gln Glu Asp Gly Gly Cys
   1 5 10
<210> 244
   <211> 12
   <212> PRT
   <213> Mus musculus
<400> 244
   Met Leu Ala Leu Pro Ser Gln Glu Asp Gly Gly Cys
   1 5 10
<210> 245
   <211> 11
   <212> PRT
   <213> Mus musculus
<400> 245
   Leu Ala Leu Pro Ser Gln Glu Asp Gly Gly Cys
   1 5 10
<210> 246
   <211> 10
   <212> PRT
   <213> Mus musculus
<400> 246
   Ala Leu Pro Ser Gln Glu Asp Gly Gly Cys
   1 5 10
<210> 247
   <211> 9
   <212> PRT
   <213> Mus musculus
<400> 247
   Leu Pro Ser Gln Glu Asp Gly Gly Cys
   1 5
<210> 248
   <211> 8
   <212> PRT
   <213> Mus musculus
<400> 248
   Pro Ser Gln Glu Asp Gly Gly Cys
   1               5
<210> 249
   <211> 7
   <212> PRT
   <213> Mus musculus
<400> 249
   Ser Gln Glu Asp Gly Gly Cys
   1               5
<210> 250
   <211> 12
   <212> PRT
   <213> Mus musculus
<400> 250
   Leu Met Leu Ala Leu Pro Ser Gln Glu Gly Gly Cys
   1               5                   10
<210> 251
   <211> 11
   <212> PRT
   <213> Mus musculus
<400> 251
   Met Leu Ala Leu Pro Ser Gln Glu Gly Gly Cys
   1               5                   10
<210> 252
   <211> 10
   <212> PRT
   <213> Mus musculus
<400> 252
   Leu Ala Leu Pro Ser Gln Glu Gly Gly Cys
   1               5                   10
<210> 253
   <211> 9
   <212> PRT
   <213> Mus musculus
<400> 253
   Ala Leu Pro Ser Gln Glu Gly Gly Cys
   1 5
<210> 254
   <211> 8
   <212> PRT
   <213> Mus musculus
<400> 254
   Leu Pro Ser Gln Glu Gly Gly Cys
   1 5
<210> 255
   <211> 11
   <212> PRT
   <213> Mus musculus
<400> 255
   Leu Met Leu Ala Leu Pro Ser Gln Gly Gly Cys
   1 5 10
<210> 256
   <211> 10
   <212> PRT
   <213> Mus musculus
<400> 256
   Met Leu Ala Leu Pro Ser Gln Gly Gly Cys
   1 5 10
<210> 257
   <211> 9
   <212> PRT
   <213> Mus musculus
<400> 257
   Leu Ala Leu Pro Ser Gln Gly Gly Cys
   1 5
<210> 258
   <211> 8
   <212> PRT
   <213> Mus musculus
<400> 258
   Ala Leu Pro Ser Gln Gly Gly Cys
   1 5
<210> 259
   <211> 10
   <212> PRT
   <213> Mus musculus
<400> 259
   Leu Met Leu Ala Leu Pro Ser Gly Gly Cys
   1 5 10
<210> 260
   <211> 9
   <212> PRT
   <213> Mus musculus
<400> 260
   Met Leu Ala Leu Pro Ser Gly Gly Cys
   1 5
<210> 261
   <211> 8
   <212> PRT
   <213> Mus musculus
<400> 261
   Leu Ala Leu Pro Ser Gly Gly Cys
   1 5
<210> 262
   <211> 14
   <212> PRT
   <213> Mus musculus
<400> 262
   Cys Gly Gly Leu Met Leu Ala Leu Pro Ser Gln Glu Asp Gly
   1 5 10
<210> 263
   <211> 13
   <212> PRT
   <213> Mus musculus
<400> 263
   Cys Gly Gly Met Leu Ala Leu Pro Ser Gln Glu Asp Gly
   1 5 10
<210> 264
   <211> 12
   <212> PRT
   <213> Mus musculus
<400> 264
   Cys Gly Gly Leu Ala Leu Pro Ser Gln Glu Asp Gly
   1 5 10
<210> 265
   <211> 11
   <212> PRT
   <213> Mus musculus
<400> 265
   Cys Gly Gly Ala Leu Pro Ser Gln Glu Asp Gly
   1 5 10
<210> 266
   <211> 10
   <212> PRT
   <213> Mus musculus
<400> 266
   Cys Gly Gly Leu Pro Ser Gln Glu Asp Gly
   1 5 10
<210> 267
   <211> 9
   <212> PRT
   <213> Mus musculus
<400> 267
   Cys Gly Gly Pro Ser Gln Glu Asp Gly
   1 5
<210> 268
   <211> 8
   <212> PRT
   <213> Mus musculus
<400> 268
   Cys Gly Gly Ser Gln Glu Asp Gly
   1 5
<210> 269
   <211> 13
   <212> PRT
   <213> Mus musculus
<400> 269
   Cys Gly Gly Leu Met Leu Ala Leu Pro Ser Gln Glu Asp
   1 5 10
<210> 270
   <211> 12
   <212> PRT
   <213> Mus musculus
<400> 270
   Cys Gly Gly Met Leu Ala Leu Pro Ser Gln Glu Asp
   1 5 10
<210> 271
   <211> 11
   <212> PRT
   <213> Mus musculus
<400> 271
   Cys Gly Gly Leu Ala Leu Pro Ser Gln Glu Asp
   1 5 10
<210> 272
   <211> 10
   <212> PRT
   <213> Mus musculus
<400> 272
   Cys Gly Gly Ala Leu Pro Ser Gln Glu Asp
   1 5 10
<210> 273
   <211> 9
   <212> PRT
   <213> Mus musculus
<400> 273
   Cys Gly Gly Leu Pro Ser Gln Glu Asp
   1 5
<210> 274
   <211> 8
   <212> PRT
   <213> Mus musculus
<400> 274
   Cys Gly Gly Pro Ser Gln Glu Asp
   1 5
<210> 275
   <211> 12
   <212> PRT
   <213> Mus musculus
<400> 275
   Cys Gly Gly Leu Met Leu Ala Leu Pro Ser Gln Glu
   1 5 10
<210> 276
   <211> 11
   <212> PRT
   <213> Mus musculus
<400> 276
   Cys Gly Gly Met Leu Ala Leu Pro Ser Gln Glu
   1 5 10
<210> 277
   <211> 10
   <212> PRT
   <213> Mus musculus
<400> 277
   Cys Gly Gly Leu Ala Leu Pro Ser Gln Glu
   1 5 10
<210> 278
   <211> 9
   <212> PRT
   <213> Mus musculus
<400> 278
   Cys Gly Gly Ala Leu Pro Ser Gln Glu
   1 5
<210> 279
   <211> 8
   <212> PRT
   <213> Mus musculus
<400> 279
   Cys Gly Gly Leu Pro Ser Gln Glu
   1 5
<210> 280
   <211> 11
   <212> PRT
   <213> Mus musculus
<400> 280
   Cys Gly Gly Leu Met Leu Ala Leu Pro Ser Gln
   1 5 10
<210> 281
   <211> 10
   <212> PRT
   <213> Mus musculus
<400> 281
   Cys Gly Gly Met Leu Ala Leu Pro Ser Gln
   1 5 10
<210> 282
   <211> 9
   <212> PRT
   <213> Mus musculus
<400> 282
   Cys Gly Gly Leu Ala Leu Pro Ser Gln
   1 5
<210> 283
   <211> 8
   <212> PRT
   <213> Mus musculus
<400> 283
   Cys Gly Gly Ala Leu Pro Ser Gln
   1 5
<210> 284
   <211> 10
   <212> PRT
   <213> Mus musculus
<400> 284
   Cys Gly Gly Leu Met Leu Ala Leu Pro Ser
   1 5 10
<210> 285
   <211> 9
   <212> PRT
   <213> Mus musculus
<400> 285
   Cys Gly Gly Met Leu Ala Leu Pro Ser
   1 5
<210> 286
   <211> 8
   <212> PRT
   <213> Mus musculus
<400> 286
   Cys Gly Gly Leu Ala Leu Pro Ser
   1 5
<210> 287
   <211> 13
   <212> PRT
   <213> Homo sapiens
<400> 287
   Cys Ser Arg His Leu Ala Gln Ala Ser Gln Glu Leu Gln
   1               5                   10
<210> 288
   <211> 12
   <212> PRT
   <213> Homo sapiens
<400> 288
   Cys Ser Arg His Leu Ala Gln Ala Ser Gln Glu Leu
   1               5                   10
<210> 289
   <211> 11
   <212> PRT
   <213> Homo sapiens
<400> 289
   Cys Ser Arg His Leu Ala Gln Ala Ser Gln Glu
   1               5                   10
<210> 290
   <211> 10
   <212> PRT
   <213> Homo sapiens
<400> 290
   Cys Ser Arg His Leu Ala Gln Ala Ser Gln
   1               5                   10
<210> 291
   <211> 9
   <212> PRT
   <213> Homo sapiens
<400> 291
   Cys Ser Arg His Leu Ala Gln Ala Ser
   1               5
<210> 292
   <211> 12
   <212> PRT
   <213> Homo sapiens
<400> 292
   Ser Arg His Leu Ala Gln Ala Ser Gln Glu Leu Gln
   1               5                   10
<210> 293
   <211> 11
   <212> PRT
   <213> Homo sapiens
<400> 293
   Ser Arg His Leu Ala Gln Ala Ser Gln Glu Leu
   1 5 10
<210> 294
   <211> 10
   <212> PRT
   <213> Homo sapiens
<400> 294
   Ser Arg His Leu Ala Gln Ala Ser Gln Glu
   1 5 10
<210> 295
   <211> 9
   <212> PRT
   <213> Homo sapiens
<400> 295
   Ser Arg His Leu Ala Gln Ala Ser Gln
   1 5
<210> 296
   <211> 8
   <212> PRT
   <213> Homo sapiens
<400> 296
   Ser Arg His Leu Ala Gln Ala Ser
   1 5
<210> 297
   <211> 11
   <212> PRT
   <213> Homo sapiens
<400> 297
   Arg His Leu Ala Gln Ala Ser Gln Glu Leu Gln
   1 5 10
<210> 298
   <211> 10
   <212> PRT
   <213> Homo sapiens
<400> 298
   Arg His Leu Ala Gln Ala Ser Gln Glu Leu
   1 5 10
<210> 299
   <211> 9
   <212> PRT
   <213> Homo sapiens
<400> 299
   Arg His Leu Ala Gln Ala Ser Gln Glu
   1               5
<210> 300
   <211> 8
   <212> PRT
   <213> Homo sapiens
<400> 300
   Arg His Leu Ala Gln Ala Ser Gln
   1               5
<210> 301
   <211> 7
   <212> PRT
   <213> Homo sapiens
<400> 301
   Arg His Leu Ala Gln Ala Ser
   1               5
<210> 302
   <211> 10
   <212> PRT
   <213> Homo sapiens
<400> 302
   His Leu Ala Gln Ala Ser Gln Glu Leu Gln
   1               5                   10
<210> 303
   <211> 9
   <212> PRT
   <213> Homo sapiens
<400> 303
   His Leu Ala Gln Ala Ser Gln Glu Leu
   1               5
<210> 304
   <211> 7
   <212> PRT
   <213> Homo sapiens
<400> 304
   His Leu Ala Gln Ala Ser Gln
   1 5
<210> 305
   <211> 6
   <212> PRT
   <213> Homo sapiens
<400> 305
   His Leu Ala Gln Ala Ser
   1 5
<210> 306
   <211> 9
   <212> PRT
   <213> Homo sapiens
<400> 306
   Leu Ala Gln Ala Ser Gln Glu Leu Gln
   1 5
<210> 307
   <211> 8
   <212> PRT
   <213> Homo sapiens
<400> 307
   Leu Ala Gln Ala Ser Gln Glu Leu
   1 5
<210> 308
   <211> 7
   <212> PRT
   <213> Homo sapiens
<400> 308
   Leu Ala Gln Ala Ser Gln Glu
   1 5
<210> 309
   <211> 6
   <212> PRT
   <213> Homo sapiens
<400> 309
   Leu Ala Gln Ala Ser Gln
   1 5
<210> 310
   <211> 5
   <212> PRT
   <213> Homo sapiens
<400> 310
   Leu Ala Gln Ala Ser
   1 5
<210> 311
   <211> 8
   <212> PRT
   <213> Homo sapiens
<400> 311
   Ala Gln Ala Ser Gln Glu Leu Gln
   1 5
<210> 312
   <211> 7
   <212> PRT
   <213> Homo sapiens
<400> 312
   Ala Gln Ala Ser Gln Glu Leu
   1 5
<210> 313
   <211> 6
   <212> PRT
   <213> Homo sapiens
<400> 313
   Ala Gln Ala Ser Gln Glu
   1 5
<210> 314
   <211> 5
   <212> PRT
   <213> Homo sapiens
<400> 314
   Ala Gln Ala Ser Gln
   1 5
<210> 315
   <211> 7
   <212> PRT
   <213> Homo sapiens
<400> 315
   Gln Ala Ser Gln Glu Leu Gln
   1 5
<210> 316
   <211> 6
   <212> PRT
   <213> Homo sapiens
<400> 316
   Gln Ala Ser Gln Glu Leu
   1 5
<210> 317
   <211> 5
   <212> PRT
   <213> Homo sapiens
<400> 317
   Gln Ala Ser Gln Glu
   1 5
<210> 318
   <211> 6
   <212> PRT
   <213> Homo sapiens
<400> 318
   Ala Ser Gln Glu Leu Gln
   1 5
<210> 319
   <211> 5
   <212> PRT
   <213> Homo sapiens
<400> 319
   Ala Ser Gln Glu Leu
   1 5
<210> 320
   <211> 5
   <212> PRT
   <213> Homo sapiens
<400> 320
   Ser Gln Glu Leu Gln
   1 5
<210> 321
   <211> 15
   <212> PRT
   <213> Homo sapiens
<400> 321
   Cys Gly Gly Ser Arg His Leu Ala Gln Ala Ser Gln Glu Leu Gln
   1 5 10 15
<210> 322
   <211> 14
   <212> PRT
   <213> Homo sapiens
<400> 322
   Cys Gly Gly Ser Arg His Leu Ala Gln Ala Ser Gln Glu Leu
   1 5 10
<210> 323
   <211> 13
   <212> PRT
   <213> Homo sapiens
<400> 323
   Cys Gly Gly Ser Arg His Leu Ala Gln Ala Ser Gln Glu
   1 5 10
<210> 324
   <211> 12
   <212> PRT
   <213> Homo sapiens
<400> 324
   Cys Gly Gly Ser Arg His Leu Ala Gln Ala Ser Gln
   1 5 10
<210> 325
   <211> 11
   <212> PRT
   <213> Homo sapiens
<400> 325
   Cys Gly Gly Ser Arg His Leu Ala Gln Ala Ser
   1 5 10
<210> 326
   <211> 14
   <212> PRT
   <213> Homo sapiens
<400> 326
   Cys Gly Gly Arg His Leu Ala Gln Ala Ser Gln Glu Leu Gln
   1 5 10
<210> 327
   <211> 13
   <212> PRT
   <213> Homo sapiens
<400> 327
   Cys Gly Gly Arg His Leu Ala Gln Ala Ser Gln Glu Leu
   1 5 10
<210> 328
   <211> 12
   <212> PRT
   <213> Homo sapiens
<400> 328
   Cys Gly Gly Arg His Leu Ala Gln Ala Ser Gln Glu
   1 5 10
<210> 329
   <211> 11
   <212> PRT
   <213> Homo sapiens
<400> 329
   Cys Gly Gly Arg His Leu Ala Gln Ala Ser Gln
   1 5 10
<210> 330
   <211> 10
   <212> PRT
   <213> Homo sapiens
<400> 330
   Cys Gly Gly Arg His Leu Ala Gln Ala Ser
   1 5 10
<210> 331
   <211> 13
   <212> PRT
   <213> Homo sapiens
<400> 331
   Cys Gly Gly His Leu Ala Gln Ala Ser Gln Glu Leu Gln
   1 5 10
<210> 332
   <211> 12
   <212> PRT
   <213> Homo sapiens
<400> 332
   Cys Gly Gly His Leu Ala Gln Ala Ser Gln Glu Leu
   1 5 10
<210> 333
   <211> 10
   <212> PRT
   <213> Homo sapiens
<400> 333
   Cys Gly Gly His Leu Ala Gln Ala Ser Gln
   1 5 10
<210> 334
   <211> 9
   <212> PRT
   <213> Homo sapiens
<400> 334
   Cys Gly Gly His Leu Ala Gln Ala Ser
   1 5
<210> 335
   <211> 12
   <212> PRT
   <213> Homo sapiens
<400> 335
   Cys Gly Gly Leu Ala Gln Ala Ser Gln Glu Leu Gln
   1 5 10
<210> 336
   <211> 11
   <212> PRT
   <213> Homo sapiens
<400> 336
   Cys Gly Gly Leu Ala Gln Ala Ser Gln Glu Leu
   1 5 10
<210> 337
   <211> 10
   <212> PRT
   <213> Homo sapiens
<400> 337
   Cys Gly Gly Leu Ala Gln Ala Ser Gln Glu
   1 5 10
<210> 338
   <211> 9
   <212> PRT
   <213> Homo sapiens
<400> 338
   Cys Gly Gly Leu Ala Gln Ala Ser Gln
   1 5
<210> 339
   <211> 8
   <212> PRT
   <213> Homo sapiens
<400> 339
   Cys Gly Gly Leu Ala Gln Ala Ser
   1 5
<210> 340
   <211> 11
   <212> PRT
   <213> Homo sapiens
<400> 340
   Cys Gly Gly Ala Gln Ala Ser Gln Glu Leu Gln
   1 5 10
<210> 341
   <211> 10
   <212> PRT
   <213> Homo sapiens
<400> 341
   Cys Gly Gly Ala Gln Ala Ser Gln Glu Leu
   1 5 10
<210> 342
   <211> 9
   <212> PRT
   <213> Homo sapiens
<400> 342
   Cys Gly Gly Ala Gln Ala Ser Gln Glu
   1 5
<210> 343
   <211> 8
   <212> PRT
   <213> Homo sapiens
<400> 343
   Cys Gly Gly Ala Gln Ala Ser Gln
   1 5
<210> 344
   <211> 10
   <212> PRT
   <213> Homo sapiens
<400> 344
   Cys Gly Gly Gln Ala Ser Gln Glu Leu Gln
   1               5                   10
<210> 345
   <211> 9
   <212> PRT
   <213> Homo sapiens
<400> 345
   Cys Gly Gly Gln Ala Ser Gln Glu Leu
   1               5
<210> 346
   <211> 8
   <212> PRT
   <213> Homo sapiens
<400> 346
   Cys Gly Gly Gln Ala Ser Gln Glu
   1               5
<210> 347
   <211> 9
   <212> PRT
   <213> Homo sapiens
<400> 347
   Cys Gly Gly Ala Ser Gln Glu Leu Gln
   1               5
<210> 348
   <211> 8
   <212> PRT
   <213> Homo sapiens
<400> 348
   Cys Gly Gly Ala Ser Gln Glu Leu
   1               5
<210> 349
   <211> 8
   <212> PRT
   <213> Homo sapiens
<400> 349
   Cys Gly Gly Ser Gln Glu Leu Gln
   1 5
<210> 350
   <211> 15
   <212> PRT
   <213> Homo sapiens
<400> 350
   Ser Arg His Leu Ala Gln Ala Ser Gln Glu Leu Gln Gly Gly Cys
   1 5 10 15
<210> 351
   <211> 14
   <212> PRT
   <213> Homo sapiens
<400> 351
   Ser Arg His Leu Ala Gln Ala Ser Gln Glu Leu Gly Gly Cys
   1 5 10
<210> 352
   <211> 13
   <212> PRT
   <213> Homo sapiens
<400> 352
   Ser Arg His Leu Ala Gln Ala Ser Gln Glu Gly Gly Cys
   1 5 10
<210> 353
   <211> 12
   <212> PRT
   <213> Homo sapiens
<400> 353
   Ser Arg His Leu Ala Gln Ala Ser Gln Gly Gly Cys
   1 5 10
<210> 354
   <211> 11
   <212> PRT
   <213> Homo sapiens
<400> 354
   Ser Arg His Leu Ala Gln Ala Ser Gly Gly Cys
   1 5 10
<210> 355
   <211> 14
   <212> PRT
   <213> Homo sapiens
<400> 355
   Arg His Leu Ala Gln Ala Ser Gln Glu Leu Gln Gly Gly Cys
   1 5 10
<210> 356
   <211> 13
   <212> PRT
   <213> Homo sapiens
<400> 356
   Arg His Leu Ala Gln Ala Ser Gln Glu Leu Gly Gly Cys
   1 5 10
<210> 357
   <211> 12
   <212> PRT
   <213> Homo sapiens
<400> 357
   Arg His Leu Ala Gln Ala Ser Gln Glu Gly Gly Cys
   1 5 10
<210> 358
   <211> 11
   <212> PRT
   <213> Homo sapiens
<400> 358
   Arg His Leu Ala Gln Ala Ser Gln Gly Gly Cys
   1 5 10
<210> 359
   <211> 10
   <212> PRT
   <213> Homo sapiens
<400> 359
   Arg His Leu Ala Gln Ala Ser Gly Gly Cys
   1 5 10
<210> 360
   <211> 13
   <212> PRT
   <213> Homo sapiens
<400> 360
   His Leu Ala Gln Ala Ser Gln Glu Leu Gln Gly Gly Cys
   1 5 10
<210> 361
   <211> 12
   <212> PRT
   <213> Homo sapiens
<400> 361
   His Leu Ala Gln Ala Ser Gln Glu Leu Gly Gly Cys
   1 5 10
<210> 362
   <211> 10
   <212> PRT
   <213> Homo sapiens
<400> 362
   His Leu Ala Gln Ala Ser Gln Gly Gly Cys
   1 5 10
<210> 363
   <211> 9
   <212> PRT
   <213> Homo sapiens
<400> 363
   His Leu Ala Gln Ala Ser Gly Gly Cys
   1 5
<210> 364
   <211> 12
   <212> PRT
   <213> Homo sapiens
<400> 364
   Leu Ala Gln Ala Ser Gln Glu Leu Gln Gly Gly Cys
   1 5 10
<210> 365
   <211> 11
   <212> PRT
   <213> Homo sapiens
<400> 365
   Leu Ala Gln Ala Ser Gln Glu Leu Gly Gly Cys
   1 5 10
<210> 366
   <211> 10
   <212> PRT
   <213> Homo sapiens
<400> 366
   Leu Ala Gln Ala Ser Gln Glu Gly Gly Cys
   1               5                   10
<210> 367
   <211> 9
   <212> PRT
   <213> Homo sapiens
<400> 367
   Leu Ala Gln Ala Ser Gln Gly Gly Cys
   1               5
<210> 368
   <211> 8
   <212> PRT
   <213> Homo sapiens
<400> 368
   Leu Ala Gln Ala Ser Gly Gly Cys
   1               5
<210> 369
   <211> 11
   <212> PRT
   <213> Homo sapiens
<400> 369
   Ala Gln Ala Ser Gln Glu Leu Gln Gly Gly Cys
   1               5                   10
<210> 370
   <211> 10
   <212> PRT
   <213> Homo sapiens
<400> 370
   Ala Gln Ala Ser Gln Glu Leu Gly Gly Cys
   1               5                   10
<210> 371
   <211> 9
   <212> PRT
   <213> Homo sapiens
<400> 371
   Ala Gln Ala Ser Gln Glu Gly Gly Cys
   1 5
<210> 372
   <211> 8
   <212> PRT
   <213> Homo sapiens
<400> 372
   Ala Gln Ala Ser Gln Gly Gly Cys
   1 5
<210> 373
   <211> 10
   <212> PRT
   <213> Homo sapiens
<400> 373
   Gln Ala Ser Gln Glu Leu Gln Gly Gly Cys
   1 5 10
<210> 374
   <211> 9
   <212> PRT
   <213> Homo sapiens
<400> 374
   Gln Ala Ser Gln Glu Leu Gly Gly Cys
   1 5
<210> 375
   <211> 8
   <212> PRT
   <213> Homo sapiens
<400> 375
   Gln Ala Ser Gln Glu Gly Gly Cys
   1 5
<210> 376
   <211> 9
   <212> PRT
   <213> Homo sapiens
<400> 376
   Ala Ser Gln Glu Leu Gln Gly Gly Cys
   1 5
<210> 377
   <211> 8
   <212> PRT
   <213> Homo sapiens
<400> 377
   Ala Ser Gln Glu Leu Gly Gly Cys
   1 5
<210> 378
   <211> 8
   <212> PRT
   <213> Homo sapiens
<400> 378
   Ser Gln Glu Leu Gln Gly Gly Cys
   1 5
<210> 379
   <211> 13
   <212> PRT
   <213> Mus musculus
<400> 379
   Cys Arg Ser Arg Pro Ser Ala Lys Ala Ser Trp Val Gln
   1 5 10
<210> 380
   <211> 12
   <212> PRT
   <213> Mus musculus
<400> 380
   Cys Arg Ser Arg Pro Ser Ala Lys Ala Ser Trp Val
   1 5 10
<210> 381
   <211> 11
   <212> PRT
   <213> Mus musculus
<400> 381
   Cys Arg Ser Arg Pro Ser Ala Lys Ala Ser Trp
   1 5 10
<210> 382
   <211> 10
   <212> PRT
   <213> Mus musculus
<400> 382
   Cys Arg Ser Arg Pro Ser Ala Lys Ala Ser
   1 5 10
<210> 383
   <211> 12
   <212> PRT
   <213> Mus musculus
<400> 383
   Arg Ser Arg Pro Ser Ala Lys Ala Ser Trp Val Gln
   1 5 10
<210> 384
   <211> 11
   <212> PRT
   <213> Mus musculus
<400> 384
   Arg Ser Arg Pro Ser Ala Lys Ala Ser Trp Val
   1 5 10
<210> 385
   <211> 10
   <212> PRT
   <213> Mus musculus
<400> 385
   Arg Ser Arg Pro Ser Ala Lys Ala Ser Trp
   1 5 10
<210> 386
   <211> 9
   <212> PRT
   <213> Mus musculus
<400> 386
   Arg Ser Arg Pro Ser Ala Lys Ala Ser
   1 5
<210> 387
   <211> 11
   <212> PRT
   <213> Mus musculus
<400> 387
   Ser Arg Pro Ser Ala Lys Ala Ser Trp Val Gln
   1 5 10
<210> 388
   <211> 10
   <212> PRT
   <213> Mus musculus
<400> 388
   Ser Arg Pro Ser Ala Lys Ala Ser Trp Val
   1 5 10
<210> 389
   <211> 9
   <212> PRT
   <213> Mus musculus
<400> 389
   Ser Arg Pro Ser Ala Lys Ala Ser Trp
   1 5
<210> 390
   <211> 8
   <212> PRT
   <213> Mus musculus
<400> 390
   Ser Arg Pro Ser Ala Lys Ala Ser
   1 5
<210> 391
   <211> 10
   <212> PRT
   <213> Mus musculus
<400> 391
   Arg Pro Ser Ala Lys Ala Ser Trp Val Gln
   1 5 10
<210> 392
   <211> 9
   <212> PRT
   <213> Mus musculus
<400> 392
   Arg Pro Ser Ala Lys Ala Ser Trp Val
   1 5
<210> 393
   <211> 8
   <212> PRT
   <213> Mus musculus
<400> 393
   Arg Pro Ser Ala Lys Ala Ser Trp
   1 5
<210> 394
   <211> 7
   <212> PRT
   <213> Mus musculus
<400> 394
   Arg Pro Ser Ala Lys Ala Ser
   1 5
<210> 395
   <211> 9
   <212> PRT
   <213> Mus musculus
<400> 395
   Pro Ser Ala Lys Ala Ser Trp Val Gln
   1 5
<210> 396
   <211> 8
   <212> PRT
   <213> Mus musculus
<400> 396
   Pro Ser Ala Lys Ala Ser Trp Val
   1 5
<210> 397
   <211> 7
   <212> PRT
   <213> Mus musculus
<400> 397
   Pro Ser Ala Lys Ala Ser Trp
   1 5
<210> 398
   <211> 6
   <212> PRT
   <213> Mus musculus
<400> 398
   Pro Ser Ala Lys Ala Ser
   1 5
<210> 399
   <211> 5
   <212> PRT
   <213> Mus musculus
<400> 399
   Ser Ala Lys Ala Ser
   1 5
<210> 400
   <211> 15
   <212> PRT
   <213> Mus musculus
<400> 400
   Cys Gly Gly Arg Ser Arg Pro Ser Ala Lys Ala Ser Trp Val Gln
   1 5 10 15
<210> 401
   <211> 14
   <212> PRT
   <213> Mus musculus
<400> 401
   Cys Gly Gly Arg Ser Arg Pro Ser Ala Lys Ala Ser Trp Val
   1 5 10
<210> 402
   <211> 13
   <212> PRT
   <213> Mus musculus
<400> 402
   Cys Gly Gly Arg Ser Arg Pro Ser Ala Lys Ala Ser Trp
   1 5 10
<210> 403
   <211> 12
   <212> PRT
   <213> Mus musculus
<400> 403
   Cys Gly Gly Arg Ser Arg Pro Ser Ala Lys Ala Ser
   1 5 10
<210> 404
   <211> 14
   <212> PRT
   <213> Mus musculus
<400> 404
   Cys Gly Gly Ser Arg Pro Ser Ala Lys Ala Ser Trp Val Gln
   1 5 10
<210> 405
   <211> 13
   <212> PRT
   <213> Mus musculus
<400> 405
   Cys Gly Gly Ser Arg Pro Ser Ala Lys Ala Ser Trp Val
   1 5 10
<210> 406
   <211> 12
   <212> PRT
   <213> Mus musculus
<400> 406
   Cys Gly Gly Ser Arg Pro Ser Ala Lys Ala Ser Trp
   1               5                   10
<210> 407
   <211> 11
   <212> PRT
   <213> Mus musculus
<400> 407
   Cys Gly Gly Ser Arg Pro Ser Ala Lys Ala Ser
   1               5                   10
<210> 408
   <211> 13
   <212> PRT
   <213> Mus musculus
<400> 408
   Cys Gly Gly Arg Pro Ser Ala Lys Ala Ser Trp Val Gln
   1               5                   10
<210> 409
   <211> 12
   <212> PRT
   <213> Mus musculus
<400> 409
   Cys Gly Gly Arg Pro Ser Ala Lys Ala Ser Trp Val
   1               5                   10
<210> 410
   <211> 11
   <212> PRT
   <213> Mus musculus
<400> 410
   Cys Gly Gly Arg Pro Ser Ala Lys Ala Ser Trp
   1               5                   10
<210> 411
   <211> 10
   <212> PRT
   <213> Mus musculus
<400> 411
   Cys Gly Gly Arg Pro Ser Ala Lys Ala Ser
   1 5 10
<210> 412
   <211> 12
   <212> PRT
   <213> Mus musculus
<400> 412
   Cys Gly Gly Pro Ser Ala Lys Ala Ser Trp Val Gln
   1 5 10
<210> 413
   <211> 11
   <212> PRT
   <213> Mus musculus
<400> 413
   Cys Gly Gly Pro Ser Ala Lys Ala Ser Trp Val
   1 5 10
<210> 414
   <211> 10
   <212> PRT
   <213> Mus musculus
<400> 414
   Cys Gly Gly Pro Ser Ala Lys Ala Ser Trp
   1 5 10
<210> 415
   <211> 9
   <212> PRT
   <213> Mus musculus
<400> 415
   Cys Gly Gly Pro Ser Ala Lys Ala Ser
   1 5
<210> 416
   <211> 8
   <212> PRT
   <213> Mus musculus
<400> 416
   Cys Gly Gly Ser Ala Lys Ala Ser
   1 5
<210> 417
   <211> 15
   <212> PRT
   <213> Mus musculus
<400> 417
   Arg Ser Arg Pro Ser Ala Lys Ala Ser Trp Val Gln Gly Gly Cys
   1 5 10 15
<210> 418
   <211> 14
   <212> PRT
   <213> Mus musculus
<400> 418
   Arg Ser Arg Pro Ser Ala Lys Ala Ser Trp Val Gly Gly Cys
   1 5 10
<210> 419
   <211> 13
   <212> PRT
   <213> Mus musculus
<400> 419
   Arg Ser Arg Pro Ser Ala Lys Ala Ser Trp Gly Gly Cys
   1 5 10
<210> 420
   <211> 12
   <212> PRT
   <213> Mus musculus
<400> 420
   Arg Ser Arg Pro Ser Ala Lys Ala Ser Gly Gly Cys
   1 5 10
<210> 421
   <211> 14
   <212> PRT
   <213> Mus musculus
<400> 421
   Ser Arg Pro Ser Ala Lys Ala Ser Trp Val Gln Gly Gly Cys
   1 5 10
<210> 422
   <211> 13
   <212> PRT
   <213> Mus musculus
<400> 422
   Ser Arg Pro Ser Ala Lys Ala Ser Trp Val Gly Gly Cys
   1 5 10
<210> 423
   <211> 12
   <212> PRT
   <213> Mus musculus
<400> 423
   Ser Arg Pro Ser Ala Lys Ala Ser Trp Gly Gly Cys
   1 5 10
<210> 424
   <211> 11
   <212> PRT
   <213> Mus musculus
<400> 424
   Ser Arg Pro Ser Ala Lys Ala Ser Gly Gly Cys
   1 5 10
<210> 425
   <211> 13
   <212> PRT
   <213> Mus musculus
<400> 425
   Arg Pro Ser Ala Lys Ala Ser Trp Val Gln Gly Gly Cys
   1 5 10
<210> 426
   <211> 12
   <212> PRT
   <213> Mus musculus
<400> 426
   Arg Pro Ser Ala Lys Ala Ser Trp Val Gly Gly Cys
   1 5 10
<210> 427
   <211> 11
   <212> PRT
   <213> Mus musculus
<400> 427
   Arg Pro Ser Ala Lys Ala Ser Trp Gly Gly Cys
   1 5 10
<210> 428
   <211> 10
   <212> PRT
   <213> Mus musculus
<400> 428
   Arg Pro Ser Ala Lys Ala Ser Gly Gly Cys
   1 5 10
<210> 429
   <211> 12
   <212> PRT
   <213> Mus musculus
<400> 429
   Pro Ser Ala Lys Ala Ser Trp Val Gln Gly Gly Cys
   1 5 10
<210> 430
   <211> 11
   <212> PRT
   <213> Mus musculus
<400> 430
   Pro Ser Ala Lys Ala Ser Trp Val Gly Gly Cys
   1 5 10
<210> 431
   <211> 10
   <212> PRT
   <213> Mus musculus
<400> 431
   Pro Ser Ala Lys Ala Ser Trp Gly Gly Cys
   1 5 10
<210> 432
   <211> 9
   <212> PRT
   <213> Mus musculus
<400> 432
   Pro Ser Ala Lys Ala Ser Gly Gly Cys
   1 5
<210> 433
   <211> 8
   <212> PRT
   <213> Mus musculus
<400> 433
   Ser Ala Lys Ala Ser Gly Gly Cys
   1 5
<210> 434
   <211> 13
   <212> PRT
   <213> Homo sapiens
<400> 434
   Cys Ser Arg His Leu Ala Gln Ala Ser Gln Glu Leu Gln
   1 5 10
<210> 435
   <211> 13
   <212> PRT
   <213> Homo sapiens
<400> 435
   Cys Ser Arg His Leu Ala Gln Ala Ser Gln Glu Leu Gln
   1 5 10
<210> 436
   <211> 12
   <212> PRT
   <213> Homo sapiens
<400> 436
   Cys Ser Arg His Leu Ala Gln Ala Ser Gln Glu Leu
   1 5 10
<210> 437
   <211> 11
   <212> PRT
   <213> Homo sapiens
<400> 437
   Cys Ser Arg His Leu Ala Gln Ala Ser Gln Glu
   1 5 10
<210> 438
   <211> 10
   <212> PRT
   <213> Homo sapiens
<400> 438
   Cys Ser Arg His Leu Ala Gln Ala Ser Gln
   1 5 10
<210> 439
   <211> 9
   <212> PRT
   <213> Homo sapiens
<400> 439
   Cys Ser Arg His Leu Ala Gln Ala Ser
   1 5
<210> 440
   <211> 12
   <212> PRT
   <213> Homo sapiens
<400> 440
   Ser Arg His Leu Ala Gln Ala Ser Gln Glu Leu Gln
   1 5 10
<210> 441
   <211> 11
   <212> PRT
   <213> Homo sapiens
<400> 441
   Ser Arg His Leu Ala Gln Ala Ser Gln Glu Leu
   1 5 10
<210> 442
   <211> 10
   <212> PRT
   <213> Homo sapiens
<400> 442
   Ser Arg His Leu Ala Gln Ala Ser Gln Glu
   1 5 10
<210> 443
   <211> 9
   <212> PRT
   <213> Homo sapiens
<400> 443
   Ser Arg His Leu Ala Gln Ala Ser Gln
   1 5
<210> 444
   <211> 8
   <212> PRT
   <213> Homo sapiens
<400> 444
   Ser Arg His Leu Ala Gln Ala Ser
   1 5
<210> 445
   <211> 11
   <212> PRT
   <213> Homo sapiens
<400> 445
   Arg His Leu Ala Gln Ala Ser Gln Glu Leu Gln
   1 5 10
<210> 446
   <211> 10
   <212> PRT
   <213> Homo sapiens
<400> 446
   Arg His Leu Ala Gln Ala Ser Gln Glu Leu
   1               5                   10
<210> 447
   <211> 9
   <212> PRT
   <213> Homo sapiens
<400> 447
   Arg His Leu Ala Gln Ala Ser Gln Glu
   1               5
<210> 448
   <211> 8
   <212> PRT
   <213> Homo sapiens
<400> 448
   Arg His Leu Ala Gln Ala Ser Gln
   1               5
<210> 449
   <211> 7
   <212> PRT
   <213> Homo sapiens
<400> 449
   Arg His Leu Ala Gln Ala Ser
   1               5
<210> 450
   <211> 10
   <212> PRT
   <213> Homo sapiens
<400> 450
   His Leu Ala Gln Ala Ser Gln Glu Leu Gln
   1               5                   10
<210> 451
   <211> 9
   <212> PRT
   <213> Homo sapiens
<400> 451
   His Leu Ala Gln Ala Ser Gln Glu Leu
   1 5
<210> 452
   <211> 7
   <212> PRT
   <213> Homo sapiens
<400> 452
   His Leu Ala Gln Ala Ser Gln
   1 5
<210> 453
   <211> 6
   <212> PRT
   <213> Homo sapiens
<400> 453
   His Leu Ala Gln Ala Ser
   1 5
<210> 454
   <211> 9
   <212> PRT
   <213> Homo sapiens
<400> 454
   Leu Ala Gln Ala Ser Gln Glu Leu Gln
   1 5
<210> 455
   <211> 8
   <212> PRT
   <213> Homo sapiens
<400> 455
   Leu Ala Gln Ala Ser Gln Glu Leu
   1 5
<210> 456
   <211> 7
   <212> PRT
   <213> Homo sapiens
<400> 456
   Leu Ala Gln Ala Ser Gln Glu
   1 5
<210> 457
   <211> 6
   <212> PRT
   <213> Homo sapiens
<400> 457
   Leu Ala Gln Ala Ser Gln
   1 5
<210> 458
   <211> 5
   <212> PRT
   <213> Homo sapiens
<400> 458
   Leu Ala Gln Ala Ser
   1 5
<210> 459
   <211> 8
   <212> PRT
   <213> Homo sapiens
<400> 459
   Ala Gln Ala Ser Gln Glu Leu Gln
   1 5
<210> 460
   <211> 7
   <212> PRT
   <213> Homo sapiens
<400> 460
   Ala Gln Ala Ser Gln Glu Leu
   1 5
<210> 461
   <211> 6
   <212> PRT
   <213> Homo sapiens
<400> 461
   Ala Gln Ala Ser Gln Glu
   1 5
<210> 462
   <211> 5
   <212> PRT
   <213> Homo sapiens
<400> 462
   Ala Gln Ala Ser Gln
   1 5
<210> 463
   <211> 7
   <212> PRT
   <213> Homo sapiens
<400> 463
   Gln Ala Ser Gln Glu Leu Gln
   1 5
<210> 464
   <211> 6
   <212> PRT
   <213> Homo sapiens
<400> 464
   Gln Ala Ser Gln Glu Leu
   1 5
<210> 465
   <211> 5
   <212> PRT
   <213> Homo sapiens
<400> 465
   Gln Ala Ser Gln Glu
   1 5
<210> 466
   <211> 6
   <212> PRT
   <213> Homo sapiens
<400> 466
   Ala Ser Gln Glu Leu Gln
   1 5
<210> 467
   <211> 5
   <212> PRT
   <213> Homo sapiens
<400> 467
   Ala Ser Gln Glu Leu
   1 5
<210> 468
   <211> 5
   <212> PRT
   <213> Homo sapiens
<400> 468
   Ser Gln Glu Leu Gln
   1 5
<210> 469
   <211> 15
   <212> PRT
   <213> Homo sapiens
<400> 469
   Cys Gly Gly Ser Arg His Leu Ala Gln Ala Ser Gln Glu Leu Gln
   1 5 10 15
<210> 470
   <211> 14
   <212> PRT
   <213> Homo sapiens
<400> 470
   Cys Gly Gly Ser Arg His Leu Ala Gln Ala Ser Gln Glu Leu
   1 5 10
<210> 471
   <211> 13
   <212> PRT
   <213> Homo sapiens
<400> 471
   Cys Gly Gly Ser Arg His Leu Ala Gln Ala Ser Gln Glu
   1 5 10
<210> 472
   <211> 12
   <212> PRT
   <213> Homo sapiens
<400> 472
   Cys Gly Gly Ser Arg His Leu Ala Gln Ala Ser Gln
   1 5 10
<210> 473
   <211> 11
   <212> PRT
   <213> Homo sapiens
<400> 473
   Cys Gly Gly Ser Arg His Leu Ala Gln Ala Ser
   1 5 10
<210> 474
   <211> 14
   <212> PRT
   <213> Homo sapiens
<400> 474
   Cys Gly Gly Arg His Leu Ala Gln Ala Ser Gln Glu Leu Gln
   1 5 10
<210> 475
   <211> 13
   <212> PRT
   <213> Homo sapiens
<400> 475
   Cys Gly Gly Arg His Leu Ala Gln Ala Ser Gln Glu Leu
   1 5 10
<210> 476
   <211> 12
   <212> PRT
   <213> Homo sapiens
<400> 476
   Cys Gly Gly Arg His Leu Ala Gln Ala Ser Gln Glu
   1 5 10
<210> 477
   <211> 11
   <212> PRT
   <213> Homo sapiens
<400> 477
   Cys Gly Gly Arg His Leu Ala Gln Ala Ser Gln
   1 5 10
<210> 478
   <211> 10
   <212> PRT
   <213> Homo sapiens
<400> 478
   Cys Gly Gly Arg His Leu Ala Gln Ala Ser
   1 5 10
<210> 479
   <211> 13
   <212> PRT
   <213> Homo sapiens
<400> 479
   Cys Gly Gly His Leu Ala Gln Ala Ser Gln Glu Leu Gln
   1 5 10
<210> 480
   <211> 12
   <212> PRT
   <213> Homo sapiens
<400> 480
   Cys Gly Gly His Leu Ala Gln Ala Ser Gln Glu Leu
   1 5 10
<210> 481
   <211> 10
   <212> PRT
   <213> Homo sapiens
<400> 481
   Cys Gly Gly His Leu Ala Gln Ala Ser Gln
   1 5 10
<210> 482
   <211> 9
   <212> PRT
   <213> Homo sapiens
<400> 482
   Cys Gly Gly His Leu Ala Gln Ala Ser
   1 5
<210> 483
   <211> 12
   <212> PRT
   <213> Homo sapiens
<400> 483
   Cys Gly Gly Leu Ala Gln Ala Ser Gln Glu Leu Gln
   1 5 10
<210> 484
   <211> 11
   <212> PRT
   <213> Homo sapiens
<400> 484
   Cys Gly Gly Leu Ala Gln Ala Ser Gln Glu Leu
   1 5 10
<210> 485
   <211> 10
   <212> PRT
   <213> Homo sapiens
<400> 485
   Cys Gly Gly Leu Ala Gln Ala Ser Gln Glu
   1 5 10
<210> 486
   <211> 9
   <212> PRT
   <213> Homo sapiens
<400> 486
   Cys Gly Gly Leu Ala Gln Ala Ser Gln
   1 5
<210> 487
   <211> 8
   <212> PRT
   <213> Homo sapiens
<400> 487
   Cys Gly Gly Leu Ala Gln Ala Ser
   1 5
<210> 488
   <211> 11
   <212> PRT
   <213> Homo sapiens
<400> 488
   Cys Gly Gly Ala Gln Ala Ser Gln Glu Leu Gln
   1 5 10
<210> 489
   <211> 10
   <212> PRT
   <213> Homo sapiens
<400> 489
   Cys Gly Gly Ala Gln Ala Ser Gln Glu Leu
   1 5 10
<210> 490
   <211> 9
   <212> PRT
   <213> Homo sapiens
<400> 490
   Cys Gly Gly Ala Gln Ala Ser Gln Glu
   1 5
<210> 491
   <211> 8
   <212> PRT
   <213> Homo sapiens
<400> 491
   Cys Gly Gly Ala Gln Ala Ser Gln
   1 5
<210> 492
   <211> 10
   <212> PRT
   <213> Homo sapiens
<400> 492
   Cys Gly Gly Gln Ala Ser Gln Glu Leu Gln
   1 5 10
<210> 493
   <211> 9
   <212> PRT
   <213> Homo sapiens
<400> 493
   Cys Gly Gly Gln Ala Ser Gln Glu Leu
   1 5
<210> 494
   <211> 8
   <212> PRT
   <213> Homo sapiens
<400> 494
   Cys Gly Gly Gln Ala Ser Gln Glu
   1 5
<210> 495
   <211> 9
   <212> PRT
   <213> Homo sapiens
<400> 495
   Cys Gly Gly Ala Ser Gln Glu Leu Gln
   1 5
<210> 496
   <211> 8
   <212> PRT
   <213> Homo sapiens
<400> 496
   Cys Gly Gly Ala Ser Gln Glu Leu
   1 5
<210> 497
   <211> 8
   <212> PRT
   <213> Homo sapiens
<400> 497
   Cys Gly Gly Ser Gln Glu Leu Gln
   1 5
<210> 498
   <211> 15
   <212> PRT
   <213> Homo sapiens
<400> 498
   Ser Arg His Leu Ala Gln Ala Ser Gln Glu Leu Gln Gly Gly Cys
   1 5 10 15
<210> 499
   <211> 14
   <212> PRT
   <213> Homo sapiens
<400> 499
   Ser Arg His Leu Ala Gln Ala Ser Gln Glu Leu Gly Gly Cys
   1 5 10
<210> 500
   <211> 13
   <212> PRT
   <213> Homo sapiens
<400> 500
   Ser Arg His Leu Ala Gln Ala Ser Gln Glu Gly Gly Cys
   1 5 10
<210> 501
   <211> 12
   <212> PRT
   <213> Homo sapiens
<400> 501
   Ser Arg His Leu Ala Gln Ala Ser Gln Gly Gly Cys
   1 5 10
<210> 502
   <211> 11
   <212> PRT
   <213> Homo sapiens
<400> 502
   Ser Arg His Leu Ala Gln Ala Ser Gly Gly Cys
   1 5 10
<210> 503
   <211> 14
   <212> PRT
   <213> Homo sapiens
<400> 503
   Arg His Leu Ala Gln Ala Ser Gln Glu Leu Gln Gly Gly Cys
   1 5 10
<210> 504
   <211> 13
   <212> PRT
   <213> Homo sapiens
<400> 504
   Arg His Leu Ala Gln Ala Ser Gln Glu Leu Gly Gly Cys
   1 5 10
<210> 505
   <211> 12
   <212> PRT
   <213> Homo sapiens
<400> 505
   Arg His Leu Ala Gln Ala Ser Gln Glu Gly Gly Cys
   1 5 10
<210> 506
   <211> 11
   <212> PRT
   <213> Homo sapiens
<400> 506
   Arg His Leu Ala Gln Ala Ser Gln Gly Gly Cys
   1 5 10
<210> 507
   <211> 10
   <212> PRT
   <213> Homo sapiens
<400> 507
   Arg His Leu Ala Gln Ala Ser Gly Gly Cys
   1 5 10
<210> 508
   <211> 13
   <212> PRT
   <213> Homo sapiens
<400> 508
   His Leu Ala Gln Ala Ser Gln Glu Leu Gln Gly Gly Cys
   1 5 10
<210> 509
   <211> 12
   <212> PRT
   <213> Homo sapiens
<400> 509
   His Leu Ala Gln Ala Ser Gln Glu Leu Gly Gly Cys
   1 5 10
<210> 510
   <211> 10
   <212> PRT
   <213> Homo sapiens
<400> 510
   His Leu Ala Gln Ala Ser Gln Gly Gly Cys
   1 5 10
<210> 511
   <211> 9
   <212> PRT
   <213> Homo sapiens
<400> 511
   His Leu Ala Gln Ala Ser Gly Gly Cys
   1 5
<210> 512
   <211> 12
   <212> PRT
   <213> Homo sapiens
<400> 512
   Leu Ala Gln Ala Ser Gln Glu Leu Gln Gly Gly Cys
   1 5 10
<210> 513
   <211> 11
   <212> PRT
   <213> Homo sapiens
<400> 513
   Leu Ala Gln Ala Ser Gln Glu Leu Gly Gly Cys
   1 5 10
<210> 514
   <211> 10
   <212> PRT
   <213> Homo sapiens
<400> 514
   Leu Ala Gln Ala Ser Gln Glu Gly Gly Cys
   1 5 10
<210> 515
   <211> 9
   <212> PRT
   <213> Homo sapiens
<400> 515
   Leu Ala Gln Ala Ser Gln Gly Gly Cys
   1 5
<210> 516
   <211> 8
   <212> PRT
   <213> Homo sapiens
<400> 516
   Leu Ala Gln Ala Ser Gly Gly Cys
   1 5
<210> 517
   <211> 11
   <212> PRT
   <213> Homo sapiens
<400> 517
   Ala Gln Ala Ser Gln Glu Leu Gln Gly Gly Cys
   1 5 10
<210> 518
   <211> 10
   <212> PRT
   <213> Homo sapiens
<400> 518
   Ala Gln Ala Ser Gln Glu Leu Gly Gly Cys
   1 5 10
<210> 519
   <211> 9
   <212> PRT
   <213> Homo sapiens
<400> 519
   Ala Gln Ala Ser Gln Glu Gly Gly Cys
   1               5
<210> 520
   <211> 8
   <212> PRT
   <213> Homo sapiens
<400> 520
   Ala Gln Ala Ser Gln Gly Gly Cys
   1               5
<210> 521
   <211> 10
   <212> PRT
   <213> Homo sapiens
<400> 521
   Gln Ala Ser Gln Glu Leu Gln Gly Gly Cys
   1               5                   10
<210> 522
   <211> 9
   <212> PRT
   <213> Homo sapiens
<400> 522
   Gln Ala Ser Gln Glu Leu Gly Gly Cys
   1               5
<210> 523
   <211> 8
   <212> PRT
   <213> Homo sapiens
<400> 523
   Gln Ala Ser Gln Glu Gly Gly Cys
   1               5
<210> 524
   <211> 9
   <212> PRT
   <213> Homo sapiens
<400> 524
   Ala Ser Gln Glu Leu Gln Gly Gly Cys
   1               5
<210> 525
   <211> 8
   <212> PRT
   <213> Homo sapiens
<400> 525
   Ala Ser Gln Glu Leu Gly Gly Cys
   1               5
<210> 526
   <211> 8
   <212> PRT
   <213> Homo sapiens
<400> 526
   Ser Gln Glu Leu Gln Gly Gly Cys
   1               5
<210> 527
   <211> 13
   <212> PRT
   <213> Mus musculus
<400> 527
   Cys Arg Ser Arg Pro Ser Ala Lys Ala Ser Trp Val Gln
   1               5                   10
<210> 528
   <211> 12
   <212> PRT
   <213> Mus musculus
<400> 528
   Cys Arg Ser Arg Pro Ser Ala Lys Ala Ser Trp Val
   1               5                   10
<210> 529
   <211> 11
   <212> PRT
   <213> Mus musculus
<400> 529
   Cys Arg Ser Arg Pro Ser Ala Lys Ala Ser Trp
   1               5                   10
<210> 530
   <211> 10
   <212> PRT
   <213> Mus musculus
<400> 530
   Cys Arg Ser Arg Pro Ser Ala Lys Ala Ser
   1               5                   10
<210> 531
   <211> 12
   <212> PRT
   <213> Mus musculus
<400> 531
   Arg Ser Arg Pro Ser Ala Lys Ala Ser Trp Val Gln
   1               5                   10
<210> 532
   <211> 11
   <212> PRT
   <213> Mus musculus
<400> 532
   Arg Ser Arg Pro Ser Ala Lys Ala Ser Trp Val
   1               5                   10
<210> 533
   <211> 10
   <212> PRT
   <213> Mus musculus
<400> 533
   Arg Ser Arg Pro Ser Ala Lys Ala Ser Trp
   1               5                   10
<210> 534
   <211> 9
   <212> PRT
   <213> Mus musculus
<400> 534
   Arg Ser Arg Pro Ser Ala Lys Ala Ser
   1               5
<210> 535
   <211> 11
   <212> PRT
   <213> Mus musculus
<400> 535
   Ser Arg Pro Ser Ala Lys Ala Ser Trp Val Gln
   1               5                   10
<210> 536
   <211> 10
   <212> PRT
   <213> Mus musculus
<400> 536
   Ser Arg Pro Ser Ala Lys Ala Ser Trp Val
   1               5                   10
<210> 537
   <211> 9
   <212> PRT
   <213> Mus musculus
<400> 537
   Ser Arg Pro Ser Ala Lys Ala Ser Trp
   1               5
<210> 538
   <211> 8
   <212> PRT
   <213> Mus musculus
<400> 538
   Ser Arg Pro Ser Ala Lys Ala Ser
   1               5
<210> 539
   <211> 10
   <212> PRT
   <213> Mus musculus
<400> 539
   Arg Pro Ser Ala Lys Ala Ser Trp Val Gln
   1               5                   10
<210> 540
   <211> 9
   <212> PRT
   <213> Mus musculus
<400> 540
   Arg Pro Ser Ala Lys Ala Ser Trp Val
   1               5
<210> 541
   <211> 8
   <212> PRT
   <213> Mus musculus
<400> 541
   Arg Pro Ser Ala Lys Ala Ser Trp
   1 5
<210> 542
   <211> 7
   <212> PRT
   <213> Mus musculus
<400> 542
   Arg Pro Ser Ala Lys Ala Ser
   1 5
<210> 543
   <211> 9
   <212> PRT
   <213> Mus musculus
<400> 543
   Pro Ser Ala Lys Ala Ser Trp Val Gln
   1 5
<210> 544
   <211> 8
   <212> PRT
   <213> Mus musculus
<400> 544
   Pro Ser Ala Lys Ala Ser Trp Val
   1 5
<210> 545
   <211> 7
   <212> PRT
   <213> Mus musculus
<400> 545
   Pro Ser Ala Lys Ala Ser Trp
   1 5
<210> 546
   <211> 6
   <212> PRT
   <213> Mus musculus
<400> 546
   Pro Ser Ala Lys Ala Ser
   1 5
<210> 547
   <211> 5
   <212> PRT
   <213> Mus musculus
<400> 547
   Ser Ala Lys Ala Ser
   1 5
<210> 548
   <211> 15
   <212> PRT
   <213> Mus musculus
<400> 548
   Cys Gly Gly Arg Ser Arg Pro Ser Ala Lys Ala Ser Trp Val Gln
   1 5 10 15
<210> 549
   <211> 14
   <212> PRT
   <213> Mus musculus
<400> 549
   Cys Gly Gly Arg Ser Arg Pro Ser Ala Lys Ala Ser Trp Val
   1 5 10
<210> 550
   <211> 13
   <212> PRT
   <213> Mus musculus
<400> 550
   Cys Gly Gly Arg Ser Arg Pro Ser Ala Lys Ala Ser Trp
   1 5 10
<210> 551
   <211> 12
   <212> PRT
   <213> Mus musculus
<400> 551
   Cys Gly Gly Arg Ser Arg Pro Ser Ala Lys Ala Ser
   1 5 10
<210> 552
   <211> 14
   <212> PRT
   <213> Mus musculus
<400> 552
   Cys Gly Gly Ser Arg Pro Ser Ala Lys Ala Ser Trp Val Gln
   1 5 10
<210> 553
   <211> 13
   <212> PRT
   <213> Mus musculus
<400> 553
   Cys Gly Gly Ser Arg Pro Ser Ala Lys Ala Ser Trp Val
   1 5 10
<210> 554
   <211> 12
   <212> PRT
   <213> Mus musculus
<400> 554
   Cys Gly Gly Ser Arg Pro Ser Ala Lys Ala Ser Trp
   1 5 10
<210> 555
   <211> 11
   <212> PRT
   <213> Mus musculus
<400> 555
   Cys Gly Gly Ser Arg Pro Ser Ala Lys Ala Ser
   1 5 10
<210> 556
   <211> 13
   <212> PRT
   <213> Mus musculus
<400> 556
   Cys Gly Gly Arg Pro Ser Ala Lys Ala Ser Trp Val Gln
   1 5 10
<210> 557
   <211> 12
   <212> PRT
   <213> Mus musculus
<400> 557
   Cys Gly Gly Arg Pro Ser Ala Lys Ala Ser Trp Val
   1 5 10
<210> 558
   <211> 11
   <212> PRT
   <213> Mus musculus
<400> 558
   Cys Gly Gly Arg Pro Ser Ala Lys Ala Ser Trp
   1 5 10
<210> 559
   <211> 10
   <212> PRT
   <213> Mus musculus
<400> 559
   Cys Gly Gly Arg Pro Ser Ala Lys Ala Ser
   1 5 10
<210> 560
   <211> 12
   <212> PRT
   <213> Mus musculus
<400> 560
   Cys Gly Gly Pro Ser Ala Lys Ala Ser Trp Val Gln
   1 5 10
<210> 561
   <211> 11
   <212> PRT
   <213> Mus musculus
<400> 561
   Cys Gly Gly Pro Ser Ala Lys Ala Ser Trp Val
   1 5 10
<210> 562
   <211> 10
   <212> PRT
   <213> Mus musculus
<400> 562
   Cys Gly Gly Pro Ser Ala Lys Ala Ser Trp
   1 5 10
<210> 563
   <211> 9
   <212> PRT
   <213> Mus musculus
<400> 563
   Cys Gly Gly Pro Ser Ala Lys Ala Ser
   1 5
<210> 564
   <211> 8
   <212> PRT
   <213> Mus musculus
<400> 564
   Cys Gly Gly Ser Ala Lys Ala Ser
   1 5
<210> 565
   <211> 15
   <212> PRT
   <213> Mus musculus
<400> 565
   Arg Ser Arg Pro Ser Ala Lys Ala Ser Trp Val Gln Gly Gly Cys
   1 5 10 15
<210> 566
   <211> 14
   <212> PRT
   <213> Mus musculus
<400> 566
   Arg Ser Arg Pro Ser Ala Lys Ala Ser Trp Val Gly Gly Cys
   1 5 10
<210> 567
   <211> 13
   <212> PRT
   <213> Mus musculus
<400> 567
   Arg Ser Arg Pro Ser Ala Lys Ala Ser Trp Gly Gly Cys
   1 5 10
<210> 568
   <211> 12
   <212> PRT
   <213> Mus musculus
<400> 568
   Arg Ser Arg Pro Ser Ala Lys Ala Ser Gly Gly Cys
   1 5 10
<210> 569
   <211> 14
   <212> PRT
   <213> Mus musculus
<400> 569
   Ser Arg Pro Ser Ala Lys Ala Ser Trp Val Gln Gly Gly Cys
   1 5 10
<210> 570
   <211> 13
   <212> PRT
   <213> Mus musculus
<400> 570
   Ser Arg Pro Ser Ala Lys Ala Ser Trp Val Gly Gly Cys
   1 5 10
<210> 571
   <211> 12
   <212> PRT
   <213> Mus musculus
<400> 571
   Ser Arg Pro Ser Ala Lys Ala Ser Trp Gly Gly Cys
   1 5 10
<210> 572
   <211> 11
   <212> PRT
   <213> Mus musculus
<400> 572
   Ser Arg Pro Ser Ala Lys Ala Ser Gly Gly Cys
   1 5 10
<210> 573
   <211> 13
   <212> PRT
   <213> Mus musculus
<400> 573
   Arg Pro Ser Ala Lys Ala Ser Trp Val Gln Gly Gly Cys
   1 5 10
<210> 574
   <211> 12
   <212> PRT
   <213> Mus musculus
<400> 574
   Arg Pro Ser Ala Lys Ala Ser Trp Val Gly Gly Cys
   1 5 10
<210> 575
   <211> 11
   <212> PRT
   <213> Mus musculus
<400> 575
   Arg Pro Ser Ala Lys Ala Ser Trp Gly Gly Cys
   1 5 10
<210> 576
   <211> 10
   <212> PRT
   <213> Mus musculus
<400> 576
   Arg Pro Ser Ala Lys Ala Ser Gly Gly Cys
   1 5 10
<210> 577
   <211> 12
   <212> PRT
   <213> Mus musculus
<400> 577
   Pro Ser Ala Lys Ala Ser Trp Val Gln Gly Gly Cys
   1 5 10
<210> 578
   <211> 11
   <212> PRT
   <213> Mus musculus
<400> 578
   Pro Ser Ala Lys Ala Ser Trp Val Gly Gly Cys
   1 5 10
<210> 579
   <211> 10
   <212> PRT
   <213> Mus musculus
<400> 579
   Pro Ser Ala Lys Ala Ser Trp Gly Gly Cys
   1 5 10
<210> 580
   <211> 9
   <212> PRT
   <213> Mus musculus
<400> 580
   Pro Ser Ala Lys Ala Ser Gly Gly Cys
   1 5
<210> 581
   <211> 8
   <212> PRT
   <213> Mus musculus
<400> 581
   Ser Ala Lys Ala Ser Gly Gly Cys
   1 5
<210> 582
   <211> 21
   <212> DNA
   <213> Artificial Sequence
<220>
   <223> Synthetic
<400> 582
   tcgtcgtttt tcggtgcttt t 21
<210> 583
   <211> 21
   <212> DNA
   <213> Artificial Sequence
<220>
   <223> Synthetic
<400> 583
   tcgtcgtttt tcggtcgttt t 21
<210> 584
   <211> 24
   <212> DNA
   <213> Artificial Sequence
<220>
   <223> Synthetic
<400> 584
   tcgtcgtttt gtcgttttgt cgtt 24
<210> 585
   <211> 24
   <212> DNA
   <213> Artificial Sequence
<220>
   <223> Synthetic
<400> 585
   tcgtcgtttc gtcgttttgt cgtt 24
<210> 586
   <211> 24
   <212> DNA
   <213> Artificial Sequence
<220>
   <223> Synthetic
<400> 586
   tcgtcgtttt gtcgtttttt tcga 24
<210> 587
   <211> 22
   <212> DNA
   <213> Artificial Sequence
<220>
   <223> Synthetic
<400> 587
   tcgcgtcgtt cggcgcgcgc cg 22
<210> 588
   <211> 23
   <212> DNA
   <213> Artificial Sequence
<220>
   <223> Synthetic
<400> 588
   tcgtcgacgt tcggcgcgcg ccg 23
<210> 589
   <211> 21
   <212> DNA
   <213> Artificial Sequence
<220>
   <223> Synthetic
<400> 589
   tcggacgttc ggcgcgcgcc g 21
<210> 590
   <211> 19
   <212> DNA
   <213> Artificial Sequence
<220>
   <223> Synthetic
<400> 590
   tcggacgttc ggcgcgccg 19
<210> 591
   <211> 20
   <212> DNA
   <213> Artificial Sequence
<220>
   <223> Synthetic
<400> 591
   tcgcgtcgtt cggcgcgccg 20
<210> 592
   <211> 20
   <212> DNA
   <213> Artificial Sequence
<220>
   <223> Synthetic
<400> 592
   tcgacgttcg gcgcgcgccg 20
<210> 593
   <211> 18
   <212> DNA
   <213> Artificial Sequence
<220>
   <223> Synthetic
<400> 593
   tcgacgttcg gcgcgccg 18
<210> 594
   <211> 18
   <212> DNA
   <213> Artificial Sequence
<220>
   <223> Synthetic
<400> 594
   tcgcgtcgtt cggcgccg 18
<210> 595
   <211> 22
   <212> DNA
   <213> Artificial Sequence
<220>
   <223> Synthetic
<400> 595
   tcgcgacgtt cggcgcgcgc cg 22
<210> 596
   <211> 22
   <212> DNA
   <213> Artificial Sequence
<220>
   <223> Synthetic
<400> 596
   tcgtcgtttt cggcgcgcgc cg 22
<210> 597
   <211> 22
   <212> DNA
   <213> Artificial Sequence
<220>
   <223> Synthetic
<400> 597
   tcgtcgtttt cggcggccgc cg 22
<210> 598
   <211> 24
   <212> DNA
   <213> Artificial Sequence
<220>
   <223> Synthetic
<400> 598
   tcgtcgtttt acggcgccgt gccg 24
<210> 599
   <211> 23
   <212> DNA
   <213> Artificial Sequence
<220>
   <223> Synthetic
<400> 599
   tcgtcgtttt cggcgcgcgc cgt 23
<210> 600
   <211> 23
   <212> DNA
   <213> Artificial Sequence
<220>
   <223> Synthetic
<400> 600
   tcgtcgacga tcggcgcgcg ccg 23